# EUROPEAN PATENT APPLICATION

(11) **EP 4 089 111 A1**
(43) Date of publication of application: **16.11.2022**
(21) Application number: 21738017.9
(22) Date of filing: 08.01.2021
(51) Int. Cl.: C07K 16/00, C07K 16/46, A61K 39/395, A61K 38/17, C12N 15/62, G01N 33/53

(54) **NEW POLYPEPTIDE COMPLEX**

(30) Priority: 09.01.2020 CN 202010020878
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: YING, Hua, Shanghai 200245 (CN); HU, Qiyue, Shanghai 200245 (CN); ZHANG, Ling, Shanghai 200245 (CN); MAO, Langyong, Shanghai 200245 (CN); SHI, Jinping, Shanghai 200245 (CN); LI, Tingting, Shanghai 200245 (CN); LAI, Weiming, Shanghai 200245 (CN); QIN, Qianshan, Shanghai 200245 (CN); JIN, Xinsheng, Shanghai 200245 (CN); TAO, Weikang, Shanghai 200245 (CN)
(74) Representative: Potter Clarkson
(86) International application number: PCT/CN2021/070832
(87) International publication number: WO 2021/139758

(57) **Abstract**

The present disclosure relates to a new polypeptide complex. Specifically, the present disclosure relates to a domain engineered antibody. At least one of the constant region domains CH1 and/or CL of the antibody is replaced, and the domain CH1/CL is replaced by a Titin T chain/Obscurin-O chain or by a Titin T chain/Obscurin-like-O chain.

## Description

The present application claims priority to the Chinese Patent Application (Application No. CN202010020878.7) filed on Jan. 09, 2020.

### TECHNICAL FIELD

The present disclosure relates to the field of antibody pharmaceuticals, and specifically comprises a novel antibody in which CH1 and CL structures are substituted and use thereof.

### BACKGROUND

The statements herein merely provide background information related to the present disclosure and may not necessarily constitute the prior art.

With the continuous improvement of antibody humanization technology, monoclonal antibodies have been developed rapidly in recent years. Various monoclonal antibodies have been used to treat major diseases such as malignant tumors and autoimmune diseases. However, the immune escape of tumors is often accompanied by a variety of different mechanisms, and a single monoclonal antibody can only bind to one specific target, which can greatly reduce the therapeutic effect of the monoclonal antibody.

The bispecific antibody (BsAb) is an artificial antibody formed by combining antibodies targeting two different antigens or two different epitopes together by using a genetic engineering means. Different from the monoclonal antibody, the bispecific antibody has the ability to target two different epitopes simultaneously, and can play special biological functions, such as immune cell recruitment, receptor co-stimulation or co-inhibition, multivalent virus neutralization, etc., and are expected to obtain better clinical therapeutic effects than single monoclonal antibodies, even antibody combinations.

A wide variety of recombinant bispecific antibodies have been developed, such as tetravalent bispecific antibodies formed by fusion of, for example, an IgG antibody and a single-chain domain (see Coloma, M.J., et al., Nature Biotech. 15 (1997) 159-163; Morrison, S.L., Nature Biotech. 25 (2007) 1233-1234). There are other bispecific forms, such as DVD-Ig, CrossMab and BiTE (Spiess et al., Molecular Immunology, 67 (2), pp. 95-106 (2015)).

Various structural models for bispecific antibodies have also been disclosed, for example, the introduction of the mutation "knobs-into-holes" into the Fc region (Ridgway et al., Protein Engineering, 9 (7), pp. 617-21 (1996)); the introduction of electrostatic design (Gunasekaran et al., Journal of Biological Chemistry, 285, (25), pp. 19637-19646, (2010)), or the introduction of negative state design (Kreudenstein et al., mAbs, 5 (5), pp. 646-654 (2013); Leaver-Fay et al., Structure, 24 (4), pp. 641-651 (2016)), the exchange of CH1 and CL domains (CrossMab platform) (Schaefer et al., Proceedings of the National Academy of Sciences of the United States of America, 108 (27), pp. 11187-11192 (2011)), and fusion with the TCR constant region (WO2019057122A1).

### SUMMARY

However, in the preparation of a bispecific antibody, a random combination of different polypeptide chains results in a variety of different products, only one of which is desired, and the resulting byproducts comprise an antibody lacking a light chain, a half-antibody, a heavy chain polymer, light-heavy-chain mispairing, and the like, causing significant challenges to the development of downstream processes. Therefore, the present disclosure provides a novel domain-engineered polypeptide complex.

In some embodiments, the present disclosure provides a polypeptide complex, which comprises a first antigen-binding moiety comprising:
A) a first polypeptide, comprising a first heavy chain variable domain (VH1) from an N-terminus to a C-terminus, the VH1 being operably linked to a first domain comprising a Titin-T chain, and
   a second polypeptide, comprising a first light chain variable domain (VL1) from an N-terminus to a C-terminus, the VL1 being operably linked to a second domain comprising a domain capable of interacting with a Titin-T chain;
   or,
B) a first polypeptide, comprising a VH1 from an N-terminus to a C-terminus, the VH1 being operably linked to a first domain comprising a domain capable of interacting with a Titin-T chain, and
   a second polypeptide, comprising a VL1 from an N-terminus to a C-terminus, the VL1 being operably linked to a second domain comprising a Titin-T chain;
   or,
C) a first polypeptide, comprising a VH1 from an N-terminus to a C-terminus, the VH1 being operably linked to a first domain comprising an Obscurin-O chain or an Obscurin-like-O chain, and
   a second polypeptide, comprising a VL1 from an N-terminus to a C-terminus, the VL1 being operably linked to a second domain comprising a domain capable of interacting with an Obscurin-O chain or an Obscurin-like-O chain;
   or,
D) a first polypeptide, comprising a VH1 from an N-terminus to a C-terminus, the VH1 being operably linked to a first domain comprising a domain capable of interacting with an Obscurin-O chain or an Obscurin-like-O chain, and
   a second polypeptide, comprising a VL1 from an N-terminus to a C-terminus, the VL1 being operably linked to a second domain comprising an Obscurin-O chain or an Obscurin-like-O chain;
   wherein the first antigen-binding moiety specifically binds to a first antigen, and the first domain and the second domain are capable of forming a dimer.

In some embodiments, for the aforementioned polypeptide complex, the domain capable of interacting with the Obscurin-O chain or the Obscurin-like-O chain is the Titin-T chain, and the domain capable of interacting with the Titin-T chain is the Obscurin-O chain or the Obscurin-like-O chain.

In some embodiments, for the aforementioned polypeptide complex, the VH1 and the VL1 of the first antigen-binding moiety form a first antigen-binding site specifically binding to the first antigen.

In some embodiments, the C-terminus of the VH1 is operably linked to an N-terminus of the first domain.

In some embodiments, the C-terminus of the VL1 is operably linked to an N-terminus of the second domain.

In some embodiments, for the aforementioned polypeptide complex, the first domain and the second domain form a dimer through natural inter-chain bonds and/or non-natural inter-chain bonds.

In some embodiments, the first domain and the second domain form a dimer through natural inter-chain bonds; in some embodiments, one or more residues at positions selected from the group consisting of 7-15, 19-24, 26, 55, 59 and 60 on the Titin-T chain and one or more residues at positions selected from the group consisting of 3-6, 9, 41, 73, 75 and 80-90 on the Obscurin-O chain are linked to each other, or one or more residues selected from the group consisting of positions 1, 7-10, 13-16, 19-26, 59-60 and 96 on the Titin-T chain and one or more residues selected from the group consisting of positions 4-5, 10, 12-13, 74, 76, 78 and 82-91 on the Obscurin-like-O chain are linked to each other; the residue position in the Titin-T chain is a natural sequence numbering position relative to a sequence SEQ ID NO: 32; the residue position in the Obscurin-O chain is a natural sequence numbering position relative to a sequence SEQ ID NO: 33; the residue position in the Obscurin-like-O chain is a natural sequence numbering position relative to a sequence SEQ ID NO: 34.

In some embodiments, for any one of the aforementioned polypeptide complexes, the first domain and the second domain form a dimer through at least one non-natural inter-chain bonds.

In some embodiments, the non-natural inter-chain bond is formed between a particular mutated residue of the first domain and a particular mutated residue of the second domain. In some embodiments, at least one of the particular mutated residue of the first domain and the particular mutated residue of the second domain is a cysteine residue.

In some embodiments, the non-natural inter-chain bond is a disulfide bond; in some embodiments, the dimer comprises 1, 2, 3, 4, 5, 6, 7, 8, 9 or 10 non-natural inter-chain bonds.

In some embodiments, for the aforementioned polypeptide complex, the particular mutated residue of the first domain and the particular mutated residue of the second domain are located at a contact interface of the first domain and the second domain.

In some embodiments, the particular mutated residue of the aforementioned first domain and the particular mutated residue of the second domain have mutations on positions selected from the group consisting of: the Titin-T chain has one or more amino acid residue mutations on positions selected from the group consisting of 8, 20, 22, 25, 26 and 39, and/or the Obscurin-O chain has one or more amino acid residue mutations on positions selected from the group consisting of 3, 9, 25, 76 and 88; or the Titin-T chain has one or more amino acid residue mutations on positions selected from the group consisting of 8, 20, 22, 25, 26 and 39, and/or the Obscurin-like-O chain has one or more amino acid residue mutations on positions selected from the group consisting of 6, 26, 74, 77, 84 and 86. The mutation position in the Titin-T chain is a natural sequence numbering position relative to a sequence SEQ ID NO: 32; the mutation position in the Obscurin-O chain is a natural sequence numbering position relative to a sequence SEQ ID NO: 33; the mutation position in the Obscurin-like-O chain is a natural sequence numbering position relative to a sequence SEQ ID NO: 34. In some specific embodiments, the aforementioned first mutated residue and the aforementioned second mutated residue have mutations on positions selected from the group consisting of: the Titin-T chain has one or more amino acid residue mutations on positions selected from the group consisting of 8C, 20C, 22C, 25S, 26C and 39T, and/or the Obscurin-O chain has one or more amino acid residue mutations on positions selected from the group consisting of 3C, 9C, 25S, 76S and 88C; or the Titin-T chain has one or more amino acid residue mutations on positions selected from the group consisting of 8C, 20C, 22C, 25S, 26C and 39T, and/or the Obscurin-like-O chain has one or more amino acid residue mutations on positions selected from the group consisting of 6E, 26S, 74C, 77S, 84C and 86C. In some embodiments, the Titin-T chain has 25S, 39T and 8C mutations, and the Obscurin-O chain has an 88C mutation;
the Titin-T chain has 25S, 39T and 20C mutations, and the Obscurin-O chain has a 3C mutation;
the Titin-T chain has 25S, 39T and 26C mutations, and the Obscurin-O chain has a 9C mutation;
the Titin-T chain has 25S, 39T and 8C mutations, and the Obscurin-O chain has 25S, 76S and 88C mutations;
the Titin-T chain has 25S, 39T and 20C mutations, and the Obscurin-O chain has 25S, 76S and 3C mutations;
the Titin-T chain has 25S, 39T and 26C mutations, and the Obscurin-O chain has 25S, 76S and 9C mutations;
the Titin-T chain has 25S, 39T and 8C mutations, and the Obscurin-like-O chain has 6E and 74C mutations;
the Titin-T chain has 25S, 39T and 20C mutations, and the Obscurin-like-O chain has 6E and 84C mutations;
the Titin-T chain has 25S, 39T and 22C mutations, and the Obscurin-like-O chain has 6E and 86C mutations;
the Titin-T chain has 25S, 39T and 8C mutations, and the Obscurin-like-O chain has 6E, 26S, 77S and 74C mutations;
the Titin-T chain has 25S, 39T and 20C mutations, and the Obscurin-like-O chain has 6E, 26S, 77S and 84C mutations; or
the Titin-T chain has 25S, 39T and 22C mutations, and the Obscurin-like-O chain has 6E, 26S, 77S and 86C mutations;
the mutation position in the Titin-T chain is a natural sequence numbering position relative to a sequence SEQ ID NO: 32; the mutation position in the Obscurin-O chain is a natural sequence numbering position relative to a sequence SEQ ID NO: 33; the mutation position in the Obscurin-like-O chain is a natural sequence numbering position relative to a sequence SEQ ID NO: 34.

In some specific embodiments, the aforementioned first mutated residue and the aforementioned second mutated residue have mutations on positions selected from the group consisting of: the Titin-T chain has one or more amino acid residue mutations on positions selected from the group consisting of A8C, V20C, T22C, C25S, A26C and C39T, and/or the Obscurin-O chain has one or more amino acid residue mutations on positions selected from the group consisting of A3C, R9C, C25S, C76S and A88C; or the Titin-T chain has one or more amino acid residue mutations on positions selected from the group consisting of A8C, V20C, T22C, C25S, A26C and C39T, and/or the Obscurin-like-O chain has one or more amino acid residue mutations on positions selected from the group consisting of C6E, C26S, C77S, V74C, G84C and A86C. The mutation position in the Titin-T chain is a natural sequence numbering position relative to a sequence SEQ ID NO: 32; the mutation position in the Obscurin-O chain is a natural sequence numbering position relative to a sequence SEQ ID NO: 33; the mutation position in the Obscurin-like-O chain is a natural sequence numbering position relative to a sequence SEQ ID NO: 34.

In some embodiments, the particular mutated residue of the aforementioned first domain and the particular mutated residue of the second domain have mutations on positions selected from the group consisting of: the Titin-T chain has C25S, C39T and A8C mutations, and the Obscurin-O chain has an A88C mutation; the Titin-T chain has C25S, C39T and V20C mutations, and the Obscurin-O chain has an A3C mutation; the Titin-T chain has C25S, C39T and A26C mutations, and the Obscurin-O chain has an R9C mutation; the Titin-T chain has C25S, C39T and A8C mutations, and the Obscurin-O chain has C25S, C76S and A88C mutations; the Titin-T chain has C25S, C39T and V20C mutations, and the Obscurin-O chain has C25S, C76S and A3C mutations; the Titin-T chain has C25S, C39T and A26C mutations, and the Obscurin-O chain has C25S, C76S and R9C mutations; the Titin-T chain has C25S, C39T and A8C mutations, and the Obscurin-like-O chain has C6E and V74C mutations; the Titin-T chain has C25S, C39T and V20C mutations, and the Obscurin-like-O chain has C6E and G84C mutations; the Titin-T chain has C25S, C39T and T22C mutations, and the Obscurin-like-O chain has C6E and A86C mutations; the Titin-T chain has C25S, C39T and A8C mutations, and the Obscurin-like-O chain has C6E, C26S, C77S and V74C mutations; the Titin-T chain has C25S, C39T and V20C mutations, and the Obscurin-like-O chain has C6E, C26S, C77S and G84C mutations; the Titin-T chain has C25S, C39T and T22C mutations, and the Obscurin-like-O chain has C6E, C26S, C77S and A86C mutations. The mutation position in the Titin-T chain is a natural sequence numbering position relative to a sequence SEQ ID NO: 32; the mutation position in the Obscurin-O chain is a natural sequence numbering position relative to a sequence SEQ ID NO: 33; the mutation position in the Obscurin-like-O chain is a natural sequence numbering position relative to a sequence SEQ ID NO: 34.

In some embodiments, the aforementioned first domain and the aforementioned second domain are selected from the group consisting of the following Titin-T chain/Obscurin-O chain, or Titin-T chain/Obscurin-like-O chain: the Titin-T chain is a polypeptide having one or more amino acid residue mutations on positions selected from the group consisting of 8, 20, 22, 25, 26 and 39 on the basis of SEQ ID NO: 32, and/or the Obscurin-O chain is a polypeptide having one or more amino acid residue mutations on positions selected from the group consisting of 3, 9, 25, 76 and 88 on the basis of SEQ ID NO: 33; or the Titin-T chain is a polypeptide having one or more amino acid residue mutations on positions selected from the group consisting of 8, 20, 22, 25, 26 and 39 on the basis of SEQ ID NO: 32, and/or the Obscurin-like-O chain is a polypeptide having one or more amino acid residue mutations on positions selected from the group consisting of 6, 26, 74, 77, 84 and 86 on the basis of SEQ ID NO: 34.

In some embodiments, the aforementioned first domain and the aforementioned second domain are selected from the group consisting of: a Titin-T chain/Obscurin-O chain, and a Titin-T chain/Obscurin-like-O chain. The Titin-T chain has one or more amino acid residue mutations on positions selected from the group consisting of A8C, V20C, T22C, C25S, A26C and C39T on the basis of SEQ ID NO: 32, and/or the Obscurin-O chain has one or more amino acid residue mutations on positions selected from the group consisting of A3C, R9C, C25S, C76S and A88C on the basis of SEQ ID NO: 33; or the Titin-T chain has one or more amino acid residue mutations on positions selected from the group consisting of A8C, V20C, T22C, C25S, A26C and C39T on the basis of SEQ ID NO: 32, and/or the Obscurin-like-O chain has one or more amino acid residue mutations on positions selected from the group consisting of C6E, C26S, C77S, V74C, G84C and A86C on the basis of SEQ ID NO: 34. The mutation position in the Titin-T chain is a natural sequence numbering position of the sequence SEQ ID NO: 32; the mutation position in the Obscurin-O chain is a natural sequence numbering position of the sequence SEQ ID NO: 33; the mutation position in the Obscurin-like-O chain is a natural sequence numbering position of the sequence SEQ ID NO: 34.

In some embodiments, the aforementioned first domain and the aforementioned second domain are selected from the group consisting of: a Titin-T chain/Obscurin-O chain, and a Titin-T chain/Obscurin-like-O chain. The Titin-T chain has C25S, C39T and A8C mutations on the basis of SEQ ID NO: 32, and the Obscurin-O chain has an A88C mutation on the basis of SEQ ID NO: 33; the Titin-T chain has C25S, C39T and V20C mutations on the basis of SEQ ID NO: 32, and the Obscurin-O chain has an A3C mutation on the basis of SEQ ID NO: 33; the Titin-T chain has C25S, C39T and A26C mutations on the basis of SEQ ID NO: 32, and the Obscurin-O chain has an R9C mutation on the basis of SEQ ID NO: 33; the Titin-T chain has C25S, C39T and A8C mutations on the basis of SEQ ID NO: 32, and the Obscurin-O chain has C25S, C76S and A88C mutations on the basis of SEQ ID NO: 33; the Titin-T chain has C25S, C39T and V20C mutations on the basis of SEQ ID NO: 32, and the Obscurin-O chain has C25S, C76S and A3C mutations on the basis of SEQ ID NO: 33; the Titin-T chain has C25S, C39T and A26C mutations on the basis of SEQ ID NO: 32, and the Obscurin-O chain has C25S, C76S and R9C mutations on the basis of SEQ ID NO: 33; the Titin-T chain has C25S, C39T and A8C mutations on the basis of SEQ ID NO: 32, and the Obscurin-like-O chain has C6E and V74C mutations on the basis of SEQ ID NO: 34; the Titin-T chain has C25S, C39T and V20C mutations on the basis of SEQ ID NO: 32, and the Obscurin-like-O chain has C6E and G84C mutations on the basis of SEQ ID NO: 34; the Titin-T chain has C25S, C39T and T22C mutations on the basis of SEQ ID NO: 32, and the Obscurin-like-O chain has C6E and A86C mutations on the basis of SEQ ID NO: 34; the Titin-T chain has C25S, C39T and A8C mutations on the basis of SEQ ID NO: 32, and the Obscurin-like-O chain has C6E, C26S, C77S and V74C mutations on the basis of SEQ ID NO: 34; the Titin-T chain has C25S, C39T and V20C mutations on the basis of SEQ ID NO: 32, and the Obscurin-like-O chain has C6E, C26S, C77S and G84C mutations on the basis of SEQ ID NO: 34; the Titin-T chain has C25S, C39T and T22C mutations on the basis of SEQ ID NO: 32, and the Obscurin-like-O chain has C6E, C26S, C77S and A86C mutations on the basis of SEQ ID NO: 34. The mutation position in the Titin-T chain is a natural sequence numbering position relative to a sequence SEQ ID NO: 32; the mutation position in the Obscurin-O chain is a natural sequence numbering position relative to a sequence SEQ ID NO: 33; the mutation position in the Obscurin-like-O chain is a natural sequence numbering position relative to a sequence SEQ ID NO: 34.

In some embodiments, for the aforementioned polypeptide complex, the Obscurin-O chain has one or more amino acid residue mutations on positions selected from the group consisting of 7, 11 and 62. In some embodiments, the Obscurin-O chain has one or more amino acid residue mutations on positions selected from the group consisting of 7R and 7K, 62K and 62H, and 11L; most preferably, the Titin-T chain has 25S, 39T and 8C mutations, and the Obscurin-O chain has 25S, 76S, 88C, 7K and 62K mutations; the Titin-T chain has 25S, 39T and 8C mutations, and the Obscurin-O chain has 25S, 76S, 88C, 7K and 62H mutations; the Titin-T chain has 25S, 39T and 8C mutations, and the Obscurin-O chain has 25S, 76S, 88C, 11L and 62K mutations; or the Titin-T chain has 25S, 39T and 8C mutations and the Obscurin-O chain has 25S, 76S, 88C, 11L and 62H mutations; the mutation position in the Titin-T chain is a natural sequence numbering position relative to a sequence SEQ ID NO: 32; the mutation position in the Obscurin-O chain is a natural sequence numbering position relative to a sequence SEQ ID NO: 33. In some embodiments, the Obscurin-O chain has one or more amino acid residue mutations on positions selected from the group consisting of L7K and L7R, K11L, T62K and T62H; in some embodiments, the Titin-T chain has C25S, C39T, and A8C mutations, and the Obscurin-O chain has C25S, C76S, A88C, L7K and T62K mutations; the Titin-T chain has C25S, C39T and A8C mutations, and the Obscurin-O chain has C25S, C76S, A88C, L7K and T62H mutations; the Titin-T chain has C25S, C39T and A8C mutations, and the Obscurin-O chain has C25S, C76S, A88C, K11L and T62K mutations; the Titin-T chain has C25S, C39T and A8C mutations, and the Obscurin-O chain has C25S, C76S, A88C, K11L and T62H mutations. The mutation position in the Titin-T chain is a natural sequence numbering position relative to a sequence SEQ ID NO: 32; the mutation position in the Obscurin-O chain is a natural sequence numbering position relative to a sequence SEQ ID NO: 33.

In some embodiments, for the aforementioned polypeptide complex, the Obscurin-O chain is an Obscurin-O chain having one or more amino acid residue mutations on positions selected from the group consisting of 7, 11 and 62 on the basis of SEQ ID NO: 45; in some embodiments, the Obscurin-O chain has one or more amino acid residue mutations on positions selected from the group consisting of L7K and L7R, K11L and T62K and T62H on the basis of SEQ ID NO: 45; in some embodiments, the Titin-T chain has C25S, C39T and A8C mutations on the basis of SEQ ID NO: 32, and the Obscurin-O chain has C25S, C76S, A88C, L7K and T62K mutations on the basis of SEQ ID NO: 45; the Titin-T chain has C25S, C39T and A8C mutations on the basis of SEQ ID NO: 32, and the Obscurin-O chain has C25S, C76S, A88C, L7K and T62H mutations on the basis of SEQ ID NO: 45; the Titin-T chain has C25S, C39T and A8C mutations on the basis of SEQ ID NO: 32, and the Obscurin-O chain has C25S, C76S, A88C, K11L and T62K mutations on the basis of SEQ ID NO: 45; the Titin-T chain has C25S, C39T and A8C mutations on the basis of SEQ ID NO: 32, and the Obscurin-O chain has C25S, C76S, A88C, K11L and T62H mutations on the basis of SEQ ID NO: 45. The mutation position in the Titin-T chain is a natural sequence numbering position relative to a sequence SEQ ID NO: 32; the mutation position in the Obscurin-O chain is a natural sequence numbering position relative to a sequence SEQ ID NO: 33.

In some embodiments, for the aforementioned polypeptide complex, the first domain and the second domain have one or more amino acid residue mutations on positions selected from the group consisting of: the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84, and/or the Obscurin-O chain has one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97. The mutation position in the Titin-T chain is a natural sequence numbering position relative to a sequence SEQ ID NO: 35; the mutation position in the Obscurin-O chain is a natural sequence numbering position relative to a sequence SEQ ID NO: 50. In some embodiments, for the aforementioned polypeptide complex, the first domain and the second domain are selected from the group consisting of the following Titin-T chain/Obscurin-O chain: the Titin-T chain is a polypeptide having one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and the Obscurin-O chain is a polypeptide having one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97 on the basis of SEQ ID NO: 50. The mutation position in the Titin-T chain is a natural sequence numbering position relative to a sequence SEQ ID NO: 35; the mutation position in the Obscurin-O chain is a natural sequence numbering position relative to a sequence SEQ ID NO: 50.

In some embodiments, the aforementioned first domain and the aforementioned second domain have one or more residue mutations on positions selected from the group consisting of: the Titin-T chain has one or more amino acid mutations on positions selected from the group consisting of 3W, 11I, 13L, 22M, 40S, 42K, 45S, 47E, 49G, 56S, 58E, 66S and 66K, 70R, 75V, 77S, 79T, 81R, 82M, 83D and 84L, and/or the Obscurin-O chain has one or more amino acid mutations on positions selected from the group consisting of 2E, 11K, 12S, 13Y, 14T, 17E, 20L, 22M and 22S, 30D, 32P, 34E, 36T, 41K, 42L, 441, 45T, 53L, 58V, 62E, 67Q and 67T, 69S, 89L, 92E, 94G and 97G. In some embodiments, the Titin-T chain has 66S and 77S amino acid mutations, and/or the Obscurin-O chain has 11K, 12S, 13Y, 14T and 22S amino acid mutations; the Titin-T chain has 66K, 70R, 79T and 81R amino acid mutations, and/or the Obscurin-O chain has 2E, 17E, 30D, 32P, 34E, 36T, 441, 45T, 58V, 62E, 67Q, 69S and 97G amino acid mutations; the Titin-T chain has 3W, 11I, 13L, 22M and 82M amino acid mutations, and/or the Obscurin-O chain has 20L, 22M and 53L amino acid mutations; the Titin-T chain has 11I, 66K, 79T and 81R amino acid mutations, and/or the Obscurin-O chain has 41K, 45T, 67Q, 69S and 89L amino acid mutations; the Titin-T chain has 40S, 42K, 45S, 47E, 49G, 56S, 58E, 75V, 83D, and 84L amino acid mutations, and/or the Obscurin-O chain has 42L, 45T, 67T, 69S, 92E, and 94G amino acid mutations; the Titin-T chain has 47E, 49G, 56S, 58E and 75V amino acid mutations, and/or the Obscurin-O chain has 42L, 45T, 67T, 69S, 92E and 94G amino acid mutations; the Titin-T chain has 56S, 58E and 75V amino acid mutations, and/or the Obscurin-O chain has 42L, 45T, 67T, 69S, 92E and 94G amino acid mutations; or the Titin-T chain has 56S, 58E, 66S and 77S amino acid mutations, and/or the Obscurin-O chain has 12S, 13Y, 22S, 42L, 45T, 67Q, 69S, 92E and 94G amino acid mutations; the mutation position in the Titin-T chain is a natural sequence numbering position relative to a sequence SEQ ID NO: 35; the mutation position in the Obscurin-O chain is a natural sequence numbering position relative to a sequence SEQ ID NO: 50. In some embodiments, the aforementioned first domain and the aforementioned second domain have one or more residue mutations on positions selected from the group consisting of: the Titin-T chain has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L, and/or the Obscurin-O chain has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G. The mutation position in the Titin-T chain is a natural sequence numbering position relative to a sequence SEQ ID NO: 35; the mutation position in the Obscurin-O chain is a natural sequence numbering position relative to a sequence SEQ ID NO: 50.

In some embodiments, the aforementioned first domain and the aforementioned second domain are selected from the group consisting of the following Titin-T chain/Obscurin-O chain: the Titin-T chain has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L on the basis of SEQ ID NO: 35, and/or the Obscurin-O chain has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G on the basis of SEQ ID NO: 35. The mutation position in the Titin-T chain is a natural sequence numbering position of a sequence SEQ ID NO: 35; the mutation position in the Obscurin-O chain is a natural sequence numbering position of a sequence SEQ ID NO: 50.

In some embodiments, the aforementioned Titin-T chain has M66S and T77S amino acid mutations, and/or the Obscurin-O chain has L11K, A12S, F13Y, V14T and T22S amino acid mutations; the Titin-T chain has M66K, K70R, S79T and G81R amino acid mutations, and/or the Obscurin-O chain has G2E, V17E, N30D, T32P, Q34E, S36T, V44I, A45T, L58V, K62E, A67Q, G69S and A97G amino acid mutations; the Titin-T chain has P3W, S11I, I13L, T22M and N82M amino acid mutations, and/or the Obscurin-O chain has D20L, T22M and A53L amino acid mutations; the Titin-T chain has S11I, M66K, S79T and G81R amino acid mutations, and/or the Obscurin-O chain has Q41K, A45T, A67Q, G69S and V89L amino acid mutations; the Titin-T chain has G40S, R42K, H45S, Q47E, Q49G, N56S, D58E, L75V, E83D and F84L amino acid mutations, and/or the Obscurin-O chain has Q42L, A45T, A67T, G69S, Q92E and D94G amino acid mutations; the Titin-T chain has Q47E, Q49G, N56S, D58E and L75V amino acid mutations, and/or the Obscurin-O chain has Q42L, A45T, A67T, G69S, Q92E and D94G amino acid mutations; the Titin-T chain has N56S, D58E and L75V amino acid mutations, and/or the Obscurin-O chain has Q42L, A45T, A67T, G69S, Q92E and D94G amino acid mutations; or the Titin-T chain has N56S, D58E, M66S and T77S amino acid mutations, and/or the Obscurin-O chain has A12S, F13Y, T22S, Q42L, A45T, A67Q, G69S, Q92E and D94G amino acid mutations. The mutation position in the Titin-T chain is a natural sequence numbering position relative to a sequence SEQ ID NO: 35; the mutation position in the Obscurin-O chain is a natural sequence numbering position relative to a sequence SEQ ID NO: 50.

In some embodiments, the aforementioned first domain and the aforementioned second domain are selected from the group consisting of the following Titin-T chain/Obscurin-O chain: the Titin-T chain has M66S and T77S amino acid mutations on the basis of SEQ ID NO: 35, and/or the Obscurin-O chain has L11K, A12S, F13Y, V14T and T22S amino acid mutations on the basis of SEQ ID NO: 50; the Titin-T chain has M66K, K70R, S79T and G81R amino acid mutations on the basis of SEQ ID NO: 35, and/or the Obscurin-O chain has G2E, V17E, N30D, T32P, Q34E, S36T, V44I, A45T, L58V, K62E, A67Q, G69S and A97G amino acid mutations on the basis of SEQ ID NO: 50; the Titin-T chain has P3W, S11I, I13L, T22M and N82M amino acid mutations on the basis of SEQ ID NO: 35, and/or the Obscurin-O chain has D20L, T22M and A53L amino acid mutations on the basis of SEQ ID NO: 50; the Titin-T chain has S11I, M66K, S79T and G81R amino acid mutations on the basis of SEQ ID NO: 35, and/or the Obscurin-O chain has Q41K, A45T, A67Q, G69S and V89L amino acid mutations on the basis of SEQ ID NO: 50; the Titin-T chain has G40S, R42K, H45S, Q47E, Q49G, N56S, D58E, L75V, E83D and F84L amino acid mutations on the basis of SEQ ID NO: 35, and/or the Obscurin-O chain has Q42L, A45T, A67T, G69S, Q92E and D94G amino acid mutations on the basis of SEQ ID NO: 50; the Titin-T chain has Q47E, Q49G, N56S, D58E and L75V amino acid mutations on the basis of SEQ ID NO: 35, and/or the Obscurin-O chain has Q42L, A45T, A67T, G69S, Q92E and D94G amino acid mutations on the basis of SEQ ID NO: 50; the Titin-T chain has N56S, D58E and L75V amino acid mutations on the basis of SEQ ID NO: 35, and/or the Obscurin-O chain has Q42L, A45T, A67T, G69S, Q92E and D94G amino acid mutations on the basis of SEQ ID NO: 50; or the Titin-T chain has N56S, D58E, M66S and T77S amino acid mutations on the basis of SEQ ID NO: 35, and/or the Obscurin-O chain has A12S, F13Y, T22S, Q42L, A45T, A67Q, G69S, Q92E and D94G amino acid mutations on the basis of SEQ ID NO: 50. The mutation position in the Titin-T chain is a natural sequence numbering position of a sequence SEQ ID NO: 35; the mutation position in the Obscurin-O chain is a natural sequence numbering position of a sequence SEQ ID NO: 50.

In some embodiments, for the aforementioned polypeptide complex, the VH1 is operably linked to the first domain through a first linking domain, and the VL1 is operably linked to the second domain through a second linking domain.

In some embodiments, the aforementioned first and/or second linking domain is selected from the group consisting of: an N-terminal fragment of the Titin-T chain, an N-terminal fragment of the Obscurin-O chain and an N-terminal fragment of the Obscurin-like-O chain, and a (GₓS)_{y} linker, wherein X is selected from the group consisting of integers from 1 to 5 (for example, 1, 2, 3, 4 or 5), and Y is selected from the group consisting of integers from 1 to 6 (for example, 1, 2, 3, 4, 5 or 6).

In some embodiments, the aforementioned first and/or second linking domain is selected from the group consisting of: "KAGIR", "DQPQF", (G₄S)₁ and (G₄S)₂.

In some embodiments, for the aforementioned polypeptide complex, when the first domain is a Titin-T chain, the first linking domain is a KAGIR polypeptide; when the second domain is an Obscurin-O chain, the second linking domain is a DQPQF polypeptide. In other embodiments, when the second domain is a Titin-T chain, the second linking domain is a KAGIR polypeptide, and when the first domain is an Obscurin-O chain, the first linking domain is a DQPQF polypeptide.

In some embodiments of the polypeptide complex, wherein,
(A) the Titin-T chain has a sequence set forth in SEQ ID NO: 32 or further has a sequence having one or more amino acid mutations on positions selected from the group consisting of 8, 20, 22, 25, 26, 39, 3, 11, 13, 40, 42, 45, 47, 49, 56, 58, 66, 70, 75, 77, 79, 81, 82, 83 and 84 and/or 5 amino acid residues of KAGIR added at the N-terminus on the basis of SEQ ID NO: 32; preferably, the Titin-T chain has a sequence having one or more amino acid mutations on positions selected from the group consisting of A8C, V20C, T22C, C25S, A26C, C39T, P3W, S11I, I13L, T22M, G40S, R42K, H45S, Q47E, Q49G, N56S, D58E, M66S and M66K, K70R, L75V, T77S, S79T, G81R, N82M, E83D and F84L and/or a KAGIR amino acid residue added at the N-terminus; and/or
(B) the Obscurin-O chain has a sequence set forth in SEQ ID NO: 33 or further has a sequence having one or more amino acid mutations on positions selected from the group consisting of 3, 9, 25, 76, 88, 7, 11, 62, 2, 12, 13, 14, 17, 20, 22, 30, 32, 34, 36, 41, 42, 44, 45, 53, 58, 67, 69, 89, 92, 94 and 97 and/or a KAGIR amino acid residue added at the N-terminus on the basis of SEQ ID NO: 33; preferably, the Obscurin-O chain has a sequence having one or more amino acid mutations on positions selected from the group consisting of A3C, R9C, C25S, C76S, A88C, L7K and L7R, T62K and T62H, G2E, L11K, A12S, F13Y, V14T, V17E, D20L, T22M and T22S, N30D, T32P, Q34E, S36T, Q41K, Q42L, V44I, A45T, A53L, L58V, T62E, A67Q and A67T, G69S, V89L, Q92E, D94G and A97G and/or a DQPQF amino acid residue added at the N-terminus; or
   the Obscurin-like-O chain has a sequence set forth in SEQ ID NO: 34 or further has a sequence having one or more amino acid mutations on positions selected from the group consisting of 6, 26, 74, 77, 84 and 86 on the basis of SEQ ID NO: 34; preferably, the Obscurin-like-O chain has a sequence having one or more amino acid mutations on positions selected from the group consisting of C6E, C26S, C77S, V74C, G84C and A86C;
   the mutation position in the Titin-T chain is a natural sequence numbering position of the sequence SEQ ID NO: 32; the mutation position in the Obscurin-O chain is a natural sequence numbering position of the sequence SEQ ID NO: 33; the mutation position in the Obscurin-like-O chain is a natural sequence numbering position of the sequence SEQ ID NO: 34.

In some embodiments of the polypeptide complex, wherein (A) the Titin-T chain has one or more amino acid residue mutations on positions selected from the group consisting of 8, 20, 22, 25, 26 and 39 on the basis of SEQ ID NO: 32, and preferably has one or more amino acid residue mutations on positions selected from the group consisting of A8C, V20C, T22C, C25S, A26C and C39T, and the mutation position in the Titin-T chain is a natural sequence numbering position of SEQ ID NO: 32; more preferably, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and preferably has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L; the mutation position in the Titin-T chain is a natural sequence numbering position of SEQ ID NO: 35, and/or
(B) the Obscurin-O chain has one or more amino acid residue mutations on positions selected from the group consisting of 3, 9, 25, 76 and 88 on the basis of SEQ ID NO: 33, and preferably has one or more amino acid residue mutations on positions selected from the group consisting of A3C, R9C, C25S, C76S and A88C, and the mutation position in the Obscurin-O chain is a natural sequence numbering position of SEQ ID NO: 33; preferably, the Obscurin-O chain has one or more amino acid residue mutations on positions selected from the group consisting of 7, 11 and 62 on the basis of SEQ ID NO: 45, and preferably has one or more amino acid residue mutations on positions selected from the group consisting of L7K and L7R, T62K and T62H, and K11L, and the mutation position in the Obscurin-O chain is a natural sequence numbering position of SEQ ID NO: 45; more preferably, the Obscurin-O chain has one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97 on the basis of SEQ ID NO: 50, and preferably has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G, and the mutation position in the Obscurin-O chain is a natural sequence numbering position of SEQ ID NO: 50; or
the Obscurin-like-O chain has one or more amino acid residue mutations on positions selected from the group consisting of 6, 26, 74, 77, 84 and 86 on the basis of SEQ ID NO: 34, and preferably has one or more amino acid residue mutations on positions selected from the group consisting of C6E, C26S, C77S, V74C, G84C and A86C, and the mutation position in the Obscurin-like-O chain is a natural sequence numbering position of SEQ ID NO: 34.

In some embodiments of the polypeptide complex, wherein the Titin-T chain comprises a polypeptide having a sequence set forth in any one of SEQ ID NOs: 32, 35-41, 65-76 or a polypeptide having a sequence having at least 80% sequence identity to any one of SEQ ID NOs: 32, 35-41, 65-76, and/or the Obscurin-O chain comprises a polypeptide having a sequence set forth in any one of SEQ ID NOs: 33, 42-58, 77-86 or a polypeptide having a sequence having at least 80% sequence identity to any one of SEQ ID NOs: 33, 42-58, 77-86; or the Titin-T chain comprises a polypeptide having a sequence set forth in any one of SEQ ID NOs: 32, 35-41, 65-76 or a polypeptide having a sequence having at least 80% sequence identity to any one of SEQ ID NOs: 32, 35-41, 65-76, and/or the Obscurin-like-O chain comprises a polypeptide having a sequence set forth in any one of SEQ ID NOs: 34, 59-64 or a polypeptide having a sequence having at least 80% sequence identity to any one of SEQ ID NOs: 34, 59-64. Wherein, the at least 80% sequence identity refers to, for example, at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity.

In some embodiments, the aforementioned Titin-T chain comprises a polypeptide having a sequence set forth in SEQ ID NO: 68, and the Obscurin-O chain comprises a polypeptide having a sequence set forth in SEQ ID NO: 80.

In other embodiments, the Titin-T chain comprises a polypeptide having a sequence set forth in SEQ ID NO: 73, and the Obscurin-O chain comprises a polypeptide having a sequence set forth in SEQ ID NO: 83.

In other embodiments, the Titin-T chain comprises a polypeptide having a sequence set forth in SEQ ID NO: 76, and the Obscurin-O chain comprises a polypeptide having a sequence set forth in SEQ ID NO: 84.

In other embodiments, the Titin-T chain comprises a polypeptide having a sequence set forth in SEQ ID NO: 76, and the Obscurin-O chain comprises a polypeptide having a sequence set forth in SEQ ID NO: 86.

In other embodiments, the Titin-T chain comprises a polypeptide having a sequence set forth in SEQ ID NO: 76, and the Obscurin-O chain comprises a polypeptide having a sequence set forth in SEQ ID NO: 86.

In other embodiments, the Titin-T chain comprises a polypeptide having a sequence set forth in SEQ ID NO: 75, and the Obscurin-O chain comprises a polypeptide having a sequence set forth in SEQ ID NO: 86.

In some embodiments, the Titin-T chain is a polypeptide having a sequence set forth in SEQ ID NO: 68, and the Obscurin-O chain is a polypeptide having a sequence set forth in SEQ ID NO: 80; the Titin-T chain is a polypeptide having a sequence set forth in SEQ ID NO: 73, and the Obscurin-O chain is a polypeptide having a sequence set forth in SEQ ID NO: 83; the Titin-T chain is a polypeptide having a sequence set forth in SEQ ID NO: 76, and the Obscurin-O chain is a polypeptide having a sequence set forth in SEQ ID NO: 84; the Titin-T chain is a polypeptide having a sequence set forth in SEQ ID NO: 76, and the Obscurin-O chain is a polypeptide having a sequence set forth in SEQ ID NO: 86; the Titin-T chain is a polypeptide having a sequence set forth in SEQ ID NO: 76, and the Obscurin-O chain is a polypeptide having a sequence set forth in SEQ ID NO: 86; or the Titin-T chain is a polypeptide having a sequence set forth in SEQ ID NO: 75, and the Obscurin-O chain is a polypeptide having a sequence set forth in SEQ ID NO: 86.

In some embodiments, for the aforementioned polypeptide complex, the first antigen is selected from the group consisting of: a foreign antigen, an endogenous antigen, an autoantigen, a neoantigen, a viral antigen and a tumor antigen.

In some embodiments, disclosed is a multispecific polypeptide complex, comprising:
a first polypeptide complex, being any one of the aforementioned polypeptide complexes and specifically binding to a first antigen, and
a second polypeptide complex, comprising a second antigen-binding moiety and specifically binding to a second antigen.

In some embodiments, the first polypeptide complex and the second polypeptide complex bind to two different antigens.

In some embodiments, the first polypeptide complex and the second polypeptide complex bind to two different epitopes on the same antigen.

In some embodiments, the multispecific polypeptide complex is a multispecific polypeptide complex, and more preferably, the multispecific polypeptide complex is a bispecific antibody.

In some embodiments, the second polypeptide complex of the multispecific polypeptide complex comprises a second heavy chain variable domain (VH2) and a second light chain variable domain (VL2). Mispairing between the VH1 of the first polypeptide complex and the VL2 of the second polypeptide complex, and/or between the VH2 of the second polypeptide complex and the VL1 of the first polypeptide complex is less susceptible to happen. In some embodiments, the VH2 and the VL2 of the second antigen-binding moiety form a second antigen-binding site specifically binding to the second antigen.

In some embodiments, for the aforementioned multispecific polypeptide complex, the second antigen-binding moiety of the second polypeptide complex comprises:
a third polypeptide, comprising a VH2 from an N-terminus to a C-terminus, the VH2 being operably linked to a third domain comprising a CH1, and
a fourth polypeptide, comprising a VL2 from an N-terminus to a C-terminus, the VL2 being operably linked to a fourth domain comprising a CL.

In some embodiments, the C-terminus of VH2 is linked to the N-terminus of CH1, and the C-terminus of VL2 is linked to the N-terminus of CL.

In some embodiments, for the aforementioned multispecific polypeptide complex, the first polypeptide complex further comprises an operably-linked first dimerization domain, and the second polypeptide complex further comprises an operably-linked second dimerization domain, the first dimerization domain and the second dimerization domain being bound together.

In some embodiments, a C-terminus of the first domain of the first polypeptide complex is operably linked to an N-terminus of the first dimerization domain, and a C-terminus of the third domain of the second polypeptide complex is operably linked to an N-terminus of the second dimerization domain.

In some embodiments, the aforementioned first dimerization domain and the aforementioned second dimerization domain are bound to form a dimer by a means of an antibody hinge region or a portion thereof, a linker, a disulfide bond, a hydrogen bond, an electrostatic interaction, a salt bridge, a hydrophobic-hydrophilic interaction, or a combination thereof.

In some embodiments, the aforementioned first dimerization domain and the aforementioned second dimerization domain are bound to form a dimer by an antibody hinge region or a portion thereof, and preferably a hinge region of IgG1, IgG2 or IgG4 or a portion thereof. In some embodiments, the hinge region of IgG1, IgG2, IgG3 or IgG4 or a portion thereof is preferred to form a dimer.

In some embodiments, for the aforementioned multispecific polypeptide complex, the first dimerization domain and/or the second dimerization domain further comprises an antibody CH2 domain and/or an antibody CH3 domain.

In some embodiments, for the aforementioned multispecific polypeptide complex, the antibody CH2 domain and/or the antibody CH3 domain is derived from IgG1, IgG2 or IgG4. In some embodiments, the antibody CH2 domain and/or the antibody CH3 domain is derived from IgG1, IgG2, IgG3 or IgG4.

In some embodiments, for the aforementioned multispecific polypeptide complex, the first polypeptide sequentially comprises VH1-L1-T chain-L2-CH2-CH3 from an N-terminus to a C-terminus, the second polypeptide sequentially comprises VL1-L3-O chain or VL1-L4-OL chain from an N-terminus to a C-terminus, the third polypeptide sequentially comprises VH2-CH1-CH2-CH3 from an N-terminus to a C-terminus, and the fourth polypeptide sequentially comprises VL1-CL from an N-terminus to a C-terminus; or the first polypeptide sequentially comprises VH1-L2-O chain-L3-CH2-CH3 or VH1-L4-OL chain-L5-CH2-CH3 from an N-terminus to a C-terminus, the second polypeptide sequentially comprises VL1-L1-T chain from an N-terminus to a C-terminus, the third polypeptide sequentially comprises VH2-CH1-CH2-CH3 from an N-terminus to a C-terminus, and the fourth polypeptide sequentially comprises VL2-CL from an N-terminus to a C-terminus; the L1 to L5 are spacers (also known as linking domains) which may or may not be present. Preferably, the spacer is selected from the group consisting of: an N-terminal fragment of a Titin-T chain, an N-terminal fragment of an Obscurin-O chain, an N-terminal fragment of an Obscurin-like-O chain, and a linker (e.g., (GₓS)_{y} linker), wherein X is selected from the group consisting of integers from 1 to 5, and Y is selected from the group consisting of integers from 1 to 6. Most preferably, the spacer is selected from the group consisting of: "KAGIR", "DQPQF", (G₄S)₁ and (G₄S)₂.

In some embodiments, for the aforementioned multispecific polypeptide complex, the first dimerization domain and the second dimerization domain are not identical and are bound in a manner that prevents homodimerization and/or favors heterodimerization.

In some embodiments, the aforementioned first dimerization domain and the aforementioned second dimerization domain are bound to form a dimer by a means of knob-into-hole, a hydrophobic interaction, an electrostatic interaction, a hydrophilic interaction or a flexibility increase.

In some embodiments, the aforementioned first dimerization domain has amino acid residues at positions 104 to 330 of a sequence set forth in SEQ ID NO: 2, and the second dimerization domain has amino acid residues at positions 104 to 330 of a sequence set forth in SEQ ID NO: 3; or the first dimerization domain has amino acid residues at positions 104 to 330 of a sequence set forth in SEQ ID NO: 3, and the second dimerization domain has amino acid residues at positions 104 to 330 of a sequence set forth in SEQ ID NO: 2.

In some embodiments, the present disclosure provides a domain engineered antibody in which at least one constant region domain CH1 and at least one constant region domain CL are substituted, wherein,
the domain CH1 is substituted with a Titin-T chain, and the domain CL is substituted with a domain capable of having an inter-protein interaction with the Titin-T chain;
the domain CL is substituted with the Titin-T chain, and the domain CH1 is substituted with a domain capable of having an inter-protein interaction with the Titin-T chain;
the domain CH1 is substituted with an Obscurin-O chain, and the domain CL is substituted with a domain capable of having an inter-protein interaction with the Obscurin-O chain;
the domain CL is substituted with the Obscurin-O chain, and the domain CH1 is substituted with a domain capable of having an inter-protein interaction with the Obscurin-O chain;
the domain CH1 is substituted with an Obscurin-like-O chain, and the domain CL is substituted with a domain capable of having an inter-protein interaction with the Obscurin-like-O chain; or
the domain CL is substituted with the Obscurin-like-O chain, and the domain CH1 is substituted with a domain capable of having an inter-protein interaction with the Obscurin-like-O chain.

In some embodiments, for the aforementioned domain engineered antibody, the domain capable of interacting with the Obscurin-O chain or the Obscurin-like-O chain is the Titin-T chain, and the domain capable of interacting with the Titin-T chain is the Obscurin-O chain or the Obscurin-like-O chain.

In some embodiments, the antibody comprises a heavy chain variable region VH1 and a light chain variable region VL1, the VH1 and the VL1 forming a primary binding site specifically binding to an antigen thereof; a C-terminus of the VH1 is operably linked to an N-terminus of the Titin-T chain, a C-terminus of the VL1 is operably linked to an N-terminus of the Obscurin-O chain or the Obscurin-like-O chain, or the C-terminus of the VL1 is operably linked to the N-terminus of the Titin-T chain, and the C-terminus of the VH1 is operably linked to the N-terminus of the Obscurin-O chain or the Obscurin-like-O chain.

In some embodiments, the present disclosure provides a Fab fragment of a domain engineered antibody in which a constant region domain CH1 and a constant region domain CL are substituted, wherein,
the domain CH1 is substituted with a Titin-T chain, and the domain CL is substituted with a domain capable of having an inter-protein interaction with the Titin-T chain; or
the domain CL is substituted with the Titin-T chain, and the domain CH1 is substituted with a domain capable of having an inter-protein interaction with the Titin-T chain; or the domain CH1 is substituted with an Obscurin-O chain, and the domain CL is substituted with a domain capable of having an inter-protein interaction with the Obscurin-O chain; or
the domain CL is substituted with the Obscurin-O chain, and the domain CH1 is substituted with a domain capable of having an inter-protein interaction with the Obscurin-O chain; or
the domain CH1 is substituted with the Obscurin-like-O chain, and the domain CL is substituted with a domain capable of having an inter-protein interaction with the Obscurin-like-O chain; or
the domain CL is substituted with the Obscurin-like-O chain, and the domain CH1 is substituted with a domain capable of having an inter-protein interaction with the Obscurin-like-O chain.

In some embodiments, for the aforementioned Fab fragment, the domain having an inter-protein interaction with the Titin-T chain is the Obscurin-O chain or the Obscurin-like-O chain; the domain having an inter-protein interaction with the Obscurin-O chain or the Obscurin-like-O chain is the Titin-T chain.

In some embodiments, the Fab comprises a heavy chain variable region VH1 and a light chain variable region VL1, the VH1 and the VL1 forming a primary binding site specifically binding to an antigen thereof; a C-terminus of the VH1 is operably linked to an N-terminus of the Titin-T chain, a C-terminus of the VL1 is operably linked to an N-terminus of the Obscurin-O chain or the Obscurin-like-O chain, or the C-terminus of the VL1 is operably linked to the N-terminus of the Titin-T chain, and the C-terminus of the VH1 is operably linked to the N-terminus of the Obscurin-O chain or the Obscurin-like-O chain.

In some embodiments, the present disclosure provides a domain engineered antibody comprising the aforementioned Fab fragment.

In some embodiments, the present disclosure provides a bispecific antibody comprising a first heavy chain and a first light chain specifically binding to a first antigen and further comprising a second heavy chain and a second light chain specifically binding to a second antigen, wherein,
a CH1 domain of the first heavy chain is substituted with a Titin-T chain, and a CL domain of the first light chain is substituted with a domain capable of having an inter-protein interaction with the Titin-T chain; or
the CL domain of the first light chain is substituted with the Titin-T chain, and the CH1 domain of the first heavy chain is substituted with a domain capable of having an inter-protein interaction with the Titin-T chain; or
the domain CH1 of the first heavy chain is substituted with the Obscurin-O chain, and the domain CL of the first light chain is substituted with a domain capable of having an inter-protein interaction with the Obscurin-O chain; or
the domain CL of the first light chain is substituted with the Obscurin-O chain, and the domain CH1 of the first heavy chain is substituted with a domain capable of having an inter-protein interaction with the Obscurin-O chain; or
the domain CH1 of the first heavy chain is substituted with the Obscurin-like-O chain, and the domain CL of the first light chain is substituted with a domain capable of having an inter-protein interaction with the Obscurin-like-O chain; or
the domain CL of the first light chain is substituted with the Obscurin-like-O chain, and the domain CH1 of the first heavy chain is substituted with a domain capable of having an inter-protein interaction with the Obscurin-like-O chain.

The bispecific antibody binds to a first antigen and a second antigen which are not identical, or binds to two different epitopes on the same antigen.

In specific embodiments, mispairing between the first heavy chain and the second light chain, and between the first light chain and the second heavy chain is less susceptible to happen.

In some embodiments, for the aforementioned bispecific antibody, the domain having an inter-protein interaction with the Titin-T chain is the Obscurin-O chain or the Obscurin-like-O chain; the domain having an inter-protein interaction with the Obscurin-O chain or the Obscurin-like-O chain is the Titin-T chain.

In some embodiments, the first heavy chain comprises a first heavy chain variable region (VH1), and the first light chain comprises a first light chain variable region (VL1), the VH1 and the VL1 forming an antigen-binding site specifically binding to a first antigen, wherein a C-terminus of the VH1 is operably linked to an N-terminus of the Titin-T chain, a C-terminus of the VL1 is operably linked to an N-terminus of the Obscurin-O chain or the Obscurin-like-O chain, or the C-terminus of the VL1 is operably linked to the N-terminus of the Titin-T chain, and the C-terminus of the VH1 is operably linked to the N-terminus of the Obscurin-O chain or the Obscurin-like-O chain.

In some embodiments, for the aforementioned bispecific antibody, the second heavy chain comprises a second heavy chain variable region (VH2) from an N-terminus to a C-terminus operably linked to the CH1; the second light chain comprises a second light chain variable region (VL2) from an N-terminus to a C-terminus operably linked to the CL.

In some embodiments, for the aforementioned bispecific antibody, the VH2 and the VL2 form an antigen-binding site specifically binding a second antigen, and the C-terminus of the VH2 is operably linked to the N-terminus of the CH1; the C-terminus of the VL2 is operably linked to the N-terminus of the CL.

In some embodiments, for the aforementioned bispecific antibody, the bispecific antibody structural model is selected from the group consisting of F(ab)₂, scFv-Fab, (scFv)₂-Fab, KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP- DART, CODV-Fab-TL, HLE-BiTE, F(ab)₂-CrossMAb, IgG-(scFv)₂, Bs4Ab, DVD-Ig, Tetravalent-DART-Fc, (scFv)₄-Fc, CODV-Ig, mAb2, F(ab)₄-CrossMAb, IgG and Fab-IgG, and the like.

In some embodiments, for the aforementioned bispecific antibody, C-termini of the first heavy chain and the second heavy chain comprise a CH2 domain and/or a CH3 domain. In some embodiments, the aforementioned CH1, CH2 and CH3 domains are derived from IgG1, IgG2 or IgG4. In some embodiments, the aforementioned CH1, CH2 and CH3 domains are derived from IgG1, IgG2, IgG3 or IgG4. In some embodiments, the aforementioned first heavy chain and the aforementioned second heavy chain are bound to form a dimer by a means of an antibody hinge region or a portion thereof, a linker, a disulfide bond, a hydrogen bond, an electrostatic interaction, a salt bridge, a hydrophobic-hydrophilic interaction, or a combination thereof.

In some embodiments, for the aforementioned bispecific antibody, the first heavy chain sequentially comprises VH1-L1-T chain-L2-CH2-CH3 from an N-terminus to a C-terminus, and the first light chain sequentially comprises VL1-L3-O chain or VL1-L4-OL chain from an N-terminus to a C-terminus; the second heavy chain sequentially comprises VH2-CH1-CH2-CH3 from an N-terminus to a C-terminus, and the second light chain sequentially comprises VL2-CL from an N-terminus to a C-terminus; or
the first light chain sequentially comprises VL1-L1-T chain from an N-terminus to a C-terminus, and the first heavy chain sequentially comprises VH1-L2-O chain-L3-CH2-CH3 or VH1-L4-OL chain-L5-CH2-CH3 from an N-terminus to a C-terminus; the second heavy chain sequentially comprises VH2-CH1-CH2-CH3 from an N-terminus to a C-terminus, and the second light chain sequentially comprises VL2-CL from an N-terminus to a C-terminus;
wherein L1 to L5 are spacers which may or may not be present. Preferably, the spacer is an N-terminal fragment of a Titin-T chain, an N-terminal fragment of an Obscurin-O chain, an N-terminal fragment of an Obscurin-like-O chain or a linker, for example, a (GₓS)_{y} linker, wherein X is selected from the group consisting of integers from 1 to 5, and Y is selected from the group consisting of integers from 1 to 6; preferably, the spacer is selected from the group consisting of: "KAGIR", "DQPQF", (G₄S)₁ and (G4S)2.

In some embodiments, for the aforementioned bispecific antibody, the CH2 and the CH3 of the first heavy chain are not identical to the CH2 and the CH3 of the second heavy chain and are bound in a manner that prevents homodimerization and/or favors heterodimerization.

In some embodiments, the aforementioned first heavy chain and the aforementioned second heavy chain are bound to form a dimer by a means of knob-into-hole, a hydrophobic interaction, an electrostatic interaction, a hydrophilic interaction or a flexibility increase.

In some embodiments, the aforementioned first heavy chain and the aforementioned second heavy chain are bound by knob-into-hole to form a dimer. In some embodiments, the CH2-CH3 domain of the aforementioned first heavy chain has amino acid residues at positions 104 to 330 of a sequence set forth in SEQ ID NO: 2, and the CH2-CH3 domain of the second heavy chain has amino acid residues at positions 104 to 330 of a sequence set forth in SEQ ID NO: 3; or the CH2-CH3 domain of the first heavy chain has amino acid residues at positions 104 to 330 of a sequence set forth in SEQ ID NO: 3, and the CH2-CH3 domain of the second heavy chain has amino acid residues at positions 104 to 330 of a sequence set forth in SEQ ID NO: 2.

In some embodiments, for the aforementioned bispecific antibody, the first heavy chain sequentially comprises VH1-L1-T chain-L2-knob-Fc from an N-terminus to a C-terminus, the first light chain sequentially comprises VL1-L3-O chain or VL1-L4-OL chain from an N-terminus to a C-terminus, the second heavy chain sequentially comprises VH2-CH1-hole-Fc from an N-terminus to a C-terminus, and the second light chain sequentially comprises VL1-CL from an N-terminus to a C-terminus; or the first heavy chain sequentially comprises VH1-L1-T chain-L2-hole-Fc from an N-terminus to a C-terminus, the first light chain sequentially comprises VL1-L3-O chain or VL1-L4-OL chain from an N-terminus to a C-terminus, the second heavy chain sequentially comprises VH2-CH1-knob-Fc from an N-terminus to a C-terminus, and the second light chain sequentially comprises VL2-CL from an N-terminus to a C-terminus; or
the first light chain sequentially comprises VL1-L1-T chain from an N-terminus to a C-terminus, the first heavy chain sequentially comprises VH1-L2-O chain-L3-knob-Fc or VH1-L4-OL chain-L5-knob-Fc from an N-terminus to a C-terminus, the second heavy chain sequentially comprises VH2-CH1-hole-Fc from an N-terminus to a C-terminus, and the second light chain sequentially comprises VL2-CL from an N-terminus to a C-terminus; the first light chain sequentially comprises VL1-L1-T chain from an N-terminus to a C-terminus, the first heavy chain sequentially comprises VH1-L2-O chain-L3-hole-Fc or VH1-L4-OL chain-L5-hole-Fc from an N-terminus to a C-terminus, the second heavy chain sequentially comprises VH2-CH1-knob-Fc from an N-terminus to a C-terminus, and the second light chain sequentially comprises VL2-CL from an N-terminus to a C-terminus;
the L1 to L5 are spacers which may or may not be present. Preferably, the spacer is an N-terminal fragment of a Titin-T chain, an N-terminal fragment of an Obscurin-O chain, an N-terminal fragment of an Obscurin-like-O chain or a linker, for example, a (GₓS)_{y} linker, wherein X is selected from the group consisting of integers from 1 to 5, and Y is selected from the group consisting of integers from 1 to 6; preferably, the spacer is selected from the group consisting of: "KAGIR", "DQPQF", (G₄S)₁ and (G₄S)₂.

In some embodiments, for any one of the aforementioned domain engineered antibody, the Fab fragment of a domain engineered antibody, or the bispecific antibody, the domain having an inter-protein interaction with a Titin-T chain is an Obscurin-O chain or an Obscurin-like-O chain; the domain having an inter-protein interaction with an Obscurin-O chain or an Obscurin-like-O chain is a Titin-T chain. For any one of the aforementioned domain engineered antibody, or the Fab fragment of a domain engineered antibody, or the bispecific antibody, the operable linkage is a linkage of two polypeptide sequences through a spacer or a direct linkage of two polypeptides.

In some embodiments, for the aforementioned domain engineered antibody, or the bispecific antibody, or the Fab fragment of a domain engineered antibody, the Titin-T chain and the Obscurin-O chain, or the Titin-T chain and the Obscurin-like-O chain, are bound through natural inter-chain bonds and/or unnatural inter-chain bonds to form a dimer.

In some embodiments, the first domain and the second domain form a dimer through natural inter-chain bonds, and one or more residues at positions selected from the group consisting of 7-15, 19-24, 26, 55, 59 and 60 on the Titin-T chain and one or more residues at positions selected from the group consisting of 3-6, 9, 41, 73, 75, and 80-90 on the Obscurin-O chain are linked to each other, or
one or more residues selected from the group consisting of positions 1, 7-10, 13-16, 19-26, 59-60 and 96 on the Titin-T chain and one or more residues selected from the group consisting of positions 4-5, 10, 12-13, 74, 76, 78 and 82-91 on the Obscurin-like-O chain are linked to each other. the residue position in the Titin-T chain is a natural sequence numbering position relative to a sequence SEQ ID NO: 32; the residue position in the Obscurin-O chain is a natural sequence numbering position relative to a sequence SEQ ID NO: 33; the residue position in the Obscurin-like-O chain is a natural sequence numbering position relative to a sequence SEQ ID NO: 34.

In some embodiments, for the aforementioned domain engineered antibody, the bispecific antibody, or the Fab fragment of a domain engineered antibody, the Titin-T chain and the Obscurin-O chain, or the Titin-T chain and the Obscurin-like-O chain, form a dimer through at least one non-natural inter-chain bonds. In some embodiments, the non-natural inter-chain bond is a disulfide bond; in some embodiments, the dimer comprises 1, 2, 3, 4, 5, 6, 7, 8, 9 or 10 non-natural inter-chain bonds.

In some embodiments, for the aforementioned domain engineered antibody, the bispecific antibody, or the Fab fragment of a domain engineered antibody, wherein,
the Titin-T chain has one or more amino acid residue mutations on positions selected from the group consisting of 8, 20, 22, 25, 26 and 39, and/or the Obscurin-O chain has one or more amino acid residue mutations on positions selected from the group consisting of 3, 9, 25, 76 and 88; or
the Titin-T chain has one or more amino acid residue mutations on positions selected from the group consisting of 8, 20, 22, 25, 26 and 39, and/or the Obscurin-like-O chain has one or more amino acid residue mutations on positions selected from the group consisting of 6, 26, 74, 77, 84 and 86.

In some embodiments, the Titin-T chain/Obscurin-O chain, or the Titin-T chain/Obscurin-like-O chain, is as follows:
the Titin-T chain is a polypeptide having one or more amino acid residue mutations on positions selected from the group consisting of 8, 20, 22, 25, 26 and 39 on the basis of SEQ ID NO: 32, and/or the Obscurin-O chain is a polypeptide having one or more amino acid residue mutations on positions selected from the group consisting of 3, 9, 25, 76 and 88 on the basis of SEQ ID NO: 33; or
the Titin-T chain is a polypeptide having one or more amino acid residue mutations on positions selected from the group consisting of 8, 20, 22, 25, 26 and 39 on the basis of SEQ ID NO: 32, and/or the Obscurin-like-O chain is a polypeptide having one or more amino acid residue mutations on positions selected from the group consisting of 6, 26, 74, 77, 84 and 86 on the basis of SEQ ID NO: 34.

In some embodiments, for the aforementioned domain engineered antibody, the bispecific antibody, or the Fab fragment of a domain engineered antibody, wherein,
the Titin-T chain has one or more amino acid residue mutations on positions selected from the group consisting of 8C, 20C, 22C, 25S, 26C and 39T, and/or the Obscurin-O chain has one or more amino acid residue mutations on positions selected from the group consisting of 3C, 9C, 25S, 76S and 88C; or
the Titin-T chain has one or more amino acid residue mutations on positions selected from the group consisting of 8C, 20C, 22C, 25S, 26C and 39T, and/or the Obscurin-like-O chain has one or more amino acid residue mutations on positions selected from the group consisting of 6E, 26S, 74C, 77S, 84C and 86C;
more preferably, the non-natural inter-chain bond is located at a mutation position selected from the group consisting of the following:
   the Titin-T chain has 25S, 39T and 8C mutations, and the Obscurin-O chain has a 88C mutation;
   the Titin-T chain has 25S, 39T and 20C mutations, and the Obscurin-O chain has a 3C mutation;
   the Titin-T chain has 25S, 39T and 26C mutations, and the Obscurin-O chain has a 9C mutation;
   the Titin-T chain has 25S, 39T and 8C mutations, and the Obscurin-O chain has 25S, 76S and 88C mutations;
   the Titin-T chain has 25S, 39T and 20C mutations, and the Obscurin-O chain has 25S, 76S and 3C mutations;
   the Titin-T chain has 25S, 39T and 26C mutations, and the Obscurin-O chain has 25S, 76S and 9C mutations;
   the Titin-T chain has 25S, 39T and 8C mutations, and the Obscurin-like-O chain has 6E and 74C mutations;
   the Titin-T chain has 25S, 39T and 20C mutations, and the Obscurin-like-O chain has 6E and 84C mutations;
   the Titin-T chain has 25S, 39T and 22C mutations, and the Obscurin-like-O chain has 6E and 86C mutations;
   the Titin-T chain has 25S, 39T and 8C mutations, and the Obscurin-like-O chain has 6E, 26S, 77S and 74C mutations;
   the Titin-T chain has 25S, 39T and 20C mutations, and the Obscurin-like-O chain has 6E, 26S, 77S and 84C mutations; or
   the Titin-T chain has 25S, 39T and 22C mutations, and the Obscurin-like-O chain has 6E, 26S, 77S and 86C mutations;
   the mutation position in the Titin-T chain is a natural sequence numbering position relative to a sequence SEQ ID NO: 32; the mutation position in the Obscurin-O chain is a natural sequence numbering position relative to a sequence SEQ ID NO: 33; the mutation position in the Obscurin-like-O chain is a natural sequence numbering position relative to a sequence SEQ ID NO: 34.

In some embodiments, for the aforementioned domain engineered antibody, the bispecific antibody, or the Fab fragment of a domain engineered antibody, wherein,
the Titin-T chain has one or more amino acid residue mutations on positions selected from the group consisting of A8C, V20C, T22C, C25S, A26C and C39T, and/or the Obscurin-O chain has one or more amino acid residue mutations on positions selected from the group consisting of A3C, R9C, C25S, C76S and A88C; or
the Titin-T chain has one or more amino acid residue mutations on positions selected from the group consisting of A8C, V20C, T22C, C25S, A26C and C39T, and/or the Obscurin-like-O chain has one or more amino acid residue mutations on positions selected from the group consisting of C6E, C26S, C77S, V74C, G84C and A86C; the mutation position in the Titin-T chain is a natural sequence numbering position relative to a sequence SEQ ID NO: 32; the mutation position in the Obscurin-O chain is a natural sequence numbering position relative to a sequence SEQ ID NO: 33; the mutation position in the Obscurin-like-O chain is a natural sequence numbering position relative to a sequence SEQ ID NO: 34.

In some embodiments, the aforementioned Titin-T chain has one or more amino acid residue mutations on positions selected from the group consisting of A8C, V20C, T22C, C25S, A26C and C39T on the basis of SEQ ID NO: 32, and/or the Obscurin-O chain has one or more amino acid residue mutations on positions selected from the group consisting of A3C, R9C, C25S, C76S and A88C on the basis of SEQ ID NO: 33; or the Titin-T chain has one or more amino acid residue mutations on positions selected from the group consisting of A8C, V20C, T22C, C25S, A26C and C39T on the basis of SEQ ID NO: 32, and/or the Obscurin-like-O chain has one or more amino acid residue mutations on positions selected from the group consisting of C6E, C26S, C77S, V74C, G84C and A86C on the basis of SEQ ID NO: 34. The mutation position in the Titin-T chain is a natural sequence numbering position of the sequence SEQ ID NO: 32; the mutation position in the Obscurin-O chain is a natural sequence numbering position of the sequence SEQ ID NO: 33; the mutation position in the Obscurin-like-O chain is a natural sequence numbering position of the sequence SEQ ID NO: 34.

In some embodiments, for the aforementioned domain engineered antibody, the bispecific antibody, or the Fab fragment of a domain engineered antibody, wherein,
the Titin-T chain has C25S, C39T and A8C mutations, and the Obscurin-O chain has an A88C mutation;
the Titin-T chain has C25S, C39T and V20C mutations, and the Obscurin-O chain has an A3C mutation;
the Titin-T chain has C25S, C39T and A26C mutations, and the Obscurin-O chain has an R9C mutation;
the Titin-T chain has C25S, C39T and A8C mutations, and the Obscurin-O chain has C25S, C76S and A88C mutations;
the Titin-T chain has C25S, C39T and V20C mutations, and the Obscurin-O chain has C25S, C76S and A3C mutations;
the Titin-T chain has C25S, C39T and A26C mutations, and the Obscurin-O chain has C25S, C76S and R9C mutations;
the Titin-T chain has C25S, C39T and A8C mutations, and the Obscurin-like-O chain has C6E and V74C mutations;
the Titin-T chain has C25S, C39T and V20C mutations, and the Obscurin-like-O chain has C6E and G84C mutations;
the Titin-T chain has C25S, C39T and T22C mutations, and the Obscurin-like-O chain has C6E and A86C mutations;
the Titin-T chain has C25S, C39T and A8C mutations, and the Obscurin-like-O chain has C6E, C26S, C77S and V74C mutations;
the Titin-T chain has C25S, C39T and V20C mutations, and the Obscurin-like-O chain has C6E, C26S, C77S and G84C mutations;
the Titin-T chain has C25S, C39T and T22C mutations, and the Obscurin-like-O chain has C6E, C26S, C77S and A86C mutations;
the mutation position in the Titin-T chain is a natural sequence position relative to a sequence SEQ ID NO: 32; the mutation position in the Obscurin-O chain is a natural sequence position relative to a sequence SEQ ID NO: 33; the mutation position in the Obscurin-like-O chain is a natural sequence position relative to a sequence SEQ ID NO: 34.

In some embodiments, for the aforementioned domain engineered antibody, the bispecific antibody, or the Fab fragment of a domain engineered antibody, the Titin-T chain has C25S, C39T and A8C mutations on the basis of SEQ ID NO: 32, and the Obscurin-O chain has an A88C mutation on the basis of SEQ ID NO: 33; the Titin-T chain has C25S, C39T and V20C mutations on the basis of SEQ ID NO: 32, and the Obscurin-O chain has an A3C mutation on the basis of SEQ ID NO: 33; the Titin-T chain has C25S, C39T and A26C mutations on the basis of SEQ ID NO: 32, and the Obscurin-O chain has an R9C mutation on the basis of SEQ ID NO: 33; the Titin-T chain has C25S, C39T and A8C mutations on the basis of SEQ ID NO: 32, and the Obscurin-O chain has C25S, C76S and A88C mutations on the basis of SEQ ID NO: 33; the Titin-T chain has C25S, C39T and V20C mutations on the basis of SEQ ID NO: 32, and the Obscurin-O chain has C25S, C76S and A3C mutations on the basis of SEQ ID NO: 33; the Titin-T chain has C25S, C39T and A26C mutations on the basis of SEQ ID NO: 32, and the Obscurin-O chain has C25S, C76S and R9C mutations on the basis of SEQ ID NO: 33; the Titin-T chain has C25S, C39T and A8C mutations on the basis of SEQ ID NO: 32, and the Obscurin-like-O chain has C6E and V74C mutations on the basis of SEQ ID NO: 34; the Titin-T chain has C25S, C39T and V20C mutations on the basis of SEQ ID NO: 32, and the Obscurin-like-O chain has C6E and G84C mutations on the basis of SEQ ID NO: 34; the Titin-T chain has C25S, C39T and T22C mutations on the basis of SEQ ID NO: 32, and the Obscurin-like-O chain has C6E and A86C mutations on the basis of SEQ ID NO: 34; the Titin-T chain has C25S, C39T and A8C mutations on the basis of SEQ ID NO: 32, and the Obscurin-like-O chain has C6E, C26S, C77S and V74C mutations on the basis of SEQ ID NO: 34; the Titin-T chain has C25S, C39T and V20C mutations on the basis of SEQ ID NO: 32, and the Obscurin-like-O chain has C6E, C26S, C77S and G84C mutations on the basis of SEQ ID NO: 34; the Titin-T chain has C25S, C39T and T22C mutations on the basis of SEQ ID NO: 32, and the Obscurin-like-O chain has C6E, C26S, C77S and A86C mutations on the basis of SEQ ID NO: 34. The mutation position in the Titin-T chain is a natural sequence numbering position relative to a sequence SEQ ID NO: 32; the mutation position in the Obscurin-O chain is a natural sequence numbering position relative to a sequence SEQ ID NO: 33; the mutation position in the Obscurin-like-O chain is a natural sequence numbering position relative to a sequence SEQ ID NO: 34.

In some embodiments, for the aforementioned domain engineered antibody, the bispecific antibody, or the Fab fragment of a domain engineered antibody, the Obscurin-O chain further has one or more amino acid residue mutations on positions selected from the group consisting of 7, 11 and 62. In some embodiments, the Obscurin-O chain has one or more amino acid residue mutations on positions selected from the group consisting of 7R and 7K, 62K and 62H, and 11L; most preferably, the Titin-T chain has 25S, 39T and 8C mutations, and the Obscurin-O chain has 25S, 76S, 88C, 7K and 62K mutations; the Titin-T chain has 25S, 39T and 8C mutations, and the Obscurin-O chain has 25S, 76S, 88C, 7K and 62H mutations; the Titin-T chain has 25S, 39T and 8C mutations, and the Obscurin-O chain has 25S, 76S, 88C, 11L and 62K mutations; or the Titin-T chain has 25S, 39T and 8C mutations, and the Obscurin-O chain has 25S, 76S, 88C, 11L and 62H mutations. In some embodiments, the Obscurin-O chain has one or more amino acid residue mutations on positions selected from the group consisting of L7K and L7R, K11L, T62K and T62H; in some embodiments, the Titin-T chain has C25S, C39T, and A8C mutations, and the Obscurin-O chain has C25S, C76S, A88C, L7K and T62K mutations; the Titin-T chain has C25S, C39T and A8C mutations, and the Obscurin-O chain has C25S, C76S, A88C, L7K and T62H mutations; the Titin-T chain has C25S, C39T and A8C mutations, and the Obscurin-O chain has C25S, C76S, A88C, K11L and T62K mutations; or the Titin-T chain has C25S, C39T and A8C mutations, and the Obscurin-O chain has C25S, C76S, A88C, K11L and T62H mutations. The mutation position in the Titin-T chain is a natural sequence numbering position relative to a sequence SEQ ID NO: 32; the mutation position in the Obscurin-O chain is a natural sequence numbering position relative to a sequence SEQ ID NO: 33.

In some embodiments, for the aforementioned domain engineered antibody, the bispecific antibody, or the Fab fragment of a domain engineered antibody, the Obscurin-O chain is an Obscurin-O chain having one or more amino acid residue mutations on positions selected from the group consisting of 7, 11 and 62 on the basis of SEQ ID NO: 45; in some embodiments, the Obscurin-O chain has one or more amino acid residue mutations on positions selected from the group consisting of L7K and L7R, K11L and T62K and T62H on the basis of SEQ ID NO: 45; in some embodiments, the Titin-T chain has C25S, C39T and A8C mutations on the basis of SEQ ID NO: 32, and the Obscurin-O chain has C25S, C76S, A88C, L7K and T62K mutations on the basis of SEQ ID NO: 45; the Titin-T chain has C25S, C39T and A8C mutations on the basis of SEQ ID NO: 32, and the Obscurin-O chain has C25S, C76S, A88C, L7K and T62H mutations on the basis of SEQ ID NO: 45; the Titin-T chain has C25S, C39T and A8C mutations on the basis of SEQ ID NO: 32, and the Obscurin-O chain has C25S, C76S, A88C, K11L and T62K mutations on the basis of SEQ ID NO: 45; the Titin-T chain has C25S, C39T and A8C mutations on the basis of SEQ ID NO: 32, and the Obscurin-O chain has C25S, C76S, A88C, K11L and T62H mutations on the basis of SEQ ID NO: 45. The mutation position in the Titin-T chain is a natural sequence numbering position relative to a sequence SEQ ID NO: 32; the mutation position in the Obscurin-O chain is a natural sequence numbering position relative to a sequence SEQ ID NO: 33.

In some embodiments, for the aforementioned domain engineered antibody, the bispecific antibody, or the Fab fragment of a domain engineered antibody, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84, and/or the Obscurin-O chain has one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97. In some embodiments, the Titin-T chain has one or more amino acid mutations on positions selected from the group consisting of 3W, 11I, 13L, 22M, 40S, 42K, 45S, 47E, 49G, 56S, 58E, 66S and 66K, 70R, 75V, 77S, 79T, 81R, 82M, 83D and 84L, and/or the Obscurin-O chain has one or more amino acid mutations on positions selected from the group consisting of 2E, 11K, 12S, 13Y, 14T, 17E, 20L, 22M and 22S, 30D, 32P, 34E, 36T, 41K, 42L, 441, 45T, 53L, 58V, 62E, 67Q and 67T, 69S, 89L, 92E, 94G and 97G. More preferably, the Titin-T chain has 66S and 77S amino acid mutations, and/or the Obscurin-O chain has 11K, 12S, 13Y, 14T and 22S amino acid mutations; the Titin-T chain has 66K, 70R, 79T and 81R amino acid mutations, and/or the Obscurin-O chain has 2E, 17E, 30D, 32P, 34E, 36T, 441, 45T, 58V, 62E, 67Q, 69S and 97G amino acid mutations; the Titin-T chain has 3W, 11I, 13L, 22M and 82M amino acid mutations, and/or the Obscurin-O chain has 20L, 22M and 53L amino acid mutations; the Titin-T chain has 11I, 66K, 79T and 81R amino acid mutations, and/or the Obscurin-O chain has 41K, 45T, 67Q, 69S and 89L amino acid mutations; the Titin-T chain has 40S, 42K, 45S, 47E, 49G, 56S, 58E, 75V, 83D, and 84L amino acid mutations, and/or the Obscurin-O chain has 42L, 45T, 67T, 69S, 92E, and 94G amino acid mutations; the Titin-T chain has 47E, 49G, 56S, 58E and 75V amino acid mutations, and/or the Obscurin-O chain has 42L, 45T, 67T, 69S, 92E and 94G amino acid mutations; the Titin-T chain has 56S, 58E and 75V amino acid mutations, and/or the Obscurin-O chain has 42L, 45T, 67T, 69S, 92E and 94G amino acid mutations; or the Titin-T chain has 56S, 58E, 66S and 77S amino acid mutations, and/or the Obscurin-O chain has 12S, 13Y, 22S, 42L, 45T, 67Q, 69S, 92E and 94G amino acid mutations. the mutation position in the Titin-T chain is a natural sequence numbering position relative to a sequence SEQ ID NO: 35; the mutation position in the Obscurin-O chain is a natural sequence numbering position relative to a sequence SEQ ID NO: 50. In some embodiments, the Titin-T chain has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L, and/or the Obscurin-O chain has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G. In some embodiments, the Titin-T chain has M66S and T77S amino acid mutations, and/or the Obscurin-O chain has L11K, A12S, F13Y, V14T and T22S amino acid mutations; the Titin-T chain has M66K, K70R, S79T and G81R amino acid mutations, and/or the Obscurin-O chain has G2E, V17E, N30D, T32P, Q34E, S36T, V44I, A45T, L58V, K62E, A67Q, G69S and A97G amino acid mutations; the Titin-T chain has P3W, S11I, I13L, T22M and N82M amino acid mutations, and/or the Obscurin-O chain has D20L, T22M and A53L amino acid mutations; the Titin-T chain has S11I, M66K, S79T and G81R amino acid mutations, and/or the Obscurin-O chain has Q41K, A45T, A67Q, G69S and V89L amino acid mutations; the Titin-T chain has G40S, R42K, H45S, Q47E, Q49G, N56S, D58E, L75V, E83D and F84L amino acid mutations, and/or the Obscurin-O chain has Q42L, A45T, A67T, G69S, Q92E and D94G amino acid mutations; the Titin-T chain has Q47E, Q49G, N56S, D58E and L75V amino acid mutations, and/or the Obscurin-O chain has Q42L, A45T, A67T, G69S, Q92E and D94G amino acid mutations; the Titin-T chain has N56S, D58E and L75V amino acid mutations, and/or the Obscurin-O chain has Q42L, A45T, A67T, G69S, Q92E and D94G amino acid mutations; or the Titin-T chain has N56S, D58E, M66S and T77S amino acid mutations, and/or the Obscurin-O chain has A12S, F13Y, T22S, Q42L, A45T, A67Q, G69S, Q92E and D94G amino acid mutations. the mutation position in the Titin-T chain is a natural sequence numbering position relative to a sequence SEQ ID NO: 35; the mutation position in the Obscurin-O chain is a natural sequence numbering position relative to a sequence SEQ ID NO: 50.

In some embodiments, for the aforementioned domain engineered antibody, the bispecific antibody, or the Fab fragment of a domain engineered antibody, the Titin-T chain is a polypeptide having one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and the Obscurin-O chain is a polypeptide having one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97 on the basis of SEQ ID NO: 50. The mutation position in the Titin-T chain is a natural sequence numbering position of a sequence SEQ ID NO: 35; the mutation position in the Obscurin-O chain is a natural sequence numbering position of a sequence SEQ ID NO: 50. In some embodiments, the Titin-T chain has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L on the basis of SEQ ID NO: 35, and/or the Obscurin-O chain has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G on the basis of SEQ ID NO: 35. In some embodiments, the Titin-T chain has M66S and T77S amino acid mutations on the basis of SEQ ID NO: 35, and/or the Obscurin-O chain has L11K, A12S, F13Y, V14T and T22S amino acid mutations on the basis of SEQ ID NO: 50; the Titin-T chain has M66K, K70R, S79T and G81R amino acid mutations on the basis of SEQ ID NO: 35, and/or the Obscurin-O chain has G2E, V17E, N30D, T32P, Q34E, S36T, V44I, A45T, L58V, K62E, A67Q, G69S and A97G amino acid mutations on the basis of SEQ ID NO: 50; the Titin-T chain has P3W, S11I, I13L, T22M and N82M amino acid mutations on the basis of SEQ ID NO: 35, and/or the Obscurin-O chain has D20L, T22M and A53L amino acid mutations on the basis of SEQ ID NO: 50; the Titin-T chain has S11I, M66K, S79T and G81R amino acid mutations on the basis of SEQ ID NO: 35, and/or the Obscurin-O chain has Q41K, A45T, A67Q, G69S and V89L amino acid mutations on the basis of SEQ ID NO: 50; the Titin-T chain has G40S, R42K, H45S, Q47E, Q49G, N56S, D58E, L75V, E83D and F84L amino acid mutations on the basis of SEQ ID NO: 35, and/or the Obscurin-O chain has Q42L, A45T, A67T, G69S, Q92E and D94G amino acid mutations on the basis of SEQ ID NO: 50; the Titin-T chain has Q47E, Q49G, N56S, D58E and L75V amino acid mutations on the basis of SEQ ID NO: 35, and/or the Obscurin-O chain has Q42L, A45T, A67T, G69S, Q92E and D94G amino acid mutations on the basis of SEQ ID NO: 50; the Titin-T chain has N56S, D58E and L75V amino acid mutations on the basis of SEQ ID NO: 35, and/or the Obscurin-O chain has Q42L, A45T, A67T, G69S, Q92E and D94G amino acid mutations on the basis of SEQ ID NO: 50; or the Titin-T chain has N56S, D58E, M66S and T77S amino acid mutations on the basis of SEQ ID NO: 35, and/or the Obscurin-O chain has A12S, F13Y, T22S, Q42L, A45T, A67Q, G69S, Q92E and D94G amino acid mutations on the basis of SEQ ID NO: 50; the mutation position in the Titin-T chain is a natural sequence numbering position of a sequence SEQ ID NO: 35; the mutation position in the Obscurin-O chain is a natural sequence numbering position of a sequence SEQ ID NO: 50.

In some embodiments, for the aforementioned domain engineered antibody, the bispecific antibody or the Fab fragment of a domain engineered antibody, N-termini of the Titin-T chain, the Obscurin-O chain or the Obscurin-like-O chain are operably linked to the VH or the VL through a linking domain. In some embodiments, the linking domain is selected from the group consisting of: an N-terminal fragment of the Titin-T chain, an N-terminal fragment of the Obscurin-O chain, an N-terminal fragment of the Obscurin-like-O chain, and a (GₓS)_{y} linker, wherein X is selected from the group consisting of integers from 1 to 5, and Y is selected from the group consisting of integers from 1 to 6. In some embodiments, the linking domain is selected from the group consisting of: "KAGIR", "DQPQF", (G₄S)₁ and (G₄S)₂.

In some embodiments, the Titin-T chain is linked to the VH or the VL through a KAGIR polypeptide, and/or the Obscurin-O chain is linked to the VL or the VH through a DQPQF polypeptide.

In some embodiments, for the aforementioned polypeptide complex, wherein,
(A) the Titin-T chain has a sequence set forth in SEQ ID NO: 32 or further has a sequence having one or more amino acid mutations on positions selected from the group consisting of 8, 20, 22, 25, 26, 39, 3, 11, 13, 40, 42, 45, 47, 49, 56, 58, 66, 70, 75, 77, 79, 81, 82, 83 and 84 and/or a KAGIR amino acid residue added at the N-terminus on the basis of SEQ ID NO: 32; preferably, the Titin-T chain has a sequence having one or more amino acid mutations on positions selected from the group consisting of A8C, V20C, T22C, C25S, A26C, C39T, P3W, S11I, I13L, T22M, G40S, R42K, H45S, Q47E, Q49G, N56S, D58E, M66S and M66K, K70R, L75V, T77S, S79T, G81R, N82M, E83D and F84L and/or a KAGIR amino acid residue added at the N-terminus; and/or
(B) the Obscurin-O chain has a sequence set forth in SEQ ID NO: 33 or further has a sequence having one or more amino acid mutations on positions selected from the group consisting of 3, 9, 25, 76, 88, 7, 11, 62, 2, 12, 13, 14, 17, 20, 22, 30, 32, 34, 36, 41, 42, 44, 45, 53, 58, 67, 69, 89, 92, 94 and 97 and/or a DQPQF amino acid residue added at the N-terminus on the basis of SEQ ID NO: 33; preferably, the Obscurin-O chain has a sequence having one or more amino acid mutations on positions selected from the group consisting of A3C, R9C, C25S, C76S, A88C, L7K and L7R, T62K and T62H, G2E, L11K, A12S, F13Y, V14T, V17E, D20L, T22M and T22S, N30D, T32P, Q34E, S36T, Q41K, Q42L, V44I, A45T, A53L, L58V, T62E, A67Q and A67T, G69S, V89L, Q92E, D94G and A97G and/or a DQPQF amino acid residue added at the N-terminus; or
   the Obscurin-like-O chain has a sequence set forth in SEQ ID NO: 34 or further has a sequence having one or more amino acid mutations on positions selected from the group consisting of 6, 26, 74, 77, 84 and 86 on the basis of SEQ ID NO: 34; preferably, the Obscurin-like-O chain has a sequence having one or more amino acid mutations on positions selected from the group consisting of C6E, C26S, C77S, V74C, G84C and A86C;
   the mutation position in the Titin-T chain is a natural sequence numbering position of the sequence SEQ ID NO: 32; the mutation position in the Obscurin-O chain is a natural sequence numbering position of the sequence SEQ ID NO: 33; the mutation position in the Obscurin-like-O chain is a natural sequence numbering position of the sequence SEQ ID NO: 34;
   preferably, wherein,
      (A) the Titin-T chain has one or more amino acid residue mutations on positions selected from the group consisting of 8, 20, 22, 25, 26 and 39 on the basis of SEQ ID NO: 32, and preferably has one or more amino acid residue mutations on positions selected from the group consisting of A8C, V20C, T22C, C25S, A26C and C39T, and the mutation position in the Titin-T chain is a natural sequence numbering position of SEQ ID NO: 32; more preferably, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and preferably has one or more amino acid mutations on positions selected from the group consisting ofP3W, S11I, I13L, T22M, G40S, R42K, H45S, Q47E, Q49G, N56S, D58E, M66S, M66K, K70R, L75V, T77S, S79T, G81R, N82M, E83D and F84L; the mutation position in the Titin-T chain is a natural sequence numbering position of SEQ ID NO: 35, and/or
      (B) the Obscurin-O chain has one or more amino acid residue mutations on positions selected from the group consisting of 3, 9, 25, 76 and 88 on the basis of SEQ ID NO: 33, and preferably has one or more amino acid residue mutations on positions selected from the group consisting of A3C, R9C, C25S, C76S and A88C, and the mutation position in the Obscurin-O chain is a natural sequence numbering position of SEQ ID NO: 33; preferably, the Obscurin-O chain has one or more amino acid residue mutations on positions selected from the group consisting of 7, 11 and 62 on the basis of SEQ ID NO: 45, and preferably has one or more amino acid residue mutations on positions selected from the group consisting of L7K and L7R, T62K and T62H, and K11L, and the mutation position in the Obscurin-O chain is a natural sequence numbering position of SEQ ID NO: 45; more preferably, the Obscurin-O chain has one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97 on the basis of SEQ ID NO: 50, and preferably has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G, and the mutation position in the Obscurin-O chain is a natural sequence numbering position of SEQ ID NO: 50; or
   the Obscurin-like-O chain has one or more amino acid residue mutations on positions selected from the group consisting of 6, 26, 74, 77, 84 and 86 on the basis of SEQ ID NO: 34, and preferably has one or more amino acid residue mutations on positions selected from the group consisting of C6E, C26S, C77S, V74C, G84C and A86C, and the mutation position in the Obscurin-like-O chain is a natural sequence numbering position of SEQ ID NO: 34.

In some embodiments, for the aforementioned domain engineered antibody, the bispecific antibody, or the Fab fragment of a domain engineered antibody, the Titin-T chain comprises a polypeptide having a sequence set forth in any one of SEQ ID NOs: 32, 35-41, 65-76 or a polypeptide having a sequence having at least 80% sequence identity to any one of SEQ ID NOs: 32, 35-41, 65-76, and/or the Obscurin-O chain comprises a polypeptide having a sequence set forth in any one of SEQ ID NOs: 33, 42-58, 77-86 or a polypeptide having a sequence having at least 80% sequence identity to any one of SEQ ID NOs: 33, 42-58, 77-86; or the Titin-T chain comprises a polypeptide having a sequence set forth in any one of SEQ ID NOs: 32, 35-41, 65-76 or a polypeptide having a sequence having at least 80% sequence identity to any one of SEQ ID NOs: 32, 35-41, 65-76, and/or the Obscurin-like-O chain comprises a polypeptide having a sequence set forth in any one of SEQ ID NOs: 34, 59-64 or a polypeptide having a sequence having at least 80% sequence identity to any one of SEQ ID NOs: 34, 59-64. Wherein, the at least 80% sequence identity refers to, for example, at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity.

In some embodiments, the Titin-T chain comprises a polypeptide having a sequence set forth in SEQ ID NO: 68, and the Obscurin-O chain comprises a polypeptide having a sequence set forth in SEQ ID NO: 80; the Titin-T chain comprises a polypeptide having a sequence set forth in SEQ ID NO: 73, and the Obscurin-O chain comprises a polypeptide having a sequence set forth in SEQ ID NO: 83; the Titin-T chain comprises a polypeptide having a sequence set forth in SEQ ID NO: 76, and the Obscurin-O chain comprises a polypeptide having a sequence set forth in SEQ ID NO: 84; the Titin-T chain comprises a polypeptide having a sequence set forth in SEQ ID NO: 76, and the Obscurin-O chain comprises a polypeptide having a sequence set forth in SEQ ID NO: 86; the Titin-T chain comprises a polypeptide having a sequence set forth in SEQ ID NO: 76, and the Obscurin-O chain comprises a polypeptide having a sequence set forth in SEQ ID NO: 86; or the Titin-T chain comprises a polypeptide having a sequence set forth in SEQ ID NO: 75, and the Obscurin-O chain comprises a polypeptide having a sequence set forth in SEQ ID NO: 86.

In some embodiments, the aforementioned bispecific antibody is a bispecific antibody selected from the group consisting of any one of the following A-G:
A. the first heavy chain of the bispecific antibody has a sequence set forth in SEQ ID NO: 89 or a sequence having at least 85% sequence identity to SEQ ID NO: 89, the first light chain has a sequence set forth in SEQ ID NO: 90 or a sequence having at least 85% sequence identity to SEQ ID NO: 90, the second heavy chain has a sequence set forth in SEQ ID NO: 87 or a sequence having at least 85% sequence identity to SEQ ID NO: 87, and the second light chain has a sequence set forth in SEQ ID NO: 88 or a sequence having at least 85% sequence identity to SEQ ID NO: 88;
B. the first heavy chain of the bispecific antibody has a sequence set forth in SEQ ID NO: 93 or a sequence having at least 85% sequence identity to SEQ ID NO: 93, the first light chain has a sequence set forth in SEQ ID NO: 94 or a sequence having at least 85% sequence identity to SEQ ID NO: 94, the second heavy chain has a sequence set forth in SEQ ID NO: 91 or a sequence having at least 85% sequence identity to SEQ ID NO: 91, and the second light chain has a sequence set forth in SEQ ID NO: 92 or a sequence having at least 85% sequence identity to SEQ ID NO: 92;
C. the first heavy chain of the bispecific antibody has a sequence set forth in SEQ ID NO: 97 or a sequence having at least 85% sequence identity to SEQ ID NO: 97, the first light chain has a sequence set forth in SEQ ID NO: 98 or a sequence having at least 85% sequence identity to SEQ ID NO: 98, the second heavy chain has a sequence set forth in SEQ ID NO: 95 or a sequence having at least 85% sequence identity to SEQ ID NO: 95, and the second light chain has a sequence set forth in SEQ ID NO: 96 or a sequence having at least 85% sequence identity to SEQ ID NO: 96;
D. the first heavy chain of the bispecific antibody has a sequence set forth in SEQ ID NO: 99 or a sequence having at least 85% sequence identity to SEQ ID NO: 99, the first light chain has a sequence set forth in SEQ ID NO: 100 or a sequence having at least 85% sequence identity to SEQ ID NO: 100, the second heavy chain has a sequence set forth in SEQ ID NO: 101 or a sequence having at least 85% sequence identity to SEQ ID NO: 101, and the second light chain has a sequence set forth in SEQ ID NO: 96 or a sequence having at least 85% sequence identity to SEQ ID NO: 96;
E. the first heavy chain of the bispecific antibody has a sequence set forth in SEQ ID NO: 102 or a sequence having at least 85% sequence identity to SEQ ID NO: 102, the first light chain has a sequence set forth in SEQ ID NO: 100 or a sequence having at least 85% sequence identity to SEQ ID NO: 100, the second heavy chain has a sequence set forth in SEQ ID NO: 95 or a sequence having at least 85% sequence identity to SEQ ID NO: 95, and the second light chain has a sequence set forth in SEQ ID NO: 96 or a sequence having at least 85% sequence identity to SEQ ID NO: 96;
F. the first heavy chain of the bispecific antibody has a sequence set forth in SEQ ID NO: 103 or a sequence having at least 85% sequence identity to SEQ ID NO: 103, the first light chain has a sequence set forth in SEQ ID NO: 104 or a sequence having at least 85% sequence identity to SEQ ID NO: 104, the second heavy chain has a sequence set forth in SEQ ID NO: 95 or a sequence having at least 85% sequence identity to SEQ ID NO: 95, and the second light chain has a sequence set forth in SEQ ID NO: 96 or a sequence having at least 85% sequence identity to SEQ ID NO: 96;
G. the first heavy chain of the bispecific antibody has a sequence set forth in SEQ ID NO: 107 or a sequence having at least 85% sequence identity to SEQ ID NO: 107, the first light chain has a sequence set forth in SEQ ID NO: 108 or a sequence having at least 85% sequence identity to SEQ ID NO: 108, the second heavy chain has a sequence set forth in SEQ ID NO: 109 or a sequence having at least 85% sequence identity to SEQ ID NO: 109, and the second light chain has a sequence set forth in SEQ ID NO: 110 or a sequence having at least 85% sequence identity to SEQ ID NO: 110;
H. the first heavy chain of the bispecific antibody has a sequence set forth in SEQ ID NO: 122 or a sequence having at least 85% sequence identity to SEQ ID NO: 122, the first light chain has a sequence set forth in SEQ ID NO: 123 or a sequence having at least 85% sequence identity to SEQ ID NO: 123, the second heavy chain has a sequence set forth in SEQ ID NO: 116 or a sequence having at least 85% sequence identity to SEQ ID NO: 116, and the second light chain has a sequence set forth in SEQ ID NO: 117 or a sequence having at least 85% sequence identity to SEQ ID NO: 117;
I. the first heavy chain of the bispecific antibody has a sequence set forth in SEQ ID NO: 118 or a sequence having at least 85% sequence identity to SEQ ID NO: 118, the first light chain has a sequence set forth in SEQ ID NO: 119 or a sequence having at least 85% sequence identity to SEQ ID NO: 119, the second heavy chain has a sequence set forth in SEQ ID NO: 124 or a sequence having at least 85% sequence identity to SEQ ID NO: 124, and the second light chain has a sequence set forth in SEQ ID NO: 125 or a sequence having at least 85% sequence identity to SEQ ID NO: 125;
wherein, the at least 85% sequence identity refers to, for example, at least 85%, 86%, 87%, 88%, 89%, 90%, 91%, 92%, 93%, 94%, 95%, 96%, 97%, 98%, 99% or 100% sequence identity.

In some embodiments, the present disclosure provides a method for preparing a multispecific antibody, which comprises substituting a CH1/CL of an antibody with a Titin-T chain and an Obscurin-O chain, or a Titin-T chain and an Obscurin-like-O chain. In some embodiments of the method for preparing a multispecific antibody, the multispecific antibody of the present disclosure has reduced mispairing between light chains and heavy chains as compared to a wild-type in which a CH1/CL is not substituted with a Titin-T chain/Obscurin-O chain, or a Titin-T chain/Obscurin-like-O chain.

In some embodiments, the present disclosure further provides a multispecific antibody obtained by the aforementioned method for preparing a multispecific antibody. In some embodiments, the multispecific antibody comprises a heavy chain variable region (VH1) and a light chain variable region (VL1), the VH1 and the VL1 forming an antigen binding site specifically binding to a first antigen; a C-terminus of the VH1 is operably linked to an N-terminus of the Titin-T chain, a C-terminus of the VL1 is operably linked to an N-terminus of the Obscurin-O chain or the Obscurin-like-O chain, or the C-terminus of the VL1 is operably linked to the N-terminus of the Titin-T chain, and the C-terminus of the VH1 is operably linked to the N-terminus of the Obscurin-O chain or the Obscurin-like-O chain.

In some embodiments, for the method for preparing a multispecific antibody, the Titin-T chain comprises a polypeptide having a sequence set forth in any one of SEQ ID NOs: 32, 35-41, 65-76 or a polypeptide having a sequence having at least 80% sequence identity to any one of SEQ ID NOs: 32, 35-41, 65-76, and/or the Obscurin-O chain comprises a polypeptide having a sequence set forth in any one of SEQ ID NOs: 33, 42-58, 77-86 or a polypeptide having a sequence having at least 80% sequence identity to any one of SEQ ID NOs: 33, 42-58, 77-86; or the Titin-T chain comprises a polypeptide having a sequence set forth in any one of SEQ ID NOs: 32, 35-41, 65-76 or a polypeptide having a sequence having at least 80% sequence identity to any one of SEQ ID NOs: 32, 35-41, 65-76, and/or the Obscurin-like-O chain comprises a polypeptide having a sequence set forth in any one of SEQ ID NOs: 34, 59-64 or a polypeptide having a sequence having at least 80% sequence identity to any one of SEQ ID NOs: 34, 59-64.

In some embodiments, the Titin-T chain comprises a polypeptide having a sequence set forth in SEQ ID NO: 68, and the Obscurin-O chain comprises a polypeptide having a sequence set forth in SEQ ID NO: 80; in some embodiments, the Titin-T chain comprises a polypeptide having a sequence set forth in SEQ ID NO: 73, and the Obscurin-O chain comprises a polypeptide having a sequence set forth in SEQ ID NO: 83; in some embodiments, the Titin-T chain comprises a polypeptide having a sequence set forth in SEQ ID NO: 76, and the Obscurin-O chain comprises a polypeptide having a sequence set forth in SEQ ID NO: 84; in some embodiments, the Titin-T chain comprises a polypeptide having a sequence set forth in SEQ ID NO: 76, and the Obscurin-O chain comprises a polypeptide having a sequence set forth in SEQ ID NO: 86; in some embodiments, the Titin-T chain comprises a polypeptide having a sequence set forth in SEQ ID NO: 75, and the Obscurin-O chain comprises a polypeptide having a sequence set forth in SEQ ID NO: 86.

In some embodiments, wherein,
(A) the Titin-T chain has a sequence set forth in SEQ ID NO: 32 or further has a sequence having one or more amino acid mutations on positions selected from the group consisting of 8, 20, 22, 25, 26, 39, 3, 11, 13, 40, 42, 45, 47, 49, 56, 58, 66, 70, 75, 77, 79, 81, 82, 83 and 84 and/or 5 amino acid residues of KAGIR added at the N-terminus on the basis of SEQ ID NO: 32; preferably, the Titin-T chain has a sequence having one or more amino acid mutations on positions selected from the group consisting of A8C, V20C, T22C, C25S, A26C, C39T, P3W, S11I, I13L, T22M, G40S, R42K, H45S, Q47E, Q49G, N56S, D58E, M66S and M66K, K70R, L75V, T77S, S79T, G81R, N82M, E83D and F84L and/or 5 amino acid residues of KAGIR added at the N-terminus; and/or
(B) the Obscurin-O chain has a sequence set forth in SEQ ID NO: 33 or further has a sequence having one or more amino acid mutations on positions selected from the group consisting of 3, 9, 25, 76, 88, 7, 11, 62, 2, 12, 13, 14, 17, 20, 22, 30, 32, 34, 36, 41, 42, 44, 45, 53, 58, 67, 69, 89, 92, 94 and 97 and/or a DQPQF amino acid residue added at the N-terminus on the basis of SEQ ID NO: 33; preferably, the Obscurin-O chain has a sequence having one or more amino acid mutations on positions selected from the group consisting of A3C, R9C, C25S, C76S, A88C, L7K and L7R, T62K and T62H, G2E, L11K, A12S, F13Y, V14T, V17E, D20L, T22M and T22S, N30D, T32P, Q34E, S36T, Q41K, Q42L, V44I, A45T, A53L, L58V, T62E, A67Q and A67T, G69S, V89L, Q92E, D94G and A97G and/or a DQPQF amino acid residue added at the N-terminus; or
   the Obscurin-like-O chain has a sequence set forth in SEQ ID NO: 34 or further has a sequence having one or more amino acid mutations on positions selected from the group consisting of 6, 26, 74, 77, 84 and 86 on the basis of SEQ ID NO: 34; preferably, the Obscurin-like-O chain has a sequence having one or more amino acid mutations on positions selected from the group consisting of C6E, C26S, C77S, V74C, G84C and A86C;
   the mutation position in the Titin-T chain is a natural sequence numbering position of the sequence SEQ ID NO: 32; the mutation position in the Obscurin-O chain is a natural sequence numbering position of the sequence SEQ ID NO: 33; the mutation position in the Obscurin-like-O chain is a natural sequence numbering position of the sequence SEQ ID NO: 34;
   preferably, wherein,
      (A) the Titin-T chain has one or more amino acid residue mutations on positions selected from the group consisting of 8, 20, 22, 25, 26 and 39 on the basis of SEQ ID NO: 32, and preferably has one or more amino acid residue mutations on positions selected from the group consisting of A8C, V20C, T22C, C25S, A26C and C39T, and the mutation position in the Titin-T chain is a natural sequence numbering position of SEQ ID NO: 32; more preferably, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and preferably has one or more amino acid mutations on positions selected from the group consisting ofP3W, S11I, I13L, T22M, G40S, R42K, H45S, Q47E, Q49G, N56S, D58E, M66S, M66K, K70R, L75V, T77S, S79T, G81R, N82M, E83D and F84L; the mutation position in the Titin-T chain is a natural sequence numbering position of SEQ ID NO: 35, and/or
      (B) the Obscurin-O chain has one or more amino acid residue mutations on positions selected from the group consisting of 3, 9, 25, 76 and 88 on the basis of SEQ ID NO: 33, and preferably has one or more amino acid residue mutations on positions selected from the group consisting of A3C, R9C, C25S, C76S and A88C, and the mutation position in the Obscurin-O chain is a natural sequence numbering position of SEQ ID NO: 33; preferably, the Obscurin-O chain has one or more amino acid residue mutations on positions selected from the group consisting of 7, 11 and 62 on the basis of SEQ ID NO: 45, and preferably has one or more amino acid residue mutations on positions selected from the group consisting of L7K and L7R, T62K and T62H, and K11L, and the mutation position in the Obscurin-O chain is a natural sequence numbering position of SEQ ID NO: 45; more preferably, the Obscurin-O chain has one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97 on the basis of SEQ ID NO: 50, and preferably has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G, and the mutation position in the Obscurin-O chain is a natural sequence numbering position of SEQ ID NO: 50; or
   the Obscurin-like-O chain has one or more amino acid residue mutations on positions selected from the group consisting of 6, 26, 74, 77, 84 and 86 on the basis of SEQ ID NO: 34, and preferably has one or more amino acid residue mutations on positions selected from the group consisting of C6E, C26S, C77S, V74C, G84C and A86C, and the mutation position in the Obscurin-like-O chain is a natural sequence numbering position of SEQ ID NO: 34.

In some embodiments, the present disclosure provides a conjugate comprising any one of the aforementioned polypeptide complex, the multispecific polypeptide complex, the domain engineered antibody, the Fab fragment of a domain engineered antibody or the bispecific antibody, or the multispecific antibody.

In some embodiments, the present disclosure provides a polypeptide having a sequence set forth in any one of SEQ ID NOs: 32, 35-41, 65-76 or a sequence having at least 80% sequence identity to any one of SEQ ID NOs: 32, 35-41, 65-76, or having a sequence set forth in any one of SEQ ID NOs: 33, 42-58, 77-86 or a sequence having at least 80% sequence identity to any one of SEQ ID NOs: 33, 42-58, 77-86, or having a sequence set forth in any one of SEQ ID NOs: 34, 59-64 or a sequence having at least 80% sequence identity to any one of SEQ ID NOs: 34, 59-64; preferably, the polypeptide can be used to substitute a CH1 and/or a CL of an antibody. More preferably, the polypeptide is a Titin-T chain and an Obscurin-O chain, or a Titin-T chain and an Obscurin-like-O chain, and the single-sided CH1 and CL of the bispecific antibody are substituted with the Titin-T chain and the Obscurin-O chain, or the Titin-T chain and the Obscurin-like-O chain. After the substitution, the mispairing between the first heavy chain and the second light chain, or between the first light chain and the second heavy chain, is less susceptible to happen.

In some embodiments, the Titin-T chain has a sequence set forth in SEQ ID NO: 32 or further has a sequence having one or more amino acid mutations on positions selected from the group consisting of 8, 20, 22, 25, 26, 39, 3, 11, 13, 40, 42, 45, 47, 49, 56, 58, 66, 70, 75, 77, 79, 81, 82, 83 and 84 on the basis of SEQ ID NO: 32; preferably, the Titin-T chain has a sequence having one or more amino acid mutations on positions selected from the group consisting of A8C, V20C, T22C, C25S, A26C, C39T, P3W, S11I, I13L, T22M, G40S, R42K, H45S, Q47E, Q49G, N56S, D58E, M66S and M66K, K70R, L75V, T77S, S79T, G81R, N82M, E83D and F84L and/or 5 amino acid residues of KAGIR added at the N-terminus; the Obscurin-O chain has a sequence set forth in SEQ ID NO: 33 or further has a sequence having one or more amino acid mutations on positions selected from the group consisting of 3, 9, 25, 76, 88, 7, 11, 62, 2, 12, 13, 14, 17, 20, 22, 30, 32, 34, 36, 41, 42, 44, 45, 53, 58, 67, 69, 89, 92, 94 and 97 and/or a DQPQF amino acid residue added at the N-terminus on the basis of SEQ ID NO: 33; preferably, the Obscurin-O chain has a sequence having one or more amino acid mutations on positions selected from the group consisting of A3C, R9C, C25S, C76S, A88C, L7K and L7R, T62K and T62H, G2E, L11K, A12S, F13Y, V14T, V17E, D20L, T22M and T22S, N30D, T32P, Q34E, S36T, Q41K, Q42L, V44I, A45T, A53L, L58V, T62E, A67Q and A67T, G69S, V89L, Q92E, D94G and A97G and/or a DQPQF amino acid residue added at the N-terminus; the Obscurin-like-O chain has a sequence set forth in SEQ ID NO: 34 or further has a sequence having one or more amino acid mutations on positions selected from the group consisting of 6, 26, 74, 77, 84 and 86 on the basis of SEQ ID NO:34; preferably, the Obscurin-like-O chain has a sequence having one or more amino acid mutations on positions selected from the group consisting of C6E, C26S, C77S, V74C, G84C and A86C.

Disclosed is a polypeptide for use in substituting a CH1 and/or a CL of an antibody, a CH1 and/or a CL of an antigen-binding fragment, or a CH1 and/or a CL of a polypeptide complex, wherein the polypeptide has a sequence set forth in any one of SEQ ID NOs: 32, 35-41, 65-76 or a sequence having at least 80% sequence identity to any one of SEQ ID NOs: 32, 35-41, 65-76; or the polypeptide has a sequence set forth in any one of SEQ ID NOs: 33, 42-58, 77-86 or a sequence having at least 80% sequence identity to any one of SEQ ID NOs: 33, 42-58, 77-86; or the polypeptide has a sequence set forth in any one of SEQ ID NOs: 34, 59-64 or a sequence having at least 80% sequence identity to any one of SEQ ID NOs: 34, 59-64; preferably, the polypeptide is a Titin-T chain and an Obscurin-O chain, or a Titin-T chain and an Obscurin-like-O chain. More preferably, after the single-sided CH1 and CL of the bispecific antibody are substituted with the polypeptide, the mispairing between the first heavy chain and the second light chain, or between the first light chain and the second heavy chain, is less susceptible to happen.

In some embodiments, the present disclosure provides a Titin-T chain, an Obscurin-O chain, and/or an Obscurin-like-O chain, wherein the Titin-T chain has a sequence set forth in any one of SEQ ID NOs: 32, 35-41, 65-76 or a sequence having at least 80% sequence identity to any one of SEQ ID NOs: 32, 35-41, 65-76, and the Obscurin-O chain has a sequence set forth in any one of SEQ ID NOs: 33, 42-58, 77-86 or a sequence having at least 80% sequence identity to any one of SEQ ID NOs: 33, 42-58, 77-86; the Obscurin-like-O chain has a sequence set forth in any one of SEQ ID NOs: 34, 59-64 or a sequence having at least 80% sequence identity to any one of SEQ ID NOs: 34, 59-64. In some embodiments, the Titin-T chain/Obscurin-O chain, or Titin-T chain/Obscurin-like-O chain, may be used to substitute a CH1 and/or a CL of an antibody.

In some embodiments, the Titin-T chain has a sequence set forth in SEQ ID NO: 32 or further has a sequence having one or more amino acid mutations on positions selected from the group consisting of 8, 20, 22, 25, 26, 39, 3, 11, 13, 40, 42, 45, 47, 49, 56, 58, 66, 70, 75, 77, 79, 81, 82, 83 and 84 on the basis of SEQ ID NO: 32; preferably, the Titin-T chain has a sequence having one or more amino acid mutations on positions selected from the group consisting of A8C, V20C, T22C, C25S, A26C, C39T, P3W, S11I, I13L, T22M, G40S, R42K, H45S, Q47E, Q49G, N56S, D58E, M66S and M66K, K70R, L75V, T77S, S79T, G81R, N82M, E83D and F84L; the Obscurin-O chain has a sequence set forth in SEQ ID NO: 33 or further has a sequence having one or more amino acid mutations on positions selected from the group consisting of 3, 9, 25, 76, 88, 7, 11, 62, 2, 12, 13, 14, 17, 20, 22, 30, 32, 34, 36, 41, 42, 44, 45, 53, 58, 67, 69, 89, 92, 94 and 97 on the basis of SEQ ID NO: 33; preferably, the Obscurin-O chain has a sequence having one or more amino acid mutations on positions selected from the group consisting of A3C, R9C, C25S, C76S, A88C, L7K and L7R, T62K and T62H, G2E, L11K, A12S, F13Y, V14T, V17E, D20L, T22M and T22S, N30D, T32P, Q34E, S36T, Q41K, Q42L, V44I, A45T, A53L, L58V, T62E, A67Q and A67T, G69S, V89L, Q92E, D94G and A97G; the Obscurin-like-O chain has a sequence set forth in SEQ ID NO: 34 or further has a sequence having one or more amino acid mutations on positions selected from the group consisting of 6, 26, 74, 77, 84 and 86 on the basis of SEQ ID NO:34; preferably, the Obscurin-like-O chain has a sequence having one or more amino acid mutations on positions selected from the group consisting of C6E, C26S, C77S, V74C, G84C and A86C.

In some embodiments, the present disclosure further discloses use of a Titin-T chain and an Obscurin-O chain, or a Titin-T chain and an Obscurin-like-O chain, in the reduction of mispairing of a light/heavy chain of a multispecific antibody; in some embodiments, the Titin-T chain comprises a polypeptide having a sequence set forth in any one of SEQ ID NOs: 32, 35-41, 65-76 or a polypeptide having a sequence having at least 80% sequence identity to any one of SEQ ID NOs: 32, 35-41, 65-76, and/or the Obscurin-O chain comprises a polypeptide having a sequence set forth in any one of SEQ ID NOs: 33, 42-58, 77-86 or a polypeptide having a sequence having at least 80% sequence identity to any one of SEQ ID NOs: 33, 42-58, 77-86; or the Titin-T chain comprises a polypeptide having a sequence set forth in any one of SEQ ID NOs: 32, 35-41, 65-76 or a polypeptide having a sequence having at least 80% sequence identity to any one of SEQ ID NOs: 32, 35-41, 65-76, and/or the Obscurin-like-O chain comprises a polypeptide having a sequence set forth in any one of SEQ ID NOs: 34, 59-64 or a polypeptide having a sequence having at least 80% sequence identity to any one of SEQ ID NOs: 34, 59-64.

In some embodiments, for the aforementioned use of a Titin-T chain and an Obscurin-O chain, or a Titin-T chain and an Obscurin-like-O chain, in the reduction of mispairing of a light/heavy chain of a multispecific antibody, the Titin-T chain has a sequence set forth in SEQ ID NO: 32 or further has a sequence having one or more amino acid mutations on positions selected from the group consisting of 8, 20, 22, 25, 26, 39, 3, 11, 13, 40, 42, 45, 47, 49, 56, 58, 66, 70, 75, 77, 79, 81, 82, 83 and 84 and/or 5 amino acid residues of KAGIR added at the N-terminus on the basis of SEQ ID NO: 32; preferably, the Titin-T chain has a sequence having one or more amino acid mutations on positions selected from the group consisting of A8C, V20C, T22C, C25S, A26C, C39T, P3W, S11I, I13L, T22M, G40S, R42K, H45S, Q47E, Q49G, N56S, D58E, M66S and M66K, K70R, L75V, T77S, S79T, G81R, N82M, E83D and F84L and/or 5 amino acid residues of KAGIR added at the N-terminus; the Obscurin-O chain has a sequence set forth in SEQ ID NO: 33 or further has a sequence having one or more amino acid mutations on positions selected from the group consisting of 3, 9, 25, 76, 88, 7, 11, 62, 2, 12, 13, 14, 17, 20, 22, 30, 32, 34, 36, 41, 42, 44, 45, 53, 58, 67, 69, 89, 92, 94 and 97 and/or a DQPQF amino acid residue added at the N-terminus on the basis of SEQ ID NO: 33; preferably, the Obscurin-O chain has a sequence having one or more amino acid mutations on positions selected from the group consisting of A3C, R9C, C25S, C76S, A88C, L7K and L7R, T62K and T62H, G2E, L11K, A12S, F13Y, V14T, V17E, D20L, T22M and T22S, N30D, T32P, Q34E, S36T, Q41K, Q42L, V44I, A45T, A53L, L58V, T62E, A67Q and A67T, G69S, V89L, Q92E, D94G and A97G and/or a DQPQF amino acid residue added at the N-terminus; the Obscurin-like-O chain has a sequence set forth in SEQ ID NO: 34 or further has a sequence having one or more amino acid mutations on positions selected from the group consisting of 6, 26, 74, 77, 84 and 86 on the basis of SEQ ID NO:34; preferably, the Obscurin-like-O chain has a sequence having one or more amino acid mutations on positions selected from the group consisting of C6E, C26S, C77S, V74C, G84C and A86C.

In some embodiments, the multispecific antibody comprises a heavy chain variable region (VH1) and a light chain variable region (VL1), the VH1 and the VL1 forming an antigen binding site specifically binding to a first antigen; a C-terminus of the VH1 is operably linked to an N-terminus of the Titin-T chain, a C-terminus of the VL1 is operably linked to an N-terminus of the Obscurin-O chain or the Obscurin-like-O chain, or the C-terminus of the VL1 is operably linked to the N-terminus of the Titin-T chain, and the C-terminus of the VH1 is operably linked to the N-terminus of the Obscurin-O chain or the Obscurin-like-O chain.

In some embodiments, the present disclosure provides a nucleic acid molecule encoding any one of the aforementioned polypeptide complex, or the multispecific polypeptide complex, or the domain engineered antibody, or the Fab fragment of a domain engineered antibody, or the bispecific antibody, or the polypeptide, or the multispecific antibody.

In some embodiments, the present disclosure provides a vector comprising the aforementioned nucleic acid molecule.

In some embodiments, the present disclosure provides a host cell obtained by transformation (transduction or transfection) with the aforementioned vector, wherein the host cell is selected from the group consisting of prokaryotic cells and eukaryotic cells, preferably eukaryotic cells, and more preferably mammalian cells. In some embodiments, the host cell does not include any animal or plant cells capable of developing into a whole individual, such as human embryonic stem cells, fertilized eggs, or germ cells.

In some embodiments, the host cell is a eukaryotic cell, and more preferably a mammalian cell including, but not limited to, CHO, 293, NSO, and a cell in which gene editing can be performed to alter the glycosylation modification of an antibody or an antigen-binding fragment thereof, thereby altering the ADCC function of the antibody or the antigen binding fragment thereof, e.g., knocking out genes such as FUT8 or GnT-III. In some embodiments, the mammalian cell does not include a human cell.

In some embodiments, the present disclosure provides a method for preparing any one of the aforementioned polypeptide complex, the multispecific polypeptide complex, the domain engineered antibody, the Fab fragment of a domain engineered antibody, the bispecific antibody, or the polypeptide, or the multispecific antibody, which comprises the following steps:
culturing the aforementioned host cell followed by purification and recovery of the polypeptide complex, the multispecific polypeptide complex, the domain engineered antibody, the Fab fragment of a domain engineered antibody, the bispecific antibody or the polypeptide, or the multispecific antibody.

In some embodiments, the present disclosure provides a pharmaceutical composition comprising any one of the aforementioned polypeptide complex, the multispecific polypeptide complex, the domain engineered antibody, the Fab fragment of a domain engineered antibody, the bispecific antibody, the nucleic acid, the multispecific antibody, or the conjugate, and one or more pharmaceutically acceptable carriers, excipients or diluents. Preferably, the unit dose of the pharmaceutical composition may contain 0.01 wt% to 99 wt% of the aforementioned polypeptide complex, the multispecific polypeptide complex, the domain engineered antibody, the Fab fragment of a domain engineered antibody, the bispecific antibody, the multispecific antibody or the conjugate, or the unit dose of the pharmaceutical composition contains the aforementioned polypeptide complex, the multispecific polypeptide complex, the domain engineered antibody, the Fab fragment of a domain engineered antibody, the bispecific antibody, the multispecific antibody or the conjugate in an amount of preferably 0.1-2000 mg, and more preferably 1-1000 mg.

In some embodiments, the present disclosure provides use of any one of the aforementioned polypeptide complex, the multispecific polypeptide complex, the domain engineered antibody, the Fab fragment of a domain engineered antibody, the bispecific antibody, the pharmaceutical composition, the nucleic acid molecule or the multispecific antibody in the preparation of a medicament for the treatment or prevention of a disease or condition. In some embodiments, the disease or condition is a tumor, an inflammatory disease, an autoimmune disease or an infectious disease. In some embodiments, the disease or condition is a bone-related disease, such as a disease or disorder of osteoporosis, osteopenia or osteoarthritis, rheumatoid arthritis, periodontal disease or multiple myeloma. In some embodiments, the tumor is selected from the group consisting of carcinoma, lymphoma, blastoma, sarcoma, leukemia and lymphoid malignancies. In some embodiments, the tumor is selected from the group consisting of: squamous cell carcinoma, myeloma, small-cell lung cancer, non-small cell lung cancer (NSCLC), head and neck squamous cell carcinoma (HNSCC), neuroglioma, Hodgkin's lymphoma, non-Hodgkin's lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), large B-cell lymphoma rich in T-cells/histiocytes, multiple myeloma, myeloid cell leukemia-1 protein (Mcl-1), myelodysplastic syndrome (MDS), gastrointestinal (tract) cancer, kidney cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, gastric cancer, bladder cancer, hepatoma, breast cancer, colon cancer, hepatocellular carcinoma (HCC), clear cell renal cell carcinoma (RCC), head and neck cancer, laryngeal cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative tumors, neuroendocrine tumors, Merkel cell carcinoma, testicular cancer, and skin cancer. In some embodiments, the inflammatory disease is selected from the group consisting of: rheumatoid arthritis, psoriasis, Crohn's disease, ankylosing spondylitis, multiple sclerosis, type I diabetes, hepatitis (e.g., hepatitis B, hepatitis A, hepatitis C), myocarditis, Sjogren's syndrome, autoimmune hemolytic anemia after transplant rejection, bullus pemphigoid, Graves' disease, Hashimoto's thyroiditis, systemic lupus erythematosus (SLE), myasthenia gravis, pemphigus and pernicious anemia. In some embodiments, the immune disease may be selected from the group consisting of: rheumatoid arthritis, psoriasis, joint psoriasis, dermatitis, systemic scleroderma and sclerosis, inflammatory bowel disease (IBD), Crohn's disease, ulcerative colitis, respiratory distress syndrome, meningitis, encephalitis, uveitis, glomerulonephritis, eczema, asthma, arteriosclerosis, leukocyte adhesion-deficient disease, multiple sclerosis, Raynaud's syndrome, Sjogren's syndrome, juvenile diabetes, Reiter's disease, Behcet's disease, immune complex nephritis, IgA nephropathy, IgM polyneuropathy, immune-mediated thrombocytopenic symptoms (such as acute idiopathic thrombocytopenic purpura and chronic idiopathic thrombocytopenic purpura), hemolytic anemia, myasthenia gravis, lupus nephritis, systemic lupus erythematosus, rheumatoid arthritis (RA), atopic dermatitis, pemphigus, Graves' disease, Hashimoto's thyroiditis, Wegener's granulomatosis, Omenn's syndrome, chronic renal failure, acute infectious mononucleosis, HIV and herpes virus related diseases, severe acute respiratory syndrome, choreoretinitis and immunological diseases caused by virus infection (such as diseases caused or mediated by B cell infection by Epstein-Barr Virus (EBV)).

In some embodiments, the present disclosure provides a method for treating or preventing a disease or condition, which comprises administering to a subject a prophylactically or therapeutically effective amount of any one of the aforementioned polypeptide complex, the multispecific polypeptide complex, the domain engineered antibody, the Fab fragment of a domain engineered antibody, the bispecific antibody, the pharmaceutical composition, the nucleic acid molecule or the multispecific antibody. In some embodiments, the disease or condition is a tumor, an inflammatory disease, an autoimmune disease or an infectious disease. In some embodiments, the disease or condition is a bone-related disease, such as a disease or disorder of osteoporosis, osteopenia or osteoarthritis, rheumatoid arthritis, periodontal disease or multiple myeloma. In some embodiments, the tumor is selected from the group consisting of carcinoma, lymphoma, blastoma, sarcoma, leukemia and lymphoid malignancies. In some embodiments, the tumor is selected from the group consisting of: squamous cell carcinoma, myeloma, small-cell lung cancer, non-small cell lung cancer (NSCLC), head and neck squamous cell carcinoma (HNSCC), neuroglioma, Hodgkin's lymphoma, non-Hodgkin's lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), large B-cell lymphoma rich in T-cells/histiocytes, multiple myeloma, myeloid cell leukemia-1 protein (Mcl-1), myelodysplastic syndrome (MDS), gastrointestinal (tract) cancer, kidney cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, gastric cancer, bladder cancer, hepatoma, breast cancer, colon cancer, hepatocellular carcinoma (HCC), clear cell renal cell carcinoma (RCC), head and neck cancer, laryngeal cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative tumors, neuroendocrine tumors, Merkel cell carcinoma, testicular cancer, and skin cancer. In some embodiments, the inflammatory disease is selected from the group consisting of: rheumatoid arthritis, psoriasis, Crohn's disease, ankylosing spondylitis, multiple sclerosis, type I diabetes, hepatitis (e.g., hepatitis B, hepatitis A, hepatitis C), myocarditis, Sjogren's syndrome, autoimmune hemolytic anemia after transplant rejection, bullus pemphigoid, Graves' disease, Hashimoto's thyroiditis, systemic lupus erythematosus (SLE), myasthenia gravis, pemphigus and pernicious anemia. In some embodiments, the immune disease may be selected from the group consisting of: rheumatoid arthritis, psoriasis, joint psoriasis, dermatitis, systemic scleroderma and sclerosis, inflammatory bowel disease (IBD), Crohn's disease, ulcerative colitis, respiratory distress syndrome, meningitis, encephalitis, uveitis, glomerulonephritis, eczema, asthma, arteriosclerosis, leukocyte adhesion-deficient disease, multiple sclerosis, Raynaud's syndrome, Sjogren's syndrome, juvenile diabetes, Reiter's disease, Behcet's disease, immune complex nephritis, IgA nephropathy, IgM polyneuropathy, immune-mediated thrombocytopenic symptoms (such as acute idiopathic thrombocytopenic purpura and chronic idiopathic thrombocytopenic purpura), hemolytic anemia, myasthenia gravis, lupus nephritis, systemic lupus erythematosus, rheumatoid arthritis (RA), atopic dermatitis, pemphigus, Graves' disease, Hashimoto's thyroiditis, Wegener's granulomatosis, Omenn's syndrome, chronic renal failure, acute infectious mononucleosis, HIV and herpes virus related diseases, severe acute respiratory syndrome, choreoretinitis and immunological diseases caused by virus infection (such as diseases caused or mediated by B cell infection by Epstein-Barr Virus (EBV)).

In some embodiments, the present disclosure provides any one of the aforementioned polypeptide complex, the multispecific polypeptide complex, the domain engineered antibody, the Fab fragment of a domain engineered antibody, the bispecific antibody, the pharmaceutical composition, the nucleic acid molecule or the multispecific antibody for use as a medicament. In some embodiments, the medicament is used for treating or preventing a disease or condition. In some embodiments, the disease or condition is a tumor, an inflammatory disease, an autoimmune disease or an infectious disease. In some embodiments, the disease or condition is a bone-related disease, such as a disease or disorder of osteoporosis, osteopenia or osteoarthritis, rheumatoid arthritis, periodontal disease or multiple myeloma. In some embodiments, the tumor is selected from the group consisting of carcinoma, lymphoma, blastoma, sarcoma, leukemia and lymphoid malignancies. In some embodiments, the tumor is selected from the group consisting of: squamous cell carcinoma, myeloma, small-cell lung cancer, non-small cell lung cancer (NSCLC), head and neck squamous cell carcinoma (HNSCC), neuroglioma, Hodgkin's lymphoma, non-Hodgkin's lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), large B-cell lymphoma rich in T-cells/histiocytes, multiple myeloma, myeloid cell leukemia-1 protein (Mcl-1), myelodysplastic syndrome (MDS), gastrointestinal (tract) cancer, kidney cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, gastric cancer, bladder cancer, hepatoma, breast cancer, colon cancer, hepatocellular carcinoma (HCC), clear cell renal cell carcinoma (RCC), head and neck cancer, laryngeal cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative tumors, neuroendocrine tumors, Merkel cell carcinoma, testicular cancer, and skin cancer. In some embodiments, the inflammatory disease is selected from the group consisting of: rheumatoid arthritis, psoriasis, Crohn's disease, ankylosing spondylitis, multiple sclerosis, type I diabetes, hepatitis (e.g., hepatitis B, hepatitis A, hepatitis C), myocarditis, Sjogren's syndrome, autoimmune hemolytic anemia after transplant rejection, bullus pemphigoid, Graves' disease, Hashimoto's thyroiditis, systemic lupus erythematosus (SLE), myasthenia gravis, pemphigus and pernicious anemia. In some embodiments, the immune disease may be selected from the group consisting of: rheumatoid arthritis, psoriasis, joint psoriasis, dermatitis, systemic scleroderma and sclerosis, inflammatory bowel disease (IBD), Crohn's disease, ulcerative colitis, respiratory distress syndrome, meningitis, encephalitis, uveitis, glomerulonephritis, eczema, asthma, arteriosclerosis, leukocyte adhesion-deficient disease, multiple sclerosis, Raynaud's syndrome, Sjogren's syndrome, juvenile diabetes, Reiter's disease, Behcet's disease, immune complex nephritis, IgA nephropathy, IgM polyneuropathy, immune-mediated thrombocytopenic symptoms (such as acute idiopathic thrombocytopenic purpura and chronic idiopathic thrombocytopenic purpura), hemolytic anemia, myasthenia gravis, lupus nephritis, systemic lupus erythematosus, rheumatoid arthritis (RA), atopic dermatitis, pemphigus, Graves' disease, Hashimoto's thyroiditis, Wegener's granulomatosis, Omenn's syndrome, chronic renal failure, acute infectious mononucleosis, HIV and herpes virus related diseases, severe acute respiratory syndrome, choreoretinitis and immunological diseases caused by virus infection (such as diseases caused or mediated by B cell infection by Epstein-Barr Virus (EBV)).

In some embodiments, the present disclosure provides a detection method, which comprises a step of contacting a sample to be tested with any one of the aforementioned polypeptide complex, the multispecific polypeptide complex, the domain engineered antibody, the Fab fragment of a domain engineered antibody, the bispecific antibody or the multispecific antibody.

In some embodiments, the present disclosure provides a reagent for use in detection or assay, wherein the reagent comprises any one of the aforementioned polypeptide complex, the multispecific polypeptide complex, the domain engineered antibody, the Fab fragment of a domain engineered antibody, the bispecific antibody or the multispecific antibody.

### BRIEF DESCRIPTION OF THE DRAWINGS

FIG. 1A and FIG. 1B are structure diagrams of an interaction interface between a Titin-T domain and an Obscurin-O domain or between a Titin-T chain domain and an Obscurin-like-O chain domain;
FIG. 2 is a schematic diagram of Fab with substituted CH1 and CL;
FIG. 3 is a schematic structural diagram of an IgG monospecific antibody with substituted double-sided CH1 and CL;
FIG. 4 is a schematic structural diagram of an IgG bispecific antibody with substituted single-sided CH1 and CL;
FIG. 5 is a schematic structural diagram of a DI-1 bispecific antibody;
FIGs. 6A to 6C are DI-1 mass spectrometry diagrams. FIG. 6A is an LC1/LC2 mass spectrogram, and FIG. 6B is an HC1/HC2 mass spectrogram; FIG. 6C is an LC1+LC2+HC1+HC2 mass spectrogram;
FIG. 7 is schematic structural diagrams of molecule structures with mispairing of light/heavy chains of an antibody;
FIG. 8 is a schematic structural diagram of a BU5 bispecific antibody;
FIGs. 9A to 9C are BU5 mass spectrometry diagrams;
FIG. 10 is an experimental result graph of osteoclast differentiation in an DI-1 bispecific antibody;
FIG. 11 is an experimental result graph of TF1 cell proliferation in a DI-1 bispecific antibody;
FIG. 12 is schematic structural diagrams of bispecific antibodies HJ-1, HJ-2, HJ-3 and HJ-4;
FIGs. 13A to 13D are mass spectrometry diagrams of four-chain co-expression of bispecific antibodies HJ-1, HJ-2, HJ-3 and HJ-4;
FIGs. 14A to 14B are mass spectrometry diagrams of three-chain co-expression of HJ-1-H1, HJ-1-H2 and HJ-1-L2 for the bispecific antibody HJ-1;
FIGs. 15A to 15B are mass spectrometry diagrams of three-chain co-expression of HJ-3-H1, HJ-3-H2 and HJ-3-L2 for the bispecific antibody HJ-3; and
FIGs. 16A to 16B are interactions between residues.

### DETAILED DESCRIPTION

In order that the present disclosure may be more readily understood, certain technical and scientific terms are specifically defined below. Unless otherwise specifically defined herein, all other technical and scientific terms used herein have the same meaning as commonly understood by those of ordinary skill in the art to which the present disclosure belongs.

The three-letter codes and one-letter codes for amino acids used herein are as described in J.biol.chem., 243, p 3558 (1968).

An "antibody (Ab)" is typically an immunoglobulin (Ig), and a naturally-occurring intact antibody is a tetrapeptide chain structure composed of two identical heavy chains and two identical light chains linked by inter-chain disulfide bonds. The heavy chain constant regions of an immunoglobulin differ in their amino acid composition and arrangement, and thus in their antigenicity. Accordingly, immunoglobulins can be classified into five classes, namely, IgM, IgD, IgG, IgA and IgE, with their corresponding heavy chains being µ chains, δ chains, γ chains, α chains and ε chains, respectively. The same class of Ig can be classified into different subclasses according to the differences of amino acid composition of the hinge region and the number and position of disulfide bonds of heavy chains, for example, IgG can be classified into IgG1, IgG2, IgG3 and IgG4. As light chains are classified into κ chains or λ chains according to the difference of constant regions, antibodies can be classified into two types. Each of the five classes of Ig may have either κ chains or λ chains. According to the difference of individual amino acids in the constant region of the λ chain, the λ chain can be classified into four subtypes of λ1, λ2, λ3 and λ4.

Antibodies disclosed herein include antibodies or antigen-binding fragments thereof, including antibodies or antigen-binding fragments thereof that have been engineered on the basis of immunoglobulins while retaining the antigen binding ability; the antibodies or antigen-binding fragments thereof include monospecific antibodies, and multispecific antibodies (e.g., bispecific antibodies), and further include monovalent antibodies, or multivalent antibodies (e.g., bivalent antibodies). An antigen-binding fragment of an antibody, for example, may be an antigen-binding fragment comprising at least one VH-CH1 and at least one VL-CL structure, wherein the VH and VL structures are capable of being close to each other based on inter-chain interactions and retaining the antigen binding ability. In some embodiments, the antigen-binding fragment of the antibody is a monovalent Fab fragment (Fab1 fragment), a divalent Fab fragment (F(ab)2), a trivalent Fab fragment (F(ab)3), a multivalent (two or more) Fab fragment, or other monospecific, bispecific or multispecific antigen-binding fragments comprising at least one Fab fragment.

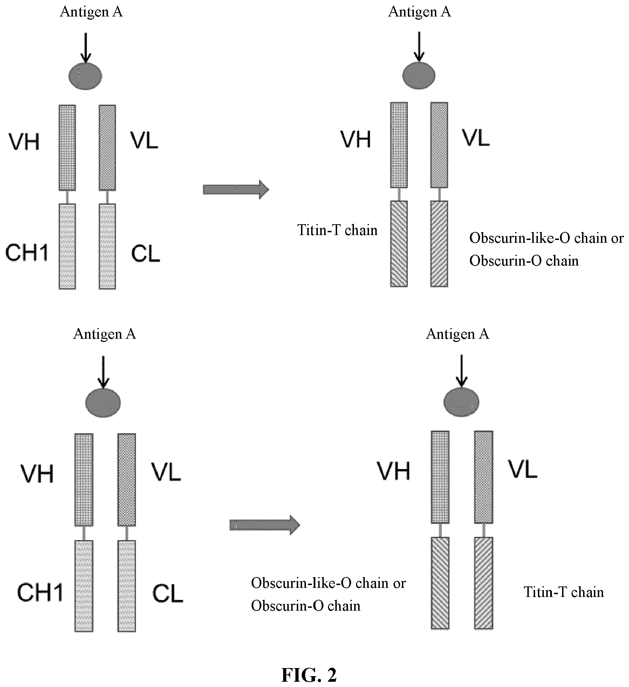

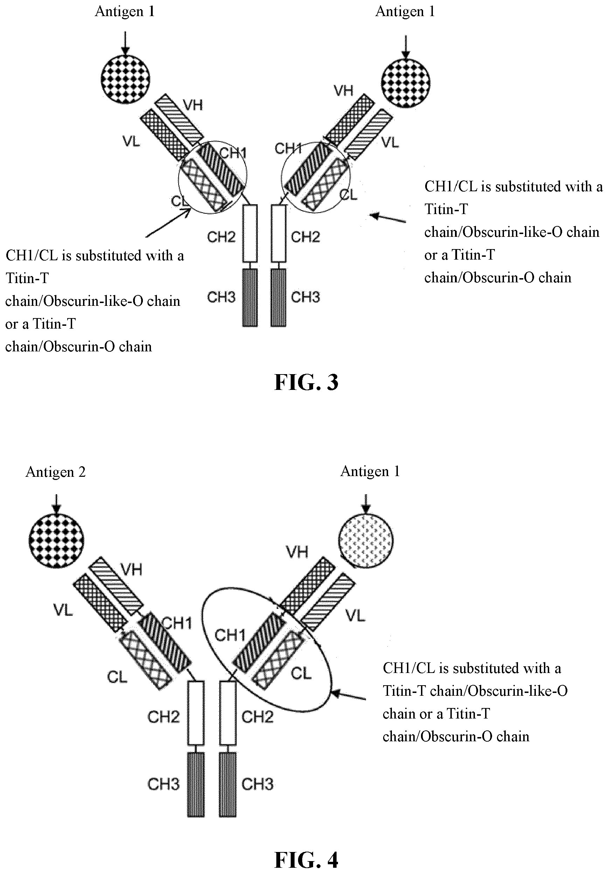

The "domain engineered antibody" described herein refers to an antibody formed after the CH1 and/or CL of the antibody has been substituted with other domains or peptide fragments, which can be used to substitute the CH1/CL domain of the antibody and generally has intermolecular interactions; alternative domain combinations include, but are not limited to, an IL13RA1 Fibronectin type-III 3 domain/IL4R Fibronectin type-III domain, an IL6ST Fibronectin type-III 2 domain/IL6R Fibronectin type-III 2 domain, an HFE Ig-like C1-type domain/B2M Ig-like C1-type domain, a CDIB Ig-like domain/B2M Ig-like C1-type domain, an Obscurin Ig-like 1 domain/Titin Ig-like 152 domain or an Obscurin-like Ig-like 1 domain/Titin Ig-like 152 domain. In some embodiments, the domain engineered antibody is a monospecific antibody, a bispecific antibody or a multispecific antibody; in some embodiments, the domain engineered antibody is a monovalent antibody, a bivalent antibody or a multivalent antibody; in some embodiments, the domain engineered antibody is a whole antibody or an antigen-binding fragment thereof; in some embodiments, the antibody antigen-binding fragment is a Fab fragment, which may be a monovalent Fab fragment (Fab1 fragment), a divalent Fab fragment (F(ab)2), a trivalent Fab fragment (F(ab)3) or a multivalent (two or more) Fab fragment. Illustratively, a schematic structural diagram of a Fab fragment of an antibody with a CH1 and CL substituted with a Titin-T chain/Obscurin-O chain or with a Titin-T chain/Obscurin-like-O chain is shown in FIG. 2; a schematic structural diagram of the antibody with the CH1 and CL substituted with the Titin-T chain/Obscurin-O chain or with the Titin-T chain/Obscurin-like-O chain is shown in FIG. 3; a schematic structural diagram of the bispecific antibody with one-side CH1 and CL substituted with the Titin-T chain/Obscurin-O chain or with the Titin-T chain/Obscurin-like-O chain is shown in FIG. 4. Other antibodies with at least one CH1 and at least one CL substituted with a Titin-T chain/Obscurin-O chain or with a Titin-T chain/Obscurin-like-O chain is, for example, a bivalent antibody with one-side CH1 and CL substituted with a Titin-T chain/Obscurin-O chain or with a Titin-T chain/Obscurin-like-O chain, a bispecific antibody with both-side CH1 and CL substituted with a Titin-T chain/Obscurin-O chain or with a Titin-T chain/Obscurin-like-O chain, a F(ab)2 fragment with two CH1/CL substituted with a Titin-T chain/an Obscurin-O chain or with a Titin-T chain/Obscurin-like-O chain, or a F(ab)3 fragment with 1 or 2 or 3 CH1/CL substituted with a Titin-T chain/Obscurin-O chain or with a Titin-T chain/Obscurin-like-O chain.

"Titin" is a large sarcomeric protein with a complex molecular folding structure. It is known to have the functions of linking thick myofilaments to a Z-wire, maintaining the integrity and stability of myofibrils, and the like. Titin is the third most abundant protein in skeletal muscle fibers, with a molecular weight of 2700 kDa (more than 25000 amino acids) and a length of 1 µm, which is about half of the sarcomere.

A "Titin Ig-like 152 domain" is an Ig-like domain on a Titin protein named as the Titin Ig-like 152 domain, which is capable of binding to an Obscurin Ig-like 1 domain or an Obscurin-like Ig-like 1 domain through intermolecular interactions to form a dimeric complex (available from the RCSB PDB database).

"Titin-T chain" or "T chain" refers to a peptide fragment of 78-118 amino acids in length comprising a Titin Ig-like 152 domain in a Titin protein or a functional variant thereof, wherein the Titin-T chain is capable of binding to the Obscurin Ig-like 1 domain or the Obscurin-like Ig-like 1 domain through intermolecular interactions to form a complex. The functional variant of the T chain is obtained through the mutation on partial amino acids of a wild-type T chain, but still has a peptide fragment of a complex formed by binding to the Obscurin Ig-like 1 domain or the Obscurin-like Ig-like 1 domain through intermolecular interactions. In the present disclosure, the Titin-T chain can be used to substitute the CH1 or CL domain of an antibody without affecting the binding of the VH and VL of the antibody and the binding of the antibody to an epitope. The partial amino acids of the Titin-T chain can be mutated so long as the Titin-T chain is still capable of associating with the Obscurin Ig-like 1 domain to form a complex. For example, amino acids of a suitable length are added or truncated at the C-terminus and/or the N-terminus of the Titin Ig-like 152 domain; for example, 1, 2, 3, 4, 5, 6, 7, 8, 9 or 10 amino acid residues can be added or truncated; for example, 5 "KAGIR" amino acids of the Titin protein immediately adjacent to the N-terminus of the Titin Ig-like 152 domain are added at the N-terminus of the Titin Ig-like 152 domain; a linker sequence of a suitable length (e.g., (GₓS)_{y} linker, wherein X is selected from the group consisting of integers from 1 to 5, and Y is selected from the group consisting of integers from 1 to 6, such as, "G₄S" or "(G₄S)₂") can also be added at the N-terminus and/or the C-terminus of the Titin Ig-like 152 domain to allow the N-terminus of the Titin Ig-like 152 domain to be linked to the VH and/or VL of the antibody through the linker sequence. Other mutations can also be made on amino acids of the Titin Ig-like 152 domain, for example, certain amino acids may be mutated to increase inter-chain disulfide bonds, improving complex stability. In some embodiments of the present disclosure, the Titin-T chain is a polypeptide having a sequence set forth in any one of SEQ ID NOs: 32, 35-41, 65-76 or a sequence having at least 80% sequence identity to any one of SEQ ID NOs: 32, 35-41, 65-76, wherein the polypeptide is capable of associating with an Obscurin-O chain or an Obscurin-like-O chain to form a complex.

"Obscurin" is a protein encoded by the OBSCN gene and belongs to the giant sarcosine signaling protein family. Obscurin is expressed in cardiac and skeletal muscle and plays an important role in the organization of myofibrils during sarcomere assembly. Obscurin is the major cytoplasmic ligand of sarcoplasmic reticulin sANK1, which can prevent the degradation of sANK1 (Lange S et al., Molecular Biology of the Cell., 23 (13): 2490-504); Obscurin acts as a signaling link between sarcoplasmic reticulum domain and sarcoplasmic reticulum domain (Bagnato P et al., The Journal of Cell Biology., 160 (2): 245-53); Obscurin participates in the formation of new sarcomere during myofibril assembly (Borisov AB, et al., Biochemical and Biophysical Research Communications., 310 (3): 910-918).

The "Obscurin Ig-like 1 domain" is a stretch of Ig-like domain in an Obscurin protein named as the Obscurin Ig-like 1 domain, which is capable of binding to a Titin Ig-like 152 domain through intermolecular interactions to form a complex (available from the RCSB PDB database).

"Obscurin-O chain" or "O chain" refers to a peptide fragment of 87-117 amino acids in length comprising an Obscurin Ig-like 1 domain in an Obscurin protein or a functional variant thereof, wherein the Obscurin-O chain is capable of binding to the Titin Ig-like 152 domain through intermolecular interactions to form a complex. The functional variant of the Obscurin-O chain is obtained through the mutation on partial amino acids of a wild type O chain, but still has a peptide fragment of a complex formed by binding to the Titin Ig-like 152 domain through intermolecular interactions. In the present disclosure, the Obscurin-O chain may substitute the CH1 or CL domain of an antibody without affecting the binding of the VH and VL of the antibody and the binding of the antibody to an epitope. The partial amino acids of the Obscurin-O chain can be mutated so long as the Obscurin-O chain is capable of associating with the Titin Ig-like 152 domain to form a complex. For example, amino acids of a suitable length are added or truncated at the C-terminus and/or the N-terminus of the Obscurin-O domain, for example, 1, 2, 3, 4, 5, 6, 7, 8, 9 or 10 amino acids are added or truncated; for example, 5 "DQPQF" amino acids of the wild-type Obscurin protein immediately adjacent to the N-terminus of the Obscurin Ig-like 1 domain are added at the N-terminus of the Obscurin-O domain. A linker sequence of a suitable length (e.g., (GₓS)_{y} linker, wherein X is selected from the group consisting of integers from 1 to 5, and Y is selected from the group consisting of integers from 1 to 6, such as, "G₄S" or "(G₄S)₂") can also be added at the N-terminus and/or the C-terminus of the Obscurin-O domain to allow the N-terminus of the Obscurin-O domain to be linked to the VH and/or VL of the antibody through the linker sequence. Other mutations can also be made on partial amino acids of the Obscurin Ig-like 1 domain, for example, certain amino acids may be mutated to increase inter-chain disulfide bonds, thereby improve antibody stability, and the like. In some embodiments of the present disclosure, the Obscurin-O chain is a polypeptide having a sequence set forth in any one of SEQ ID NOs: 33, 42-58, 77-86 or a sequence having at least 80% sequence identity to any one of SEQ ID NOs: 33, 42-58, 77-86, wherein the polypeptide is capable of associating with the Titin-T chain to form a complex.

"Obscurin-like 1" is a protein encoded by the OBSL1 gene located in SPEG of human chromosome 2q35 and is closely related to Obscurin. Alternative splicing of "Obscurin-like 1" produces multiple isoforms with a predicted molecular weight ranging from 130 kD to 230 kD (Geisler SB et al., (2007)., Genomics., 89 (4): 521-31). The "Obscurin-like Ig-like 1 domain" is a stretch of Ig-like domain in an Obscurin-like 1 protein named as the Obscurin-like Ig-like 1 domain, which is capable of binding to a Titin Ig-like 152 domain through natural intermolecular interactions to form a complex (available from the RCSB PDB database).

"Obscurin-like-O chain" or "OL chain" refers to a peptide fragment of 78-118 amino acids in length comprising an Obscurin-like Ig-like 1 domain in an Obscurin-like 1 protein or a functional variant thereof. The Obscurin-like-O chain is capable of binding to the Titin Ig-like 152 domain through intermolecular interactions to form a complex. The functional variant of the Obscurin-like-O chain is obtained through the mutation on partial amino acids of a wild-type OL chain, but still has a peptide of a complex formed by binding to the Titin Ig-like 152 domain through interactions. In the present disclosure, the Obscurin-like-O chain may substitute the CH1 or CL domain of an antibody without affecting the binding of the VH and VL of the antibody and the binding of the antibody to an epitope. The partial amino acids of the Obscurin-like-O chain can be mutated so long as the Obscurin-like-O chain is capable of associating with the Titin Ig-like 152 domain to form a complex. In some embodiments, for example, amino acids of a suitable length are added or truncated at the C-terminus and/or the N-terminus of the Obscurin-O domain, for example, 1, 2, 3, 4, 5, 6, 7, 8, 9 or 10 amino acids are added or truncated. A linker sequence of a suitable length (e.g., (GₓS)_{y} linker, wherein X is selected from the group consisting of integers from 1 to 5, and Y is selected from the group consisting of integers from 1 to 6, such as, "G₄S" or "(G₄S)₂") can also be added at the N-terminus and/or the C-terminus of the Obscurin-like-O domain to allow the N-terminus of the Obscurin-like-O domain to be linked to the VH and/or VL of the antibody through the linker sequence. Other mutations can also be made on partial amino acids of the Obscurin-like Ig-like 1 domain, for example, certain amino acids may be mutated to increase inter-chain disulfide bonds, thereby improve antibody stability, and the like. In some embodiments of the present disclosure, the Obscurin-like-O chain is a polypeptide having a sequence set forth in any one of SEQ ID NOs: 34, 59-64 or a sequence having at least 80% sequence identity to any one of SEQ ID NOs: 34, 59-64, wherein the polypeptide is capable of associating with the Titin-T chain to form a complex.

In addition, partial amino acids in the Titin Ig-like 152 domain, the Obscurin Ig-like 1 domain or the Obscurin-like Ig-like 1 domain may be mutated so long as it still enables the Titin Ig-like 152 domain and the Obscurin Ig-like 1 domain, or the Titin Ig-like 152 domain and the Obscurin-like Ig-like 1 domain, to be associated with each other to form a complex. In some embodiments, amino acids of a suitable length (e.g., 1, 2, 3, 4, 5, 6, 7, 8, 9 or 10 amino acids of the wild-type protein immediately adjacent to a domain) may be added or truncated at the C-terminus and/or the N-terminus of the Titin Ig-like 152 domain, the Obscurin Ig-like 1 domain or the Obscurin-like Ig-like 1 domain so long as it still enables the Titin Ig-like 152 domain and the Obscurin Ig-like 1 domain, or the Titin Ig-like 152 domain and the Obscurin-like Ig-like 1 domain, to be associated with each other to form a complex. For example, 5 "KAGIR" amino acids of the wild-type Titin protein immediately adjacent to the N-terminus of the Titin Ig-like 152 domain are added to the N-terminus of the Titin Ig-like 152 domain; or 5 "DQPQF" amino acids of the wild-type Obscurin protein immediately adjacent to the N-terminus of the Obscurin Ig-like 1 domain are added at the N-terminus of the Obscurin Ig-like 1 domain. In some embodiments, a linker sequence of a suitable length (e.g., (GₓS)_{y} linker, wherein X is selected from the group consisting of integers from 1 to 5, and Y is selected from the group consisting of integers from 1 to 6, such as, "G₄S" or "(G₄S)₂ ") may be added at the N-terminus and/or the C-terminus of the Titin Ig-like 152 domain, the Obscurin Ig-like 1 domain or the Obscurin-like Ig-like 1 domain to allow the N-terminus of the Titin Ig-like 152 domain, the Obscurin Ig-like 1 domain or the Obscurin-like Ig-like 1 domain to be linked to the VH or VL of the antibody through a linker sequence. In some embodiments, other mutations can also be made on partial amino acids of the Titin Ig-like 152 domain, the Obscurin Ig-like 1 domain or the Obscurin-like Ig-like 1 domain so long as it still enables the Titin Ig-like 152 domain and the Obscurin Ig-like 1 domain, or the Titin Ig-like 152 domain and the Obscurin-like Ig-like 1 domain to be interacted with each other to form a complex, for example, partial amino acids are mutated to increase inter-chain disulfide bonds, thereby improving antibody stability, and the like.

The "Fab fragment" refers to a fragment capable of binding to an antigen that can be produced by enzymatic treatment of a monoclonal antibody and comprises light chain (VL-CL) and heavy chain (VH-CH1) structures.

The "domain engineered Fab fragment" refers to a polypeptide fragment of a Fab fragment of an antibody in which a CL and/or CH1 have been substituted with other domains or peptide fragments, and still retains the interaction between VH and VL and retains the binding ability of the antibody to the antigen. In some embodiments, the domain engineered Fab fragment may be part of a multivalent antibody or multivalent polypeptide complex. In other embodiments, the domain engineered Fab fragment is a single protein molecule. The Fab fragment may be a monovalent Fab fragment (Fab fragment 1), a bivalent Fab fragment (F(ab)2), a trivalent Fab fragment (F(ab)3) or a multivalent (two or more) Fab fragment. Exemplarily, the schematic diagram of the Fab fragment with substituted CH1 and CL is shown in FIG. 2.

The term "specific antibody" or "specific polypeptide complex" refers to an antibody or polypeptide complex capable of specifically binding to a target antigen or an epitope thereof. Antibodies are classified into monospecific antibodies, bispecific antibodies, trispecific antibodies, tetraspecific antibodies, ..., multispecific (binding to two or more different target antigens or different epitopes thereof) antibodies according to the number of different target antigens or different epitopes thereof to which the antibodies bind. Polypeptide complexes are classified into monospecific polypeptide complexes, bispecific polypeptide complexes, trispecific polypeptide complexes, tetraspecific polypeptide complexes, ..., multispecific (binding to two or more different target antigens or different epitopes thereof) polypeptide complexes according to the number of different target antigens or different epitopes thereof to which the polypeptide complexes bind. For example, a "bispecific antibody" refers to an antibody (including an antibody or an antigen-binding fragment thereof, such as, a single chain antibody) capable of specifically binding to two different antigens or two different epitopes of the same antigen. Bispecific antibodies of various structures have been disclosed in the prior art; the bispecific antibodies can be classified into IgG-like bispecific antibodies and antibody-fragment-type bispecific antibodies according to the integrity of IgG molecules; the bispecific antibodies can be classified into bivalent, trivalent, tetravalent or higher-valent bispecific antibodies in configuration according to the number of the antigen-binding regions; the bispecific antibodies can be classified into symmetric bispecific antibodies and asymmetric bispecific antibodies according to whether their structures are symmetric or asymmetric. Among them, the bispecific antibodies based on antibody fragments, such as Fab fragments lacking Fc fragments, formed by binding 2 or more Fab fragments in one molecule, have low immunogenicity, small molecular weight and high tumor tissue permeability, and typical antibody structures of this type comprise bispecific antibodies, such as F(ab)₂, scFv-Fab and (scFv)₂-Fab; IgG-like bispecific antibodies (e.g., having Fc fragments) have relatively large molecular weight, and the Fc fragments facilitate later purification of the antibody and increase their solubility and stability, and the Fc fragments may further bind to the receptor FcRn and increase the serum half-life of the antibody, and typical structural models of bispecific antibodies comprise bispecific antibodies such as KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP- DART, CODV-Fab-TL, HLE-BiTE, F(ab)₂-CrossMAb, IgG-(scFv)₂, Bs4Ab, DVD-Ig, Tetravalent-DART-Fc, (scFv)4-Fc, CODV-Ig, mAb2 and F(ab)4-CrossMAb (see Aran F. Labrijn et al., Nature Reviews Drug Discovery, volume 18, pages 585-608 (2019); Chen S1 et al., JImmunol Res., Feb. 11, 2019; 2019: 4516041).

The term "monovalent", "divalent", "trivalent" or "multivalent" refers to the presence of a specified number of antigen binding sites in an antibody or a polypeptide complex. For example, the "monovalent antibody" refers to the presence of one antigen binding site in an antibody, the "monovalent polypeptide complex" refers to the presence of one antigen binding site in a polypeptide complex; the "bivalent antibody" refers to the presence of two antigen binding sites in an antibody, and the "bivalent polypeptide complex" refers to the presence of two antigen binding sites in a polypeptide complex; the "trivalent antibody" refers to the presence of three antigen binding sites in an antibody, and the "trivalent polypeptide complex" refers to the presence of three antigen binding sites in a polypeptide complex; the "multivalent antibody" refers to the presence of multiple (e.g., three or more) antigen binding sites in an antibody, and the "multivalent polypeptide complex" refers to the presence of multiple (e.g., two or more) antigen binding sites in a polypeptide complex.

The term "polypeptide", "peptide" or "protein" is used interchangeably in the present disclosure and refers to a polymer of amino acid residues, or a collection of polymers of multiple amino acid residues. These terms are used to describe amino acid polymers in which one or more amino acid residue is an artificial chemical mimetic of a corresponding naturally-occurring amino acid, as well as naturally-occurring amino acid polymers and non-naturally-occurring amino acid polymers. A polypeptide sequence is generally described as having an amino terminus (N-terminus) at the left end and having a carboxyl terminus (C-terminus) at the right end. The "polypeptide complex" disclosed herein refers to a complex comprising one or more polypeptides that are associated with implementing certain functions. In some embodiments, the polypeptide complex is an antibody or an antigen-binding fragment thereof capable of specifically binding to an antigen.

The term "binding region" or "binding site" or "binding domain" for an antigen refers to a region or portion of a specific protein molecule (e.g., an antibody molecule) that is capable of specifically binding to an antigen, and the antigen binding region can be a portion of a ligand binding domain that is capable of directly binding to an antigen, or can be a domain that comprises a variable region of an antibody that is capable of directly binding to an antigen.

The term "operably linked" or "operable linkage" means that two or more biological sequences are linked in a manner such that they function in the intended manner, regardless of the presence or absence of a spacer (also referred to as a linker or a linker sequence). When used to describe polypeptides, the term means that two polypeptide sequences are linked in such a manner that the linked product has the desired biological function, with or without a spacer between these two sequences. For example, an antibody variable region can be operably linked to a constant region to form a stable product with antigen binding activity. The term may also be used to describe polynucleotides. For example, when a polynucleotide encoding a polypeptide is operably linked to a regulatory sequence (e.g., a promoter sequence, an enhancer sequence or a silencer sequence), the term means that the polynucleotide sequence is linked in a manner that allows for the regulated expression of the polypeptide by the polynucleotide.

The term "dimerization domain" refers to a polypeptide domain capable of promoting association to form a dimer. In some embodiments, the first dimerization domain may be associated with the second dimerization domain. The association may be bound or linked or bonded by any suitable means, for example, through a linker, a disulfide bond, a hydrogen bond, an electrostatic interaction, a salt bridge, a hydrophobic-hydrophilic interaction, or a combination thereof. Exemplary dimerization domains include, but are not limited to, antibody hinge regions, antibody CH2 domains, antibody CH3 domains and other suitable protein monomers or polypeptides capable of dimerizing and interacting with each other. The hinge regions, CH2 and CH3 may be derived from any antibody isotype, e.g., IgG1, IgG2, IgG3 and IgG4.

The term "interaction domain" refers to a domain of a polypeptide that is capable of facilitating the interaction or association of two or more homologous or heterologous polypeptides. For example, interaction domains are dimerization domains that facilitate association with each other to form a dimer. An inter-protein interaction domain is a domain of a polypeptide that interacts or associates between two or more proteins, for example, an Obscurin-like Ig-like 1 domain in an Obscurin-like protein that is capable of forming a complex with a Titin Ig-like 152 domain of a Titin protein through intermolecular interactions. In some embodiments, the domain that interacts with the Titin-T chain is an Obscurin-O chain or an Obscurin-like-O chain.

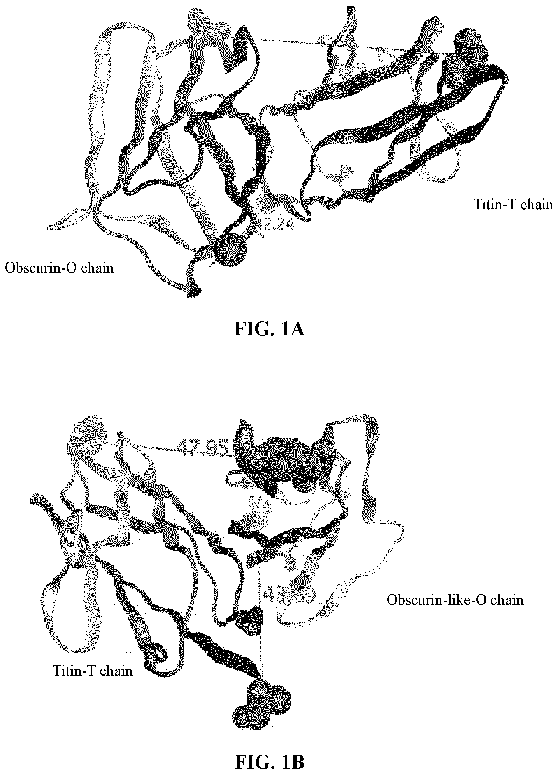

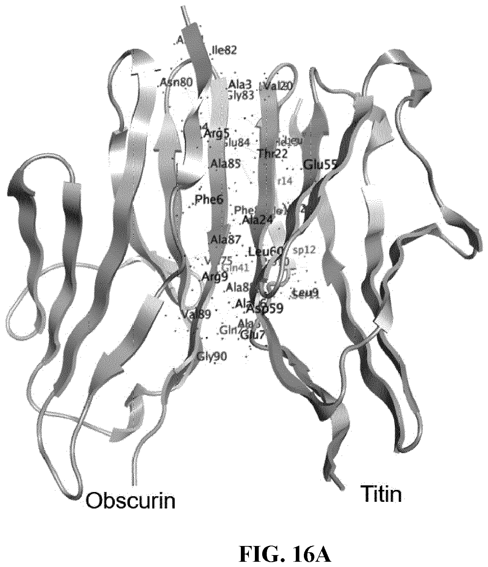

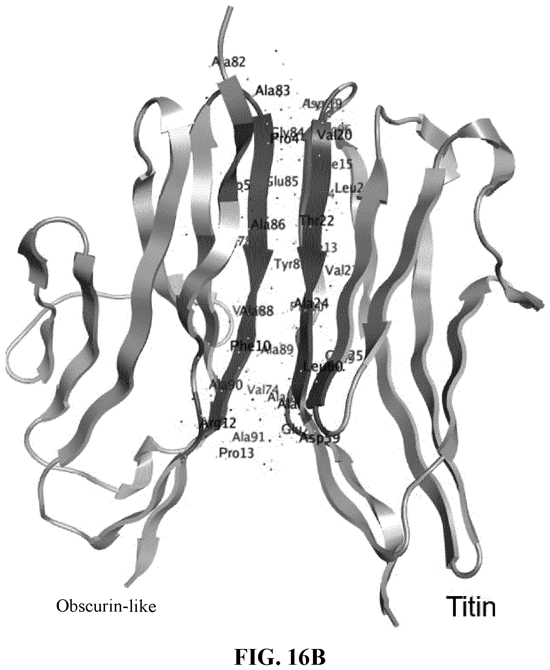

The term "dimer" refers to a structure formed by covalent or non-covalent interactions of two molecules (e.g., polypeptides or proteins). The homodimer or homodimerization is formed from two identical molecules while the heterodimer or heterodimerization is formed from two different molecules. Two molecules can form a dimer through natural inter-chain bonds, and can also form a dimer through unnatural inter-chain bonds. In some embodiments of the present disclosure, the Titin-T chain and the Obscurin-O chain, or the Titin-T chain and the Obscurin-like-O chain, may form a dimer through natural inter-chain bonds. It is well known to those skilled in the art that, for two domains associated with each other to form a dimer, contact interface residues within 6 angstroms between the first domain and the second domain (especially residues within 4.5 angstroms) play a critical role in maintaining the association of these two domains (Yan, Changhui et al., "Characterization of protein-protein interfaces", the Protein Journal, vol., 27,1 (2008): 59-70). Through analysis of the complex formed by associating the Titin-T chain with the Obscurin-O chain or associating the Titin-T chain with the Obscurin-like-O chain by the MOE (molecular operating environment) system (see FIG. 1A and FIG. 1B), it has been found that one or more of residues at positions 7-15, 19-24, 26, 55, 59 and 60 on the Titin-T chain and one or more of residues at positions 3-6, 9, 41, 73, 75 and 80-90 on the Obscurin-O chain interact with each other, and one or more of residues at positions 1, 7-10, 13-16, 19-26, 59-60 and 96 on the Titin-T chain and one or more of residues at positions 4-5, 10, 12-13, 74, 76, 78 and 82-91 on the Obscurin-like-O chain interact with each other (see FIG. 16A and FIG. 16B), wherein the contact interface residues at these sites are separated at a distance within 4.5 angstroms, playing a critical role in maintaining the association of these two domains. In addition, in some other embodiments, the Titin-T chain and the Obscurin-O chain, or the Titin-T chain and the Obscurin-like-O chain, may form a dimer through non-natural inter-chain bonds, wherein the dimer comprises 1, 2, 3, 4, 5, 6, 7, 8, 9, 10 or more non-natural inter-chain bonds. A disulfide bond can be formed between the Titin-T chain and the Obscurin-O chain or between the Titin-T chain and the Obscurin-like-O chain by mutating partial amino acids on the Titin-T chain, the Obscurin-O chain or the Obscurin-like-O chain, thereby stabilizing the dimer. In some embodiments, the Titin-T chain has one or more amino acid residue mutations on positions selected from the group consisting of 8, 20, 22, 25, 26 and 39, and/or the Obscurin-O chain has one or more amino acid residue mutations on positions selected from the group consisting of 3, 9, 25, 76 and 88; or the Titin-T chain has one or more amino acid residue mutations on positions selected from the group consisting of 8, 20, 22, 25, 26 and 39, and/or the Obscurin-like-O chain has one or more amino acid residue mutations on positions selected from the group consisting of 6, 26, 74, 77, 84 and 86. In some embodiments, the Titin-T chain has one or more amino acid residue mutations on positions selected from the group consisting of A8C, V20C, T22C, C25S, A26C and C39T, and/or the Obscurin-O chain has one or more amino acid residue mutations on positions selected from the group consisting of A3C, R9C, C25S, C76S and A88C; or the Titin-T chain has one or more amino acid residue mutations on positions selected from the group consisting of A8C, V20C, T22C, C25S, A26C and C39T, and/or the Obscurin-like-O chain has one or more amino acid residue mutations on positions selected from the group consisting of C6E, C26S, C77S, V74C, G84C and A86C. In some embodiments, the Titin-T chain has C25S, C39T and A8C mutations, and the Obscurin-O chain has an A88C mutation; the Titin-T chain has C25S, C39T and V20C mutations, and the Obscurin-O chain has an A3C mutation; the Titin-T chain has C25S, C39T and A26C mutations, and the Obscurin-O chain has an R9C mutation; the Titin-T chain has C25S, C39T and A8C mutations, and the Obscurin-O chain has C25S, C76S and A88C mutations; the Titin-T chain has C25S, C39T and V20C mutations, and the Obscurin-O chain has C25S, C76S and A3C mutations; the Titin-T chain has C25S, C39T and A26C mutations, and the Obscurin-O chain has C25S, C76S and R9C mutations; the Titin-T chain has C25S, C39T and A8C mutations, and the Obscurin-like-O chain has C6E and V74C mutations; the Titin-T chain has C25S, C39T and V20C mutations, and the Obscurin-like-O chain has C6E and G84C mutations; the Titin-T chain has C25S, C39T and T22C mutations, and the Obscurin-like-O chain has C6E and A86C mutations; the Titin-T chain has C25S, C39T and A8C mutations, and the Obscurin-like-O chain has C6E, C26S, C77S and V74C mutations; the Titin-T chain has C25S, C39T and V20C mutations, and the Obscurin-like-O chain has C6E, C26S, C77S and G84C mutations; the Titin-T chain has C25S, C39T and T22C mutations, and the Obscurin-like-O chain has C6E, C26S, C77S and A86C mutations. In some embodiments, the Obscurin-O chain further has one or more amino acid residue mutations on positions selected from the group consisting of 7, 11 and 62; preferably, the Obscurin-O chain has one or more amino acid residue mutations on positions selected from the group consisting of L7K and L7R, K11L, T62K and T62H; most preferably, the Titin-T chain has C25S, C39T and A8C mutations, and the Obscurin-O chain has C25S, C76S, A88C, L7K and T62K mutations; the Titin-T chain has C25S, C39T and A8C mutations, and the Obscurin-O chain has C25S, C76S, A88C, L7K and T62H mutations; the Titin-T chain has C25S, C39T and A8C mutations, and the Obscurin-O chain has C25S, C76S, A88C, K11L and T62K mutations; or the Titin-T chain has C25S, C39T and A8C mutations, and the Obscurin-O chain has C25S, C76S, A88C, K11L and T62H mutations. The mutation position in the Titin-T chain is a natural sequence numbering position relative to a T.0 chain (SEQ ID NO: 32); the mutation position in the Obscurin-O chain is a natural sequence numbering position relative to an O.0 chain (SEQ ID NO: 33); the mutation position in the Obscurin-like-O chain is a natural sequence numbering position relative to an OL.0 chain (SEQ ID NO: 34).

In the present disclosure, "mispairing" means that two or more homologous or heterologous polypeptides are interacted or associated together to form an undesired dimer or multimer mispairing. "Mispairing is less susceptible to happen" means, for example, that when polypeptides A1, B1 and B2 are co-expressed, if the desired amount of A1-B1 dimer expression is greater than the amount of A1-B2 dimer, preferential mispairing between A1-B1 is considered to happen, i.e., mispairing between A1-B2 is less susceptible to happen. In some embodiments of the present disclosure, in the multispecific polypeptide complex, mispairing between VH1 of the first polypeptide complex and VL2 of the second polypeptide complex is less susceptible to happen, and/or mispairing between VL1 of the first polypeptide complex and VH2 of the second polypeptide complex is less susceptible to happen. However, preferential mispairing between VH1-VL1 and preferential mispairing between VH2-VL2 are considered. The "disulfide bond" refers to a covalent bond formed between sulfur atoms in the structure R-S-S-R'. The amino acid cysteine comprises a thiol group which may form a disulphide bond with a second thiol group, for example with a thiol group of another cysteine residue. Disulfide bonds can be formed between the thiol groups of two cysteine residues located on two polypeptide chains, respectively, thereby forming an inter-chain bridge or an inter-chain bond.

Electrostatic interactions are non-covalent interactions and play a critical role in protein folding, stability, flexibility and function, including ionic interactions, hydrogen bonding and halogen bonding. Electrostatic interactions may be formed in polypeptides, for example, between Lys and Asp, between Lys and Glu, between Glu and Arg, or between Glu and Trp on a first chain and Arg, Val or Thr on a second chain.

Salt bridges are close-range electrostatic interactions, primarily from the anionic carboxylate of Asp or Glu and from the cationic ammonium of Lys or the guanidino group of Arg, which are pairs of oppositely charged residues in close spatial proximity in the native protein structure. Charged and polar residues in the hydrophobic interface can act as hot spots for binding. Among them, residues having ionizable side chains, such as His, Tyr and Ser, can also participate in the formation of salt bridges.

The hydrophilic interaction means that molecules with polar groups have large affinity to water and can form transient bonding with water through hydrogen bonds. The hydrophobic interaction is a non-covalent interaction between non-polar molecules. These non-polar molecules (e.g., some neutral amino acid residues, also known as hydrophobic residues) have a tendency to aggregate with each other in an aqueous environment and keep away from water. For example, hydrophobic interactions may be formed between one or more of Val, Tyr and Ala in the first chain and one or more of Val, Leu and Trp in the second chain, or His and Ala in the first chain and Thr and Phe in the second chain. (See Brinkmann et al., 2017).

The term "hydrogen bond" is formed by electrostatic attraction between two polar groups when a hydrogen atom is covalently bonded to a highly electronegative atom, such as, nitrogen, oxygen or fluorine. Hydrogen bonds can be formed between backbone oxygens (e.g., chalcogen groups) and amide hydrogens (nitrogen groups) of two residues of a polypeptide, for example, a hydrogen bond can be formed between a nitrogen group in Asn and an oxygen group in His, or an oxygen group in Asn and a nitrogen group in Lys. The interactions between hydrogen bonds are stronger than Van der Waals interactions, but weaker than interactions between covalent or ionic bonds, and are critical for maintaining secondary and tertiary structures. For example, an α-helix is formed when the spacing of amino acid residues occurs regularly between positions i and i+4, and a sheet of twisted folds formed by peptide fragments of 3-10 amino acids in length when two peptides are linked through at least two or three backbone hydrogen bonds is a β-sheet.

As used herein, "knob-into-hole" refers to an interaction between two polypeptides, one of which has a bulge (i.e., a "knob") due to the presence of amino acid residues with bulky side chains (e.g., tyrosine or tryptophan). The other polypeptide has a cavity (i.e., a "hole") in which small side-chain amino acid residues (e.g., alanine or threonine) are present, and a bulge can be positioned in the cavity to facilitate the interaction of the two polypeptides to form a heterodimer or a complex. Methods for producing polypeptides with bulges into the holes are known in the art and can be found in Patents US5731169, WO1996027011, etc.

"Non-natural inter-chain bonds" refer to inter-chain bonds not found in wild-type polypeptide polymers. For example, a non-natural inter-chain bond may be formed between a mutated amino acid residue of one polypeptide and a wild-type amino acid residue or a mutated amino acid residue of another polypeptide. In certain embodiments, at least one non-natural inter-chain bond is a "disulfide bond" formed following amino acid mutation. In some embodiments of the present disclosure, non-natural inter-chain bonds between the Titin-T chain and the Obscurin-O chain, or the Titin-T chain and the Obscurin-like-O chain, are formed by mutation on one or more amino acid residues on positions selected from the group consisting of 8, 20, 22, 25, 26 and 39 on the Titin-T chain, and/or positions selected from the group consisting of 3, 9, 25, 76 and 88 on the Obscurin-O chain, or formed by mutation on one or more amino acid residues on positions selected from the group consisting of 8, 20, 22, 25, 26 and 39 on the Titin-T chain, and/or positions selected from the group consisting of 6, 26, 74, 77, 84 and 86 on the Obscurin-like-O chain.

The term "contact interface" refers to a specific region on polypeptides where the polypeptides are in contact with or interacted with each other. The contact interface comprises one or more amino acid residues, and when an interaction occurs, amino acid residues of the contact interface on a polypeptide are capable of interacting with the corresponding amino acid residues with which they are in contact. The amino acid residues of the contact interface may be in a continuous or discontinuous sequence. For example, when the interface is three-dimensional, amino acid residues within the interface can be separated at different positions on the linear sequence.

The "linking domain" or "spacer" refers to a border or a border region where two polypeptide sequences are fused or combined. For example, the linking domain may comprise at least a portion of a C-terminal fragment from the first fusion polypeptide that is fused to at least a portion of an N-terminal fragment from the second fusion polypeptide, with or without other spacers therebetween. For example, the spacer may be rich in glycine and proline residues, such as a spacer having a single or repeated sequence consisting of threonine/serine and glycine, GGGGS, TGGGG, SGGGG, or a tandem repeat thereof (e.g., 2, 3, 4, 5 or more repeats). In certain embodiments, the spacer comprises a GGGGS or 2 or more tandem repeats thereof.

A natural intact antibody comprises two heavy chains and two light chains. Each heavy chain consists of a variable region ("HCVR", VH) and first, second and third constant regions (CH1, CH2 and CH3), second (CH2) and third (CH3) constant regions of the antibody constitute the "Fc", and the N-terminus of CH2 may or may not comprise a hinge region portion of the antibody which is rich in proline, including inter-chain disulfide bonds; while each light chain consists of a variable region ("LCVR", VL) and a constant region (CL). A portion near the N-terminus of the heavy and light chains has a variable sequence of about 110 amino acids is a variable region (Fv region); a portion near the C-terminus has a relatively stable sequence of the remaining amino acids is a constant region. The variable region includes 3 hypervariable regions (HVRs) and 4 framework regions (FRs) with relatively conserved sequences. The 3 hypervariable regions, which determine the specificity of the antibody, are also known as complementarity determining regions (CDRs). Each of the light chain variable region (VL) and the heavy chain variable region (VH) is composed of 3 CDR regions and 4 FR regions, and the sequence from the amino terminus to the carboxyl terminus is FR1-CDR1-FR2-CDR2-FR3-CDR3-FR4. The 3 CDR regions of the light chain refer to LCDR1, LCDR2 and LCDR3; the 3 CDR regions of the heavy chain refer to HCDR1, HCDR2 and HCDR3.

The antibodies disclosed herein may, for example, be antibodies derived from animals (e.g., antibodies of murine, avian, rabbit, camel and monkey), chimeric antibodies, humanized antibodies and fully human antibodies. The antibodies disclosed herein include, in addition to full-length antibodies, antigen-binding fragments binding to an antigen.

The term "murine antibody" used herein refers to a monoclonal antibody to an antigen prepared according to the knowledge and skill in the art. During the preparation, a test subject is injected with an antigen, and then hybridoma of antibodies expressing the desired sequence or functional properties is isolated. In a preferred embodiment of the present disclosure, the murine antibody or an antigen-binding fragment thereof may further comprise a light chain constant region of a murine κ and λ chain or a variant thereof, or further comprises a heavy chain constant region of murine IgG1, IgG2 or IgG3 or a variant thereof.

The term "chimeric antibody" is an antibody formed by fusion of the variable region of an antibody of another first species to the constant region of an antibody of a second species. For example, the establishment of a human-mouse chimeric antibody comprises establishing a hybridoma secreting a mouse-derived specific monoclonal antibody, cloning a variable region gene from a mouse hybridoma cell, then cloning a constant region gene of a human antibody as required, linking the mouse variable region gene and the human constant region gene into a chimeric gene and then inserting the chimeric gene into an expression vector, and finally expressing a chimeric antibody molecule in a eukaryotic system or a prokaryotic system. In a preferred embodiment of the present disclosure, the light chain of the chimeric antibody further comprises a light chain constant region of human κ and λ chains or variants thereof. The heavy chain of the antigen chimeric antibody further comprises a heavy chain constant region of human IgG1, IgG2, IgG3 or IgG4 or a variant thereof, and preferably comprises a heavy chain constant region of human IgG1, IgG2 or IgG4, or an IgG1, IgG2 or IgG4 variant using an amino acid mutation (e.g., L234A and/or L235A mutation, and/or S228P mutation). The term "humanized antibody", also known as CDR-grafted antibody, refers to an antibody produced by grafting murine CDR sequences into a human antibody variable region framework, i.e., a different type of human germline antibody framework sequence. Such the antibody can overcome the heterogeneous reaction induced by the chimeric antibody because of carrying a large amount of mouse protein components. Such the framework sequences can be obtained from public DNA databases or published references that include germline antibody gene sequences. Germline DNA Sequences for human heavy and light chain variable region genes can be obtained from the "VBase" human germline sequence database and found in Kabat, E.A. et al., 1991, Sequences of Proteins of Immunological Interest, 5th edition. To avoid decreased immunogenicity and decreased activity at the same time, the human antibody variable region framework sequences may be minimally reversely-mutated or back-mutated to retain activity. The humanized antibodies disclosed herein also include humanized antibodies formed after further affinity maturation mutations on the CDRs from yeast display.

In one embodiment of the present disclosure, the antibody or antigen-binding fragment thereof may further comprise a light chain constant region of a human or murine κ and λ chain or a variant thereof, or further comprise a heavy chain constant region of a human or murine IgG1, IgG2, IgG3 or IgG4 or a variant thereof, and preferably comprises a heavy chain constant region of human IgG1, IgG2 or IgG4, or an IgG1, IgG2 or IgG4 variant using an amino acid mutation (e.g., L234A and/or L235A mutation, and/or S228P mutation).

The "conventional variants" of the human antibody heavy chain constant region and the human antibody light chain constant region, including the same, in the present disclosure refer to variants of the heavy chain constant region or light chain constant region that have been disclosed in the prior art and derived from human without altering the structure and function of the antibody variable region, and exemplary variants include variants of the heavy chain constant region of IgG1, IgG2, IgG3 or IgG4 with the heavy chain constant region having site-directed engineering and amino acid substitution, and specifically, substitutions comprise such as YTE mutations known in the prior art, L234A and/or L235A mutations, S228P mutations, and/or mutations that obtain a knob-into-hole structure (such that the heavy chain of the antibody has a combination of knob-Fc and hole-Fc), all of which have been confirmed to give the antibody new properties without altering the function of the antibody variable region. The "human antibody" (HuMAb) and "fully human antibody" can be used interchangeably and can be either a human derived antibody or an antibody obtained from a transgenic organism that is "engineered" to produce specific human antibodies in response to antigenic challenge and can be produced by any method known in the art. The transgenic organism can synthesize human antibodies, and the organism can be used to produce human antibody-secreting hybridomas. A human antibody can also be an antibody in which the heavy and light chains are encoded by nucleotide sequences derived from one or more human DNA sources. Fully human antibodies can also be constructed by gene or chromosome transfection methods and phage display techniques, or by in-vitro activated B cells, all of which are known in the art.

The terms "full-length antibody", "intact antibody", "complete antibody" and "whole antibody" are used interchangeably herein and refer to a substantially intact form of an antibody, as distinguished from antigen-binding fragments defined below. The term especially refers to antibodies in which the light and heavy chains comprise constant regions. The "antibody" disclosed herein encompasses a "full-length antibody" and an antigen-binding fragment thereof.

In some embodiments, a full-length antibody disclosed herein includes a full-length antibody formed by a light chain having a light chain variable region linked to a light chain constant region and a heavy chain having a heavy chain variable region linked to a heavy chain constant region. Those skilled in the art can select light chain constant regions and heavy chain constant regions derived from different antibodies according to actual needs, such as light chain constant regions and heavy chain constant regions of human antibodies.

The term "antigen-binding fragment" of an antibody refers to one or more fragments of an antibody that retain the ability to bind to an antigen (e.g., an antigen). It has been shown that fragments of full-length antibodies can be used to perform the antigen binding function of the antibody. Examples of binding fragments encompassed in the term "antigen-binding fragment" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region, (iii) an Fd fragment consisting of the VH and CH1 domains; (iv) an Fv fragment consisting of the VH and VL domains of a single arm of an antibody; (v) dsFv, a stable antigen-binding fragment formed by inter-chain disulfide bonds from VH and VL; and (vi) diabodies, bispecific antibodies and multispecific antibodies comprising scFv, dsFv, Fab and other fragments. Furthermore, although the two domains of the Fv fragment, VL and VH, are encoded by separate genes, they can be linked by a synthetic linker using a recombinant method, such that it is capable of generating a single protein chain in which the VL and VH regions are paired to form monovalent molecules (referred to as single chain Fv (scFv); see, e.g., Bird et al., (1988) Science, 242: 423-426; and Huston et al., (1988) Proc.Natl.Acad.Sci USA 85: 5879-5883). Such single chain antibodies are also included in the term "antigen-binding fragment" of an antibody. Such antibody fragments are obtained using conventional techniques known to those skilled in the art, and the fragments are screened for utility in the same manner as for intact antibodies. Antigen-binding moieties may be produced by recombinant DNA techniques or by enzymatic or chemical cleavage of intact immunoglobulins. The antibodies can be antibodies of different types, for example, an IgG (e.g., IgG1, IgG2, IgG3 or IgG4 subtype), IgA1, IgA2, IgD, IgE or IgM antibody. F(ab')₂ is an antibody fragment having a molecular weight of about 100,000 and having antigen binding activity and comprising two Fab regions linked at the hinge position, which is obtained by enzymatically digesting the lower portion of two disulfide bonds in the IgG hinge region.

Fab' is an antibody fragment having a molecular weight of about 50,000 and having antigen-binding activity, which is obtained by cleaving the disulfide bonds in the hinge region of the above-mentioned F(ab')₂. Fab' disclosed herein can be produced by treating F(ab')₂ disclosed herein that specifically recognizes an antigen with a reducing agent such as dithiothreitol.

In addition, the Fab' can be produced by inserting DNA encoding the Fab' fragment of the antibody into a prokaryotic expression vector or a eukaryotic expression vector and introducing the vector into a prokaryote or a eukaryote to express the Fab'.

The term "single chain antibody", "single chain Fv" or "scFv" refers to a molecule comprising an antibody heavy chain variable domain (or region; VH) and an antibody light chain variable domain (or region; VL) linked by a linker. Such scFv molecules may have the general structure: NH2-VL-linker-VH-COOH or NH₂-VH-linker-VL-COOH. A suitable linker in the prior art consists of repeated GGGGS amino acid sequences or variants thereof, for example using variants of 1-4 repeats (Holliger et al., (1993), Proc. Natl. Acad. Sci. USA 90: 6444-6448). Other linkers useful in the present disclosure are described in Alfthan et al., (1995), Protein Eng., 8: 725-731, Choi et al., (2001), Eur.J.Immunol., 31: 94-106, Hu et al., (1996), Cancer Res., 56: 3055-3061, Kipriyanov et al., (1999), J.Mol.Biol., 293: 41-56 and Roovers et al., (2001), Cancer Immunol.

Diabodies are antibody fragments in which an scFv or Fab is dimerized, and are antibody fragments having bivalent antigen binding activity. In the divalent antigen binding activity, the two antigens may be the same or different.

The diabodies disclosed herein can be produced by the following steps: obtaining cDNA encoding VH and VL of the antibodies disclosed herein, constructing DNA encoding scFv, inserting the DNA into an expression vector, and then introducing the expression vector into a prokaryote or a eukaryote to express the diabodies.

The dsFv is obtained by linking polypeptides in which one amino acid residue in each of VH and VL is substituted with a cysteine residue via a disulfide bond between cysteine residues. The amino acid residue substituted with a cysteine residue may be selected based on the prediction of the three-dimensional structure of the antibody according to a known method (Protein Engineering, 7, 697 (1994)).

The full-length antibody, the antigen-binding fragment, the bispecific antibody, the multispecific antibody, the polypeptide, the polypeptide complex or the multispecific polypeptide complex disclosed herein can be produced by the following steps: obtaining cDNA encoding an antibody of the present disclosure, constructing DNA encoding a dsFv, inserting the DNA into an expression vector, and then introducing the expression vector into a prokaryote or eukaryote to express the dsFv.

The term "amino acid difference" or "amino acid mutation" refers to the presence of an amino acid change or mutation in a variant protein or polypeptide as compared to the original protein or polypeptide, including the occurrence of 1, 2, 3 or more amino acid insertions, deletions or substitutions on the basis of the original protein or polypeptide. The term "antibody framework" or "FR region" refers to a portion of a variable domain VL or VH that serves as a scaffold for the antigen-binding loops (CDRs) of the variable domain. It is essentially a variable domain without CDRs. The term "complementarity determining region", "CDR" or "hypervariable region" refers to one of the 6 hypervariable regions within the variable domain of an antibody which primarily contribute to antigen binding. Typically, there are three CDRs (HCDR1, HCDR2 and HCDR3) per heavy chain variable region and three CDRs (LCDR1, LCDR2 and LCDR3) per light chain variable region. Any of a variety of well-known protocols may be used to determine the amino acid sequence boundaries of the CDRs, including the "Kabat" numbering scheme (see Kabat et al., (1991), "Sequences of Proteins of Immunological Interest", 5th edition, Public Health Service, National Institutes of Health, Bethesda, MD), "Chothia" numbering scheme (see Al-Lazikani et al., (1997) JMB 273: 927-948) and ImMunoGenTics (IMGT) numbering scheme (Lefranc M.P., Immunologist, 7, 132-136 (1999); Lefranc, M.P. et al., Dev. Comp. Immunol., 27, 55-77 (2003)), etc.

The term "epitope" or "antigenic determinant" refers to a site on an antigen to which an immunoglobulin or antibody specifically binds (e.g., a specific site on an antigen molecule). Epitopes typically comprise at least 3, 4, 5, 6, 7, 8, 9, 10, 11, 12, 13, 14 or 15 contiguous or non-contiguous amino acids in a unique spatial conformation. See, e.g., Epitope Mapping Protocols in Methods in Molecular B iology, volume 66, G.E.Morris, Ed. (1996).

The term "specific binding", "selective binding", "selectively binds" or "specifically binds" refers to the binding of an antibody to an epitope on a predetermined antigen. Typically, the antibody binds with an affinity (KD) of less than about 10⁻⁷ M, e.g., less than about 10⁻⁸ M, 10⁻⁹ M, 10⁻¹⁰ M, 10⁻¹¹ M, 10⁻¹² M or less.

The term "KD" refers to the dissociation equilibrium constant for a particular antibody-antigen interaction. Typically, an antibody disclosed herein binds to an antigen with a dissociation equilibrium constant (KD) of less than about 10⁻⁷ M, e.g., less than about 10⁻⁸ M or 10⁻⁹ M, for example, the affinity of an antibody to an antigen in the present disclosure is determined by FACS method for KD values.

The term "competition", when used in the context of antigen binding proteins that compete for the same epitope (e.g., neutralizing antigen binding proteins or neutralizing antibodies), refers to competition between the antigen binding proteins, as determined by the following assay: in such assay, the antigen binding protein (e.g., antibody or immunologically functional fragment thereof) to be detected prevents or inhibits (e.g., reduces) specific binding of a reference antigen binding protein (e.g., ligand or reference antibody) to a common antigen. Numerous types of competitive binding assays are available for determining whether an antigen binding protein competes with another, such as, solid phase direct or indirect radioimmunoassay (RIA), solid phase direct or indirect enzyme immunoassay (EIA), sandwich competition assay (see, e.g., Stahli et al., 1983, Methods in Enzymology 9: 242-253); solid phase direct biotin-avidin EIA (see, e.g., Kirkland et al., 1986, J.Immunol., 137: 3614-3619), solid phase direct labeling assay, solid phase direct labeling sandwich assay (see, e.g., Harlow and Lane, 1988, Antibodies, A Laboratory Manual (antibody, Lab Manual), Cold Spring Harbor Press); solid phase direct labeling RIA with 1-125 label (see, e.g., Morel et al., 1988, Molec.Immunol., 25: 7-15); solid phase direct biotin-avidin EIA (see, e.g., Cheung et al., 1990, Virology, 176: 546-552); and direct-labeled RIA (Moldenhauer et al., 1990, Scand.J.Immunol., 32: 77-82). Typically, the assay involves the use of purified antigen bound to a solid surface or cell carrying either an unlabeled test antigen binding protein or a labeled reference antigen binding protein. Competitive inhibition is measured by measuring the amount of label that binds to a solid surface or cell in the presence of the measured antigen binding protein. Typically, the measured antigen binding protein is present in excess. Antigen binding proteins identified by competitive assays (competing antigen binding proteins) include an antigen binding protein binding to the same epitope as a reference antigen binding protein and an antigen binding protein binding to a proximal epitope sufficiently close to the binding epitope of the reference antigen binding protein, wherein the two epitopes sterically hinder binding from occurring. Additional details regarding methods for determining competitive binding are provided in the examples herein. Typically, when a competing antigen binding protein is present in excess, it will inhibit (e.g., reduce) at least 40%-45%, 45%-50%, 50%-55%, 55%-60%, 60%-65%, 65%-70%, 70%-75% or 75% or more of specific binding of a reference antigen binding protein to a common antigen. In certain instances, the term "nucleic acid molecule" as used herein refers to DNA molecules and RNA molecules in which binding is inhibited by at least 80%-85%, 85%-90%, 90%-95%, 95%-97% or 97% or more. The nucleic acid molecule may be single-stranded or double-stranded, and preferably double-stranded DNA or single-stranded mRNA or modified mRNA. A nucleic acid is "effectively linked" when it is placed into a functional relationship with another nucleic acid sequence. For example, a promoter or enhancer is effectively linked to a coding sequence if it affects the transcription of the coding sequence.

The term "expression vector" refers to a nucleic acid molecule capable of transporting another nucleic acid to which it has been linked. In one embodiment, the vector is a "plasmid" which refers to a circular double-stranded DNA loop to which additional DNA fragments can be linked. In another embodiment, the vector is a viral vector, wherein additional DNA fragments may be linked to the viral genome. The vectors disclosed herein are capable of autonomous replication in a host cell into which they have been introduced (e.g., bacterial vectors having a bacterial origin of replication and episomal mammalian vectors) or being integrated into the genome of a host cell upon introduction into the host cell and thereby replicated along with the host genome (e.g., non-episomal mammalian vectors).

Methods for producing and purifying antibodies and antigen-binding fragments are well known in the art, such as Cold Spring Harbor Antibody Technical Guide, Chapters 5-8 and 15. For example, a mouse may be immunized with an antigen, and the resulting antibody can be renatured, purified and amino acid sequenced using conventional methods. Antigen-binding fragments can likewise be prepared using conventional methods. The antibody or antigen-binding fragment disclosed herein is genetically engineered to incorporate one or more human FR regions in a non-human CDR region. Human FR germline sequences can be obtained from the website of ImMunoGeneTics (IMGT) by aligning the IMGT human antibody variable region germline gene database with MOE software, or from Immunoglobulin Journal, 2001ISBN012441351.

The term "host cell" refers to a cell into which an expression vector has been introduced. Host cells may include bacterial, microbial, plant or animal cells. Bacteria susceptible to transformation include members of the species *enterobacteria,* such as strains of *Escherichia coli* or *Salmonella; Bacillus* such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese hamster ovary cell lines), 293 cells and NS0 cells.

Engineered antibodies or antigen-binding fragments disclosed herein may be prepared and purified using conventional methods. For example, cDNA sequences encoding the heavy and light chains can be cloned and recombined into an expression vector. The recombinant expression vector can stably transfect CHO cells. As a more recommended prior art, mammalian expression systems may result in glycosylation of antibodies, particularly at the highly conserved N-terminal site of the Fc region. Stable clones are obtained by expression of antibodies specifically binding to the antigen. Positive clones are expanded in a serum-free medium of a bioreactor to produce antibodies. The antibody-secreting culture solution can be purified using conventional techniques. For example, purification is carried out on an A or G Sepharose FF column. Non-specifically bound fractions are washed away. The bound antibody is eluted using a pH-gradient method, the antibody fragment is detected using SDS-PAGE and collected. The antibody can be filtered and concentrated using a conventional method. Soluble mixtures and polymers can also be removed using conventional methods, such as molecular sieves and ion exchange. The resulting product is immediately frozen, e.g., at -70 °C, or lyophilized.

"Administration", "administering" and "treatment", when applied to an animal, a human, an experimental subject, a cell, a tissue, an organ or a biological fluid, refer to contact of an exogenous drug, a therapeutic agent, a diagnostic agent or a composition with the animal, the human, the subject, the cell, the tissue, the organ or the biological fluid. "Administration", "administering" and "treatment" can refer to, for example, therapeutic, pharmacokinetic, diagnostic, research and experimental methods. The treatment of cells includes contacting a reagent with cells and contacting the reagent with a fluid, wherein the fluid contacts the cells. "Administration", "administering" and "treatment" also refer to the treatment of, e.g., cells in vitro and ex vivo, by using an agent, a diagnostic agent, a binding composition, or by another cell. "Treatment", when applied to a human, veterinary or a research subject, refers to therapeutic treatment, prophylactic or preventative measures, research and diagnostic applications.

"Treatment" refers to administration of a therapeutic agent, e.g., a composition comprising any one of the binding compositions disclosed herein, either internally or externally to a subject having one or more disease symptoms for which the therapeutic agent is known to have a therapeutic effect. Typically, the therapeutic agent is administered in an amount effective to alleviate one or more symptoms of the disease in the treated subject or population to induce regression of such symptoms or to inhibit the development of such symptoms to any clinically measurable degree. The amount of therapeutic agent effective to alleviate any particular disease symptom (also referred to as the "therapeutically effective amount") may vary depending on factors such as the disease state, age and weight of the subject, and the ability of the drug to produce a desired therapeutic effect in the subject. Whether a symptom of a disease has been alleviated or not can be assessed by using any clinical test method commonly used by physicians or other health care professionals to assess the severity or progression of the symptom. Although embodiments of the present disclosure (e.g., treatment methods or articles of manufacture) may be ineffective in alleviating the symptoms of each disease of interest, they shall alleviate the symptoms of the disease of interest in a statistically significant number of subjects as determined by any statistical test method known in the art, such as the Student's t-test, chi-square test, U-test by Mann and Whitney, Kruskal-Wallis test (H-test), Jonckheere-Terpstra test and Wilcoxon test.

"Conservative modifications" or "conservative replacements or substitutions" refer to the replacement of amino acids in a protein with other amino acids having similar characteristics (e.g., charge, side chain size, hydrophobicity/hydrophilicity, backbone chain conformation and rigidity) such that changes may be made frequently without altering the biological activity of the protein. It is known to those skilled in the art that, in general, the replacement of a single amino acid in a non-essential region of a polypeptide does not substantially alter the biological activity (see, for example, Watson et al., (1987) Molecular Biology of The Gene, The Benjamin/Cummings pub. Co., page 224, (4th edition)). In addition, replacement of structurally or functionally similar amino acids is unlikely to destroy biological activity. Exemplary conservative substitutions are set forth in the following table "Exemplary amino acid conservative substitutions". Exemplary amino acid conservative substitutions:
Original residue Conservative substitution
Ala (A) Gly; Ser
Arg (R) Lys; His
Asn (N) Gln; His; Asp
Asp (D) Glu; Asn
Cys (C) Ser; Ala; Val
Gln (Q) Asn; Glu
Glu (E) Asp; Gln
Gly (G) Ala
His (H) Asn; Gln; Lys; Arg
Ile (I) Leu; Val
Leu (L) Ile; Val
Lys (K) Arg; His
Met (M)Leu; Ile; Tyr
Phe (F) Tyr; Met; Leu
Pro (P) Ala
Ser (S) Thr
Thr (T) Ser
Trp (W) Tyr; Phe
Tyr (Y) Trp; Phe
Val (V) Ile; Leu.

The "effective amount" or "effective dose" refers to the amount of a drug, compound or pharmaceutical composition necessary to achieve any one or more beneficial or desired therapeutic results. For prophylactic use, beneficial or desired results include elimination or reduction of risk, lessening the severity, or delaying the onset of the condition, including biochemical, histological and/or behavioral symptoms of conditions, complications thereof and intermediate pathological phenotypes exhibited during the development of the condition. For therapeutic use, beneficial or desired results include clinical results, such as reducing the incidence of or ameliorating one or more symptoms of various target antigen-associated conditions of the present disclosure, reducing the dosage of other agents required to treat the conditions, enhancing the therapeutic effect of another agent, and/or delaying the progression of a target antigen-associated condition of the present disclosure in a subject.

"Exogenous" refers to a substance produced outside an organism, a cell or a human accordingly.

"Endogenous" refers to a substance produced in an organism, a cell or a human accordingly.

"Homology" or "identity" refers to sequence similarity between two polynucleotide sequences or between two polypeptides. When positions in both compared sequences are occupied by the same base or amino acid monomer subunit, e.g., if each position of two DNA molecules is occupied by adenine, then the molecules are homologous at that position. The percent homology between two sequences is a function of the number of pairing or homologous positions shared by the two sequences divided by the number of positions compared × 100%. For example, if there are 6 matches or homologies at 10 positions in two sequences when the sequences are optimally aligned, then the two sequences are 60% homologous; two sequences are 95% homologous if there are 95 matches or homologies at 100 positions in the two sequences. Typically, two sequences, when aligned, are compared to give the maximum percent homology. For example, the comparison may be made by the BLAST algorithm, wherein the parameters of the algorithm are selected to give the maximum match between the reference sequences over the entire length of each sequence. The following references relate to the BLAST algorithm often used for sequence analysis: the BLAST algorithms: Altschul, S.F. et al., (1990) J. Mol. Biol., 215: 403-410; Gish, W, et al., (1993) Nature Genet., 3: 266-272; Madden, T.L. et al., (1996) Meth. Enzymol., 266: 131-141; Altschul, S.F. et al., (1997) Nucleic Acids Res., 25: 3389-3402; Zhang, J. et al., (1997) Genome Res., 7: 649-656. Other conventional BLAST algorithms, such as one provided by NCBI BLAST, are also well known to those skilled in the art. In the present disclosure, for example, the "natural sequence numbering position relative to the XX sequence" includes both a position corresponding to the natural sequence number on the XX sequence and a position in the mutated sequence of the XX sequence at a position equivalent to the XX sequence, wherein the equivalent position can be obtained by aligning the mutated sequence of the XX sequence with the XX sequence to obtain the highest percent identity, and the position on the mutated sequence of the XX sequence corresponding to the XX sequence is the equivalent position.

As used herein, the expressions of "cells", "cell lines" and "cell cultures" can be used interchangeably, and all such designations include progeny. Thus, the words "transformant" and "transformed cell" include the primary subject cells and cultures derived therefrom, regardless of the number of metastases. It shall be further understood that all progeny may not be precisely identical in DNA content due to deliberate or inadvertent mutations. Mutated progeny having the same function or biological activity as screened for in the originally transformed cells are included. Where different names are intended, they are clear from the context.

"Polymerase chain reaction" or "PCR" as used herein refers to a procedure or technique in which minute amounts of a particular portion of nucleic acid, RNA and/or DNA are amplified as described, for example, in US4683195. In general, it is desirable to obtain sequence information from the terminus of or beyond the target region so that oligonucleotide primers can be designed; these primers are identical or similar in sequence to the corresponding chains of the template to be amplified. The 5'-terminal nucleotide of 2 primers may coincide with the terminus of the material to be amplified. PCR can be used to amplify specific RNA sequences, specific DNA sequences from total genomic DNA, and cDNA, phage or plasmid sequences transcribed from total cellular RNA, and the like. See generally Mullis et al., (1987) Cold Spring Harbor Symp. Ouant. Biol., 51: 263; Erlich ed., (1989) PCR TECHNOLOGY (Stockton Press, N.Y.). PCR, as used herein, is considered to be one example, but not the only example, of a nucleic acid polymerase reaction method for amplifying a nucleic acid test sample that comprises using a known nucleic acid as a primer and a nucleic acid polymerase to amplify or generate a specific portion of the nucleic acid.

The terms "conjugate" and "drug conjugate" refer to a novel drug formed by linking a ligand to a biologically active drug via a stable linking unit. For example, an "antibody drug conjugate" (ADC) is a drug formed by linking an antibody or antibody fragment to a biologically active drug via a linking unit. The antibody may be coupled to the drug directly or via a linker. The average number of drug moieties per antibody may range, for example, from about 0 to about 20 drug moieties per antibody, in some embodiments, from 1 to about 10 drug moieties per antibody, and in some embodiments, from 1 to about 8 drug moieties per antibody. For the conjugate disclosed herein, the average drug load per antibody is about 2 to about 5 or about 3 to about 4.

"Isolated" refers to a state of purification, and in this case means that the specified molecule is substantially free of other biomolecules, such as nucleic acids, proteins, lipids, carbohydrates or other materials, such as cell debris and growth media. Generally, the term "isolated" is not intended to refer to the complete absence of such materials or the absence of water, buffers or salts, unless they are present in amounts that significantly interfere with the experimental or therapeutic use of the compounds as described herein.

The term "optional" or "optionally" means that the event or circumstance subsequently described may, but not necessarily, occur, and that the description includes instances where the event or circumstance occurs or does not occur.

The term "pharmaceutical composition" refers to a mixture containing one or more of the compounds described herein or a physiologically/pharmaceutically acceptable salt or pro-drug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activities. The term "pharmaceutically acceptable carrier" refers to any inactive substance suitable for use in formulations for delivery of the antibody or antigen-binding fragment. The carrier may be an anti-adherent, a binder, a coating, a disintegrating agent, a filler or a diluent, a preservative (e.g., an antioxidant, an antibacterial agent or an antifungal agent), a sweetening agent, an absorption delaying agent, a wetting agent, an emulsifying agent, a buffer, or the like. Examples of suitable pharmaceutically acceptable carriers include water, ethanol, polyols (e.g., glycerol, propylene glycol and polyethylene glycol), dextrose, vegetable oils (e.g., olive oil), saline, buffers, buffered saline, and isotonic agents, such as sugars, polyols, sorbitol and sodium chloride.

In addition, the present disclosure comprises a pharmaceutical formulation for use in treating a disease or condition, e.g., an agent for treating a disease related to an antigen-positive cell of interest, wherein the formulation comprises the polypeptide complex, the multispecific polypeptide complex, the domain engineered antibody, the Fab fragment of a domain engineered antibody, the bispecific antibody, the pharmaceutical composition, the nucleic acid molecule or the multispecific antibody disclosed herein as an active ingredient.

The disease disclosed herein is not limited, for example, a therapeutic response induced using the molecules disclosed herein can be achieved by binding to a human antigen, then blocking the binding of the antigen to a receptor/ligand thereof, or killing tumor cells that overexpress the antigen.

In addition, the present disclosure relates to a method for immunodetection or assay of a target antigen, a reagent for use in immunodetection or assay of a target antigen, a method for immunodetection or assay of a cell expressing a target antigen, and a diagnostic agent for diagnosis of a disease related to a target antigen-positive cell, which comprises the polypeptide complex, the multispecific polypeptide complex, the domain engineered antibody, the Fab fragment of a domain engineered antibody, the bispecific antibody, the pharmaceutical composition, the nucleic acid molecule or the multispecific antibody disclosed herein as an active ingredient.

In the present disclosure, the method for detection or assay of an amount of a target antigen may be any known method. For example, it includes immunodetection or assay methods.

The immunodetection or assay method is a method for detection or assay of an amount of an antibody or an amount of an antigen using a labeled antigen or antibody. Examples of the immunodetection or assay methods include a radioactive substance-labeled immune antibody method (RIA), an enzyme immunoassay (EIA or ELISA), a fluorescence immunoassay (FIA), a luminescence immunoassay, a Western blotting method, physicochemical methods, and the like.

The above-mentioned diseases related to antigen-positive cells can be diagnosed by detection or assay of cells expressing an antigen with the antibody disclosed herein. For detection of cells expressing the polypeptide, known immunodetection methods can be used, and immunoprecipitation, fluorescent cell staining, immunohistological staining and other methods are preferably used. In addition, a fluorescent antibody staining method using FMAT8100HTS system (Applied Biosystem) and the like can be used.

In the present disclosure, the living sample for use in detection or assay of a target antigen is not particularly limited as long as it has a possibility of comprising cells expressing the target antigen, such as tissue cells, blood, plasma, serum, pancreatic juice, urine, feces, interstitial fluid or culture fluid.

The diagnostic agent comprising the antibody or the antibody fragment thereof disclosed herein may further comprise a reagent for use in performing an antigen-antibody reaction or a reagent for use in detection of a reaction, depending on the desired diagnostic method. The reagent for use in performing an antigen-antibody reaction include buffers, salts and the like. The reagent for use in detection includes reagents generally used in immunodetection or assay methods, such as a labeled second antibody recognizing the monoclonal antibody, an antibody fragment thereof or a binding substance thereof and a substrate corresponding to the label, and the like.

The details of one or more embodiments of the present disclosure are set forth in the specification above. Although any methods and materials similar or equivalent to those described herein can be used in the practice or testing of the present disclosure, the preferred methods and materials are described below. Other features, objects and advantages of the present disclosure will be apparent from the specification and the claims. In the specification and claims, the singular forms "a", "an" and "the" include plural referents unless the context clearly dictates otherwise. Unless defined otherwise, all technical and scientific terms used herein have the same general meaning as commonly understood by one of ordinary skill in the art to which the present disclosure belongs. All patents and publications cited in the specification are incorporated by reference. The following examples are set forth in order to more fully illustrate the preferred embodiments of the present disclosure. These examples should not be construed in any way as limiting the scope of the present disclosure.

The present invention is further described below with reference to examples, but these examples are not intended to limit the scope of the present invention. The experimental methods in the examples of the present invention, in which specific conditions are not specified, are generally performed under conventional conditions such as Cold Spring Harbor Antibody Technical Guide and Molecular Cloning Manual, or under conditions recommended by the manufacturer of the raw material or the goods. Reagents without specific sources indicated are conventional reagents purchased from the market. Although the antibodies used in the examples target specific antigens, those skilled in the art, in light of the teachings of the present disclosure, will appreciate that the technical effect is achieved independently of the specific CDR sequences and independently of the specific antigen sequences, but with the benefit of substitution of the Titin T chain/Obscurin-O chain, or the Titin T chain/Obscurin-like-O chain for CH1/CL to improve the mispairing between the heavy/light chains.

### Example 1: Preparation Method for Antibody or Polypeptide Protein

The method comprises: designing a primer PCR to build a gene fragment (such as an antibody VH/VK gene fragment), carrying out homologous recombination on the gene fragment and an expression vector (such as pHr (with a signal peptide and a constant region gene (such as CH1-Fc/CL) fragment)) to construct an expression vector (such as VH-CH1-Fc-pHr/VK-CL-pHr), introducing the constructed expression vector into a prokaryote or a eukaryote for expression, and finally purifying a product to obtain the required antibody or polypeptide protein. Without limitation, the antibody constant region may be selected from the group consisting of a light chain constant region of a human κ and λ chain and a heavy chain constant region selected from the group consisting of IgG1, IgG2, IgG3 and IgG4. Non-limiting examples further include the optimization design of human antibody constant regions, such as mutations on positions L234A/L235A or L234F/L235E of the heavy chain constant region. Illustratively, antibody light/heavy chain constant region sequences are as follows:
> IgG1 heavy chain constant region (abbreviated as hIgG1): 






 (SEQ ID NO: 1);
 Note: in the sequence, the single underlined part is CH1, the dotted underlined part is CH2, and the italicized part is CH3.
> knob-IgG1 heavy chain constant region (abbreviated as knob-IgGl): ASTKGPSVFPLAPSSKSTSGGTAALGCLVKDYFPEPVTVSWNSGALTSGVHTFP 
AVLQSSGLYSLSSVVTVPSSSLGTQTYICNVNHKPSNTKVDKKVEPKSCDKTHT
CPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNK
ALPAPIEKTISKAKGQPREPQVYTLPPCREEMTKNQVSLWCLVKGFYPSDIAVEW
ESNGQPENNYKTTPPVLDSDGSFFLYSKLTVDKSRWQQGNVFSCSVMHEALHNHYT
QKSLSLSPGK (SEQ ID NO: 2);
 Note: in the sequence, the single underlined part is CH1, the dotted underlined part is CH2, and the italicized part is CH3.
> hole-IgG1 heavy chain constant region (abbreviated as hole-IgGl): 
ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHT
CPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNK
ALPAPIEKTISKAKGQPREPQVCTLPPSREEMTKNQVSLSCAVKGFYPSDIAVEWE
SNGQPENNYKTTPPVLDSDGSFFLVSKLTVDKSRWQQGNVFSCSVMHEALHNHYTQ
KSLSLSPGK (SEQ ID NO: 3);
 Note: in the sequence, the single underlined part is CH1, the dotted underlined part is CH2, and the italicized part is CH3.
> kappa light chain constant region (abbreviated as kappa or hκ): 


(SEQ ID NO: 4).
> lambda light chain constant region (abbreviated as lambda or hλ):

GQPKAAPSVTLFPPSSEELQANKATLVCLISDFYPGAVTVAWKADSSPVKAGVE
TTTPSKQSNNKYAASSYLSLTPEQWKSHRSYSCQVTHEGSTVEKTVAPTECS
(SEQ ID NO: 5).

The amino acid sequences of the light and heavy chain variable regions of antibodies against various antigens such as B7H3 and CD3 mentioned in the examples or test examples of the present disclosure are as follows:
> VH amino acid sequence of F0 antibody: 
QVQLVQSGGGVVQPGTSLRLSCAASGFIFSSSAMHWVRQAPGKGLEWVAVISY
DGSNKYYVDSVKGRFTISRDNSKNTLYLQMNSLRAEDTAVYYCARSARLYASF
DYWGQGALVTVSS (SEQ ID NO: 6);
> VL amino acid sequence of F0 antibody: 
DTVVTQEPSFSVSPGGTVTLTCGLSSGSVSTSHYPSWYQQTPGQAPRMLIYNTN
TRSSGVPDRFSGSILGNKAALTITGAQADDESDYYCAIHVDRDIWVFGGGTKLT
VL (SEQ ID NO: 7);
> VH amino acid sequence of N0 antibody: 
DVQLVQSGGGLVQPGGSLRLSCAGSGFTSSLSWMSWLRQAPGKGLEWVANIN
QDGSEKNYVDSVKGRFTISRDNAQNSLYLQMSSLRAEDTAVYYCARGLWTFD
SWGQGTLLTVSP (SEQ ID NO: 8);
> VL amino acid sequence of N0 antibody: 
EIVLTQSPGTLSLSPGERATLSCRASQNINNRFLAWYQQRPGQAPRLLIYAASSR
ATGVPDRFSGSGSGTDFTLTISRLEAEDFAMYHCQQYGSFPRTFGQGTMVDIK
(SEQ ID NO: 9);
> VH amino acid sequence of S0 antibody: 


 (SEQ ID NO: 10);
> VL amino acid sequence of S0 antibody: 


(SEQ ID NO: 11);
> VH amino acid sequence of V0 antibody: 
QVQLQQSAAELARPGASVKMSCKASGYSFTSYTIHWVKQRPGQGLEWIGYINP
NSRNTDYNQKFKDETTLTADRSSSTAYMQLISLTSEDSAVYYCARYSGSTPYW
YFDVWGAGTTVTVSS (SEQ ID NO: 12);
> VL amino acid sequence of V0 antibody: 
QIVLTQSPAILSASPGEKVTMTCRASSSVSYMNWYQQKPGSSPKPWIYATSNLA
SGVPARFSVSVSGTSHSLTISRVEAEDAATYYCQQWSSNPLTFGAGTKLELK
(SEQ ID NO: 13);
> VH amino acid sequence of J0 antibody: 
EVQLVESGGGLVKPGGSLRLSCAASGFTFSDYWMHWVRQAPGKGLEWVGHIK
SKTDAGTTDYAAPVKGRFTISRDDSKNTLYLQMNSLKTEDTAVYYCAREIYYY
AFDSWGQGTLVTVSS (SEQ ID NO: 14);
> VL amino acid sequence of J0 antibody: 
SYELTQPLSVSVALGQTARITCSGDNIGSKYVHWYQQKPGQAPVLVIYGDNERP
SGIPERFSGSNSGNTATLTISRAQAGDEADYYCQAADWVDFYVFGGGTKLTVL
(SEQ ID NO: 15);
> VH amino acid sequence of H0 antibody: 
EVQLVESGGGLVQPGGSLRLSCAASGFTFSHYYMAWVRQAPGKGLEWVTSISY
EGDITYYGDSVKGRFTISRDNSKNTLYLQMNSLRAEDTATYYCASQTLRESFDY
WGQGTLVTVSS (SEQ ID NO: 16);
> VL amino acid sequence of H0 antibody: 
DIQMTQSPSSVSASVGDRVTITCRASQDIANYLSWYQQKPGKSPKLLIYGTSNLE
VGVPSRFSGSRSGTDFTLTISSLQPEDFATYYCLQDKEFPRTFGGGTKVEIK (SEQ
ID NO: 17);
> VH amino acid sequence of R0 antibody: 


 (SEQ ID NO: 18);
> VL amino acid sequence of R0 antibody: 


(SEQ ID NO: 19);
> VH amino acid sequence of Bmab (abbreviated as B0) antibody: 
QVQLQQSGAEVKKPGSSVRVSCKASGGTFNNNAINWVRQAPGQGLEWMGGII
PMFGTAKYSQNFQGRVAITADESTGTASMELSSLRSEDTAVYYCARSRDLLLFP
HHALSPWGRGTMVTVSS (SEQ ID NO: 20);
> VL amino acid sequence of Bmab (abbreviated as B0) antibody: 
SSELTQDPAVSVALGQTVRVTCQGDSLRSYYASWYQQKPGQAPVLVIYGKNN
RPSGIPDRFSGSSSGNTASLTITGAQAEDEADYYCSSRDSSGNHWVFGGGTELT
VL (SEQ ID NO: 21);
> VH amino acid sequence of Umab (abbreviated as U0) antibody: 
EVQLVQSGAEVKKPGESLKISCKGSGYSFTTYWLGWVRQMPGKGLDWIGIMSP
VDSDIRYSPSFQGQVTMSVDKSITTAYLQWNSLKASDTAMYYCARRRPGQGYF
DFWGQGTLVTVSS (SEQ ID NO: 22);
> VL amino acid sequence of Umab (abbreviated as U0) antibody: 
DIQMTQSPSSLSASVGDRVTITCRASQGISSWLAWYQQKPEKAPKSLIYAASSLQ
SGVPSRFSGSGSGTDFTLTISSLQPEDFATYYCQQYNIYPYTFGQGTKLEIK (SEQ
ID NO: 23);
> VH amino acid sequence of Dmab (abbreviated as D0) antibody: 
EVQLLESGGGLVQPGGSLRLSCAASGFTFSSYAMSWVRQAPGKGLEWVSGITG
SGGSTYYADSVKGRFTISRDNSKNTLYLQMNSLRAEDTAVYYCAKDPGTTVIM
SWFDPWGQGTLVTVSS (SEQ ID NO: 24);
> VL amino acid sequence of Dmab (abbreviated as D0) antibody: 
EIVLTQSPGTLSLSPGERATLSCRASQSVRGRYLAWYQQKPGQAPRLLIYGASS
RATGIPDRFSGSGSGTDFTLTISRLEPEDFAVFYCQQYGSSPRTFGQGTKVEIK
(SEQ ID NO: 25);
> VH amino acid sequence of Tmab (abbreviated as I0) antibody: 


 (SEQ ID NO: 26);
> VL amino acid sequence of Tmab (abbreviated as I0) antibody: 

 (SEQ
ID NO: 27);
> VH amino acid sequence of C0 antibody: 
EVQLVQSGAEVKKPGASVKVSCKASGYTFTDYHMNWVRQAPGQRLEWMGDI
NPDIGGTSYNQNFKGRVTITRDTSASTAYMELSSLRSEDTAVYYCARWDFDSFA
NWGQGTLVTVSS (SEQ ID NO: 28);
> VL amino acid sequence of C0 antibody: DIVMTQSPDSLAVSLGERATINCRASESVSIIGTNLIHWYQQKPGQPPKLLIYHAS 
NLETGVPDRFSGSGSGTDFTLTISSLQAEDVAVYYCLQSRKIPYTFGGGTKVEIK
(SEQ ID NO: 29);
> VH amino acid sequence of A0 antibody: 
EVQLVESGGGLVQPGGSLKLSCAASGFTFNKYAMNWVRQAPGKGLEWVARIR
SKYNNYATYYADSVKDRFTISRDDSKNTAYLQMNNLKTEDTAVYYCVRHGNF
GNEYISYWAYWGQGTLVTVSS (SEQ ID NO: 105);
> VL amino acid sequence of A0 antibody:

QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVL (SEQ ID NO: 106);

The heavy chain constant region sequences of the antibodies F0, N0, S0, V0, J0, H0, R0, B0, U0, D0, I0, C0 and A0 are IgG1 heavy chain constant regions (SEQ ID NO: 1), and the light chain constant region sequences of the antibodies N0, S0, H0, R0, U0, I0 and C0 are kappa light chain constant regions (SEQ ID NO: 4); the light chain constant region sequences of the antibodies F0, V0, J0, B0, D0 and A0 are lambda light chain constant regions (SEQ ID NO: 5).

In addition, the sequence of hB7H3 antigen protein is as follows: 
LEVQVPEDPVVALVGTDATLCCSFSPEPGFSLAQLNLIWQLTDTKQLVHSFAEG
QDQGSAYANRTALFPDLLAQGNASLRLQRVRVADEGSFTCFVSIRDFGSAAVS
LQVAAPYSKPSMTLEPNKDLRPGDTVTITCSSYQGYPEAEVFWQDGQGVPLTG
NVTTSQMANEQGLFDVHSILRVVLGANGTYSCLVRNPVLQQDAHSSVTITPQR
SPTGAVEVQVPEDPVVALVGTDATLRCSFSPEPGFSLAQLNLIWQLTDTKQLVH
SFTEGRDQGSAYANRTALFPDLLAQGNASLRLQRVRVADEGSFTCFVSIRDFGS
AAVSLQVAAPYSKPSMTLEPNKDLRPGDTVTITCSSYRGYPEAEVFWQDGQGV
PLTGNVTTSQMANEQGLFDVHSVLRVVLGANGTYSCLVRNPVLQQDAHGSVTI
TGQPMT DYKDDDDKHHHHHH (SEQ ID NO: 111);
 the hCD3 antigen protein is a heterodimer consisting of a δ subunit and an ε subunit of hCD3 antigen, wherein the sequence of the δ subunit of hCD3 antigen is as follows: 
FKIPIEELEDRVFVNCNTSITWVEGTVGTLLSDITRLDLGKRILDPRGIYRCNGTD
IYKDKESTVQVHYRMCQSCVELDPATVASAENAQCEKELQALEKENAQLEWE
LQALEKELAQDYKDDDDK (SEQ ID NO: 112);
 the sequence of the ε subunit of hCD3 antigen is as follows:

DGNEEMGGITQTPYKVSISGTTVILTCPQYPGSEILWQHNDKNIGGDEDDKNIGS
DEDHLSLKEFSELEQSGYYVCYPRGSKPEDANFYLYLRARVCENCMEMDAKN
AQCKKKLQALKKKNAQLKWKLQALKKKLAQHHHHHH (SEQ ID NO: 113).

### Example 2: Substitution of Antibody CH1/CL Domain

CH1/CL domain engineered antibodies were obtained by substituting the CH1/CL domain of the antibody with the Ig-like domains of the natural Titin/Obscurin complex, Titin/Obscurin-like O complex and IL6Ra/IL6Rb complex, which possess natural intermolecular interactions, respectively.

In the IL6Ra/IL6Rb complex, the Ig-like domain in IL6Ra protein for substituting the antibody CH1 or CL is IL6Ra.0 chain, and the Ig-like domain in IL6Rb protein for substituting antibody CL or CH1 is IL6Rb.0 chain; in the Titin/Obscurin complex, the Ig-like domain (Titin Ig-like 152 domain) in the Titin protein used for substituting antibody CH1 or CL is T.0 chain, and the Ig-like domain (Obscurin Ig-like-1 domain) in the Obscurin protein used for substituting antibody CL or CH1 is O.0 chain; in the Titin/Obscurin-like O complex, the Ig-like domain (Obscurin-like-Ig-like-1 domain) in the Obscurin-like protein used for substituting antibody CH1 or CL is OL.0 chain, and the Ig-like domain (Titin Ig-like 152 domain) in the Titin protein used for substituting antibody CL or CH1 is T.0 chain, wherein:
the > IL6Ra.0 chain sequence is: 
GILQPDPPANITVTAVARNPRWLSVTWQDPHSWNSSFYRLRFELRYRAERSKTF
TTWMVKDLQHHCVIHDAWSGLRHVVQLRAQEEFGQGEWSEWSPEAMGTPW
(SEQ ID NO: 30);
the > IL6Rb.0 chain sequence is: 
FDPVYKVKPNPPHNLSVINSEELSSILKLTWTNPSIKSVIILKYNIQYRTKDASTW
SQIPPEDTASTRSSFTVQDLKPFTEYVFRIRCMKEDGKGYWSDWSEEASGIT
(SEQ ID NO: 31);
the > T.0 chain sequence is: 
GIPPKIEALPSDISIDEGKVLTVACAFTGEPTPEVTWSCGGRKIHSQEQGRFHIEN
TDDLTTLIIMDVQKQDGGLYTLSLGNEFGSDSATVNIHIRSI (SEQ ID NO: 32);
the > O.0 chain sequence is: 
SGAPRFLTRPKAFVVSVGKDATLSCQIVGNPTPQVSWEKDQQPVAAGARFRLA
QDGDLYRLTILDLALGDSGQYVCRARNAIGEAFAAVGLQVDAEA (SEQ ID NO:
33);
the > OL.0 chain sequence is:

QGSPPCFLRFPRPVRVVSGAEAELKCVVLGEPPPVVVWEKGGQQLAASERLSFP
ADGAEHGLLLTAALPTDAGVYVCRARNAAGEAYAAAAVTVLEPP (SEQ ID
NO: 34);

A plurality of antibodies with substituted CH1/CL were constructed, and the purity and the binding activity to the corresponding antigen of the antibodies to be tested were detected according to the methods of Test Examples 2 and 4. The experimental results are shown in Table 1 below, and the experimental results showed that, after CH1/CL of antibodies such as F0, V0, S0, N0, J0, H0, R0 and C0 which bound to different antigens were simultaneously substituted with T.0 chain/O.0 chain or T.0 chain/OL.0 chain, the antibodies still maintained good binding activity and had high antibody purity.

**Table 1. Purity and affinity of antibodies with substituted CH1/CL domain**

| Name of antibody | | Antibody Domain substitution | | Purity of antibody SEC (%) | Binding assay to the corresponding antigen EC50 (nM) |
|---|---|---|---|---|---|
| | | Substitution of heavy chain CH1 | Substitution of light chain CL | | |
| Substitution | F0 | CH1 | CL981.29 | | |
| of CH1/CL of F0 | F1 | IL6Rb.0 | IL6Ra.0 | 47 | 71.6 |
| | F2 | IL6Ra.0 | IL6Rb.0 | 67 | 59.28 |
| | F3 | T.0 | O.0 | 100 | 2.83 |
| | F4 | T.0 | OL.0 | 100 | 2.17 |
| Substitution of CH1/CL of V0 | V0 | CH1 | CL86 | | 1.8 |
| | V1 | IL6Rb.0 | IL6Ra.0 | 38 | 38.4 |
| | V2 | IL6Ra.0 | IL6Rb.0 | NA | - |
| | V3 | T.0 | O.0 | 99 | 3.6 |
| | V4 | T.0 | OL.0 | 97 | 2.9 |
| | V5 | O.0 | T.0 | 98 | 3.2 |
| | V6 | OL.0 | T.0 | 97 | 4.7 |
| Substitution of CH1/CL of S0 | S0 | CH1 | CL971.01 | | |
| | S1 | IL6Rb.0 | IL6Ra.0 | NA | - |
| | S2 | IL6Ra.0 | IL6Rb.0 | NA | - |
| | S3 | T.0 | OL.0 | 83 | 3.5 |
| | S4 | OL.0 | T.0 | 90 | 1.96 |
| Substitution of CH1/CL of N0 | N0 | CH1 | CL990.82 | | |
| | N1 | IL6Rb.0 | IL6Ra.0 | 100 | 1229 |
| | N2 | IL6Ra.0 | IL6Rb.0 | 67 | 766.5 |
| | N3 | T.0 | O.0 | 90 | 6.45 |
| | N4 | T.0 | OL.0 | 83 | 5.93 |
| | N5 | O.0 | T.0 | 90 | 5.24 |
| Substitution of CH1/CL of J0 | J0 | CH1 | CL950.08 | | |
| | J1 | IL6Rb.0 | IL6Ra.0 | NA | - |
| | J2 | IL6Ra.0 | IL6Rb.0 | NA | 179 |
| | J3 | OL.0 | T.0 | 93 | 0.25 |
| Substitution of CH1/CL of H0 | H0 | CH1 | CL | NA | 1.74 |
| | H1 | IL6Rb.0 | IL6Ra.0 | 75 | 19.35 |
| | H2 | IL6Ra.0 | IL6Rb.0 | 80 | 24.38 |
| | H3 | T.0 | O.0 | 95 | 2.753 |
| | H4 | T.0 | OL.0 | 95 | 2.702 |
| Substitution of CH1/CL of R0 | R0 | CH1 | CL | NA | 1.75 |
| | R1 | IL6Rb.0 | IL6Ra.0 | 75 | 24.45 |
| | R2 | IL6Ra.0 | IL6Rb.0 | 60 | 18.4 |
| | R3 | O.0 | T.0 | 98 | 1.576 |
| | R4 | OL.0 | T.0 | 98 | 1.667 |
| | R5 | T.0 | O.0 | 98 | 1.589 |
| | R6 | T.0 | OL.0 | 97 | 2.016 |
| Substitution of CH1/CL of C0 | C0 | CH1 | CL965.81 | | |
| | C1 | O.0 | T.0 | 95 | 17.7 |
| | C2 | OL.0 | T.0 | 99 | 16.48 |

| | | | | | |
|---|---|---|---|---|---|
| Note: For example, F1 indicates that the heavy chain CH1 of the F0 antibody was substituted with the IL6Rb.0 chain (SEQ ID NO: 31) and the light chain CL was substituted with the IL6Ra.0 chain (SEQ ID NO: 30), and the other portions remained the same as those of F0, and so on. In the table, "-" indicates no detection, and SEC indicates the purity of the antibody detected by size exclusion chromatography (NA indicates no detection due to underexpression). | | | | | |

### Example 3: Optimization Design of CH1/CL Domain Engineered Antibody

Individual amino acids of the Titin Ig-like 152 domain (T.0 chain), the Obscurin Ig-like 1 domain (O.0 chain) and the Obscurin-like Ig-like 1 domain (OL.0 chain) were mutated, including insertion, deletion or substitution of amino acids, to verify the activity of the domain engineered antibody.

Illustratively, firstly, inter-chain disulfide bonds were increased by mutation on a small number of amino acids on the T.0 chain, the O.0 chain and the OL.0 chains; secondly, other individual amino acids in the domain were also mutated, for example, amino acid mutations on positions 7, 62 and 11 of the Obscurin Ig-like 1 domain; amino acids were increased or decreased at the N-terminus of the Titin Ig-like 152 domain (e.g., 5 "KAGIR" amino acids of the wild-type Titin protein immediately adjacent to the N-terminus of the Titin Ig-like 152 domain were added); amino acids were increased or decreased at the N-terminus of the Obscurin Ig-like 1 domain (e.g., 5 "DQPQF" amino acids of the wild-type Obscurin protein immediately adjacent to the N-terminus of the Obscurin Ig-like 1 domain were added); in addition, a linker sequence was added at the N-terminus or the C-terminus of the Titin Ig-like 152 domain, the Obscurin Ig-like 1 domain or the Obscurin-like Ig-like 1 domain (e.g., a "G₄S" or "(G₄S)₂" linker sequence was added at the N-terminus), and the C-terminus of the antibody VH or VL was linked to the Titin Ig-like 152 domain, the Obscurin Ig-like 1 domain or the Obscurin-like Ig-like 1 domain via the linker sequence. The optimization design of the specific domain is shown in Table 2.

**Table 2. Amino acid mutations of T.0 chain, O.0 chain and OL.0 chain**

| Titin-T chain | | Obscurin-O chain | | Obscurin-like-O chain | |
|---|---|---|---|---|---|
| Name | Mode of mutation | Name | Mode of mutation | Name | Mode of mutation |
| T.0 | Wild type (SEQ ID NO: 32) | O.0 | Wild type (SEQ ID NO: 33) | OL.0 | Wild type (SEQ ID NO: 34) |
| T.1 | C25S, C39T, A8C | O.1 | A88C | OL.1 | C6E, V74C |
| T.2 | C25S, C39T, V20C | O.2 | A3C | OL.2 | C6E, G84C |
| T.3 | C25S, C39T, A26C | O.3 | R9C | OL.3 | C6E, A86C |
| T.4 | C25S, C39T, T22C | O.4 | C25S, C76S, A88C | OL.4 | C6E, C26S, C77S, V74C |
| T.5 | C25S, C39T, A8C +Titin_KAGIR | O.5 | C25S, C76S, A3C | OL.5 | C6E, C26S, C77S, G84C |
| T.6 | C25S, C39T, A8C + (G₄S)₁ linker sequence | O.6 | C25S, C76S, R9C | OL.6 | C6E, C26S, C77S, A86C |
| T.7 | C25S, C39T, A8C + (G₄S)₂ linker sequence | O.7 | C25S, C76S, A88C, L7K, T62K | | |
| | | O.8 | C25S, C76S, A88C, L7K, T62H | | |
| | | O.9 | C25S, C76S, A88C, K11L, T62K | | |
| | | O.10 | C25S, C76S, A88C, K11L, T62H | | |
| | | O.11 | C25S, C76S, A88C+Obscurin_ DQP QF | | |
| | | O.12 | C25S, C76S, A88C, L7K, T62K+Obscurin_ DQP QF | | |
| | | 0.13 | C25S, C76S, A88C, L7K, T62H+Obscurin_ DQP QF | | |
| | | O.14 | C25S, C76S, A88C, L7R, T62K+Obscurin_ DQP QF | | |
| | | O.15 | C25S, C76S, A88C, L7R, T62H+Obscurin_ DQP QF | | |
| | | O.16 | C25S, C76S, A88C + (G₄S)₁ linker sequence | | |
| | | O.17 | C25S, C76S, A88C + (G₄S)₂ linker sequence | | |

| | | | | | |
|---|---|---|---|---|---|
| Note: The O.1 mode of mutation "A88C" in the Obscurin-O chain in the table indicates that the 88^{th} amino acid residue of O.0 (SEQ ID NO: 33) sequence was mutated from A to C; the O.11 mode of mutation "C25S, C76S, A88C + Obscurin DQPQF" indicates that O.0 (SEQ ID NO: 33) sequence was subjected to C25S, C76S and A88C amino acid mutations, and 5 "DQPQF" amino acids were added at the N-terminus, and so on. | | | | | |

The sequences of the Titin-T chain, the Obscurin-O chain and the Obscurin-like-O chain after mutations on partial amino acids are as follows:
> T.1 (T.0 has C25S, C39T and A8C mutations) 
GIPPKIECLPSDISIDEGKVLTVASAFTGEPTPEVTWSTGGRKIHSQEQGRFHIENT
DDLTTLIIMDVQKQDGGLYTLSLGNEFGSDSATVNIHIRSI (SEQ ID NO: 35);
> T.2 (T.0 has C25S, C39T and V20C mutations) 
GIPPKIEALPSDISIDEGKCLTVASAFTGEPTPEVTWSTGGRKIHSQEQGRFHIENT
DDLTTLIIMDVQKQDGGLYTLSLGNEFGSDSATVNIHIRSI (SEQ ID NO: 36);
> T.3 (T.0 has C25S, C39T and A26C mutations) 
GIPPKIEALPSDISIDEGKVLTVASCFTGEPTPEVTWSTGGRKIHSQEQGRFHIENT
DDLTTLIIMDVQKQDGGLYTLSLGNEFGSDSATVNIHIRSI (SEQ ID NO: 37);
> T.4 (T.0 has C25S, C39T and T22C mutations) 
GIPPKIEALPSDISIDEGKVLCVASAFTGEPTPEVTWSTGGRKIHSQEQGRFHIEN
TDDLTTLIIMDVQKQDGGLYTLSLGNEFGSDSATVNIHIRSI (SEQ ID NO: 38);
> T.5 (T.0 has C25S, C39T and A8C mutations; KAGIR is added at the N-terminus) 
KAGIRGIPPKIECLPSDISIDEGKVLTVASAFTGEPTPEVTWSTGGRKIHSQEQGR
FHIENTDDLTTLIIMDVQKQDGGLYTLSLGNEFGSDSATVNIHIRSI (SEQ ID NO:
39);
> T.6 (T.0 has C25S, C39T and A8C mutations; (G₄S)₁ linker is added at the N-terminus) 
GGGGSGIPPKIECLPSDISIDEGKVLTVASAFTGEPTPEVTWSTGGRKIHSQEQGR
FHIENTDDLTTLIIMDVQKQDGGLYTLSLGNEFGSDSATVNIHIRSI (SEQ ID NO:
40);
> T.7 (T.0 has C25S, C39T and A8C mutations; (G₄S)₂ linker is added at the N-terminus) 
GGGGSGGGGSGIPPKIECLPSDISIDEGKVLTVASAFTGEPTPEVTWSTGGRKIHS
QEQGRFHIENTDDLTTLIIMDVQKQDGGLYTLSLGNEFGSDSATVNIHIRSI (SEQ
ID NO: 41);
> O.1 (O.0 has A88C mutation) 
SGAPRFLTRPKAFVVSVGKDATLSCQIVGNPTPQVSWEKDQQPVAAGARFRLA
QDGDLYRLTILDLALGDSGQYVCRARNAIGEAFACVGLQVDAEA (SEQ ID NO:
42);
> O.2 (O.0 has A3C mutation) 
SGCPRFLTRPKAFVVSVGKDATLSCQIVGNPTPQVSWEKDQQPVAAGARFRLA
QDGDLYRLTILDLALGDSGQYVCRARNAIGEAFAAVGLQVDAEA (SEQ ID NO:
43);
> O.3 (O.0 has R9C mutation) 
SGAPRFLTCPKAFVVSVGKDATLSCQIVGNPTPQVSWEKDQQPVAAGARFRLA
QDGDLYRLTILDLALGDSGQYVCRARNAIGEAFAAVGLQVDAEA (SEQ ID NO:
44);
> O.4 (O.0 has C25S, C76S and A88C mutations) 
SGAPRFLTRPKAFVVSVGKDATLSSQIVGNPTPQVSWEKDQQPVAAGARFRLA
QDGDLYRLTILDLALGDSGQYVSRARNAIGEAFACVGLQVDAEA (SEQ ID NO:
45);
> O.5 (O.0 has C25S, C76S and A3C mutations) 
SGCPRFLTRPKAFVVSVGKDATLSSQIVGNPTPQVSWEKDQQPVAAGARFRLA
QDGDLYRLTILDLALGDSGQYVSRARNAIGEAFAAVGLQVDAEA (SEQ ID NO:
46);
> O.6 (O.0 has C25S, C76S and R9C mutations) 
SGAPRFLTCPKAFVVSVGKDATLSSQIVGNPTPQVSWEKDQQPVAAGARFRLA
QDGDLYRLTILDLALGDSGQYVSRARNAIGEAFAAVGLQVDAEA (SEQ ID NO:
47);
> O.7 (O.0 has C25S, C76S, A88C, L7K and T62K mutations) 
SGAPRFKTRPKAFVVSVGKDATLSSQIVGNPTPQVSWEKDQQPVAAGARFRLA
QDGDLYRLKILDLALGDSGQYVSRARNAIGEAFACVGLQVDAEA (SEQ ID NO:
48);
> O.8 (O.0 has C25S, C76S, A88C, L7K and T62H mutations) 
SGAPRFKTRPKAFVVSVGKDATLSSQIVGNPTPQVSWEKDQQPVAAGARFRLA
QDGDLYRLHILDLALGDSGQYVSRARNAIGEAFACVGLQVDAEA (SEQ ID NO:
49);
> O.9 (O.0 has C25S, C76S, A88C, K11L and T62K mutations) 
SGAPRFLTRPLAFVVSVGKDATLSSQIVGNPTPQVSWEKDQQPVAAGARFRLA
QDGDLYRLKILDLALGDSGQYVSRARNAIGEAFACVGLQVDAEA (SEQ ID NO:
50);
> O.10 (O.0 has C25S, C76S, A88C, K11L and T62H mutations) 
SGAPRFLTRPLAFVVSVGKDATLSSQIVGNPTPQVSWEKDQQPVAAGARFRLA
QDGDLYRLHILDLALGDSGQYVSRARNAIGEAFACVGLQVDAEA (SEQ ID NO:
51);
> O.11 (O.0 has C25S, C76S and A88C mutations; DQPQF is added at the N-terminus) 
DQPQFSGAPRFLTRPKAFVVSVGKDATLSSQIVGNPTPQVSWEKDQQPVAAGA
RFRLAQDGDLYRLTILDLALGDSGQYVSRARNAIGEAFACVGLQVDAEA (SEQ
ID NO: 52);
> 0.12 (O.0 has C25S, C76S, A88C, L7K and T62K mutations; DQPQF is added at the N-terminus) 
DQPQFSGAPRFKTRPKAFVVSVGKDATLSSQIVGNPTPQVSWEKDQQPVAAGA
RFRLAQDGDLYRLKILDLALGDSGQYVSRARNAIGEAFACVGLQVDAEA (SEQ
ID NO: 53);
> 0.13 (O.0 has C25S, C76S, A88C, L7K and T62H mutations; DQPQF is added at the N-terminus) 
DQPQFSGAPRFKTRPKAFVVSVGKDATLSSQIVGNPTPQVSWEKDQQPVAAGA
RFRLAQDGDLYRLHILDLALGDSGQYVSRARNAIGEAFACVGLQVDAEA (SEQ
ID NO: 54);
> 0.14 (O.0 has C25S, C76S, A88C, L7R and T62K mutations; DQPQF is added at the N-terminus) 
DQPQFSGAPRFRTRPKAFVVSVGKDATLSSQIVGNPTPQVSWEKDQQPVAAGA
RFRLAQDGDLYRLKILDLALGDSGQYVSRARNAIGEAFACVGLQVDAEA (SEQ
ID NO: 55);
> 0.15 (O.0 has C25S, C76S, A88C, L7R and T62H mutations; DQPQF is added at the N-terminus) 
DQPQFSGAPRFRTRPKAFVVSVGKDATLSSQIVGNPTPQVSWEKDQQPVAAGA
RFRLAQDGDLYRLHILDLALGDSGQYVSRARNAIGEAFACVGLQVDAEA (SEQ
ID NO: 56);
> 0.16 (O.0 has C25S, C76S and A88C mutations; (G₄S)₁ linker is added at the N-terminus) 
GGGGSSGAPRFLTRPKAFVVSVGKDATLSSQIVGNPTPQVSWEKDQQPVAAGA
RFRLAQDGDLYRLTILDLALGDSGQYVSRARNAIGEAFACVGLQVDAEA (SEQ
ID NO: 57);
> 0.17 (O.0 has C25S, C76S and A88C mutations; (G₄S)₂ linker is added at the N-terminus) 
GGGGSGGGGSSGAPRFLTRPKAFVVSVGKDATLSSQIVGNPTPQVSWEKDQQP
VAAGARFRLAQDGDLYRLTILDLALGDSGQYVSRARNAIGEAFACVGLQVDAE
 A (SEQ ID NO: 58);
> OL.1 (OL.0 has C6E and V74C mutations) 
QGSPPEFLRFPRPVRVVSGAEAELKCVVLGEPPPVVVWEKGGQQLAASERLSFP
ADGAEHGLLLTAALPTDAGCYVCRARNAAGEAYAAAAVTVLEPP (SEQ ID
NO: 59);
> OL.2 (OL.0 has C6E and G84C mutations) 
QGSPPEFLRFPRPVRVVSGAEAELKCVVLGEPPPVVVWEKGGQQLAASERLSFP
ADGAEHGLLLTAALPTDAGVYVCRARNAACEAYAAAAVTVLEPP (SEQ ID
NO: 60);
> OL.3 (OL.0 has C6E and A86C mutations) 
QGSPPEFLRFPRPVRVVSGAEAELKCVVLGEPPPVVVWEKGGQQLAASERLSFP
ADGAEHGLLLTAALPTDAGVYVCRARNAAGECYAAAAVTVLEPP (SEQ ID
NO: 61);
> OL.4 (OL.0 has C6E, C26S, C77S and V74C mutations) 
QGSPPEFLRFPRPVRVVSGAEAELKSVVLGEPPPVVVWEKGGQQLAASERLSFP
ADGAEHGLLLTAALPTDAGCYVSRARNAAGEAYAAAAVTVLEPP (SEQ ID
NO: 62);
> OL.5 (OL.0 has C6E, C26S, C77S and G84C mutations) 
QGSPPEFLRFPRPVRVVSGAEAELKSVVLGEPPPVVVWEKGGQQLAASERLSFP
ADGAEHGLLLTAALPTDAGVYVSRARNAACEAYAAAAVTVLEPP (SEQ ID
NO: 63);
> OL.6 (OL.0 has C6E, C26S, C77S and A86C mutations)

QGSPPEFLRFPRPVRVVSGAEAELKSVVLGEPPPVVVWEKGGQQLAASERLSFP
ADGAEHGLLLTAALPTDAGVYVSRARNAAGECYAAAAVTVLEPP (SEQ ID
NO: 64).

A plurality of antibodies with substituted CH1/CL were constructed by utilizing the mutated or domain-engineered Titin-T chain, Obscurin-O chain and Obscurin-like-O chain, the binding activity of each optimized mutated antibody and the corresponding antigen was detected by using the method of Test Example 4 disclosed herein, and the experimental results are shown in Table 3. The results showed that the disulfide-bond-modified antibody maintained good antigen binding activity.

**Table 3. Affinity of antibodies engineered with CH1/CL domain**

| Name of antibody | | Antibody Domain substitution | | Binding assay to the corresponding antigen EC50 (nM) |
|---|---|---|---|---|
| | | Substitution of heavy chain CH1 | Substitution of light chain CL | |
| Substitution of CH1/CL of F0 | F3 | T.0 | O.0 | 2.64 |
| | F5 | T.1 | O.1 | 1.21 |
| | F6 | T.2 | O.2 | 2.71 |
| | F7 | T.3 | O.3 | 3.38 |
| | F8 | T.1 | O.4 | 3.14 |
| | F9 | T.2 | O.5 | 3.52 |
| | F10 | T.3 | 0.6 | 3.94 |
| | F4 | T.0 | OL.0 | 3.01 |
| | F11 | T.1 | OL.1 | 6.15 |
| | F12 | T.2 | OL.2 | 8.19 |
| | F13 | T.4 | OL.3 | 6.08 |
| | F14 | T.1 | OL.4 | 5.15 |
| | F15 | T.2 | OL.5 | 4.44 |
| Substitution of CH1/CL of H0 | H3 | T.0 | O.0 | 3.24 |
| | H5 | T.1 | O.1 | 3.40 |
| | H6 | T.2 | 0.2 | 2.10 |
| | H7 | T.3 | 0.3 | 2.70 |
| | H8 | T.1 | 0.4 | 2.11 |
| | H9 | T.2 | 0.5 | 2.92 |
| | H10 | T.3 | 0.6 | 2.86 |
| | H4 | T.0 | OL.0 | 3.28 |
| | H11 | T.1 | OL.1 | 3.38 |
| | H12 | T.2 | OL.2 | 4.34 |
| | H13 | T.4 | OL.3 | 3.56 |
| | H14 | T.1 | OL.4 | 2.61 |
| | H15 | T.4 | OL.6 | 4.26 |
| Substitution of CH1/CL of R0 | R5 | T.0 | O.0 | 4.13 |
| | R7 | T.1 | O.1 | 4.89 |
| | R8 | T.2 | 0.2 | 3.84 |
| | R9 | T.3 | 0.3 | 3.95 |
| | R10 | T.1 | 0.4 | 3.20 |
| | R11 | T.2 | 0.5 | 3.30 |
| | R12 | T.3 | 0.6 | 4.36 |
| Substitution of CH1/CL of N0 | N3 | T.0 | O.0 | 4.5 |
| | N6 | T.2 | 0.5 | 8.4 |
| | N4 | T.0 | OL.0 | 4.6 |
| | N7 | T.5 | OL.1 | 2.8 |
| | N8 | T.5 | OL.4 | 2.6 |

| | | | | |
|---|---|---|---|---|
| Note: For example, antibody "F5" in the table indicates that the heavy chain CH1 of the F0 antibody was substituted with the T.1 chain (SEQ ID NO: 35) and the light chain CL was substituted with the O.1 chain (SEQ ID NO: 42), and the other portions remained the same as those of F0, and so on. | | | | |

In addition, the antibodies with substituted CH1/CL were constructed by utilizing the mutated or domain-engineered Titin-T chain and Obscurin-O chain, and the functional activity of the antibodies with other substituted CH1/CL was detected by using the methods of Test Example 2 and Test Example 4 disclosed herein. The experimental results are shown in Table 4, and the experimental results showed that the antibodies with substituted CH1/CL still maintained good antigen binding activity, and the SEC (%) of the antibody purity was relatively high.

**Table 4. Detection results of purity and binding activity of antibodies with domain engineered CH1/CL**

| Name of antibody | | Antibody Domain substitution | | Purity of antibody SEC (%) | Binding assay to the corresponding antigen EC50 (nM) |
|---|---|---|---|---|---|
| | | Substitution of heavy chain CH1 | Substitution of light chain CL | | |
| Substitution of CH1/CL of F0 | F16 | T.5 | O.4 | 65 | 2.0 |
| | F17 | T.5 | O.7 | 72 | 4.8 |
| | F18 | T.1 | O.8 | 70 | 3.9 |
| | F19 | T.5 | O.8 | 68 | 2.2 |
| | F20 | T.1 | O.9 | 71 | 1.9 |
| | F21 | T.5 | O.9 | 68 | 3.4 |
| | F22 | T.1 | O.10 | 74 | 2.4 |
| | F23 | T.5 | O.10 | 73 | 4.1 |
| | F24 | T.1 | O.12 | 71 | 2.4 |
| | F25 | T.5 | O.12 | 66 | 2.6 |
| | F26 | T.1 | O.13 | 73 | 2.6 |
| | F27 | T.5 | O.13 | 71 | 1.2 |
| | F28 | T.1 | O.14 | 75 | 2.4 |
| | F29 | T.5 | O.14 | 68 | 3.6 |
| | F30 | T.1 | O.15 | 75 | 3.4 |
| | F31 | T.5 | O.15 | 69 | 2.4 |
| | F32 | T.6 | O.17 | 72 | 3.69 |
| | F33 | T.7 | O.16 | 69 | 1.92 |
| | F34 | T.7 | O.17 | 73 | 2.38 |
| Substitution of CH1/CL of N0 | N10 | T.5 | O.4 | 89 | 3.0 |
| | N11 | T.1 | O.7 | 86 | 5.0 |
| | N12 | T.1 | O.8 | 84 | 5.3 |
| | N13 | T.5 | O.8 | 82 | 2.9 |
| | N14 | T.1 | O.9 | 92 | 1.9 |
| | N15 | T.5 | O.9 | 92 | 2.6 |
| | N16 | T.5 | O.10 | 91 | 3.4 |
| | N17 | T.1 | O.11 | 90 | 2.2 |
| | N18 | T.5 | O.11 | 89 | 3.2 |
| | N19 | T.1 | O.12 | 84 | 4.3 |
| | N20 | T.5 | O.12 | 84 | 2.8 |
| | N21 | T.5 | O.13 | 84 | 5.2 |
| | N22 | T.5 | O.14 | 87 | 4.5 |
| | N23 | T.1 | O.15 | 86 | 9.4 |
| | N24 | T.5 | O.15 | 84 | 6.7 |
| | N25 | T.6 | O.17 | 92 | 0.62 |
| | N26 | T.7 | O.16 | 93 | 0.84 |
| | N27 | T.7 | O.17 | 94 | 0.67 |
| Substitution of CH1/CL of R0 | R10 | T.1 | O.4 | 84 | 0.37 |
| | R13 | T.5 | O.4 | 76 | 0.4 |
| | R14 | T.5 | O.7 | 78 | 0.2 |
| | R15 | T.5 | O.8 | 82 | 0.3 |
| | R16 | T.5 | O.9 | 80 | 0.33 |
| | R17 | T.5 | O.10 | 77 | 0.4 |
| | R18 | T.5 | O.11 | 71 | 0.44 |
| | R19 | T.5 | O.12 | 78 | 0.36 |
| | R20 | T.5 | O.13 | 75 | 0.39 |
| | R21 | T.5 | O.14 | 77 | 0.37 |
| | R22 | T.5 | O.15 | 74 | 0.39 |

| | | | | | |
|---|---|---|---|---|---|
| Note: For example, antibody "F16" in the table indicates that the heavy chain CH1 of the F0 antibody was substituted with the T.5 chain (SEQ ID NO: 39) and the light chain CL was substituted with the 0.4 chain (SEQ ID NO: 45), and the other portions remained the same as those of F0, and so on. | | | | | |

In addition, mutations were made on other amino acids in the Titin Ig-like 152 domain and the Obscurin Ig-like 1 domain, and the specific amino acid mutations were designed as shown in Table 5.

**Table 5. Amino acid mutation design of T.1 chain/O.9 chain**

| Titin-T chain | | Obscurin-O chain | |
|---|---|---|---|
| Name | Mode of mutation | Name | Mode of mutation |
| T.1 | T.1 (SEQ ID NO: 35) | O.9 | 0.9 (SEQ ID NO: 50) |
| T.8 | T.1 + (M66S, T77S) | O.18 | 0.9 + (L11K, A12S, F13Y, V14T, T22S) |
| T.9 | T.1 + (M66K, K70R, S79T, G81R) | O.19 | 0.9 + (G2E, V17E, N30D, T32P, Q34E, S36T, V44I, A45T, L58V, K62E, A67Q, G69S, A97G) |
| T.10 | T.1 + (P3W, S11I, I13L, T22M, N82M) | O.20 | 0.9 + (D20L, T22M, A53L) |
| T.11 | T.1 + (S11I, M66K, S79T, G81R) | O.21 | 0.9 + (Q41K, A45T, A67Q, G69S, V89L) |
| T.12 | T.1 + (G40S, R42K, H45S, Q47E, Q49G, N56S, D58E, L75V, E83D, F84L) | O.22 | 0.9 + (Q42L, A45T, A67T, G69S, Q92E, D94G) |
| T. 13 | T.1 + (Q47E, Q49G, N56S, D58E, L75V) | O.23 | 0.9 + (A12S, F13Y, T22S, Q42L, A45T, A67Q, G69S, Q92E, D94G) |
| T.14 | T.1 + (N56S, D58E, L75V) | O.24 | 0.9 + (Q41K, A45T, A67Q, G69S and V89L) + (G₄S)₁ linker sequence |
| T.15 | T.1 + (N56S, D58E, M66S, T77S) | O.25 | 0.9 + (A12S, F13Y, T22S, Q42L, A45T, A67Q, G69S, Q92E and D94G), + (G₄S)₁ linker sequence |
| T.16 | T.1 + (S11I, M66K, S79T and G81R), + (G₄S)₁ linker sequence | O.26 | 0.9 + (A12S, F13Y, T22S, Q42L, A45T, A67Q, G69S, Q92E, D94G), + DQPQF |
| T.17 | T.1 + (N56S, D58E, M66S, T77S), + KAGIR | O.27 | 0.9 + (A12S, F13Y, T22S, Q42L, A45T, A67Q, G69S, Q92E and D94G), + DQPQF, + (G₄S)₁ linker sequence |
| T.18 | T.1 + (N56S, D58E, M66S and T77S), + KAGIR, + (G₄S)₁ linker sequence | | |
| T.19 | T.1 + (N56S, D58E, M66S and T77S), + (G₄S)₁ linker sequence | | |

| | | | |
|---|---|---|---|
| Note: In the table, for example, T.8 indicates a Titin-T chain obtained by amino acid mutation on the T.1 (SEQ ID NO: 35) sequence at M66S and T77S; O.24 indicates an Obscurin-O chain obtained by amino acid mutations on O.9 (SEQ ID NO: 50) at A12S, F13Y, T22S, Q42L, A45T, A67Q, G69S, Q92E and D94G, then addition of a "DQPQF" sequence at the N-terminus, and finally addition of a "GGGGS" linker sequence at the N-terminus, and so on. | | | |

The mutant sequences of the T.1 chain and the O.9 chain are as follows:
> T.8 (T.1 has M66S and T77S mutations) 
GIPPKIECLPSDISIDEGKVLTVASAFTGEPTPEVTWSTGGRKIHSQEQGRFHIENT
DDLTTLIISDVQKQDGGLYSLSLGNEFGSDSATVNIHIRSI (SEQ ID NO: 65);
> T.9 (T.1 has M66K, K70R, S79T and G81R mutations) 
GIPPKIECLPSDISIDEGKVLTVASAFTGEPTPEVTWSTGGRKIHSQEQGRFHIENT
DDLTTLIIKDVQRQDGGLYTLTLRNEFGSDSATVNIHIRSI (SEQ ID NO: 66);
> T.10 (T.1 has P3W, S11I, I13L, T22M and N82M mutations) 
GIWPKIECLPIDLSIDEGKVLMVASAFTGEPTPEVTWSTGGRKIHSQEQGRFHIE
NTDDLTTLIIMDVQKQDGGLYTLSLGMEFGSDSATVNIHIRSI (SEQ ID NO: 67);
> T.11 (T.1 has S11I, M66K, S79T and G81R mutations) 
GIPPKIECLPIDISIDEGKVLTVASAFTGEPTPEVTWSTGGRKIHSQEQGRFHIENT
DDLTTLIIKDVQKQDGGLYTLTLRNEFGSDSATVNIHIRSI (SEQ ID NO: 68);
> T.12 (T.1 has G40S, R42K, H45S, Q47E, Q49G, N56S, D58E, L75V, E83D and F84L mutations) 
GIPPKIECLPSDISIDEGKVLTVASAFTGEPTPEVTWSTSGKKISSEEGGRFHIESTE
DLTTLIIMDVQKQDGGVYTLSLGNDLGSDSATVNIHIRSI (SEQ ID NO: 69);
> T.13 (T.1 has Q47E, Q49G, N56S, D58E and L75V mutations) 
GIPPKIECLPSDISIDEGKVLTVASAFTGEPTPEVTWSTGGRKIHSEEGGRFHIEST
EDLTTLIIMDVQKQDGGVYTLSLGNEFGSDSATVNIHIRSI (SEQ ID NO: 70);
> T.14 (T. 1 has N56S, D58E and L75V mutations) 
GIPPKIECLPSDISIDEGKVLTVASAFTGEPTPEVTWSTGGRKIHSQEQGRFHIEST
EDLTTLIIMDVQKQDGGVYTLSLGNEFGSDSATVNIHIRSI (SEQ ID NO: 71);
> T.15 (T.1 has N56S, D58E, M66S and T77S mutations) 
GIPPKIECLPSDISIDEGKVLTVASAFTGEPTPEVTWSTGGRKIHSQEQGRFHIEST
EDLTTLIISDVQKQDGGLYSLSLGNEFGSDSATVNIHIRSI (SEQ ID NO: 72);
> T.16 (T.1 has S11I, M66K, S79T and G81R mutations + (G₄S)₁ linker sequence) 
GGGGSGIPPKIECLPIDISIDEGKVLTVASAFTGEPTPEVTWSTGGRKIHSQEQGR
FHIENTDDLTTLIIKDVQKQDGGLYTLTLRNEFGSDSATVNIHIRSI (SEQ ID NO:
73);
> T.17 (T.1 has N56S, D58E, M66S and T77S mutations, + KAGIR) 
KAGIRGIPPKIECLPSDISIDEGKVLTVASAFTGEPTPEVTWSTGGRKIHSQEQGR
FHIESTEDLTTLIISDVQKQDGGLYSLSLGNEFGSDSATVNIHIRSI (SEQ ID NO:
74);
> T.18 (T.1 has N56S, D58E, M66S and T77S mutations, + KAGIR, + (G₄S)₁ linker sequence) 
GGGGSKAGIRGIPPKIECLPSDISIDEGKVLTVASAFTGEPTPEVTWSTGGRKIHS
QEQGRFHIESTEDLTTLIISDVQKQDGGLYSLSLGNEFGSDSATVNIHIRSI (SEQ
ID NO: 75);
> T.19 (T.1 has N56S, D58E, M66S and T77S mutations, + (G₄S)₁ linker sequence) 
GGGGSGIPPKIECLPSDISIDEGKVLTVASAFTGEPTPEVTWSTGGRKIHSQEQGR
FHIESTEDLTTLIISDVQKQDGGLYSLSLGNEFGSDSATVNIHIRSI (SEQ ID NO:
76);
> O.18 (O.9 has L11K, A12S, F13Y, V14T and T22S mutations) 
SGAPRFLTRPKSYTVSVGKDASLSSQIVGNPTPQVSWEKDQQPVAAGARFRLA
QDGDLYRLKILDLALGDSGQYVSRARNAIGEAFACVGLQVDAEA (SEQ ID NO:
77);
> 0.19 (O.9 has G2E, V17E, N30D, T32P, Q34E, S36T, V44I, A45T, L58V, K62E, A67Q, G69S and A97G mutations) 
SEAPRFLTRPLAFVVSEGKDATLSSQIVGDPPPEVTWEKDQQPITAGARFRLAQ
DGDVYRLEILDLQLSDSGQYVSRARNAIGEAFACVGLQVDAEG (SEQ ID NO:
78);
> O.20 O.9 has D20L, T22M and A53L mutations) 
SGAPRFLTRPLAFVVSVGKLAMLSSQIVGNPTPQVSWEKDQQPVAAGARFRLL
QDGDLYRLKILDLALGDSGQYVSRARNAIGEAFACVGLQVDAEA (SEQ ID NO:
79);
> O.21 (O.9 has Q41K, A45T, A67Q, G69S and V89L mutations) 
SGAPRFLTRPLAFVVSVGKDATLSSQIVGNPTPQVSWEKDKQPVTAGARFRLA
QDGDLYRLKILDLQLSDSGQYVSRARNAIGEAFACLGLQVDAEA (SEQ ID NO:
80);
> O.22 (O.9 has Q42L, A45T, A67T, G69S, Q92E and D94G mutations) 
SGAPRFLTRPLAFVVSVGKDATLSSQIVGNPTPQVSWEKDQLPVTAGARFRLAQ
DGDLYRLKILDLTLSDSGQYVSRARNAIGEAFACVGLEVGAEA (SEQ ID NO:
81);
> O.23 (O.9 has A12S, F13Y, T22S, Q42L, A45T, A67Q, G69S, Q92E and D94G mutations) 
SGAPRFLTRPLSYVVSVGKDASLSSQIVGNPTPQVSWEKDQLPVTAGARFRLAQ
DGDLYRLKILDLQLSDSGQYVSRARNAIGEAFACVGLEVGAEA (SEQ ID NO:
82);
> O.24 (O.9 has Q41K, A45T, A67Q, G69S and V89L mutations; + (G₄S)₁ linker sequence) 
GGGGSSGAPRFLTRPLAFVVSVGKDATLSSQIVGNPTPQVSWEKDKQPVTAGA
RFRLAQDGDLYRLKILDLQLSDSGQYVSRARNAIGEAFACLGLQVDAEA (SEQ
ID NO: 83);
> O.25 (O.9 has A12S, F13Y, T22S, Q42L, A45T, A67Q, G69S, Q92E and D94G mutations, + (G₄S)₁ linker sequence) GGGGSSGAPRFLTRPLSYVVSVGKDASLSSQIVGNPTPQVSWEKDQLPVTAGA 
RFRLAQDGDLYRLKILDLQLSDSGQYVSRARNAIGEAFACVGLEVGAEA (SEQ
ID NO: 84);
> O.26 (O.9 has A12S, F13Y, T22S, Q42L, A45T, A67Q, G69S, Q92E and D94G mutations; + DQPQF) 
DQPQFSGAPRFLTRPLSYVVSVGKDASLSSQIVGNPTPQVSWEKDQLPVTAGAR
FRLAQDGDLYRLKILDLQLSDSGQYVSRARNAIGEAFACVGLEVGAEA (SEQ
ID NO: 85);
> O.27 (O.9 has A12S, F13Y, T22S, Q42L, A45T, A67Q, G69S, Q92E and D94G mutations; + DQPQF; and + (G₄S)₁ linker sequence)

GGGGSDQPQFSGAPRFLTRPLSYVVSVGKDASLSSQIVGNPTPQVSWEKDQLPV
TAGARFRLAQDGDLYRLKILDLQLSDSGQYVSRARNAIGEAFACVGLEVGAEA
(SEQ ID NO: 86).

The antibodies with substituted CH1/CL were constructed by utilizing the mutated or domain-engineered Titin-T chain and Obscurin-O chain, the antibodies with substituted CH1/CL were detected by using the method of Test Example 4 disclosed herein, and the experimental results are shown in Table 6. The results showed that the antibodies still maintained good antigen binding activity after the CH1/CL was substituted with the optimized Titin-T chain and the Obscurin-O chain.

**Table 6. Experimental results of binding of antibodies with substituted CH1/CL to corresponding antigens**

| Antibody | | Antibody Domain substitution | | Binding assay to the corresponding antigen EC50 (nM) |
|---|---|---|---|---|
| | | Substitution of heavy chain CH1 | Substitution of light chain CL | |
| Substitution of CH1/CL of B0 | B0 | CH1 | CL | 0.2 |
| | B1 | T.1 | 0.9 | 0.28 |
| | B2 | T.8 | 0.18 | 0.19 |
| | B3 | T.9 | 0.19 | 0.5 |
| | B4 | T.11 | O.21 | 0.25 |
| | B5 | T.12 | O.22 | 0.29 |
| | B6 | T.13 | O.22 | 0.19 |
| | B7 | T.14 | O.22 | 0.35 |
| | B8 | T.15 | O.23 | 0.16 |
| Substitution of CH1/CL of U0 | U0 | CH1 | CL0.06 | |
| | U1T.10.90.03 | | | |
| | U2T.80.180.07 | | | |
| | U3T.90.190.02 | | | |
| | U4 | T.11 | O.21 | 0.06 |
| | U5 | T.12 | O.22 | 0.11 |
| | U6 | T.13 | O.22 | 0.11 |
| | U7 | T.14 | O.22 | 0.07 |
| | U8 | T.15 | O.23 | 0.16 |
| Substitution of CH1/CL of D0 | D0 | CH1 | CL0.089 | |
| | D1T.10.90.026 | | | |
| | D2T.90.190.056 | | | |
| | D3 | T.11 | O.21 | 0.083 |
| | D4 | T.12 | O.22 | 0.103 |
| | D5 | T.13 | O.22 | 0.134 |
| | D6 | T.14 | O.22 | 0.132 |
| | D7 | T.15 | O.23 | 0.102 |
| Substitution of CH1/CL of 10 | 10 | CH1 | CL | 1.424 |
| | 12 | T.8 | 0.18 | 3.195 |
| | 13 | T.11 | O.21 | 2.989 |
| | 14 | T.12 | O.22 | 4.132 |
| | 15 | T.13 | O.22 | 3.592 |
| | 16 | T.14 | O.22 | 4.121 |
| | 17 | T.15 | O.23 | 3.153 |

| | | | | |
|---|---|---|---|---|
| Note: In the table, for example, antibody "B1" indicates an antibody obtained by substitution of the heavy chain CH1 of B0 antibody with T.1 chain (SEQ ID NO: 35) and substitution of the light chain CL with O.9 chain (SEQ ID NO: 50), and the other portions were the same as those of B0, and so on. | | | | |

In addition, the protein expression level of the antibody with substituted CH1/CL was measured by using the method of Test Example 1 disclosed herein, and the experimental results are shown in Table 7. The results showed that the expression level of antibodies was greatly improved after partial amino acid mutations of the Titin Ig-like 152 domain and the Obscurin Ig-like 1 domain.

**Table 7. Expression level of CH1/CL domain engineered antibodies**

| Antibody | | Antibody Domain substitution | | Expression level of antibody (mg/L) |
|---|---|---|---|---|
| | | Substitution of heavy chain CH1 | Substitution of light chain CL | |
| Substitution of CH1/CL of B0 | B1 | T.1 | 0.9 | 9.4 |
| | B2 | T.8 | 0.18 | 25.6 |
| | B3 | T.9 | 0.19 | 13.9 |
| | B4 | T.11 | O.21 | 21.4 |
| Substitution of CH1/CL of D0 | D1 | T.1 | 0.9 | 5.9 |
| | D2 | T.9 | 0.19 | 44.4 |
| | D3 | T.11 | O.21 | 40.5 |
| | D4 | T.12 | O.22 | 17.8 |
| | D5 | T.13 | O.22 | 21.4 |
| | D6 | T.14 | O.22 | 23.3 |
| | D7 | T.15 | O.23 | 34.3 |
| Substitution of CH1/CL of I0 | I1 | T.1 | 0.9 | 2.1 |
| | 12 | T.8 | 0.18 | 11.0 |
| | 13 | T.11 | O.21 | 10.4 |
| | 14 | T.12 | O.22 | 4.0 |
| | 15 | T.13 | O.22 | 4.6 |
| | 16 | T.14 | O.22 | 8.2 |
| | 17 | T.15 | O.23 | 6.9 |

### Example 4: Half-Life of CH1/CL Domain Engineered Antibody

To evaluate whether the stability of the antibody in the animal body was affected after CH1/CL of the antibody was substituted with a Titin-T chain/Obscurin-O chain or a Titin-T chain/Obscurin-like-O chain, we examined the half-life of the antibody in the rat body before and after the substitution. Illustratively, we examined the half-lives in rats of antibody F20, in which CH1/CL of F0 antibody was substituted with T.1/O.9, and of antibody C3, in which CH1/CL of C0 antibody was substituted with T.1/0.9, respectively. The specific method comprises the following steps:
The 3mpk antibody was administered intravenously to rats, and 0.3 mL of whole blood was collected 5 min, 8 h, 1 d, 2 d, 4 d, 7 d, 10 d, 14 d, 21 d and 28 d after administration, respectively, without anticoagulation, and after blood collection, the blood was left at 4 °C for 30 min and centrifuged at 1000 g for 15 min, and the supernatant (serum) was placed in an EP tube and stored at -80 °C. The detection method for antibody drug concentrations at each time point adopted sandwich ELISA, namely, corresponding antigens were coated on an ELISA plate, and the plate was incubated overnight at 4 °C, washed with a washing liquid, then added with a blocking buffer and incubated at room temperature for 1-3 h, then washed with a washing liquid, added with 100 µL of standard substance or serum sample to be detected and incubated at 37 °C for 3 h, then washed with a washing liquid, added with a secondary antibody anti-Human IgG FC (HRP) mouse preadsorbed (Abcam, product No. ab98624, 1:10000 dilution) and incubated at 37 °C for 1-1.5 h, then washed with a washing buffer, added with TMB and incubated in dark at room temperature for 10 min, and then added with a stop buffer; OD450 values were read.

The experimental results are shown in Table 8, and the results showed that, after CH1/CL of the antibody was substituted with a Titin-T chain/Obscurin-O chain, the half-life of the original antibody in the rat body was still maintained, which indicates that the antibody with substituted CH1/CL had good stability in the body like the original antibody.

**Table 8. Half-life of antibodies with substituted CH1/CL domain**

| Name of antibody | *In-vivo* half-life of rat (3mpk) T1/2 (day) |
|---|---|
| F0 | 13.3 ± 1.0 |
| F20 | 12.8 ± 0.3 |
| C0 | 17.3 ± 2.2 |
| C3 | 15.4 ± 4.1 |

### Example 5: Construction and Detection of Bispecific Antibodies

A bispecific antibody was constructed through bridge PCR of an antibody binding to a first antigen and an antibody binding to a second antigen. Illustratively, we constructed a variety of bispecific antibodies.

### I. Construction of bispecific antibodies

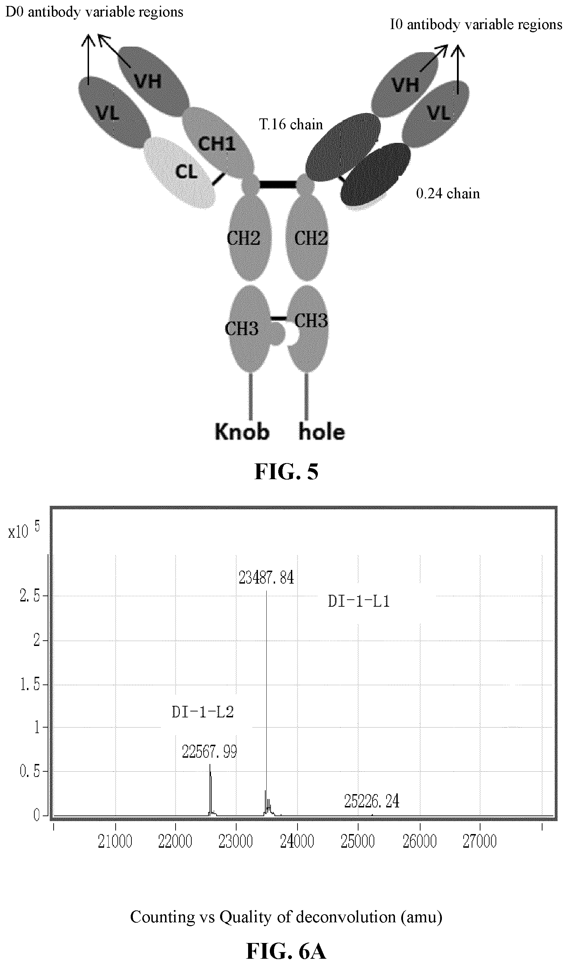

Firstly, we constructed a DI-1 bispecific antibody which is an IgG-like bispecific antibody composed of a heavy chain (with knob modification in Fc region) and a light chain of a D0 antibody, and a heavy chain (with hole modification in Fc region) and a light chain of a modified antibody obtained by substituting CH1/CL of an I0 antibody with a Titin-T chain/Obscurin-O chain; its structural schematic diagram is shown in FIG. 5, and its sequence is shown as follows:
Amino acid sequence of DI-1 chain 1 (DI-1-H1): 
EVQLLESGGGLVQPGGSLRLSCAASGFTFSSYAMSWVRQAPGKGLEWVSG
ITGSGGSTYYADSVKGRFTISRDNSKNTLYLQMNSLRAEDTAVYYCAKDPG
TTVIMSWFDPWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVN
HKPSNTKVDKKVEPKSCDKTHTCPPCPAPEAAGGPSVFLFPPKPKDTLMISRTPE
VTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPCREEMTKNQVS
LWCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 87);
 Note: In the sequence, the bold part is VH of D0, the single underlined part is CH1, the dotted underlined part is CH2, and the italicized part is CH3.
Amino acid sequence of DI-1 chain 2 (DI-1-L1): 
EIVLTQSPGTLSLSPGERATLSCRASQSVRGRYLAWYQQKPGQAPRLLIYG
ASSRATGIPDRFSGSGSGTDFTLTISRLEPEDFAVFYCQQYGSSPRTFGQGT
KVEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKSF
NRGEC   (SEQ ID NO: 88);
 Note: In the sequence, the bold part is VL of D0, and the single underlined part is light chain constant region CL of D0.
Amino acid sequence of DI-1 chain 3 (DI-1-H2): 
QVQLQESGPGLVKPSETLSLTCTVSGFSLIGYDLNWIRQPPGKGLEWIGIIW
GDGTTDYNSAVKSRVTISKDTSKNQFSLKLSSVTAADTAVYYCARGGYWY
ATSYYFDYWGQGTLVTVSSGGGGSGIPPKIECLPIDISIDEGKVLTVASAFTGEP
TPEVTWSTGGRKIHSQEQGRFHIENTDDLTTLIIKDVQKQDGGLYTLTLRNEFGS
DSATVNIHIRSIDKTHTCPPCPAPEAAGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVCTLPPSREEMTKNQVSLSCAVK
GFYPSDIAVEWESNGQPENNYKTTPPVLDSDGSFFLVSKLTVDKSRWQQGNVFSCSV
MHEALHNHYTQKSLSLSPGK (SEQ ID NO: 89);
 Note: In the sequence, the bold part is VH of I0, the single underlined part is T.16, the dotted underlined part is CH2, and the italicized part is CH3.
Amino acid sequence of DI-1 chain 4 (DI-1-L2): 
DIQMTQSPSSLSASVGDRVTITCRASQSISNNLNWYQQKPGKAPKLLIYYTS
RFHSGVPSRFSGSGSGTDFTFTISSLQPEDIATYYCQQEHTLPYTFGQGTKL
 
EIKGGGGSSGAPRFLTRPLAFVVSVGKDATLSSQIVGNPTPQVSWEKDKQPVTA
GARFRLAQDGDLYRLKILDLQLSDSGQYVSRARNAIGEAFACLGLQVDAEA
(SEQ ID NO: 90);
 Note: In the sequence, the bold part is VL of I0, and the single underlined part is O.24.

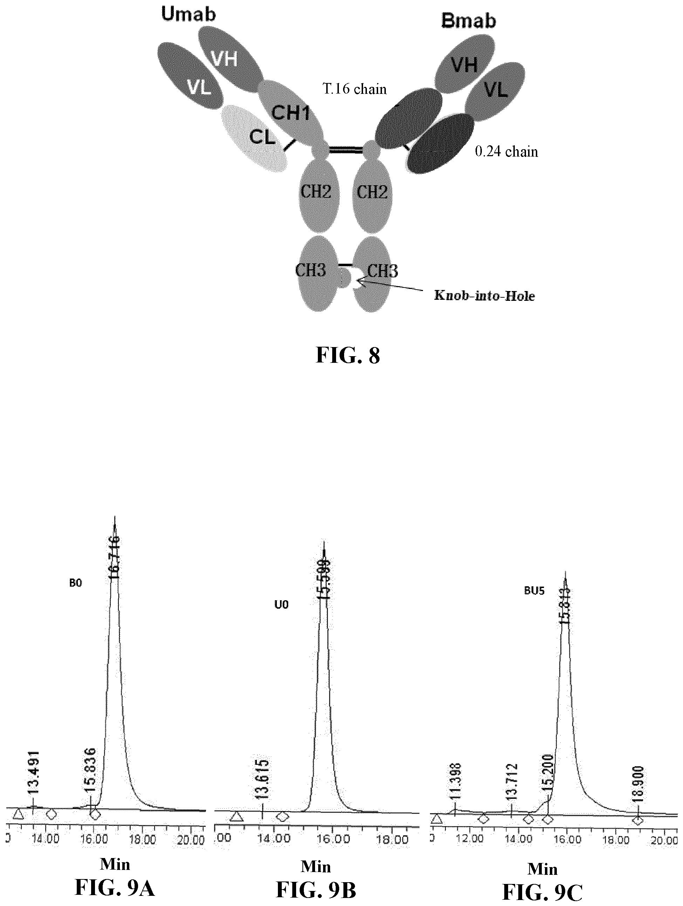

In addition, an IgG-like bispecific antibody was constructed from B0 or the CH1/CL domain engineered antibody of B0 and U0 or the CH1/CL domain engineered antibody of U0 (illustratively, the schematic structural diagram of BU5 is shown in FIG. 8), and four chain sequences of the obtained bispecific antibody are shown in Table 9 below:

**Table 9. IgG-like bispecific antibody sequence listing**

| Name of diabody | Four-chain sequence of IgG-like bispecific antibody | | | |
|---|---|---|---|---|
| | Chain 1 | Chain 2 | Chain 3 | Chain 4 |
| BU1 | Heavy chain of B0 (knob) | Light chain of B0 | Heavy chain of U0 (hole), and CH1 is substituted with T.19 | Light chain of U0, and CL is substituted with O.25 |
| | QVQLQQSGAEVKKPG SSVRVSCKASGGTFNN NAINWVRQAPGQGLE WMGGIIPMFGTAKYS QNFQGRVAITADESTG TASMELSSLRSEDTAV YYCARSRDLLLFPHHA LSPWGRGTMVTVSSAS TKGPSVFPLAPSSKSTSG GTAALGCLVKDYFPEPV TVSWNSGALTSGVHTFP AVLQSSGLYSLSSVVTV PSSSLGTQTYICNVNHKP SNTKVDKKVEPKSCDKT HTCPPCPAPEAAGGPSV FLFPPKPKDTLMISRTPE VTCVVVDVSHEDPEVKF NWYVDGVEVHNAKTKP REEQYNSTYRVVSVLTV LHQDWLNGKEYKCKVS NKALPAPIEKTISKAKGQ PREPQVYTLPPCREEMTK NQVSLWCLVKGFYPSDIA VEWESNGQPENNYKTTPP VLDSDGSFFLYSKLTVDK SRWQQGNVFSCSVMHEA LHNHYTQKSLSLSPGK (SEQ ID NO: 91) | SSELTQDPA VSVALGQTV RVTCQGDSL RSYYASWY QQKPGQAP VLVIYGKNN RPSGIPDRFS GSSSGNTAS LTITGAQAE DEADYYCSS RDSSGNHW VFGGGTELT VLGQPKAAP SVTLFPPSSE ELQANKATL VCLISDFYPG AVTVAWKA DSSPVKAGV ETTTPSKQSN NKYAASSYL SLTPEQWKS HRSYSCQVT HEGSTVEKT VAPTECS (SEQ ID NO: 92) | EVQLVQSGAEVKKPGESL KISCKGSGYSFTTYWLGW VRQMPGKGLDWIGIMSP VDSDIRYSPSFQGQVTMS VDKSITTAYLQWNSLKAS DTAMYYCARRRPGQGYF DFWGQGTLVTVSSGGGGS GIPPKIECLPSDISIDEGKVL TVASAFTGEPTPEVTWSTG GRKIHSQEQGRFHIESTEDL TTLIISDVQKQDGGLYSLSL GNEFGSDSATVNIHIRSIDK THTCPPCPAPEAAGGPSVFL FPPKPKDTLMISRTPEVTCV VVDVSHEDPEVKFNWYVD GVEVHNAKTKPREEQYNST YRVVSVLTVLHQDWLNGK EYKCKVSNKALPAPIEKTIS KAKGQPREPQVCTLPPSREE MTKNQVSLSCAVKGFYPSDI AVEWESNGQPENNYKTTPPV LDSDGSFFLVSKLTVDKSRW QQGNVFSCSVMHEALHNHY TQKSLSLSPGK (SEQ ID NO: 93) | DIQMTQSPS SLSASVGDR VTITCRASQ GISSWLAW YQQKPEKA PKSLIYAASS LQSGVPSRF SGSGSGTDF TLTISSLQPE DFATYYCQ QYNIYPYTF GQGTKLEIK GGGGSSGAP RFLTRPLSYV VSVGKDASL SSQIVGNPTP QVSWEKDQ LPVTAGARF RLAQDGDLY RLKILDLQLS DSGQYVSRA RNAIGEAFA CVGLEVGAE A (SEQ ID NO: 94) |
| BU2 | Heavy chain of U0 (knob) | Light chain of U0 | Heavy chain of B0 (hole), and CH1 is substituted with T.19 | Light chain of B0, and CL is substituted with O.27 |
| | EVQLVQSGAEVKKPGE SLKISCKGSGYSFTTY WLGWVRQMPGKGLD WIGIMSPVDSDIRYSPS FQGQVTMSVDKSITTA YLQWNSLKASDTAMY YCARRRPGQGYFDFW | DIQMTQSPS SLSASVGDR VTITCRASQ GISSWLAWY QQKPEKAP KSLIYAASSL QSGVPSRFS | QVQLQQSGAEVKKPGSSV RVSCKASGGTFNNNAINW VRQAPGQGLEWMGGIIP MFGTAKYSQNFQGRVAIT ADESTGTASMELSSLRSED TAVYYCARSRDLLLFPHH ALSPWGRGTMVTVSSGG | SSELTQDPA VSVALGQT VRVTCQGD SLRSYYASW YQQKPGQA PVLVIYGKN NRPSGIPDR |
| | GQGTLVTVSSASTKGPS VFPLAPSSKSTSGGTAAL GCLVKDYFPEPVTVSW NSGALTSGVHTFPAVLQ SSGLYSLSSVVTVPSSSL GTQTYICNVNHKPSNTK VDKKVEPKSCDKTHTCP PCPAPEAAGGPSVFLFPP KPKDTLMISRTPEVTCV VVDVSHEDPEVKFNWY VDGVEVHNAKTKPREE QYNSTYRVVSVLTVLH QDWLNGKEYKCKVSNK ALPAPIEKTISKAKGQPR EPQVYTLPPCREEMTKNQ VSLWCLVKGFYPSDIAVE WESNGQPENNYKTTPPVL DSDGSFFLYSKLTVDKSR WQQGNVFSCSVMHEALH NHYTQKSLSLSPGK (SEQ ID NO: 95) | GSGSGTDFT LTISSLQPED FATYYCQQY NIYPYTFGQ GTKLEIKRT VAAPSVFIFP PSDEQLKSGT ASVVCLLNN FYPREAKVQ WKVDNALQS GNSQESVTE QDSKDSTYS LSSTLTLSKA DYEKHKVYA CEVTHQGLS SPVTKSFNRG EC (SEQ ID NO: 96) | GGSGIPPKIECLPSDISIDEG KVLTVASAFTGEPTPEVTW STGGRKIHSQEQGRFHIEST EDLTTLIISDVQKQDGGLYS LSLGNEFGSDSATVNIHIRSI DKTHTCPPCPAPEAAGGPS VFLFPPKPKDTLMISRTPEV TCVVVDVSHEDPEVKFNW YVDGVEVHNAKTKPREEQ YNSTYRVVSVLTVLHQDW LNGKEYKCKVSNKALPAPI EKTISKAKGQPREPQVCTLP PSREEMTKNQVSLSCAVKGF YPSDIAVEWESNGQPENNYK TTPPVLDSDGSFFLVSKLTVD KSRWQQGNVFSCSVMHEAL HNHYTQKSLSLSPGK (SEQ ID NO: 97) | FSGSSSGNT ASLTITGAQ AEDEADYY CSSRDSSGN HWVFGGGT ELTVLGGGG SDQPQFSGA PRFLTRPLSY VVSVGKDAS LSSQIVGNPT PQVSWEKDQ LPVTAGARF RLAQDGDLY RLKILDLQLS DSGQYVSRA RNAIGEAFA CVGLEVGAE A (SEQ ID NO: 98) |
| BU3 | Heavy chain of B0 (knob), and CH1 is substituted with T.19 | Light chain of B0, and CL is substituted with O.27 | Heavy chain of U0 (hole) | Light chain of U0 |
| | QVQLQQSGAEVKKPG SSVRVSCKASGGTFNN NAINWVRQAPGQGLE WMGGIIPMFGTAKYS QNFQGRVAITADESTG TASMELSSLRSEDTAV YYCARSRDLLLFPHHA LSPWGRGTMVTVSSGG GGSGIPPKIECLPSDISID EGKVLTVASAFTGEPTP EVTWSTGGRKIHSQEQG RFHIESTEDLTTLIISDVQ KQDGGLYSLSLGNEFGS DSATVNIHIRSIDKTHTC PPCPAPEAAGGPSVFLFP PKPKDTLMISRTPEVTC VVVDVSHEDPEVKFNW YVDGVEVHNAKTKPRE EQYNSTYRVVSVLTVLH QDWLNGKEYKCKVSNK ALPAPIEKTISKAKGQPR EPQVYTLPPCREEMTKNQ VSLWCLVKGFYPSDIAVE WESNGQPENNYKTTPPVL DSDGSFFLYSKLTVDKSR WQQGNVFSCSVMHEALH NHYTQKSLSLSPGK (SEQ ID NO: 99) | SSELTQDPA VSVALGQTV RVTCQGDSL RSYYASWY QQKPGQAP VLVIYGKNN RPSGIPDRFS GSSSGNTAS LTITGAQAE DEADYYCSS RDSSGNHW VFGGGTELT VLGGGGSDQ PQFSGAPRFL TRPLSYVVSV GKDASLSSQI VGNPTPQVS WEKDQLPVT AGARFRLAQ DGDLYRLKIL DLQLSDSGQ YVSRARNAI GEAFACVGL EVGAEA (SEQ ID NO: 100) | EVQLVQSGAEVKKPGESL KISCKGSGYSFTTYWLGW VRQMPGKGLDWIGIMSP VDSDIRYSPSFQGQVTMS VDKSITTAYLQWNSLKAS DTAMYYCARRRPGQGYF DFWGQGTLVTVSSASTKG PSVFPLAPSSKSTSGGTAAL GCLVKDYFPEPVTVSWNSG ALTSGVHTFPAVLQSSGLY SLSSVVTVPSSSLGTQTYIC NVNHKPSNTKVDKKVEPK SCDKTHTCPPCPAPEAAGG PSVFLFPPKPKDTLMISRTP EVTCVVVDVSHEDPEVKFN WYVDGVEVHNAKTKPREE QYNSTYRVVSVLTVLHQD WLNGKEYKCKVSNKALPA PIEKTISKAKGQPREPQVCT LPPSREEMTKNQVSLSCAVK GFYPSDIAVEWESNGQPENN YKTTPPVLDSDGSFFLVSKLT VDKSRWQQGNVFSCSVMHE ALHNHYTQKSLSLSPGK (SEQ ID NO: 101) | DIQMTQSPS SLSASVGDR VTITCRASQ GISSWLAW YQQKPEKA PKSLIYAASS LQSGVPSRF SGSGSGTDF TLTISSLQPE DFATYYCQ QYNIYPYTF GQGTKLEIK RTVAAPSVFI FPPSDEQLKS GTASVVCLL NNFYPREAK VQWKVDNA LQSGNSQES VTEQDSKDS TYSLSSTLTL SKADYEKHK VYACEVTHQ GLSSPVTKSF NRGEC (SEQ ID NO: 96) |
| BU4 | Heavy chain of U0 (knob) | Light chain of U0 | Heavy chain of B0 (hole), and CH1 is substituted with T.18 | Light chain of B0, and CL is substituted with O.27 |
| | EVQLVQSGAEVKKPGE SLKISCKGSGYSFTTY WLGWVRQMPGKGLD | DIQMTQSPS SLSASVGDR VTITCRASQ | QVQLQQSGAEVKKPGSSV RVSCKASGGTFNNNAINW VRQAPGQGLEWMGGIIP | SSELTQDPA VSVALGQT VRVTCQGD |
| | WIGIMSPVDSDIRYSPS FQGQVTMSVDKSITTA YLQWNSLKASDTAMY YCARRRPGQGYFDFW GQGTLVTVSSASTKGPS VFPLAPSSKSTSGGTAAL GCLVKDYFPEPVTVSW NSGALTSGVHTFPAVLQ SSGLYSLSSVVTVPSSSL GTQTYICNVNHKPSNTK VDKKVEPKSCDKTHTCP PCPAPEAAGGPSVFLFPP KPKDTLMISRTPEVTCV VVDVSHEDPEVKFNWY VDGVEVHNAKTKPREE QYNSTYRVVSVLTVLH QDWLNGKEYKCKVSNK ALPAPIEKTISKAKGQPR EPQVYTLPPCREEMTKNQ VSLWCLVKGFYPSDIAVE WESNGQPENNYKTTPPVL DSDGSFFLYSKLTVDKSR WQQGNVFSCSVMHEALH NHYTQKSLSLSPGK (SEQ ID NO: 95) | GISSWLAWY QQKPEKAP KSLIYAASSL QSGVPSRFS GSGSGTDFT LTISSLQPED FATYYCQQY NIYPYTFGQ GTKLEIKRT VAAPSVFIFP PSDEQLKSGT ASVVCLLNN FYPREAKVQ WKVDNALQS GNSQESVTE QDSKDSTYS LSSTLTLSKA DYEKHKVYA CEVTHQGLS SPVTKSFNRG EC (SEQ ID NO: 96) | MFGTAKYSQNFQGRVAIT ADESTGTASMELSSLRSED TAVYYCARSRDLLLFPHH ALSPWGRGTMVTVSSGG GGSKAGIRGIPPKIECLPSDI SIDEGKVLTVASAFTGEPTP EVTWSTGGRKIHSQEQGRF HIESTEDLTTLIISDVQKQD GGLYSLSLGNEFGSDSATV NIHIRSIDKTHTCPPCPAPEA AGGPSVFLFPPKPKDTLMIS RTPEVTCVVVDVSHEDPEV KFNWYVDGVEVHNAKTKP REEQYNSTYRVVSVLTVLH QDWLNGKEYKCKVSNKAL PAPIEKTISKAKGQPREPQV CTLPPSREEMTKNQVSLSCA VKGFYPSDIAVEWESNGQPE NNYKTTPPVLDSDGSFFLVSK LTVDKSRWQQGNVFSCSVM HEALHNHYTQKSLSLSPGK (SEQ ID NO: 102) | SLRSYYASW YQQKPGQA PVLVIYGKN NRPSGIPDR FSGSSSGNT ASLTITGAQ AEDEADYY CSSRDSSGN HWVFGGGT ELTVLGGGG SDQPQFSGA PRFLTRPLSY VVSVGKDAS LSSQIVGNPT PQVSWEKDQ LPVTAGARF RLAQDGDLY RLKILDLQLS DSGQYVSRA RNAIGEAFA CVGLEVGAE A (SEQ ID NO: 100) |
| BU5 | Heavy chain of B0 (hole), and CH1 is substituted with T16 | Light chain of B0, and CL is substituted with O.24 | Heavy chain of U0 (knob) | Light chain of U0 |
| | QVQLQQSGAEVKKPG SSVRVSCKASGGTFNN NAINWVRQAPGQGLE WMGGIIPMFGTAKYS QNFQGRVAITADESTG TASMELSSLRSEDTAV YYCARSRDLLLFPHHA LSPWGRGTMVTVSSG GGGSGIPPKIECLPIDISI DEGKVLTVASAFTGEPT PEVTWSTGGRKIHSQEQ GRFHIENTDDLTTLIIKD VQKQDGGLYTLTLRNEF GSDSATVNIHIRSIDKTH TCPPCPAPEAAGGPSVFL FPPKPKDTLMISRTPEVT CVVVDVSHEDPEVKFN WYVDGVEVHNAKTKPR EEQYNSTYRVVSVLTVL HQDWLNGKEYKCKVSN KALPAPIEKTISKAKGQP REPQVCTLPPSREEMTKN QVSLSCAVKGFYPSDIAVE WESNGQPENNYKTTPPVL DSDGSFFLVSKLTVDKSR WQQGNVFSCSVMHEALH NHYTQKSLSLSPGK (SEQ ID NO: 103) | SSELTQDPA VSVALGQTV RVTCQGDSL RSYYASWY QQKPGQAP VLVIYGKNN RPSGIPDRFS GSSSGNTAS LTITGAQAE DEADYYCSS RDSSGNHW VFGGGTELT VLGGGGSSG APRFLTRPLA FVVSVGKDA TLSSQIVGNP TPQVSWEKD KQPVTAGAR FRLAQDGDL YRLKILDLQL SDSGQYVSR ARNAIGEAF ACLGLQVDA EA (SEQ ID NO: 104) | EVQLVQSGAEVKKPGESL KISCKGSGYSFTTYWLGW VRQMPGKGLDWIGIMSP VDSDIRYSPSFQGQVTMS VDKSITTAYLQWNSLKAS DTAMYYCARRRPGQGYF DFWGQGTLVTVSSASTKG PSVFPLAPSSKSTSGGTAAL GCLVKDYFPEPVTVSWNSG ALTSGVHTFPAVLQSSGLY SLSSVVTVPSSSLGTQTYIC NVNHKPSNTKVDKKVEPK SCDKTHTCPPCPAPEAAGG PSVFLFPPKPKDTLMISRTP EVTCVVVDVSHEDPEVKFN WYVDGVEVHNAKTKPREE QYNSTYRVVSVLTVLHQD WLNGKEYKCKVSNKALPA PIEKTISKAKGQPREPQVYTL PPCREEMTKNQVSLWCLVK GFYPSDIAVEWESNGQPENN YKTTPPVLDSDGSFFLYSKLT VDKSRWQQGNVFSCSVMHE ALHNHYTQKSLSLSPGK (SEQ ID NO: 95) | DIQMTQSPS SLSASVGDR VTITCRASQ GISSWLAW YQQKPEKA PKSLIYAASS LQSGVPSRF SGSGSGTDF TLTISSLQPE DFATYYCQ QYNIYPYTF GQGTKLEIK RTVAAPSVFI FPPSDEQLKS GTASVVCLL NNFYPREAK VQWKVDNA LQSGNSQES VTEQDSKDS TYSLSSTLTL SKADYEKHK VYACEVTHQ GLSSPVTKSF NRGEC (SEQ ID NO: 96) |

| | | | | |
|---|---|---|---|---|
| Note: In the sequence, the bold part is the variable region sequence, the single underlined part is CH1 or Titin-T chain, the dotted underlined part is CH2, the italicized part is CH3, and the double underlined part is CL or Obscurin-O chain. | | | | |

Furthermore, an FA-1 bispecific antibody was constructed, which is an IgG-like bispecific antibody composed of a heavy chain (knob modification of Fc region) and a light chain of A0 antibody, and a heavy chain (hole modification of Fc region, substitution of CH1 with T.16) and a light chain (substitution of CL with O.24) of F0 antibody. The four-chain sequences of FA-1 are shown in Table 10 below:

**Table 10. FA-1 bispecific antibody sequence listing**

| Name of diabody | Four-chain sequence of IgG-like | bispecific antibody | | |
|---|---|---|---|---|
| | Chain 1 | Chain 2 | Chain 3 | Chain 4 |
| FA-1 | Heavy chain of F0 (hole), and CH1 is substituted with T.16 | Light chain of F0, and CL is substituted with O.24 | Heavy chain of A0 (knob) | Light chain of A0 |
| | QVQLVQSGGGVVQPGTS LRLSCAASGFIFSSSAMH WVRQAPGKGLEWVAVI SYDGSNKYYVDSVKGRF TISRDNSKNTLYLQMNS LRAEDTAVYYCARSARL YASFDYWGQGALVTVSS GGGGSGIPPKIECLPIDISID EGKVLTVASAFTGEPTPE VTWSTGGRKIHSQEQGRF HIENTDDLTTLIIKDVQKQ DGGLYTLTLRNEFGSDSA TVNIHIRSIDKTHTCPPCPA PEAAGGPSVFLFPPKPKDT LMISRTPEVTCVVVDVSH EDPEVKFNWYVDGVEVH NAKTKPREEQYNSTYRVV SVLTVLHQDWLNGKEYK CKVSNKALPAPIEKTISKA KGQPREPQVCTLPPSREEM TKNQVSLSCAVKGFYPSDIA VEWESNGQPENNYKTTPPV LDSDGSFFLVSKLTVDKSR WQQGNVFSCSVMHEALHN HYTQKSLSLSPGK (SEQ ID NO: 107) | DTVVTQEPSF SVSPGGTVTL TCGLSSGSVST SHYPSWYQQT PGQAPRMLIY NTNTRSSGVP DRFSGSILGN KAALTITGAQ ADDESDYYCA IHVDRDIWVF GGGTKLTVLG GGGSSGAPRFL TRPLAFVVSVG KDATLSSQIVG NPTPQVSWEK DKQPVTAGAR FRLAQDGDLY RLKILDLQLSD SGQYVSRARN AIGEAFACLGL QVDAEA (SEQ ID NO: 108) | EVQLVESGGGLVQPG GSLKLSCAASGFTFNK YAMNWVRQAPGKGL EWVARIRSKYNNYAT YYADSVKDRFTISRDD SKNTAYLQMNNLKTE DTAVYYCVRHGNFGN EYISYWAYWGQGTLV TVSSASTKGPSVFPLAPS SKSTSGGTAALGCLVK DYFPEPVTVSWNSGALT SGVHTFPAVLQSSGLYS LSSVVTVPSSSLGTQTYI CNVNHKPSNTKVDKKV EPKSCDKTHTCPPCPAP EAAGGPSVFLFPPKPKD TLMISRTPEVTCVVVDV SHEDPEVKFNWYVDGV EVHNAKTKPREEQYNS TYRVVSVLTVLHQDWL NGKEYKCKVSNKALPA PIEKTISKAKGQPREPQV YTLPPCREEMTKNQVSL WCLVKGFYPSDIAVEWES NGQPENNYKTTPPVLDS DGSFFLYSKLTVDKSRWQ QGNVFSCSVMHEALHNH YTQKSLSLSPGK (SEQ ID NO: 109) | QTVVTQEPS LTVSPGGTV TLTCGSSTG AVTSGNYPN WVQQKPGQ APRGLIGGT KFLAPGTPA RFSGSLLGG KAALTLSGV QPEDEAEYY CVLWYSNR WVFGGGTK LTVLRTVAA PSVFIFPPSDE QLKSGTASV VCLLNNFYP REAKVQWK VDNALQSGN SQESVTEQD SKDSTYSLSS TLTLSKADY EKHKVYACE VTHQGLSSP VTKSFNRGE C (SEQ ID NO: 110) |

| | | | | |
|---|---|---|---|---|
| Note: In the sequence, the bold part is the variable region sequence, the single underlined part is CH1 or Titin-T chain, the dotted underlined part is CH2, the italicized part is CH3, and the double underlined part is CL or Obscurin-O chain. | | | | |

### II. Detection of bispecific antibodies

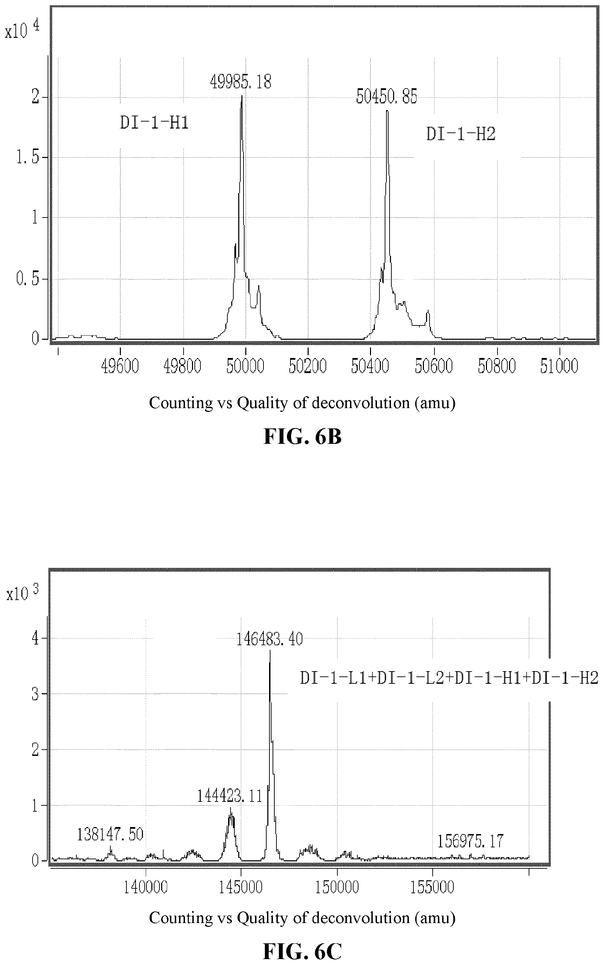

DI-1 was detected by using the method of Test Example 3 disclosed herein, and four chains of DI-1 were co-transfected into cells for expression and then subjected to mass spectrometry. The experimental results are shown in Table 11 and FIGs. 6A-6C, homodimers and mispaired molecules were undetectable in mass spectrometry results, and DI-1 was correctly assembled.

**Table 11. DI-1 mass spectrometry data**

| DI-1 chain | **Chain 1** (DI-1-H1) | **Chain 2** (DI-1-L1) | **Chain 3** (DI-1-H2) | **Chain 4** (DI-1-L2) | DI-1-L1 +DI-1-L2+ DI-1-H1+DI-1-H2 | MS results |
|---|---|---|---|---|---|---|
| Molecular weight | 49983.82 | 23487.38 | 50450.05 | 22567.45 | 146488.70 | Correct, homodimers and mispaired molecules undetectable |

In addition, BU5 expression was detected by using the methods of Test Example 3 and Test Example 2 disclosed herein. Firstly, the four chains of BU5 were co-transfected into cells for expression of the bispecific antibody, with B0 and U0 as controls. Then, the expressed product was subjected to purity and mass spectrometry analysis. The experimental results are shown in Table 12 and FIGs. 9A-9C, and the results show that the bispecific antibody BU5 was successfully expressed, and four chains of BU5 were successfully assembled into the target molecules without mispairing. In addition, the purity of the bispecific molecule BU5 was high, and the SEC% reached 88%.

**Table 12. BU5 mass spectrometry results**

| Antibody | Mass spectrometry |
|---|---|
| Control B0 | ✔ |
| Control U0 | ✔ |
| BU5 | ✔ |

| | |
|---|---|
| Note: In the table, "✔" indicates correct pairing. | |

In addition, the constructed BU1-BU4 bispecific antibody was detected for antibody purity and antigen binding activity by using the methods of Test Example 2 and Test Example 4 disclosed herein. The experimental results are shown in Table 13, and the results showed that the bispecific antibody with substituted CH1/CL still maintained good antigen binding activity and had high antibody purity.

**Table 13. Experimental results of antigen binding activity and purity of bispecific antibody**

| Name of antibody | Purity of antibody SEC (%) | Antigen binding EC50 (nM) | |
|---|---|---|---|
| | | Antigen BAFF | Antigen P40 |
| B0 | 99.05 | 0.2193 | - |
| U0 | 99.63 | - | 0.08881 |
| BU1 | 95.23 | 0.3561 | 0.08397 |
| BU2 | 91.09 | 0.4693 | 0.05674 |
| BU3 | 83.45 | 0.4131 | 0.2238 |
| BU4 | 91.85 | 0.5057 | 0.04429 |

### Test Examples

### Test Example 1: Method for Measuring Expression Level of Antibody

HEK293E cells were transfected with expression plasmids of monoclonal antibodies or bispecific antibodies, and after 6 days expression supernatants were collected and centrifuged at high speed to remove impurities. The supernatant was purified using Protein A column (GE Healthcare). The column was washed with PBS until the A280 reading dropped to a baseline, and the column was washed with 100 mM acetate buffer (pH 3.5) and neutralized with 1 M Tris-HCl, pH 8.0. The expression level of the antibody was quantified from the amount of the antibody/expression volume obtained by the final purification.

### Test Example 2: Method for Detecting Purity of Antibody

The purity of an antibody was monitored using SEC-HPLC. Detection was performed according to the instrument operating instructions using a Waters e2695 chromatograph, wherein Waters Xbridge BEH 200A SEC column was applied, PBS was taken as a mobile phase (pH was adjusted to 6.8 with dilute hydrochloric acid), 100 µg of protein was injected, and gradient elution was performed, with a flow rate of 0.5 mL/min. The purity of antibody was indicated as the percentage of the peak area of the main peak to the total peak area (SEC (%)).

### Test Example 3: Method for Detecting Antibody by Mass Spectrometry

(I) Sample preparation:
1. Determination of complete molecular weight after deglycosylation: taking 30 µg of expressed antibody, adding 10 µL of 8 M Gua-HCl after freeze-drying, and performing water bath denaturation at 70 °C for 10 min; adding 90 µL of distilled water, taking 30 µL and adding 0.8 µL of PNG enzyme F (peptide N-glycosidase F), placing the resulting solution in a water bath at 37 °C for incubation for 2 h, and taking 0.5 µg for determination of complete molecular weight after deglycosylation.
2. Determination of molecular weight after deglycosylation and reduction: taking 30 µg of expressed antibody, adding 10 µL of 8 M Gua-HCl after freeze-drying, and performing water bath denaturation at 70 °C for 10 min; adding 90 µL of distilled water, taking 30 µL and adding 0.8 µL of PNG enzyme F (peptide N-glycosidase F), and placing in a water bath at 37 °C for incubation for 2 h; and then adding 2 µL of 0.025 M DTT, reducing for 10 min in a water bath at 70 °C, and taking 0.5 µg for determination of the molecular weight after deglycosylation and reduction.

(II) Chromatography-mass spectrometry conditions:
Chromatographic conditions are as follows:

| | |
|---|---|
| Chromatographic column: Poroshell | 300SB-C8 5 µm 2.1 × 75 mm |
| Mobile phase: A: 0.1% HCOOH/H₂O | B: 0.1% HCOOH/CAN |

Column temperature: 75 °C
Gradient:

| Time (min) | 0 | 5 | 8 | 12 | 12.1 | 15 |
|---|---|---|---|---|---|---|
| B% | 5 | 5 | 95 | 95 | 5 | 5 |

Mass spectrometry conditions are as follows:
Mass spectrum: Agilent 6530 Q-TOF LC-MS
Ionization mode ESI
Acquisition mode: 1 GHz (mass range: 500-5000 m/z)
Temperature of drying gas: 325 °C
Flow rate of drying gas: 10 L/min
Atomizer: 40 psi
Temperature of sheath gas: 350 °C
Flow rate of sheath gas: 12 L/min
Spray voltage: 500 V
Capillary voltage: 3500 V
Cleavage voltage: 200 V
Skimmer voltage: 65 V
Rf voltage: 7500 V

Mass spectrometry was performed on the antibody to be detected, and the experimental results showed that the antibody remained being correctly assembled after CH1/CL was substituted with Titin-T/Obscurin-O.

### Test Example 4: Method for Detecting Antibody Binding Activity

The binding activity of an antibody against a membrane protein target to an antigen may be detected using FACS. For example, C0, N0, F0, V0 and S0 and CH1/CL domain engineered antibodies thereof may be detected using FACS method. FACS buffer (98% PBS, 2% FBS) resuspended cells (2 × 10⁶ cells/mL, 90 µL) were added to a 96-well U-shaped bottom plate (corning, 3795), and 10 µL of gradiently-diluted antibody was added; the reaction mixture was incubated at 4 °C for 1 h, washed 2 times with an FACS buffer, then added with Alexa Fluor 488 goat anti-human IgG (H + L) (invitrogen, Cat # 2015982, 1:1000 dilution) per well, incubated at 4 °C for 1 h and then washed 2 times, cells were resuspended with an FACS buffer, and finally fluorescence signal values were read using FACS CantoII (BD). Finally, the data were processed and analyzed using FlowJo 7.6 and Graphpad Prism 5.

For antibodies targeting soluble proteins, the binding activity of the antibodies to the soluble proteins was measured using ELISA, for example, D0, I0, B0, U0, H0, R0 and J0 and CH1/CL domain-engineered antibodies thereof may be measured using ELISA. The method comprises the following steps: diluting the protein to 1 µg/mL with a PBS (Basalmedia, B320) buffer (pH 7.4), adding the resulting solution to a 96-well microplate (Corning, 9018) at a volume of 100 µL/well, and incubating at 4 °C overnight; after the solution was discarded, adding 300 µL of 5% skim milk (BD, 232100) diluted with PBS to each well for blocking, and incubating at 37 °C for 2 h; after blocking was completed, discarding the blocking solution, and after the plate was washed 3 times with a PBST buffer (pH 7.4, PBS containing 0.1% tween-20), adding 100 µL of gradiently-diluted antibody solution to each well and incubating at 37 °C for 1 h; after the incubation was completed, washing the plate 3 times with PBST, adding 100 µL of mouse anti-human IgG (H + L) (Jackson ImmunoResearch, 209-035-088, 1:8000 dilution) to each well, and incubating at 37 °C for 1 h; and after the plate was washed 3 times with PBST, adding 100 µL of TMB chromogenic substrate (KPL, 5120-0077) to each well and incubating at room temperature for 10-15 min, adding 50 µL of 1 M H₂SO₄ to stop the reaction, reading absorption values at 450 nm using a microplate reader, and fitting a binding curve of antibodies to antigens using software to calculate EC50 values.

The antigens corresponding to the antibodies in this test example are as follows: the antigen binding to D0 and a CH1/CL domain-engineered antibody thereof was hRANKL (available from Sino biological, 11682-HNCH); the antigen binding to 10 and a CH1/CL domain-engineered antibody thereof was hNGF (available from Sino biological, 11050-HNAC); the antigen binding to B0 and a CH1/CL domain-engineered antibody thereof was hBAFF (available from Sino biological, 10056-HNCH); the antigen binding to U0 and a CH1/CL domain-engineered antibody thereof was hP40 (available from Sino biological, 10052-H08H); the antigen binding to H0 and R0 and CH1/CL domain-engineered antibodies thereof was hIL-5 (available from R&D systems, 205-IL-025/CF); the antigen binding to J0 and a CH1/CL domain-engineered antibody thereof was hTSLP (available from Sino biological, 16135-H08H); the antigen binding to C0 and N0 and CH1/CL domain-engineered antibodies thereof was CEA (overexpression in CEA cells MKN45 (available from Nanjing Cobioer, CBP60488)); the antigen binding to F0, V0 and S0 and CH1/CL domain-engineered antibodies thereof was B7H3 (overexpression in B7H3 recombinant cell lines CT26-B7H3 (CT26 was derived from the Chinese Academy of Cell Bank, and the sequence of B7H3 is shown in ID: XP_005254757.1)).

### Test Example 5: Experimental Method for Mispairing Reduction in CH1/CL Domain Engineered Antibody

### I. Detection method for expression of light/heavy chain cross-mispaired molecules of different antibodies

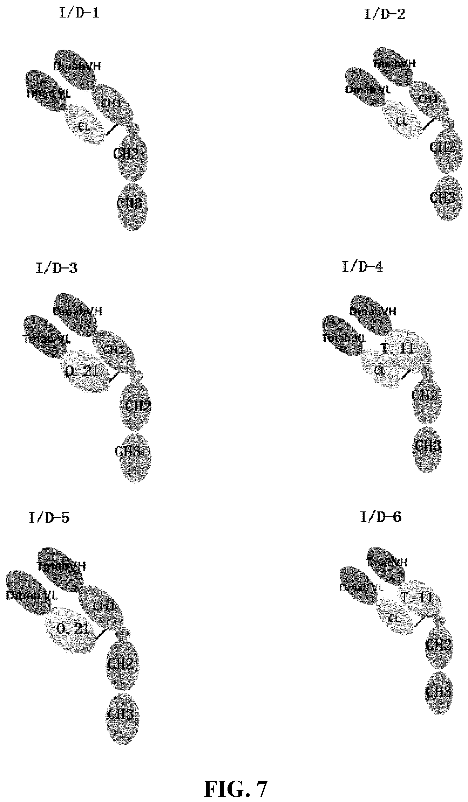

The experiment verified that CH1/CL could effectively reduce the heavy/light chain mispairing after being substituted with a Titin-T chain/Obscurin-O chain or a Titin-T chain/Obscurin-like-O chain through analysis of the paired expression of different light/heavy chain antibodies. Specifically, when the transfected cells expressed the antibodies, heavy/light chains of D0 and I0 were respectively exchanged (i.e., I/D-1 (heavy chain of D0 + light chain of I0); I/D-2 (heavy chain of I0 + light chain of D0)) was to detect whether cross-mispaired molecules would be formed in wild-type CH1 and CL; meanwhile, heavy and light chains of antibody D3 (heavy chain CH1 of D0 was substituted with T.11, light chain CL was substituted with 0.21) and antibody I3 (heavy chain CH1 of I0 was substituted with T.11, and light chain CL was substituted with 0.21) were respectively exchanged after CH1/CL of D0 and I0 was substituted with the Titin-T chain/Obscurin-O chain, and the mispaired molecules I/D-3 (heavy chain of D0 + light chain of 13), I/D-4 (heavy chain of D3 + light chain of I0), I/D-5 (heavy chain of I0 + light chain of 13) and I/D-6 (heavy chain of I3 + light chain of D0) were constructed; the schematic structural diagrams of the constructed antibodies are shown in FIG. 7.

The expression level of the antibodies was measured by using the method of Test Example 1, and whether the mispaired molecules would be formed was determined by mass spectrometry of the expression product by using the method of Test Example 3. The experimental results are shown in Table 14, and the experimental results showed that when the heavy chain of D0 + the light chain of I0 or the heavy chain of I0 + the light chain of D0 were co-expressed, complete IgG molecules (150 kDa) could be formed, with high expression levels of 74.4 mg/L and 73.8 mg/L respectively; and LC-MS results showed that the obtained proteins were mismatched IgG molecules, which indicated that if CH1-CL was not engineered or replaced, a large amount of light/heavy chain mispaired molecules could be formed when four different heavy and light chains of the bispecific antibody were co-expressed. After CH1/CL was substituted with a Titin-T chain/Obscurin-O chain, no target molecules (150 kDa) were generated in four forms of I/D-3 (heavy chain of D0 + light chain of 13), I/D-4 (heavy chain of D3 + light chain of I0), I/D-5 (heavy chain of I0 + light chain of 13) and I/D-6 (heavy chain of I3 + light chain of D0) through cross-mispairing expression, and no mispaired molecules were generated in the other three forms except that a small amount of error molecules (the molecular weight was 100 kDa) of two heavy chains were formed by the combination of the heavy chain of D3 and the light chain of I0, which indicated that the non-homologous pairing after modification cannot form mispaired molecules in an IgG structural form. Thus, substitution of CH1-CL with a Titin-T chain/Obscurin-O chain effectively avoids or reduces the formation of non-homologous cross-pairing molecules between the light/heavy chains of IgG-like bispecific antibodies.

**Table 14. Expression level of antibodies with different cross-paired light/heavy chains and mass spectrometry analysis**

| Name of mispairing | Heavy chain | Light chain | Expression level of antibody (mg/L) | Mass spectrometry |
|---|---|---|---|---|
| I/D-1 | Heavy chain of D0 | Light chain of 10 | 74.4 | √ |
| I/D-2 | Heavy chain of 10 | Light chain of D0 | 73.8 | √ |
| I/D-3 | Heavy chain of D0 | Light chain of 13 | 0.04 | × |
| I/D-4 | Heavy chain of D3 | Light chain of 10 | 12.75 | Only heavy chain, without light chain |
| I/D-5 | Heavy chain of 10 | Light chain of D3 | 0.04 | × |
| I/D-6 | Heavy chain of 13 | Light chain of D0 | 0.25 | × |

| | | | | |
|---|---|---|---|---|
| Note: In the table, "√" indicates that the mispaired molecule was detected, and "×" indicates that the mispaired molecule was undetectable. | | | | |

### II. Detection method for expression of mispaired light/heavy chains of bispecific antibodies

To verify that there was no mispairing or mispairing reduction in CH1/CL which had been substituted with the Titin-T chain/Obscurin-O chain, the test example designed three forms of light/heavy chain cross-pairing expression, namely, two different heavy chains and one identical VL-CL light chain for bispecific antibody expression, or two different heavy chains and one identical light chain with CL substituted with the Obscurin-O chain for expression, or two different heavy chains and two different light chains for normal expression as control (see Table 15 for specific pairings).

The expression product was then subjected to mass spectrometry (for the method, see Test Example 3 disclosed herein) and the antibody purity SEC was detected (for the method, see Test Example 2 disclosed herein). The experimental results are shown in Table 15, and the results showed that the correct molecules were formed when the light chain substituted with the Obscurin-O chain and the light chain not substituted with CL were added together, and the correct target molecules were not found in the cross-pairing when only one light chain not substituted with CL was added or only one light chain with CL substituted with the Obscurin-O chain was added. This indicates that the light chain with CL substituted with the Obscurin-O chain could be more easily bound to the heavy chain with CH1 substituted with the Titin-T chain to form a molecule, but would not cross-mispaired with the heavy chain with CH1 not substituted with the Titin-T chain; the light chain with CL not substituted with the Obscurin-O chain was more easily bound to the heavy chain with CH1 not substituted with the Titin-T chain, but would not bound to the heavy chain with CH1 substituted with the Titin-T chain.

**Table 15. Detection results of light/heavy chain mispairing of bispecific antibodies**

| Name of mispairing | First heavy chain (B-H1) | Second heavy chain (B-H2) | First light chain (B-L1) | Second light chain (B-L2) | Mass spectrometry | | | | Purity of antibody SEC (%) | Expression level of antibody (mg/L) |
|---|---|---|---|---|---|---|---|---|---|---|
| | | | | | B-H1 | B-H1 | B-H1 | B-H2 | | |
| | | | | | B-H2 | B-H2 | B-H2 | B-H2 | | |
| | | | | | B-L1 | B-L1 | B-L2 | B-L2 | | |
| | | | | | B-L2 | B-L1 | B-L2 | B-L2 | | |
| B/U-1 | Chain formed by substitution of CH1 of heavy chain of B0 with T11 (with hole modification) | Heavy chain of U0 (with knob modification) | Chain formed by substitution of CL of light chain of B0 with O.21 | Light chain of U0 | ✔ | -- | -- | -- | 91 | 25.6 |
| B/U-2 | Heavy chain of B0 (with hole modification) | Heavy chain of U0 (with knob modification) | Chain formed by substitution of CL of light chain of B0 with O.21 | Chain formed by substitution of CL of light chain of B0 with O.21 | -- | -- | -- | -- | -- | 0.5 |
| B/U-3 | Chain formed by substitution of CH1 of heavy chain of B0 with T11 (with hole modification) | Heavy chain of U0 (with knob modification) | Light chain of U0 | Light chain of U0 | -- | -- | -- | ✔ | 33 | 15.6 |

| | | | | | | | | | | |
|---|---|---|---|---|---|---|---|---|---|---|
| Note: In the table, "✔' indicates that the four-chain-paired molecule was detected, and "-" indicates that the four-chain-paired molecule was undetectable. | | | | | | | | | | |

### Test Example 6: Detection of Affinity of Bispecific Antibody to Antigen

The affinity of the antibody to be detected to the antigen protein was determined by using Biacore T200 (GE) instrument. According to the method in the specification of a human anti-capture kit (GE, Cat # BR-1008-39), a Protein A biosensor chip (Cat # 29127556, GE) was used for affinity capture of an antibody to be detected, then soluble antigens with a series of concentration gradients flowed through the surface of the chip, and a Biacore T200 instrument was used for real-time detection of reaction signals, so that a binding-dissociation curve was obtained. After dissociation was completed for each cycle, the biosensor chip was washed with a glycine-hydrochloric acid regeneration solution (pH 1.5, Cat # BR-1003-54, GE). The data obtained from the experiment were fitted to the data using BIAevaluation version 4.1, GE software using a (1:1) Langmuir model to obtain affinity values, and the experimental results are shown in Table 16.

The experimental results indicate that the domain engineered bispecific antibody disclosed herein fully retained the affinity of the two parent monoclonal antibodies to antigens thereof. In this experiment, included were antigens hRANKL (available from Sino biological, 11682-HNCH), hNGF (available from Sino biological, 11050-HNAC), hBAFF (available from Sino biological, 10056-HNCH), hP40 (available from Sino biological, 10052-H08H), hB7H3 (prepared in the laboratory, for the sequence, see SEQ ID NO: 111), and hCD3 (prepared in the laboratory, a heterodimer consisting of the δ subunit (for the sequence, see SEQ ID NO: 112) and the ε subunit (for the sequence, see SEQ ID NO: 113) of hCD3).

**Table 16. Detection results of affinity of bispecific antibody**

| Antibody | Antigen | ka (1/Ms) | kd (1/s) | KD (M) |
|---|---|---|---|---|
| B0 | hBAFF | 1.77E+05 | 1.52E-04 | 8.55E-10 |
| U0 | hP40 | 1.44E+06 | 1.14E-04 | 7.93E-11 |
| BU5 | hBAFF | 1.94E+05 | 7.62E-05 | 3.93E-10 |
| | hP40 | 1.54E+06 | 1.02E-04 | 6.64E-11 |
| BU1 | hBAFF | 3.38E+05 | 1.69E-04 | 4.99E-10 |
| | hP40 | 1.38E+06 | 4.81E-05 | 3.49E-11 |
| D0 | hRANKL | 2.36E+04 | 2.10E-04 | 8.89E-09 |
| I0 | hNGF | 1.92E+06 | 1.19E-04 | 6.21E-11 |
| DI-1 | hRANKL | 3.05E+04 | 9.36E-05 | 3.06E-09 |
| | hNGF | 2.56E+06 | 8.95E-05 | 3.50E-11 |
| A0 | hCD3 | 8.01E+04 | 4.92E-03 | 6.14E-08 |
| F0 | hB7H3 | 1.57E+05 | 2.06E-03 | 1.31E-08 |
| FA-1 | hCD3 | 7.06E+04 | 4.25E-03 | 6.02E-08 |
| | hB7H3 | 1.58E+05 | 7.12E-03 | 4.50E-08 |

### Test Example 7: Experiment for Simultaneous Binding of CH1-CL Domain-Engineered Diabodies to Two Target Antigens

The CH1-CL domain-engineered bispecific antibodies disclosed herein were verified to bind to two target antigens simultaneously by determining the affinity of the antibody to be detected to the antigenic protein using a Biacore T200 (GE) instrument. The specific experimental steps were as follows: the antibody BU5 was affinity-captured by using a Protein A biosensor chip (Cat # 29127556, GE), then the first antigen molecule hP40 (available from Sino biological, 10052-H08H) of the bispecific antibody flowed through the surface of the chip to reach saturation, and then a second antigen molecule hBAFF (available from Sino biological, 10056-HNCH) was injected at a certain concentration, and reaction signals were detected in real time by using a Biacore T200 instrument to obtain a binding-dissociation curve. After dissociation was completed for each cycle, the biosensor chip was washed with a glycine-hydrochloric acid regeneration solution (pH 1.5, Cat # BR-1003-54, GE), and the data fitting model was obtained using BIAevaluation version 4.1, GE software using a (1:1) Langmuir model. Then, the antibody BU5 was affinity-captured by using a Protein A biosensor chip (Cat # 29127556, GE), the first antigen molecule hP40 (available from Sino biological, 10052-H08H) of the bispecific antibody flowed through the surface of the chip to reach saturation, a second antigen molecule hBAFF (available from Sino biological, 10056-HNCH) was then injected at a certain concentration, and the reaction signal was detected in real time by using a Biacore T200 instrument to obtain a binding-dissociation curve. After dissociation was completed for each cycle, the biosensor chip was washed with a glycine-hydrochloric acid regeneration solution (pH 1.5, Cat # BR-1003-54, GE), and the data fitting model was obtained using BIAevaluation version 4.1, GE software using a (1:1) Langmuir model.

The experimental results are shown in Table 17, and the results showed that the bispecific antibody BU5 could continue to bind to the antigen hBAFF after binding to the antigen hP40 to reach saturation, and had the ability of rebinding to hBAFF comparable to the monoclonal antibody B0. Similarly, the bispecific antibody BU5 could continue to bind to the antigen hP40 after binding to the antigen hBAFF to reach saturation, and had the ability of rebinding to hP40 comparable to U0. This indicates that the bispecific antibody disclosed herein with CH1/CL substituted with a Titin-T chain/Obscurin-O chain had a dual-target feature and could bind to two target antigens simultaneously.

**Table 17. Detection results of affinity of antibodies**

| Antibody | Antigen flowed through chip surface | Affinity detection data | | | |
|---|---|---|---|---|---|
| | | Antigen | ka (1/Ms) | kd (1/s) | KD (M) |
| Bmab | hBAFF | hBAFF | 1.77E+05 | 1.53E-04 | 8.64E-10 |
| Umab | hP40 | hP40 | 1.59E+06 | 1.09E-04 | 6.89E-11 |
| BU5 | Injecting hBAFF for binding to reach saturation, reinjecting hP40 antigen | hP40 | 1.57E+06 | 1.04E-04 | 6.61E-11 |
| | Injecting hP40 for binding to reach saturation, | hBAFF | 1.57E+05 | 8.44E-05 | 5.37E-10 |
| | reinjecting hBAFF antigen | | | | |

### Test Example 8: Experiment of Osteoclast Differentiation of DI-1 Bispecific Antibody

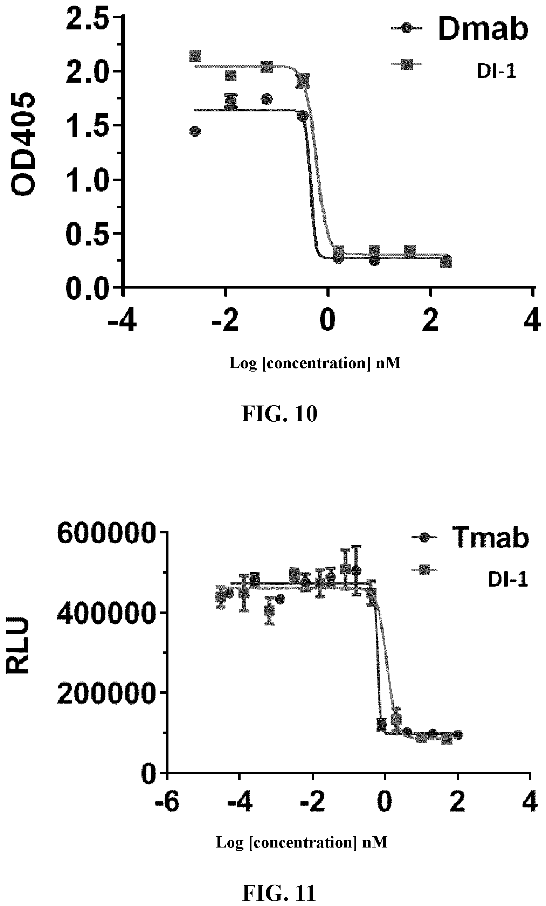

Raw264.7 cells (the Chinese Academy of Cell Bank, SCSP-5036) were digested and counted in resuspension, plated in a 24-well cell culture plate (Corning, 3524) and cultured overnight in a cell incubator at 37 °C. The next day, the antibody solution was diluted to different concentrations and mixed homogeneously with RANKL (Sino biological, 11682-HNCH) in the cell culture plate to a final concentration of 50 ng/mL, and the cells were incubated at 37 °C. After 96 h, the solution in the 24-well plate was removed, 100 µL of cell lysate (Beyotime, P0013J) was added to each well, the mixture was pipetted and mixed homogeneously, the lysate was transferred to an EP tube and centrifuged at 12000 g for 5 min, the supernatant was taken and measured using an anti-tartaric acid phosphatase test kit (Beyotime, P0332) according to the method described in the specification, and the absorption values at 405 nm were read using a microplate reader. The measured values were fitted to a curve by using software, and the IC50 values were calculated. The experimental results are shown in FIG. 10, and the experimental results showed that the bispecific antibody DI-1 with substituted CH1/CL maintained good activity and effectively inhibited osteoclast differentiation.

### Test Example 9: Experiment of TF1 Cell Proliferation of DI-1 Bispecific Antibody

The activity of the antibody at the cellular level was assessed in an NGF-induced TF-1 cell (ATCC, CRL-2003) proliferation experiment. The experimental method was as follows: TF1 cells were digested and collected, resuspended, then counted, plated in a 96-well plate (Corning, 3903), and incubated overnight in an incubator at 37 °C. The next day, the antibody solution was diluted to different concentrations and mixed homogeneously with NGF (Sino biological, 11050-HNAC) and added to the cell culture plate to a final concentration of 10 ng/mL, and the cells were incubated in the incubator. After 72 h, the cell culture plate was removed, 50 µL of Cell-titer Glo (Promega, G755B) assay solution was added to each well, the reaction solution was incubated on a shaker for 10 min and allowed to stand at room temperature for 10 min. Luminescence signals were detected using a microplate reader (PerkinElmer, Victor 3). The detected signal values were fitted to a graph using software, and the IC50 values were calculated. The experimental results are shown in FIG. 11, and the results showed that the bispecific antibody DI-1 with substituted CH1/CL had good activity.

### Test Example 10: Detection of Light and Heavy Chain Mispairing of Bispecific Antibody with CH1/CL Substituted with Different Domains

The following experiments were performed to analyze the light and heavy chain mispairing of bispecific antibodies with CH1/CL substituted with a Titin-T chain/Obscurin-O chain or with TCRα/TCRβ, and the specific experiments were as follows:

### I. Experiment of light and heavy chain mispairing during co-expression of four chains of bispecific antibody

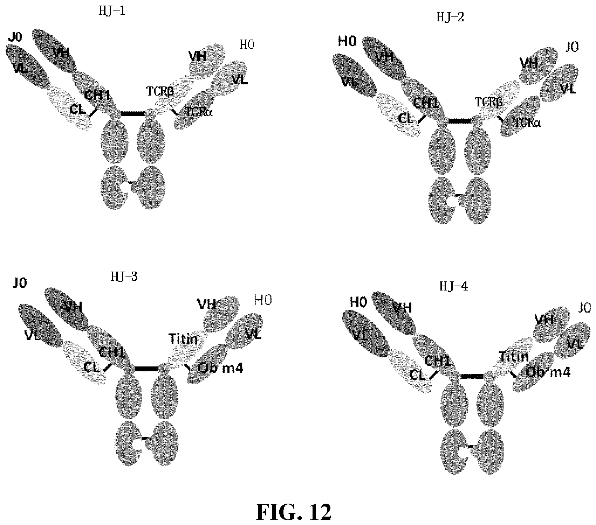

Bispecific antibodies HJ-1 and HJ-2 each consisting of four chains were constructed by substituting CH1/CL of H0 or J1 with TCRα/TCRβ, and bispecific antibodies HJ-3 and HJ-4 each consisting of four chains were constructed by substituting CH1/CL of H0 or J1 with the Titin-T chain/Obscurin-O chain; the schematic structural diagrams of the bispecific antibodies are shown in FIG. 12, and the full-length sequences of the bispecific antibodies are shown in Table 18:

**Table 18. Bispecific antibody sequence listing**

| Name of diabody | Four-chain sequence of IgG-like bispecific antibody | | | |
|---|---|---|---|---|
| | Chain 1 | Chain 3 | Chain 2 | Chain 4 |
| HJ-1 | Heavy chain of H0 (knob), and CH1 is substituted with TCRβ | Light chain of H0, and CL is substituted with TCRα | Heavy chain of J1 (hole) | Light chain of J1 |
| HJ-1-H1: EVQLVESGGGLVQPG GSLRLSCAASGFTFSH YYMAWVRQAPGKGL EWVTSISYEGDITYYG DSVKGRFTISRDNSKN TLYLQMNSLRAEDTA TYYCASQTLRESFDY WGQGTLVTVSSLEDL KNVFPPEVAVFEPSEA EISHTQKATLVCLATG FYPDHVELSWWVNGK EVHSGVCTDPQPLKEQ PALQDSRYALSSRLRV SATFWQNPRNHFRCQ VQFYGLSENDEWTQD RAKPVTQIVSAEAWG RASDKTHTCPPCPAPE AAGGPSVFLFPPKPKD TLMISRTPEVTCVVVD VSHEDPEVKFNWYVD GVEVHNAKTKPREEQ YNSTYRVVSVLTVLH QDWLNGKEYKCKVSN KALPAPIEKTISKAKGQ PREPQVYTLPPCREEM TKNQVSLWCLVKGFY PSDIAVEWESNGQPEN NYKTTPPVLDSDGSFF LYSKLTVDKSRWQQG NVFSCSVMHEALHNH YTQKSLSLSPGKHHHH HH (SEQ ID NO: 114) | HJ-1-L1: DIQMTQSPSSVS ASVGDRVTITCR ASQDIANYLSW YQQKPGKSPKLL IYGTSNLEVGVP SRFSGSRSGTDF TLTISSLQPEDFA TYYCLQDKEFPR TFGGGTKVEIKK PDIQNPDPAVYQ LRDSKSSDKSVC LFTDFDSQTQVS QSKDSDVYITDK CVLDMRSMDFK SNSAVAWSQKS DFACANAFQNSI IPEDTFFPSPESS (SEQ ID NO: 115) | | HJ-1-H2: EVQLVQSGAEVKKPGSS VKVSCKASGYTFSNYLI EWVRQAPGQGLEWIGV IDPGVGDTNYNENFKGR ATLTADKSTSTAYIELSS LRSEDTAVYYCAREDN TGTAFDYWGQGTTVTV SSASTKGPSVFPLAPSSK STSGGTAALGCLVKDYF PEPVTVSWNSGALTSGV HTFPAVLQSSGLYSLSS VVTVPSSSLGTQTYICN VNHKPSNTKVDKKVEP KSCDKTHTCPPCPAPEA AGGPSVFLFPPKPKDTL MISRTPEVTCVVVDVSH EDPEVKFNWYVDGVEV HNAKTKPREEQYNSTY RVVSVLTVLHQDWLNG KEYKCKVSNKALPAPIE KTISKAKGQPREPQVCT LPPSREEMTKNQVSLSC AVKGFYPSDIAVEWESN GQPENNYKTTPPVLDSD GSFFLVSKLTVDKSRWQ QGNVFSCSVMHEALHN HYTQKSLSLSPGKDYKD DDDK (SEQ ID NO: 116) | HJ-1-L2: SIVMTQTPLS LSVTPGQPA SISCKASQSV SSDVTWYLQ KPGQSPQLLI YYVSEHYTG VPDRFSGSG YGTDFTLKIS RVEAEDVGV YYCQQHHRF PLTFGQGTK LEIKRTVAAP SVFIFPPSDE QLKSGTASV VCLLNNFYP REAKVQWK VDNALQSGN SQESVTEQD SKDSTYSLSS TLTLSKADY EKHKVYACE VTHQGLSSP VTKSFNRGE C (SEQ ID NO: 117) |
| HJ-2 | Heavy chain of H0 (hole) | Light chain of H0 | Heavy chain of J1 (knob), and CH1 is substituted with TCRβ | Light chain of J1, and CL is substituted with TCRα |
| | HJ-2-H1: | HJ-2-L1: | HJ-1-H2: | HJ-2-L2: |
| | EVQLVESGGGLVQPG GSLRLSCAASGFTFSH YYMAWVRQAPGKGL EWVTSISYEGDITYYG DSVKGRFTISRDNSKN TLYLQMNSLRAEDTA TYYCASQTLRESFDY WGQGTLVTVSSASTK GPSVFPLAPSSKSTSGG TAALGCLVKDYFPEPV TVSWNSGALTSGVHTF PAVLQSSGLYSLSSVV TVPSSSLGTQTYICNV NHKPSNTKVDKKVEP KSCDKTHTCPPCPAPE AAGGPSVFLFPPKPKD TLMISRTPEVTCVVVD VSHEDPEVKFNWYVD GVEVHNAKTKPREEQ YNSTYRVVSVLTVLH QDWLNGKEYKCKVSN KALPAPIEKTISKAKGQ PREPQVCTLPPSREEM TKNQVSLSCAVKGFYP SDIAVEWESNGQPENN YKTTPPVLDSDGSFFL VSKLTVDKSRWQQGN VFSCSVMHEALHNHY TQKSLSLSPGKDYKDD DDK (SEQ ID NO: 118) | DIQMTQSPSSVS ASVGDRVTITCR ASQDIANYLSW YQQKPGKSPKLL IYGTSNLEVGVP SRFSGSRSGTDF TLTISSLQPEDFA TYYCLQDKEFPR TFGGGTKVEIKR TVAAPSVFIFPPS DEQLKSGTASVV CLLNNFYPREAK VQWKVDNALQS GNSQESVTEQDS KDSTYSLSSTLT LSKADYEKHKV YACEVTHQGLSS PVTKSFNRGEC (SEQ ID NO: 119) | EVQLVQSGAEVKKPGSS VKVSCKASGYTFSNYLI EWVRQAPGQGLEWIGV IDPGVGDTNYNENFKGR ATLTADKSTSTAYIELSS LRSEDTAVYYCAREDN TGTAFDYWGQGTTVTV SSLEDLKNVFPPEVAV FEPSEAEISHTQKATLV CLATGFYPDHVELSW WVNGKEVHSGVCTDP QPLKEQPALQDSRYAL SSRLRVSATFWQNPRN HFRCQVQFYGLSENDE WTQDRAKPVTQIVSAE AWGRASDKTHTCPPCP APEAAGGPSVFLFPPKP KDTLMISRTPEVTCVVV DVSHEDPEVKFNWYVD GVEVHNAKTKPREEQY NSTYRVVSVLTVLHQD WLNGKEYKCKVSNKAL PAPIEKTISKAKGQPREP QVYTLPPCREEMTKNQ VSLWCLVKGFYPSDIAV EWESNGQPENNYKTTPP VLDSDGSFFLYSKLTVD KSRWQQGNVFSCSVMH EALHNHYTQKSLSLSPG KHHHHHH (SEQ ID NO: 120) | SIVMTQTPLS LSVTPGQPA SISCKASQSV SSDVTWYLQ KPGQSPQLLI YYVSEHYTG VPDRFSGSG YGTDFTLKIS RVEAEDVGV YYCQQHHRF PLTFGQGTK LEIKKPDIQN PDPAVYQLR DSKSSDKSV CLFTDFDSQ TQVSQSKDS DVYITDKCV LDMRSMDF KSNSAVAW SQKSDFACA NAFQNSIIPE DTFFPSPESS (SEQ ID NO: 121) |
| HJ-3 | Heavy chain of H0 (knob), and CH1 is substituted with T.11 | Light chain of H0, and CL is substituted with O.21 | Heavy chain of J1 (hole) | Light chain of J1 |
| | HJ-3-H1: EVQLVESGGGLVQPG GSLRLSCAASGFTFSH YYMAWVRQAPGKGL EWVTSISYEGDITYYG DSVKGRFTISRDNSKN TLYLQMNSLRAEDTA TYYCASQTLRESFDY WGQGTLVTVSSGGGG SGIPPKIECLPIDISIDE GKVLTVASAFTGEPT PEVTWSTGGRKIHSQ EQGRFHIENTDDLTT LIIKDVQKQDGGLYT LTLRNEFGSDSATVNI HIRSIDKTHTCPPCPAP EAAGGPSVFLFPPKPK DTLMISRTPEVTCVVV DVSHEDPEVKFNWYV DGVEVHNAKTKPREE QYNSTYRVVSVLTVL HQDWLNGKEYKCKVS NKALPAPIEKTISKAKG QPREPQVYTLPPCREE MTKNQVSLWCLVKGF | HJ-3-L1: DIQMTQSPSSVS ASVGDRVTITCR ASQDIANYLSW YQQKPGKSPKLL IYGTSNLEVGVP SRFSGSRSGTDF TLTISSLQPEDFA TYYCLQDKEFPR TFGGGTKVEIKG GGGSSGAPRFL TRPLAFVVSVG KDATLSSQIVG NPTPQVSWEKD KQPVTAGARFR LAQDGDLYRLK ILDLQLSDSGQY VSRARNAIGEA FACLGLQVDAE A (SEQ ID NO: 123) | HJ-3-H2: EVQLVQSGAEVKKPGSS VKVSCKASGYTFSNYLI EWVRQAPGQGLEWIGV IDPGVGDTNYNENFKGR ATLTADKSTSTAYIELSS LRSEDTAVYYCAREDN TGTAFDYWGQGTTVTV SSASTKGPSVFPLAPSSK STSGGTAALGCLVKDYF PEPVTVSWNSGALTSGV HTFPAVLQSSGLYSLSS VVTVPSSSLGTQTYICN VNHKPSNTKVDKKVEP KSCDKTHTCPPCPAPEA AGGPSVFLFPPKPKDTL MISRTPEVTCVVVDVSH EDPEVKFNWYVDGVEV HNAKTKPREEQYNSTY RVVSVLTVLHQDWLNG KEYKCKVSNKALPAPIE KTISKAKGQPREPQVCT LPPSREEMTKNQVSLSC AVKGFYPSDIAVEWESN GQPENNYKTTPPVLDSD | HJ-3-L2: SIVMTQTPLS LSVTPGQPA SISCKASQSV SSDVTWYLQ KPGQSPQLLI YYVSEHYTG VPDRFSGSG YGTDFTLKIS RVEAEDVGV YYCQQHHRF PLTFGQGTK LEIKRTVAAP SVFIFPPSDE QLKSGTASV VCLLNNFYP REAKVQWK VDNALQSGN SQESVTEQD SKDSTYSLSS TLTLSKADY EKHKVYACE VTHQGLSSP VTKSFNRGE C (SEQ ID |
| | YPSDIAVEWESNGQPE NNYKTTPPVLDSDGSF FLYSKLTVDKSRWQQ GNVFSCSVMHEALHN HYTQKSLSLSPGKHHH HHH (SEQ ID NO: 122) | | GSFFLVSKLTVDKSRWQ QGNVFSCSVMHEALHN HYTQKSLSLSPGKDYKD DDDK(SEQ ID NO: 116) | NO: 117) |
| HJ-4 | Heavy chain of H0 (hole) | Light chain of H0 | Heavy chain of J1 (knob), and CH1 is substituted with T.11 | Light chain of J1, and CL is substituted with O.21 |
| | HJ-4-H1: EVQLVESGGGLVQPG GSLRLSCAASGFTFSH YYMAWVRQAPGKGL EWVTSISYEGDITYYG DSVKGRFTISRDNSKN TLYLQMNSLRAEDTA TYYCASQTLRESFDY WGQGTLVTVSSASTK GPSVFPLAPSSKSTSGG TAALGCLVKDYFPEPV TVSWNSGALTSGVHTF PAVLQSSGLYSLSSVV TVPSSSLGTQTYICNV NHKPSNTKVDKKVEP KSCDKTHTCPPCPAPE AAGGPSVFLFPPKPKD TLMISRTPEVTCVVVD VSHEDPEVKFNWYVD GVEVHNAKTKPREEQ YNSTYRVVSVLTVLH QDWLNGKEYKCKVSN KALPAPIEKTISKAKGQ PREPQVCTLPPSREEM TKNQVSLSCAVKGFYP SDIAVEWESNGQPENN YKTTPPVLDSDGSFFL VSKLTVDKSRWQQGN VFSCSVMHEALHNHY TQKSLSLSPGKDYKDD DDK (SEQ ID NO: 118) | HJ-4-L1: DIQMTQSPSSVS ASVGDRVTITCR ASQDIANYLSW YQQKPGKSPKLL IYGTSNLEVGVP SRFSGSRSGTDF TLTISSLQPEDFA TYYCLQDKEFPR TFGGGTKVEIKR TVAAPSVFIFPPS DEQLKSGTASVV CLLNNFYPREAK VQWKVDNALQS GNSQESVTEQDS KDSTYSLSSTLT LSKADYEKHKV YACEVTHQGLSS PVTKSFNRGEC (SEQ ID NO: 119) | HJ-4-H2: EVQLVQSGAEVKKPGSS VKVSCKASGYTFSNYLI EWVRQAPGQGLEWIGV IDPGVGDTNYNENFKGR ATLTADKSTSTAYIELSS LRSEDTAVYYCAREDN TGTAFDYWGQGTTVTV SSGGGGSGIPPKIECLP IDISIDEGKVLTVASAF TGEPTPEVTWSTGGR KIHSQEQGRFHIENTD DLTTLIIKDVQKQDGG LYTLTLRNEFGSDSAT VNIHIRSIDKTHTCPPCP APEAAGGPSVFLFPPKP KDTLMISRTPEVTCVVV DVSHEDPEVKFNWYVD GVEVHNAKTKPREEQY NSTYRVVSVLTVLHQD WLNGKEYKCKVSNKAL PAPIEKTISKAKGQPREP QVYTLPPCREEMTKNQ VSLWCLVKGFYPSDIAV EWESNGQPENNYKTTPP VLDSDGSFFLYSKLTVD KSRWQQGNVFSCSVMH EALHNHYTQKSLSLSPG KHHHHHH (SEQ ID NO: 124) | HJ-4-L2: SIVMTQTPLS LSVTPGQPA SISCKASQSV SSDVTWYLQ KPGQSPQLLI YYVSEHYTG VPDRFSGSG YGTDFTLKIS RVEAEDVGV YYCQQHHRF PLTFGQGTK LEIKGGGGS SGAPRFLTR PLAFVVSVG KDATLSSQI VGNPTPQVS WEKDKQPV TAGARFRL AQDGDLYR LKILDLQLS DSGQYVSRA RNAIGEAFA CLGLQVDA EA (SEQ ID NO: 125) |

| | | | | |
|---|---|---|---|---|
| Note: In the sequence, the bold part is a substituted portion, the dotted underlined part is a constant region portion, and a single underlined part is a variable region. | | | | |

Heavy chain of J1: 
EVQLVQSGAEVKKPGSSVKVSCKASGYTFSNYLIEWVRQAPGQGLEWIGVIDP
GVGDTNYNENFKGRATLTADKSTSTAYIELSSLRSEDTAVYYCAREDNTGTAF
DYWGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLYITREPEVTCVVVDV
SHEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFS
CSVMHEALHNHYTQKSLSLSPGK
(SEQ ID NO: 126);
Light chain of J1:

SIVMTQTPLSLSVTPGQPASISCKASQSVSSDVTWYLQKPGQSPQLLIYYVSEHY
TGVPDRFSGSGYGTDFTLKISRVEAEDVGVYYCQQHHRFPLTFGQGTKLEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
(SEQ ID NO: 117);

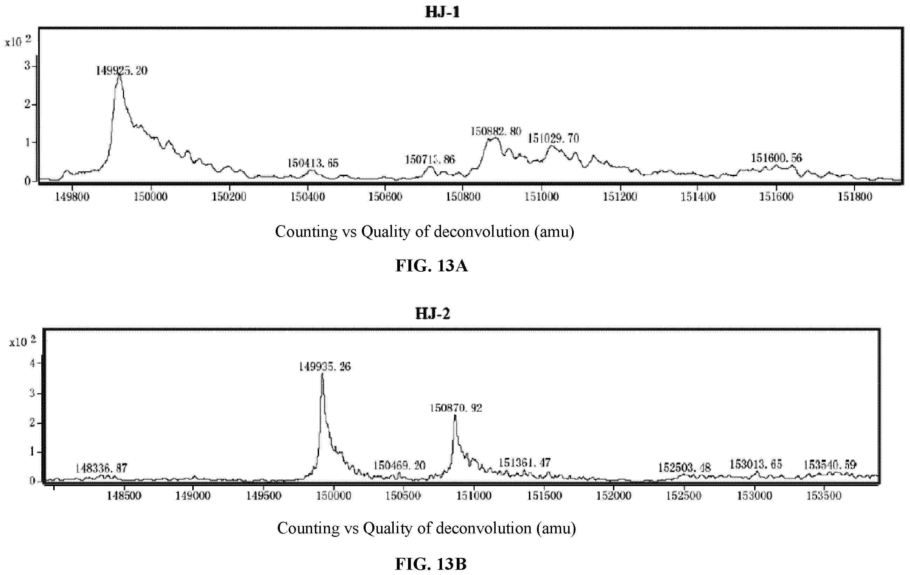

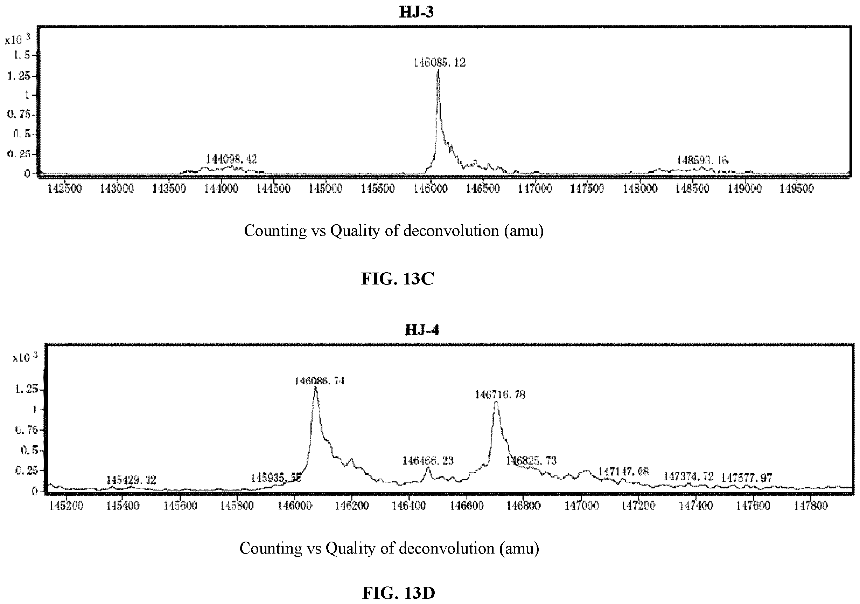

The four chains of HJ-1, HJ-2, HJ-3 and HJ-4 were co-transfected for expression respectively, and then mass spectrometry was carried out on the expression products (for the method, see Test Example 3 disclosed herein) to detect the presence of the light and heavy chain mispaired molecules. The experimental results are shown in FIGs. 13A to 13D and Table 19.

**Table 19. Experimental results of mass spectrometry of expression of bispecific antibodies**

| **Name of diabody** | **Co-transfected chains** | **Results of mass spectrometry** | | |
|---|---|---|---|---|
| | | **HJ (1+2+3+4)** | **Mispaired molecule 1 (1+2+3+3)** | **Mispaired molecule 2 (1 + 2+4+4)** |
| HJ-1 | 1:HJ-1-H1 | √ | **X** | √ |
| | 2: HJ-1-H2 | | | |
| | 3: HJ-1-L1 | | | |
| | 4: HJ-1-L2 | | | |
| HJ-2 | 1: HJ-2-H2 | √ | **X** | √ |
| | 2: HJ-2-H1 | | | |
| | 3: HJ-2-L2 | | | |
| | 4: HJ-2-L1 | | | |
| HJ-3 | 1: HJ-3-H1 | √ | **X** | **X** |
| | 2: HJ-3-H2 | | | |
| | 3: HJ-3-L1 | | | |
| | 4: HJ-3-L2 | | | |
| HJ-4 | 1: HJ-4-H2 | √ | **X** | **X** |
| | 2: HJ-4-H1 | | | |
| | 3: HJ-4-L2 | | | |
| | 4: HJ-4-L1 | | | |

| | | | | |
|---|---|---|---|---|
| Note: In the table, "√" indicates that the molecule was detected, "X" indicates that the molecule was undetectable, "HJ (1 + 2 + 3 + 4)" indicates a bispecific antibody formed by the four corresponding chains numbered 1, 2, 3 and 4 on the left side thereof, "mispaired molecule 1 (1 + 2 + 3 + 3)" indicates a mispaired molecule 1 formed by the four corresponding chains numbered 1, 2, 3 and 3 on the left side thereof; and "mispaired molecule 2 (1 + 2 + 4 + 4)" indicates a mispaired molecule 2 formed by the four corresponding chains numbered 1, 2, 4 and 4 on the left side thereof. | | | | |

Experimental results showed that when four chains of the bispecific antibody with CH1/CL substituted with TCRβ/TCRα were co-transfected for expression, the light and heavy chains of the antibody were mispaired. However, the bispecific antibody with CH1/CL substituted with the Titin-T chain/Obscurin-O chain had no mispairing of light and heavy chains. This indicates that the bispecific antibody disclosed herein with CH1/CL substituted with the Titin-T chain/Obscurin-O chain had excellent ability of reducing the mispairing of light and heavy chains.

### II. Experiment of three-chain co-expression of bispecific antibody

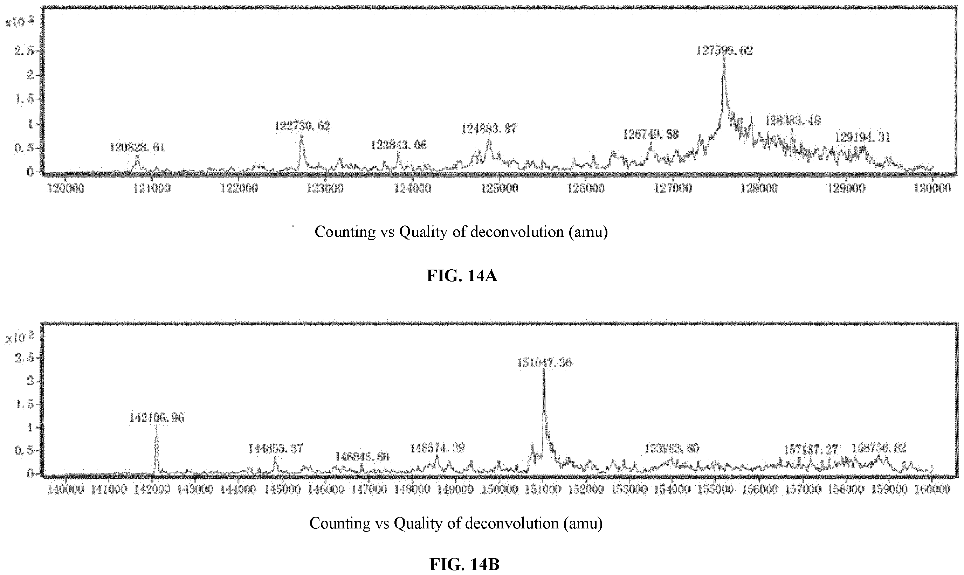

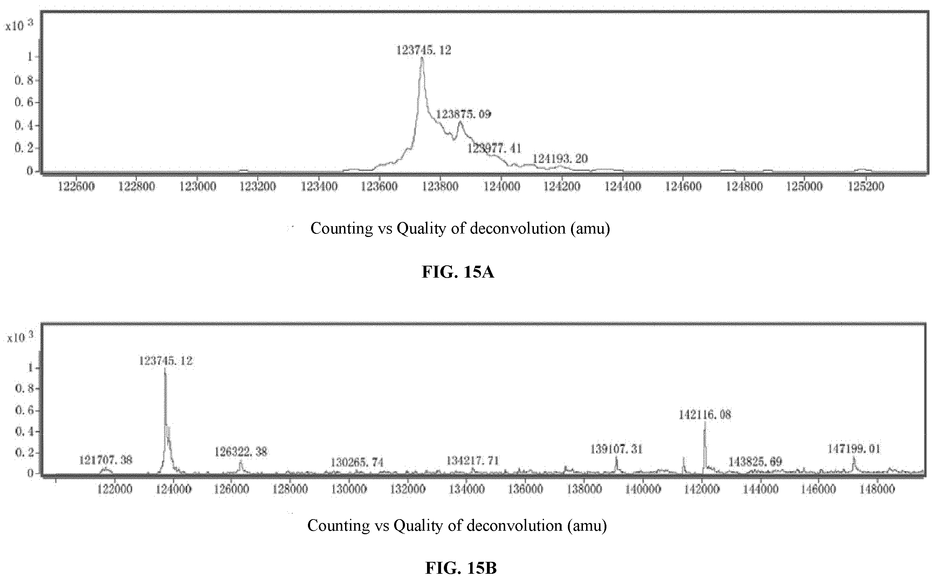

The experiment was performed by co-transfecting and expressing three chains (1 heavy chain with CH1 substituted with TCRβ or Titin-T chain, 1 heavy chain with CH1 not substituted, and 1 wild-type light chain (VL-CL) with CL not substituted, and specific sequences are shown in Table 18), then performing mass spectrometry on the expression product (for the method, see Test Example 3 disclosed herein) and detecting antibody purity SEC (for the method, see Test Example 2 disclosed herein) to verify whether the wild-type light chain (VL-CL) and the heavy chain with CH1 substituted with TCRβ or Titin-T chain were bound to form a mispaired molecule. The experimental results are shown in FIG. 14A, FIG. 14B, FIG. 15A, FIG. 15B and Table 20.

**Table 20. Experimental results of three-chain co-expression of bispecific antibody**

| **Group** | **Co-transfected chains** | **Antibody purity SEC (%)** | **Results of mass spectrometry** | |
|---|---|---|---|---|
| | | | **Molecule (1+2+3)** | **Mispaired molecule (1+2+3+3)** |
| 1 | 1: HJ-1-H1 | 75.85 | √ | √ |
| | 2: HJ-1-H2 | | | |
| | 3: HJ-1-L2 | | | |
| 2 | 1: HJ-3-H1 | 96.82 | √ | **X** |
| | 2: HJ-3-H2 | | | |
| | 3: HJ-3-L2 | | | |

| | | | | |
|---|---|---|---|---|
| Note: In the table, "√" indicates that the molecule was detected, "X" indicates that the molecule was undetectable, "molecule (1 + 2 + 3)" indicates a molecule formed by the three corresponding chains numbered 1, 2 and 3 on the left side thereof, and "mispaired molecule (1 + 2 + 3 + 3)" indicates a mispaired molecule formed by the four corresponding chains 1, 2, 3 and 3 on the left side thereof. | | | | |

The experimental results showed that VL-CL was readily bound to the heavy chain with CH1 substituted with TCRβ to express a mispaired molecule with two identical light chains. However, the VL-CL light chain did not bind to the heavy chain with CH1 substituted with the Titin-T chain to form a mispaired molecule with two identical light chains, which further indirectly indicated that the bispecific antibody with CH1 of heavy chain substituted with the Titin-T chain disclosed herein had more excellen ability of reducing the mispairing of light and heavy chains compared to the bispecific antibody with CH1 of heavy chain substituted with TCRβ.

## Claims

1. A polypeptide complex, comprising a first antigen-binding moiety comprising:
A) a first polypeptide, comprising a first heavy chain variable domain (VH1) from an N-terminus to a C-terminus, the VH1 being operably linked to a first domain comprising a Titin-T chain, and
a second polypeptide, comprising a first light chain variable domain (VL1) from an N-terminus to a C-terminus, the VL1 being operably linked to a second domain comprising a domain capable of interacting with a Titin-T chain;
or,
B) a first polypeptide, comprising a VH1 from an N-terminus to a C-terminus, the VH1 being operably linked to a first domain comprising a domain capable of interacting with a Titin-T chain, and
a second polypeptide, comprising a VL1 from an N-terminus to a C-terminus, the VL1 being operably linked to a second domain comprising a Titin-T chain;
or,
C) a first polypeptide, comprising a VH1 from an N-terminus to a C-terminus, the VH1 being operably linked to a first domain comprising an Obscurin-O chain or an Obscurin-like-O chain, and
a second polypeptide, comprising a VL1 from an N-terminus to a C-terminus, the VL1 being operably linked to a second domain comprising a domain capable of interacting with an Obscurin-O chain or an Obscurin-like-O chain;
or,
D) a first polypeptide, comprising a VH1 from an N-terminus to a C-terminus, the VH1 being operably linked to a first domain comprising a domain capable of interacting with an Obscurin-O chain or an Obscurin-like-O chain, and
a second polypeptide, comprising a VL1 from an N-terminus to a C-terminus, the VL1 being operably linked to a second domain comprising an Obscurin-O chain or an Obscurin-like-O chain;
wherein the first antigen-binding moiety specifically binds to a first antigen, and the first domain and the second domain are capable of forming a dimer.

2. The polypeptide complex according to claim 1, wherein,
the domain capable of interacting with an Obscurin-O chain or an Obscurin-like-O chain is a Titin-T chain, and
the domain capable of interacting with a Titin-T chain is an Obscurin-O chain or an Obscurin-like-O chain.

3. The polypeptide complex according to claim 1 or 2, wherein the VH1 and the VL1 of the first antigen-binding moiety form a first antigen-binding site specifically binding to the first antigen; the C-terminus of the VH1 is operably linked to an N-terminus of the first domain, and the C-terminus of the VL1 is operably linked to an N-terminus of the second domain.

4. The polypeptide complex according to any one of claims 1 to 3, wherein the first domain and the second domain form a dimer through natural inter-chain bonds and/or non-natural inter-chain bonds;
preferably, the first domain and the second domain form a dimer through natural inter-chain bonds;
more preferably, one or more residues selected from the group consisting of positions 7-15, 19-24, 26, 55, 59 and 60 on the Titin-T chain and one or more residues selected from the group consisting of positions 3-6, 9, 41, 73, 75 and 80-90 on the Obscurin-O chain are linked to each other; or
one or more residues selected from the group consisting of positions 1, 7-10, 13-16, 19-26, 59-60 and 96 on the Titin-T chain and one or more residues selected from the group consisting of positions 4-5, 10, 12-13, 74, 76, 78 and 82-91 on the Obscurin-like-O chain are linked to each other;
the residue position in the Titin-T chain is a natural sequence numbering position relative to a sequence SEQ ID NO: 32; the residue position in the Obscurin-O chain is a natural sequence numbering position relative to a sequence SEQ ID NO: 33; the residue position in the Obscurin-like-O chain is a natural sequence numbering position relative to a sequence SEQ ID NO: 34.

5. The polypeptide complex according to any one of claims 1 to 4, wherein the first domain and the second domain form a dimer through at least one non-natural inter-chain bonds;
preferably, the non-natural inter-chain bond is formed between a particular mutated residue of the first domain and a particular mutated residue of the second domain;
more preferably, at least one pair of the mutated residues is cysteine residues;
most preferably, the non-natural inter-chain bond is a disulfide bond;
more preferably, wherein the dimer comprises 1, 2, 3, 4, 5, 6, 7, 8, 9 or 10 non-natural inter-chain bonds.

6. The polypeptide complex according to claim 5, wherein the particular mutated residue of the first domain and the particular mutated residue of the second domain are selected from the group consisting of the following mutations:
(A) the Titin-T chain has one or more amino acid residue mutations on positions selected from the group consisting of 8, 20, 22, 25, 26 and 39, and/or the Obscurin-O chain has one or more amino acid residue mutations on positions selected from the group consisting of 3, 9, 25, 76 and 88; or
(B) the Titin-T chain has one or more amino acid residue mutations on positions selected from the group consisting of 8, 20, 22, 25, 26 and 39, and/or the Obscurin-like-O chain has one or more amino acid residue mutations on positions selected from the group consisting of 6, 26, 74, 77, 84 and 86;
preferably, the Titin-T chain has one or more amino acid residue mutations on positions selected from the group consisting of A8C, V20C, T22C, C25S, A26C and C39T, and/or the Obscurin-O chain has one or more amino acid residue mutations on positions selected from the group consisting of A3C, R9C, C25S, C76S and A88C; or
the Titin-T chain has one or more amino acid residue mutations on positions selected from the group consisting of A8C, V20C, T22C, C25S, A26C and C39T, and/or the Obscurin-like-O chain has one or more amino acid residue mutations on positions selected from the group consisting of C6E, C26S, C77S, V74C, G84C and A86C;
more preferably, the mutated residue of the first domain and the mutated residue of the second domain are selected from the group consisting of the following mutations:
the Titin-T chain has C25S, C39T and A8C mutations, and the Obscurin-O chain has an A88C mutation;
the Titin-T chain has C25S, C39T and V20C mutations, and the Obscurin-O chain has an A3C mutation;
the Titin-T chain has C25S, C39T and A26C mutations, and the Obscurin-O chain has an R9C mutation;
the Titin-T chain has C25S, C39T and A8C mutations, and the Obscurin-O chain has C25S, C76S and A88C mutations;
the Titin-T chain has C25S, C39T and V20C mutations, and the Obscurin-O chain has C25S, C76S and A3C mutations;
the Titin-T chain has C25S, C39T and A26C mutations, and the Obscurin-O chain has C25S, C76S and R9C mutations;
the Titin-T chain has C25S, C39T and A8C mutations, and the Obscurin-like-O chain has C6E and V74C mutations;
the Titin-T chain has C25S, C39T and V20C mutations, and the Obscurin-like-O chain has C6E and G84C mutations;
the Titin-T chain has C25S, C39T and T22C mutations, and the Obscurin-like-O chain has C6E and A86C mutations;
the Titin-T chain has C25S, C39T and A8C mutations, and the Obscurin-like-O chain has C6E, C26S, C77S and V74C mutations;
the Titin-T chain has C25S, C39T and V20C mutations, and the Obscurin-like-O chain has C6E, C26S, C77S and G84C mutations; or
the Titin-T chain has C25S, C39T and T22C mutations, and the Obscurin-like-O chain has C6E, C26S, C77S and A86C mutations;
the mutation position in the Titin-T chain is a natural sequence numbering position relative to a sequence SEQ ID NO: 32; the mutation position in the Obscurin-O chain is a natural sequence numbering position relative to a sequence SEQ ID NO: 33; the mutation position in the Obscurin-like-O chain is a natural sequence numbering position relative to a sequence SEQ ID NO: 34.

7. The polypeptide complex according to claim 6, wherein the Obscurin-O chain further has one or more amino acid residue mutations on positions selected from the group consisting of 7, 11 and 62;
preferably, the Obscurin-O chain has one or more amino acid residue mutations on positions selected from the group consisting of L7R and L7K, T62K and T62H, and K11L;
most preferably, the Titin-T chain has C25S, C39T and A8C mutations, and the Obscurin-O chain has C25S, C76S, A88C, L7K and T62K mutations;
the Titin-T chain has C25S, C39T and A8C mutations, and the Obscurin-O chain has C25S, C76S, A88C, L7K and T62H mutations;
the Titin-T chain has C25S, C39T and A8C mutations, and the Obscurin-O chain has C25S, C76S, A88C, K11L and T62K mutations; or
the Titin-T chain has C25S, C39T and A8C mutations, and the Obscurin-O chain has C25S, C76S, A88C, K11L and T62H mutations;
the mutation position in the Titin-T chain is a natural sequence numbering position relative to a sequence SEQ ID NO: 32; the mutation position in the Obscurin-O chain is a natural sequence numbering position relative to a sequence SEQ ID NO: 33.

8. The polypeptide complex according to any one of claims 1 to 7, wherein the first domain and the second domain have one or more amino acid residue mutations on positions selected from the group consisting of:
the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84, and/or the Obscurin-O chain has one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97;
preferably, the first domain and the second domain have one or more residue mutations on positions selected from the group consisting of:
the Titin-T chain has one or more amino acid mutations on positions selected from the group consisting of P3W, S11I, I13L, T22M, G40S, R42K, H45S, Q47E, Q49G, N56S, D58E, M66S and M66K, K70R, L75V, T77S, S79T, G81R, N82M, E83D and F84L, and/or the Obscurin-O chain has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G;
more preferably, the Titin-T chain has M66S and T77S amino acid mutations, and/or the Obscurin-O chain has L11K, A12S, F13Y, V14T and T22S amino acid mutations;
the Titin-T chain has M66K, K70R, S79T and G81R amino acid mutations, and/or the Obscurin-O chain has G2E, V17E, N30D, T32P, Q34E, S36T, V44I, A45T, L58V, K62E, A67Q, G69S and A97G amino acid mutations;
the Titin-T chain has P3W, S11I, I13L, T22M and N82M amino acid mutations, and/or the Obscurin-O chain has D20L, T22M and A53L amino acid mutations;
the Titin-T chain has S11I, M66K, S79T and G81R amino acid mutations, and/or the Obscurin-O chain has Q41K, A45T, A67Q, G69S and V89L amino acid mutations;
the Titin-T chain has G40S, R42K, H45S, Q47E, Q49G, N56S, D58E, L75V, E83D and F84L amino acid mutations, and/or the Obscurin-O chain has Q42L, A45T, A67T, G69S, Q92E and D94G amino acid mutations;
the Titin-T chain has Q47E, Q49G, N56S, D58E and L75V amino acid mutations, and/or the Obscurin-O chain has Q42L, A45T, A67T, G69S, Q92E and D94G amino acid mutations;
the Titin-T chain has N56S, D58E and L75V amino acid mutations, and/or the Obscurin-O chain has Q42L, A45T, A67T, G69S, Q92E and D94G amino acid mutations; or
the Titin-T chain has N56S, D58E, M66S and T77S amino acid mutations, and/or the Obscurin-O chain has A12S, F13Y, T22S, Q42L, A45T, A67Q, G69S, Q92E and D94G amino acid mutations;
the mutation position in the Titin-T chain is a natural sequence numbering position relative to a sequence SEQ ID NO: 35; the mutation position in the Obscurin-O chain is a natural sequence numbering position relative to a sequence SEQ ID NO: 50.

9. The polypeptide complex according to any one of claims 1 to 8, wherein the VH1 is operably linked to the first domain through a first linking domain, and the VL1 is operably linked to the second domain through a second linking domain;
preferably, the first linking domain and/or the second linking domain is selected from the group consisting of: an N-terminal fragment of the Titin-T chain, an N-terminal fragment of the Obscurin-O chain, an N-terminal fragment of the Obscurin-like-O chain, and a (GₓS)_{y} linker, wherein X is selected from the group consisting of integers from 1 to 5, and Y is selected from the group consisting of integers from 1 to 6;
more preferably, the first linking domain and/or the second linking domain is selected from the group consisting of the following polypeptide fragments: "KAGIR", "DQPQF", (G₄S)₁ and (G₄S)₂;
most preferably, the first domain is the Titin-T chain, the first linking domain is the KAGIR polypeptide, the second domain is the Obscurin-O chain, and the second linking domain is the DQPQF polypeptide; or
the second domain is the Titin-T chain, the second linking domain is the KAGIR polypeptide, the first domain is the Obscurin-O chain, and the first linking domain is the DQPQF polypeptide.

10. The polypeptide complex according to any one of claims 1 to 9, wherein,
(A) the Titin-T chain has a sequence set forth in SEQ ID NO: 32 or further has a sequence having one or more amino acid mutations on positions selected from the group consisting of 3, 8, 11, 13, 20, 22, 25, 26, 39, 40, 42, 45, 47, 49, 56, 58, 66, 70, 75, 77, 79, 81, 82, 83 and 84 and/or a KAGIR amino acid residue added at the N-terminus on the basis of SEQ ID NO: 32;
preferably, the Titin-T chain has a sequence having one or more amino acid mutations on positions selected from the group consisting of A8C, V20C, T22C, C25S, A26C, C39T, P3W, S11I, I13L, T22M, G40S, R42K, H45S, Q47E, Q49G, N56S, D58E, M66S and M66K, K70R, L75V, T77S, S79T, G81R, N82M, E83D and F84L and/or a KAGIR amino acid residue added at the N-terminus; and/or
(B) the Obscurin-O chain has a sequence set forth in SEQ ID NO: 33 or further has a sequence having one or more amino acid mutations on positions selected from the group consisting of 2, 3, 7, 9, 11, 12, 13, 14, 17, 20, 22, 25, 30, 32, 34, 36, 41, 42, 44, 45, 53, 58, 62, 67, 69, 76, 88, 89, 92, 94 and 97 and/or a DQPQF amino acid residue added at the N-terminus on the basis of SEQ ID NO: 33; preferably, the Obscurin-O chain has a sequence having one or more amino acid mutations on positions selected from the group consisting of A3C, R9C, C25S, C76S, A88C, L7K, T62K and T62H, G2E, L11K, A12S, F13Y, V14T, V17E, D20L, T22M and T22S, N30D, T32P, Q34E, S36T, Q41K, Q42L, V44I, A45T, A53L, L58V, T62E, A67Q and A67T, G69S, V89L, Q92E, D94G and A97G and/or a DQPQF amino acid residue added at the N-terminus; or
the Obscurin-like-O chain has a sequence set forth in SEQ ID NO: 34 or further has a sequence having one or more amino acid mutations on positions selected from the group consisting of 6, 26, 74, 77, 84 and 86 on the basis of SEQ ID NO: 34; preferably, the Obscurin-like-O chain has a sequence having one or more amino acid mutations on positions selected from the group consisting of C6E, C26S, C77S, V74C, G84C and A86C;
the mutation position in the Titin-T chain is a natural sequence numbering position of the sequence SEQ ID NO: 32; the mutation position in the Obscurin-O chain is a natural sequence numbering position of the sequence SEQ ID NO: 33; the mutation position in the Obscurin-like-O chain is a natural sequence numbering position of the sequence SEQ ID NO: 34;
preferably, wherein,
A) the Titin-T chain has one or more amino acid residue mutations on positions selected from the group consisting of 8, 20, 22, 25, 26 and 39 on the basis of SEQ ID NO: 32, and preferably has one or more amino acid residue mutations on positions selected from the group consisting of A8C, V20C, T22C, C25S, A26C and C39T, and the mutation position in the Titin-T chain is a natural sequence numbering position of SEQ ID NO: 32;
more preferably, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and preferably has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L; the mutation position in the Titin-T chain is a natural sequence numbering position of SEQ ID NO: 35, and/or
B) the Obscurin-O chain has one or more amino acid residue mutations on positions selected from the group consisting of 3, 9, 25, 76 and 88 on the basis of SEQ ID NO: 33, and preferably has one or more amino acid residue mutations on positions selected from the group consisting of A3C, R9C, C25S, C76S and A88C, and the mutation position in the Obscurin-O chain is a natural sequence numbering position of SEQ ID NO: 33;
more preferably, the Obscurin-O chain has one or more amino acid residue mutations on positions selected from 7, 11 and 62 on the basis of SEQ ID NO: 45, and preferably has one or more amino acid residue mutations on positions selected from the group consisting of L7K and L7R, T62K and T62H, and K11L, and the mutation position in the Obscurin-O chain is a natural sequence numbering position of SEQ ID NO: 45;
most preferably, the Obscurin-O chain has one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97 on the basis of SEQ ID NO: 50, and preferably has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G, and the mutation position in the Obscurin-O chain is a natural sequence numbering position of SEQ ID NO: 50;
or,
A) the Titin-T chain has one or more amino acid residue mutations on positions selected from the group consisting of 8, 20, 22, 25, 26 and 39 on the basis of SEQ ID NO: 32, and preferably has one or more amino acid residue mutations on positions selected from the group consisting of A8C, V20C, T22C, C25S, A26C and C39T, and the mutation position in the Titin-T chain is a natural sequence numbering position of SEQ ID NO: 32;
more preferably, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and preferably has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L; the mutation position in the Titin-T chain is a natural sequence numbering position of SEQ ID NO: 35, and/or
B) the Obscurin-like-O chain has one or more amino acid residue mutations on positions selected from the group consisting of 6, 26, 74, 77, 84 and 86 on the basis of SEQ ID NO: 34, and preferably has one or more amino acid residue mutations on positions selected from the group consisting of C6E, C26S, C77S, V74C, G84C and A86C, and the mutation position in the Obscurin-like-O chain is a natural sequence numbering position of SEQ ID NO: 34.

11. The polypeptide complex according to any one of claims 1 to 10, wherein the Titin-T chain comprises a sequence set forth in any one of SEQ ID NOs: 32, 35-41, 65-76 or a sequence having at least 80% sequence identity to any one of SEQ ID NOs: 32, 35-41, 65-76, and/or the Obscurin-O chain comprises a sequence set forth in any one of SEQ ID NOs: 33, 42-58, 77-86 or a sequence having at least 80% sequence identity to any one of SEQ ID NOs: 33, 42-58, 77-86; or the Titin-T chain comprises a sequence set forth in any one of SEQ ID NOs: 32, 35-41, 65-76 or a sequence having at least 80% sequence identity to any one of SEQ ID NOs: 32, 35-41, 65-76, and/or the Obscurin-like-O chain comprises a sequence set forth in any one of SEQ ID NOs: 34, 59-64 or a sequence having at least 80% sequence identity to any one of SEQ ID NOs: 34, 59-64;
more preferably, the Titin-T chain comprises a sequence set forth in SEQ ID NO: 68, and the Obscurin-O chain comprises a sequence set forth in SEQ ID NO: 80;
the Titin-T chain comprises a sequence set forth in SEQ ID NO: 73, and the Obscurin-O chain comprises a sequence set forth in SEQ ID NO: 83;
the Titin-T chain comprises a sequence set forth in SEQ ID NO: 76, and the Obscurin-O chain comprises a sequence set forth in SEQ ID NO: 84;
the Titin-T chain comprises a sequence set forth in SEQ ID NO: 76, and the Obscurin-O chain comprises a sequence set forth in SEQ ID NO: 86; or
the Titin-T chain comprises a sequence set forth in SEQ ID NO: 75, and the Obscurin-O chain comprises a sequence set forth in SEQ ID NO: 86.

12. The polypeptide complex according to any one of claims 1 to 11, wherein the first antigen is selected from the group consisting of: a foreign antigen, an endogenous antigen, an autoantigen, a neoantigen, a viral antigen and a tumor antigen.

13. A multispecific polypeptide complex, comprising:
a first polypeptide complex, being the polypeptide complex according to any one of claims 1 to 12 and specifically binding to a first antigen, and
a second polypeptide complex, comprising a second antigen-binding moiety and specifically binding to a second antigen,
the first polypeptide complex and the second polypeptide complex binding to two different antigens or binding to two different epitopes on the same antigen; wherein
preferably, the second polypeptide complex comprises a second heavy chain variable domain (VH2) and a second light chain variable domain (VL2), and mispairing between the VH1 of the first polypeptide complex and the VL2 of the second polypeptide complex, and/or between the VH2 of the second polypeptide complex and the VL1 of the first polypeptide complex is less susceptible to happen; more preferably, the multispecific polypeptide complex is a bispecific polypeptide complex; most preferably, the VH2 and the VL2 of the second antigen-binding moiety form a second antigen-binding site specifically binding to the second antigen.

14. The multispecific polypeptide complex according to claim 13, wherein the second antigen-binding moiety of the second polypeptide complex comprises:
a third polypeptide, comprising a VH2 from an N-terminus to a C-terminus, the VH2 being operably linked to a third domain comprising a CHI, and
a fourth polypeptide, comprising a VL2 from an N-terminus to a C-terminus, the VL2 being operably linked to a fourth domain comprising a CL; wherein
preferably, the C-terminus of the VH2 is operably linked to the N-terminus of the CHI, and the C-terminus of the VL2 is operably linked to the N-terminus of the CL.

15. The multispecific polypeptide complex according to claim 13 or 14, wherein the first polypeptide complex further comprises a first dimerization domain, and the second polypeptide complex further comprises a second dimerization domain, the first dimerization domain and the second dimerization domain being bound together; preferably, a C-terminus of the first domain of the first polypeptide complex is operably linked to an N-terminus of the first dimerization domain, and a C-terminus of the third domain of the second polypeptide complex is operably linked to an N-terminus of the second dimerization domain;
more preferably, the first dimerization domain and the second dimerization domain are bound by a means selected from the group consisting of: an antibody hinge region and a portion thereof, a linker, a disulfide bond, a hydrogen bond, an electrostatic interaction, a salt bridge, a hydrophobic-hydrophilic interaction, and a combination thereof;
most preferably, the first dimerization domain and the second dimerization domain are bound by a means selected from the group consisting of: an antibody hinge region and a portion thereof, and preferably hinge regions and portions thereof of IgG1, IgG2, IgG3 and IgG4.

16. The multispecific polypeptide complex according to claim 15, wherein the first dimerization domain comprises an antibody CH2 domain and/or an antibody CH3 domain, and/or the second dimerization domain comprises an antibody CH2 domain and/or an antibody CH3 domain;
preferably, the antibody CH2 domain and/or the antibody CH3 domain is derived from IgG1, IgG2, IgG3 or IgG4.

17. The multispecific polypeptide complex according to claim 15 or 16, wherein the first dimerization domain and the second dimerization domain are not identical and are bound in a manner that prevents homodimerization and/or favors heterodimerization;
preferably, the first dimerization domain and the second dimerization domain are bound by a means selected from the group consisting of: knob-into-hole, a hydrophobic interaction, an electrostatic interaction, a hydrophilic interaction, and a flexibility increase;
more preferably, the first dimerization domain has amino acid residues at positions 104 to 330 of a sequence set forth in SEQ ID NO: 2, and the second dimerization domain has amino acid residues at positions 104 to 330 of a sequence set forth in SEQ ID NO: 3; or
the first dimerization domain has amino acid residues at positions 104 to 330 of a sequence set forth in SEQ ID NO: 3, and the second dimerization domain has amino acid residues at positions 104 to 330 of a sequence set forth in SEQ ID NO: 2.

18. A domain engineered antibody, wherein at least one heavy chain constant region domain CH1 and at least one light chain constant region domain CL of the antibody are substituted, wherein,
A) the domain CH1 is substituted with a Titin-T chain, and the domain CL is substituted with a domain capable of having an inter-protein interaction with the Titin-T chain;
B) the domain CL is substituted with the Titin-T chain, and the domain CH1 is substituted with a domain capable of having an inter-protein interaction with the Titin-T chain;
C) the domain CH1 is substituted with an Obscurin-O chain, and the domain CL is substituted with a domain capable of having an inter-protein interaction with the Obscurin-O chain;
D) the domain CL is substituted with the Obscurin-O chain, and the domain CH1 is substituted with a domain capable of having an inter-protein interaction with the Obscurin-O chain;
E) the domain CH1 is substituted with an Obscurin-like-O chain, and the domain CL is substituted with a domain capable of having an inter-protein interaction with the Obscurin-like-O chain; or
F) the domain CL is substituted with the Obscurin-like-O chain, and the domain CH1 is substituted with a domain capable of having an inter-protein interaction with the Obscurin-like-O chain;
preferably, the domain capable of having an inter-protein interaction with the Obscurin-O chain or the Obscurin-like-O chain is the Titin-T chain, and
the domain capable of having an inter-protein interaction with the Titin-T chain is the Obscurin-O chain or the Obscurin-like-O chain;
more preferably, the antibody comprises a heavy chain variable region VH1 and a light chain variable region VL1; a C-terminus of the VH1 is operably linked to an N-terminus of the Titin-T chain, a C-terminus of the VL1 is operably linked to an N-terminus of the Obscurin-O chain or the Obscurin-like-O chain, or
the C-terminus of the VL1 is operably linked to the N-terminus of the Titin-T chain, and the C-terminus of the VH1 is operably linked to the N-terminus of the Obscurin-O chain or the Obscurin-like-O chain.

19. A Fab fragment of a domain engineered antibody, wherein a constant region domain CH1 and a constant region domain CL of the Fab fragment are substituted, wherein,
the domain CH1 is substituted with a Titin-T chain, and the domain CL is substituted with a domain capable of having an inter-protein interaction with the Titin-T chain;
the domain CL is substituted with the Titin-T chain, and the domain CH1 is substituted with a domain capable of having an inter-protein interaction with the Titin-T chain;
the domain CH1 is substituted with an Obscurin-O chain, and the domain CL is substituted with a domain capable of having an inter-protein interaction with the Obscurin-O chain;
the domain CL is substituted with the Obscurin-O chain, and the domain CH1 is substituted with a domain capable of having an inter-protein interaction with the Obscurin-O chain;
the domain CH1 is substituted with an Obscurin-like-O chain, and the domain CL is substituted with a domain capable of having an inter-protein interaction with the Obscurin-like-O chain; or
the domain CL is substituted with the Obscurin-like-O chain, and the domain CH1 is substituted with a domain capable of having an inter-protein interaction with the Obscurin-like-O chain;
preferably, the domain having an inter-protein interaction with the Titin-T chain is the Obscurin-O chain or the Obscurin-like-O chain;
the domain having an inter-protein interaction with the Obscurin-O chain or the Obscurin-like-O chain is the Titin-T chain;
more preferably, the Fab comprises a heavy chain variable region VH1 and a light chain variable region VL1, the VH1 and the VL1 forming an antigen-binding site; a C-terminus of the VH1 is operably linked to an N-terminus of the Titin-T chain, and a C-terminus of the VL1 is operably linked to an N-terminus of the Obscurin-O chain or the Obscurin-like-O chain, or
the C-terminus of the VL1 is operably linked to the N-terminus of the Titin-T chain, and the C-terminus of the VH1 is operably linked to the N-terminus of the Obscurin-O chain or the Obscurin-like-O chain.

20. A domain engineered antibody, comprising the Fab fragment according to claim 19.

21. A bispecific antibody, comprising a first heavy chain and a first light chain specifically binding to a first antigen, and further comprising a second heavy chain and a second light chain specifically binding to a second antigen, wherein,
a CH1 domain of the first heavy chain is substituted with a Titin-T chain, and a CL domain of the first light chain is substituted with a domain capable of having an inter-protein interaction with the Titin-T chain;
the CL domain of the first light chain is substituted with the Titin-T chain, and the CH1 domain of the first heavy chain is substituted with a domain capable of having an inter-protein interaction with the Titin-T chain;
the CH1 domain of the first heavy chain is substituted with an Obscurin-O chain, and
the CL domain of the first light chain is substituted with a domain capable of having an inter-protein interaction with the Obscurin-O chain;
the CL domain of the first light chain is substituted with the Obscurin-O chain, and the CH1 domain of the first heavy chain is substituted with a domain capable of having an inter-protein interaction with the Obscurin-O chain;
the CH1 domain of the first heavy chain is substituted with an Obscurin-like-O chain, and the CL domain of the first light chain is substituted with a domain capable of having an inter-protein interaction with the Obscurin-like-O chain; or
the CL domain of the first light chain is substituted with the Obscurin-like-O chain, and
the CH1 domain of the first heavy chain is substituted with a domain capable of having an inter-protein interaction with the Obscurin-like-O chain;
the first antigen and the second antigen are not identical, or the first antigen and the second antigen are two different epitopes on the same antigen;
preferably, mispairing between the first heavy chain and the second light chain, and between the first light chain and the second heavy chain is less susceptible to happen;
more preferably, the domain having an inter-protein interaction with the Titin-T chain is the Obscurin-O chain or the Obscurin-like-O chain;
the domain having an inter-protein interaction with the Obscurin-O chain or the Obscurin-like-O chain is the Titin-T chain.

22. The bispecific antibody according to claim 21, wherein the first heavy chain comprises a first heavy chain variable region (VH1), and the first light chain comprises a first light chain variable region (VL1), the VH1 and the VL1 forming an antigen-binding site specifically binding to the first antigen, and
a C-terminus of the VH1 is operably linked to an N-terminus of the Titin-T chain, and a C-terminus of the VL1 is operably linked to an N-terminus of the Obscurin-O chain or the Obscurin-like-O chain, or
the C-terminus of the VL1 is operably linked to the N-terminus of the Titin-T chain, and
the C-terminus of the VH1 is operably linked to the N-terminus of the Obscurin-O chain or the Obscurin-like-O chain.

23. The bispecific antibody according to claim 21 or 22, wherein the second heavy chain comprises a second heavy chain variable region (VH2) from an N-terminus to a C-terminus, the VH2 being operably linked to the CHI; the second light chain comprises a second light chain variable region (VL2) from an N-terminus to a C-terminus, the VL2 being operably linked to the CL;
preferably, the VH2 and the VL2 form an antigen-binding site specifically binding to the second antigen, a C-terminus of the VH2 being operably linked to an N-terminus of the CHI; a C-terminus of the VL2 is operably linked to an N-terminus of the CL.

24. The bispecific antibody according to any one of claims 21 to 23, wherein C-termini of the first heavy chain and the second heavy chain comprise a CH2 domain and/or a CH3 domain;
preferably, the CH1 domain, the CH2 domain and the CH3 domain are derived from IgG1, IgG2, IgG3 or IgG4;
more preferably, the first heavy chain and the second heavy chain are bound by a means selected from the group consisting of: an antibody hinge region and a portion thereof, a linker, a disulfide bond, a hydrogen bond, an electrostatic interaction, a salt bridge, a hydrophobic-hydrophilic interaction, and a combination thereof.

25. The bispecific antibody according to claim 24, wherein,
the first heavy chain sequentially comprises VH1-L1-Titin-T chain-L2-CH2-CH3 from an N-terminus to a C-terminus, and
the first light chain sequentially comprises VL1-L3+Obscurin-O chain or VL1-L4-Obscurin-like-O chain from an N-terminus to a C-terminus;
the second heavy chain sequentially comprises VH2-CH1-CH2-CH3 from an N-terminus to a C-terminus, and
the second light chain sequentially comprises VL2-CL from an N-terminus to a C-terminus;
or,
the first light chain sequentially comprises VL1-L1-Titin-T chain from an N-terminus to a C-terminus, and
the first heavy chain sequentially comprises VH1-L2+Obscurin-O chain-L3-CH2-CH3 or VH1-L4-Obscurin-like-O chain-L5-CH2-CH3 from an N-terminus to a C-terminus;
the second heavy chain sequentially comprises VH2-CH1-CH2-CH3 from an N-terminus to a C-terminus, and
the second light chain sequentially comprises VL2-CL from an N-terminus to a C-terminus;
the L1 to L5 are spacers which may or may not be present;
preferably, the spacer is selected from the group consisting of: an N-terminal fragment of the Titin-T chain, an N-terminal fragment of the Obscurin-O chain, an N-terminal fragment of the Obscurin-like-O chain, and a (GₓS)_{y} linker; wherein X is selected from the group consisting of integers from 1 to 5, and Y is selected from the group consisting of integers from 1 to 6;
most preferably, the spacer is selected from the group consisting of: "KAGIR", "DQPQF", (G₄S)₁ and (G₄S)₂.

26. The bispecific antibody according to claim 25, wherein the CH2 and the CH3 of the first heavy chain are not identical to the CH2 and the CH3 of the second heavy chain and are bound in a manner that prevents homodimerization and/or favors heterodimerization;
preferably, the first heavy chain and the second heavy chain are bound by a means selected from the group consisting of: knob-into-hole, a hydrophobic interaction, an electrostatic interaction, a hydrophilic interaction, and a flexibility increase;
more preferably, the first heavy chain and the second heavy chain are bound by a means comprising knob-into-hole;
most preferably, the first heavy chain CH2-CH3 domain has amino acid residues at positions 104 to 330 of a sequence set forth in SEQ ID NO: 2, and the second heavy chain CH2-CH3 domain has amino acid residues at positions 104 to 330 of a sequence set forth in SEQ ID NO: 3; or
the first heavy chain CH2-CH3 domain has amino acid residues at positions 104 to 330 of a sequence set forth in SEQ ID NO: 3, and the second heavy chain CH2-CH3 domain has amino acid residues at positions 104 to 330 of a sequence set forth in SEQ ID NO: 2.

27. The domain engineered antibody according to claim 18 or 20, or the Fab fragment of a domain engineered antibody according to claim 19, or the bispecific antibody according to any one of claims 21 to 26, wherein the operable linkage is a linkage of two polypeptide sequences through a linking domain or a direct linkage of two polypeptides.

28. The domain engineered antibody, or the bispecific antibody, or the Fab fragment of a domain engineered antibody according to claim 27, wherein,
the Titin-T chain and the Obscurin-O chain, or the Titin-T chain and the Obscurin-like-O chain, are bound through natural inter-chain bonds and/or through unnatural inter-chain bonds to form a dimer;
preferably, the Titin-T chain and the Obscurin-O chain or the Obscurin-like-O chain form a dimer through natural inter-chain bonds, and one or more residues at positions selected from the group consisting of 7-15, 19-24, 26, 55, 59 and 60 on the Titin-T chain and one or more residues at positions selected from the group consisting of 3-6, 9, 41, 73, 75 and 80-90 on the Obscurin-O chain are linked to each other, or
one or more residues selected from the group consisting of positions 1, 7-10, 13-16, 19-26, 59-60 and 96 on the Titin-T chain and one or more residues selected from the group consisting of positions 4-5, 10, 12-13, 74, 76, 78 and 82-91 on the Obscurin-like-O chain are linked to each other;
the residue position in the Titin-T chain is a natural sequence numbering position relative to a sequence SEQ ID NO: 32; the residue position in the Obscurin-O chain is a natural sequence numbering position relative to a sequence SEQ ID NO: 33; the residue position in the Obscurin-like-O chain is a natural sequence numbering position relative to a sequence SEQ ID NO: 34.

29. The domain engineered antibody, the bispecific antibody or the Fab fragment of a domain engineered antibody according to claim 27 or 28, wherein the Titin-T chain and the Obscurin-O chain, or the Titin-T chain and the Obscurin-like-O chain, form a dimer through at least one non-natural inter-chain bonds;
preferably, the non-natural inter-chain bond is a disulfide bond;
more preferably, wherein the dimer comprises 1, 2, 3, 4, 5, 6, 7, 8, 9 or 10 non-natural inter-chain bonds.

30. The domain engineered antibody, the bispecific antibody or the Fab fragment of a domain engineered antibody according to any one of claims 27 to 29, wherein,
the Titin-T chain has one or more amino acid residue mutations on positions selected from the group consisting of 8, 20, 22, 25, 26 and 39, and/or the Obscurin-O chain has one or more amino acid residue mutations on positions selected from the group consisting of 3, 9, 25, 76 and 88; or
the Titin-T chain has one or more amino acid residue mutations on positions selected from the group consisting of 8, 20, 22, 25, 26 and 39, and/or the Obscurin-like-O chain has one or more amino acid residue mutations on positions selected from the group consisting of 6, 26, 74, 77, 84 and 86;
preferably, the Titin-T chain has one or more amino acid residue mutations on positions selected from the group consisting of A8C, V20C, T22C, C25S, A26C and C39T, and/or
the Obscurin-O chain has one or more amino acid residue mutations on positions selected from the group consisting of A3C, R9C, C25S, C76S and A88C; or
the Titin-T chain has one or more amino acid residue mutations on positions selected from the group consisting of A8C, V20C, T22C, C25S, A26C and C39T, and/or
the Obscurin-like-O chain has one or more amino acid residue mutations on positions selected from the group consisting of C6E, C26S, C77S, V74C, G84C and A86C; more preferably, the Titin-T chain has C25S, C39T and A8C mutations, and the Obscurin-O chain has an A88C mutation;
the Titin-T chain has C25S, C39T and V20C mutations, and the Obscurin-O chain has an A3C mutation;
the Titin-T chain has C25S, C39T and A26C mutations, and the Obscurin-O chain has an R9C mutation;
the Titin-T chain has C25S, C39T and A8C mutations, and the Obscurin-O chain has C25S, C76S and A88C mutations;
the Titin-T chain has C25S, C39T and V20C mutations, and the Obscurin-O chain has C25S, C76S and A3C mutations;
the Titin-T chain has C25S, C39T and A26C mutations, and the Obscurin-O chain has C25S, C76S and R9C mutations;
the Titin-T chain has C25S, C39T and A8C mutations, and the Obscurin-like-O chain has C6E and V74C mutations;
the Titin-T chain has C25S, C39T and V20C mutations, and the Obscurin-like-O chain has C6E and G84C mutations;
the Titin-T chain has C25S, C39T and T22C mutations, and the Obscurin-like-O chain has C6E and A86C mutations;
the Titin-T chain has C25S, C39T and A8C mutations, and the Obscurin-like-O chain has C6E, C26S, C77S and V74C mutations;
the Titin-T chain has C25S, C39T and V20C mutations, and the Obscurin-like-O chain has C6E, C26S, C77S and G84C mutations; or
the Titin-T chain has C25S, C39T and T22C mutations, and the Obscurin-like-O chain has C6E, C26S, C77S and A86C mutations;
the mutation position in the Titin-T chain is a natural sequence position relative to a sequence SEQ ID NO: 32; the mutation position in the Obscurin-O chain is a natural sequence position relative to a sequence SEQ ID NO: 33; the mutation position in the Obscurin-like-O chain is a natural sequence position relative to a sequence SEQ ID NO: 34.

31. The domain engineered antibody, the bispecific antibody, or the Fab fragment of a domain engineered antibody according to claim 29 or 30, wherein,
the Obscurin-O chain further has one or more amino acid residue mutations on positions selected from the group consisting of 7, 11 and 62;
preferably, the Obscurin-O chain has one or more amino acid residue mutations on positions selected from the group consisting of L7K and L7R, K11L, and T62K and T62H;
most preferably, the Titin-T chain has C25S, C39T and A8C mutations, and the Obscurin-O chain has C25S, C76S, A88C, L7K and T62K mutations;
the Titin-T chain has C25S, C39T and A8C mutations, and the Obscurin-O chain has C25S, C76S, A88C, L7K and T62H mutations;
the Titin-T chain has C25S, C39T and A8C mutations, and the Obscurin-O chain has C25S, C76S, A88C, K11L and T62K mutations; or
the Titin-T chain has C25S, C39T and A8C mutations, and the Obscurin-O chain has C25S, C76S, A88C, K11L and T62H mutations;
the mutation position in the Titin-T chain is a natural sequence numbering position relative to a sequence SEQ ID NO: 32; the mutation position in the Obscurin-O chain is a natural sequence numbering position relative to a sequence SEQ ID NO: 33.

32. The domain engineered antibody, the bispecific antibody or the Fab fragment of a domain engineered antibody according to any one of claims 27 to 31, wherein,
the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84, and/or
the Obscurin-O chain has one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97;
preferably, the Titin-T chain has one or more amino acid mutations on positions selected from the group consisting of P3W, S11I, I13L, T22M, G40S, R42K, H45S, Q47E, Q49G, N56S, D58E, M66S and M66K, K70R, L75V, T77S, S79T, G81R, N82M, E83D and F84L, and/or
the Obscurin-O chain has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G;
more preferably, the Titin-T chain has M66S and T77S amino acid mutations, and/or the Obscurin-O chain has L11K, A12S, F13Y, V14T and T22S amino acid mutations; the Titin-T chain has M66K, K70R, S79T and G81R amino acid mutations, and/or the Obscurin-O chain has G2E, V17E, N30D, T32P, Q34E, S36T, V44I, A45T, L58V, K62E, A67Q, G69S and A97G amino acid mutations;
the Titin-T chain has P3W, S11I, I13L, T22M and N82M amino acid mutations, and/or the Obscurin-O chain has D20L, T22M and A53L amino acid mutations;
the Titin-T chain has S11I, M66K, S79T and G81R amino acid mutations, and/or the Obscurin-O chain has Q41K, A45T, A67Q, G69S and V89L amino acid mutations;
the Titin-T chain has G40S, R42K, H45S, Q47E, Q49G, N56S, D58E, L75V, E83D and F84L amino acid mutations, and/or the Obscurin-O chain has Q42L, A45T, A67T, G69S, Q92E and D94G amino acid mutations;
the Titin-T chain has Q47E, Q49G, N56S, D58E and L75V amino acid mutations, and/or the Obscurin-O chain has Q42L, A45T, A67T, G69S, Q92E and D94G amino acid mutations;
the Titin-T chain has N56S, D58E and L75V amino acid mutations, and/or the Obscurin-O chain has Q42L, A45T, A67T, G69S, Q92E and D94G amino acid mutations; or
the Titin-T chain has N56S, D58E, M66S and T77S amino acid mutations, and/or the Obscurin-O chain has A12S, F13Y, T22S, Q42L, A45T, A67Q, G69S, Q92E and D94G amino acid mutations;
the mutation position in the Titin-T chain is a natural sequence numbering position relative to a sequence SEQ ID NO: 35; the mutation position in the Obscurin-O chain is a natural sequence numbering position relative to a sequence SEQ ID NO: 50.

33. The domain engineered antibody, the bispecific antibody or the Fab fragment of a domain engineered antibody according to any one of claims 27 to 32, wherein N-termini of the Titin-T chain, the Obscurin-O chain or the Obscurin-like-O chain are operably linked to the VH1 or the VL1 through a linking domain;
preferably, the linking domain is selected from the group consisting of: an N-terminal fragment of the Titin-T chain, an N-terminal fragment of the Obscurin-O chain, an N-terminal fragment of the Obscurin-like-O chain, and a (GₓS)_{y} linker, wherein X is selected from the group consisting of integers from 1 to 5, and Y is selected from the group consisting of integers from 1 to 6;
more preferably, the linking domain is selected from the group consisting of: "KAGIR", "DQPQF", (G₄S)₁ and (G₄S)₂;
most preferably, the Titin-T chain is linked to the VH1 or the VL1 through a KAGIR polypeptide, and/or the Obscurin-O chain is linked to the VL1 or the VH1 through a DQPQF polypeptide.

34. The domain engineered antibody, the bispecific antibody or the Fab fragment of a domain engineered antibody according to any one of claims 27 to 33, wherein,
A) the Titin-T chain has a sequence set forth in SEQ ID NO: 32 or further has a sequence having one or more amino acid mutations on positions selected from the group consisting of 3, 8, 11, 13, 20, 22, 25, 26, 39, 40, 42, 45, 47, 49, 56, 58, 66, 70, 75, 77, 79, 81, 82, 83 and 84 and/or 5 amino acid residues of KAGIR added at the N-terminus on the basis of SEQ ID NO: 32; preferably, the Titin-T chain has a sequence having one or more amino acid mutations on positions selected from the group consisting of A8C, V20C, T22C, C25S, A26C, C39T, P3W, S11I, I13L, T22M, G40S, R42K, H45S, Q47E, Q49G, N56S, D58E, M66S and M66K, K70R, L75V, T77S, S79T, G81R, N82M, E83D and F84L and/or a KAGIR amino acid residue added at the N-terminus; and/or
B) the Obscurin-O chain has a sequence set forth in SEQ ID NO: 33 or further has a sequence having one or more amino acid mutations on positions selected from the group consisting of 2, 3, 7, 9, 11, 12, 13, 14, 17, 20, 22, 25, 30, 32, 34, 36, 41, 42, 44, 45, 53, 58, 62, 67, 69, 76, 88, 89, 92, 94 and 97 and/or a DQPQF amino acid residue added at the N-terminus on the basis of SEQ ID NO: 33; preferably, the Obscurin-O chain has a sequence having one or more amino acid mutations on positions selected from the group consisting of A3C, R9C, C25S, C76S, A88C, L7K and L7R, T62K and T62H, G2E, L11K, A12S, F13Y, V14T, V17E, D20L, T22M and T22S, N30D, T32P, Q34E, S36T, Q41K, Q42L, V44I, A45T, A53L, L58V, T62E, A67Q and A67T, G69S, V89L, Q92E, D94G and A97G and/or a DQPQF amino acid residue added at the N-terminus; or
the Obscurin-like-O chain has a sequence set forth in SEQ ID NO: 34 or further has a sequence having one or more amino acid mutations on positions selected from the group consisting of 6, 26, 74, 77, 84 and 86 on the basis of SEQ ID NO: 34; preferably, the Obscurin-like-O chain has a sequence having one or more amino acid mutations on positions selected from the group consisting of C6E, C26S, C77S, V74C, G84C and A86C;
the mutation position in the Titin-T chain is a natural sequence numbering position of the sequence SEQ ID NO: 32; the mutation position in the Obscurin-O chain is a natural sequence numbering position of the sequence SEQ ID NO: 33; the mutation position in the Obscurin-like-O chain is a natural sequence numbering position of the sequence SEQ ID NO: 34;
preferably, wherein,
A) the Titin-T chain has one or more amino acid residue mutations on positions selected from the group consisting of 8, 20, 22, 25, 26 and 39 on the basis of SEQ ID NO: 32, and preferably has one or more amino acid residue mutations on positions selected from the group consisting of A8C, V20C, T22C, C25S, A26C and C39T, and the mutation position in the Titin-T chain is a natural sequence numbering position of SEQ ID NO: 32;
more preferably, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and preferably has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L; the mutation position in the Titin-T chain is a natural sequence numbering position of SEQ ID NO: 35, and/or
B) the Obscurin-O chain has one or more amino acid residue mutations on positions selected from the group consisting of 3, 9, 25, 76 and 88 on the basis of SEQ ID NO: 33, and preferably has one or more amino acid residue mutations on positions selected from the group consisting of A3C, R9C, C25S, C76S and A88C, and the mutation position in the Obscurin-O chain is a natural sequence numbering position of SEQ ID NO: 33;
preferably, the Obscurin-O chain has one or more amino acid residue mutations on positions selected from the group consisting of 7, 11 and 62 on the basis of SEQ ID NO: 45, and preferably has one or more amino acid residue mutations on positions selected from the group consisting of L7K and L7R, T62K and T62H, and K11L, and the mutation position in the Obscurin-O chain is a natural sequence numbering position of SEQ ID NO: 45;
more preferably, the Obscurin-O chain has one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97 on the basis of SEQ ID NO: 50, and preferably has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G, and the mutation position in the Obscurin-O chain is a natural sequence numbering position of SEQ ID NO: 50;
or,
A) the Titin-T chain has one or more amino acid residue mutations on positions selected from the group consisting of 8, 20, 22, 25, 26 and 39 on the basis of SEQ ID NO: 32, and preferably has one or more amino acid residue mutations on positions selected from the group consisting of A8C, V20C, T22C, C25S, A26C and C39T, and the mutation position of the Titin-T chain is a natural sequence numbering position of SEQ ID NO: 32;
more preferably, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and preferably has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L; the mutation position in the Titin-T chain is a natural sequence numbering position of SEQ ID NO: 35, and/or
B) the Obscurin-like-O chain has one or more amino acid residue mutations on positions selected from the group consisting of 6, 26, 74, 77, 84 and 86 on the basis of SEQ ID NO: 34, and preferably has one or more amino acid residue mutations on positions selected from the group consisting of C6E, C26S, C77S, V74C, G84C and A86C, and the mutation position in the Obscurin-like-O chain is a natural sequence numbering position of SEQ ID NO: 34.

35. The domain engineered antibody, the bispecific antibody or the Fab fragment of a domain engineered antibody according to any one of claims 27 to 34, wherein,
the Titin-T chain comprises a sequence set forth in any one of SEQ ID NOs: 32, 35-41, 65-76 or a sequence having at least 80% sequence identity to any one of SEQ ID NOs: 32, 35-41, 65-76, and/or
the Obscurin-O chain comprises a sequence set forth in SEQ ID NOs: 33, 42-58, 77-86 or a sequence having at least 80% sequence identity to any one of SEQ ID NOs: 33, 42-58, 77-86; or
the Titin-T chain comprises a sequence set forth in any one of SEQ ID NOs: 32, 35-41, 65-76 or a sequence having at least 80% sequence identity to any one of SEQ ID NOs: 32, 35-41, 65-76, and/or
the Obscurin-like-O chain comprises a sequence set forth in any one of SEQ ID NOs: 34, 59-64 or a sequence having at least 80% sequence identity to any one of SEQ ID NOs: 34, 59-64;
more preferably,
the Titin-T chain comprises a polypeptide set forth in SEQ ID NO: 68, and the Obscurin-O chain comprises a polypeptide set forth in SEQ ID NO: 80;
the Titin-T chain comprises a polypeptide set forth in SEQ ID NO: 73, and the Obscurin-O chain comprises a polypeptide set forth in SEQ ID NO: 83;
the Titin-T chain comprises a polypeptide set forth in SEQ ID NO: 76, and the Obscurin-O chain comprises a polypeptide set forth in SEQ ID NO: 84;
the Titin-T chain comprises a polypeptide set forth in SEQ ID NO: 76, and the Obscurin-O chain comprises a polypeptide set forth in SEQ ID NO: 86;
the Titin-T chain comprises a polypeptide set forth in SEQ ID NO: 75, and the Obscurin-O chain comprises a polypeptide set forth in SEQ ID NO: 86.

36. The bispecific antibody according to any one of claims 21 to 35, wherein the bispecific antibody is selected from the group consisting of any one of the following A-G:
A) the first heavy chain of the bispecific antibody has a sequence set forth in SEQ ID NO: 89 or a sequence having at least 85% sequence identity to SEQ ID NO: 89, the first light chain has a sequence set forth in SEQ ID NO: 90 or a sequence having at least 85% sequence identity to SEQ ID NO: 90, the second heavy chain has a sequence set forth in SEQ ID NO: 87 or a sequence having at least 85% sequence identity to SEQ ID NO: 87, and the second light chain has a sequence set forth in SEQ ID NO: 88 or a sequence having at least 85% sequence identity to SEQ ID NO: 88;
B) the first heavy chain of the bispecific antibody has a sequence set forth in SEQ ID NO: 93 or a sequence having at least 85% sequence identity to SEQ ID NO: 93, the first light chain has a sequence set forth in SEQ ID NO: 94 or a sequence having at least 85% sequence identity to SEQ ID NO: 94, the second heavy chain has a sequence set forth in SEQ ID NO: 91 or a sequence having at least 85% sequence identity to SEQ ID NO: 91, and the second light chain has a sequence set forth in SEQ ID NO: 92 or a sequence having at least 85% sequence identity to SEQ ID NO: 92;
C) the first heavy chain of the bispecific antibody has a sequence set forth in SEQ ID NO: 97 or a sequence having at least 85% sequence identity to SEQ ID NO: 97, the first light chain has a sequence set forth in SEQ ID NO: 98 or a sequence having at least 85% sequence identity to SEQ ID NO: 98, the second heavy chain has a sequence set forth in SEQ ID NO: 95 or a sequence having at least 85% sequence identity to SEQ ID NO: 95, and the second light chain has a sequence set forth in SEQ ID NO: 96 or a sequence having at least 85% sequence identity to SEQ ID NO: 96;
D) the first heavy chain of the bispecific antibody has a sequence set forth in SEQ ID NO: 99 or a sequence having at least 85% sequence identity to SEQ ID NO: 99, the first light chain has a sequence set forth in SEQ ID NO: 100 or a sequence having at least 85% sequence identity to SEQ ID NO: 100, the second heavy chain has a sequence set forth in SEQ ID NO: 101 or a sequence having at least 85% sequence identity to SEQ ID NO: 101, and the second light chain has a sequence set forth in SEQ ID NO: 96 or a sequence having at least 85% sequence identity to SEQ ID NO: 96;
E) the first heavy chain of the bispecific antibody has a sequence set forth in SEQ ID NO: 102 or a sequence having at least 85% sequence identity to SEQ ID NO: 102, the first light chain has a sequence set forth in SEQ ID NO: 100 or a sequence having at least 85% sequence identity to SEQ ID NO: 100, the second heavy chain has a sequence set forth in SEQ ID NO: 95 or a sequence having at least 85% sequence identity to SEQ ID NO: 95, and the second light chain has a sequence set forth in SEQ ID NO: 96 or a sequence having at least 85% sequence identity to SEQ ID NO: 96;
F) the first heavy chain of the bispecific antibody has a sequence set forth in SEQ ID NO: 103 or a sequence having at least 85% sequence identity to SEQ ID NO: 103, the first light chain has a sequence set forth in SEQ ID NO: 104 or a sequence having at least 85% sequence identity to SEQ ID NO: 104, the second heavy chain has a sequence set forth in SEQ ID NO: 95 or a sequence having at least 85% sequence identity to SEQ ID NO: 95, and the second light chain has a sequence set forth in SEQ ID NO: 96 or a sequence having at least 85% sequence identity to SEQ ID NO: 96; and
G) the first heavy chain of the bispecific antibody has a sequence set forth in SEQ ID NO: 107 or a sequence having at least 85% sequence identity to SEQ ID NO: 107, the first light chain has a sequence set forth in SEQ ID NO: 108 or a sequence having at least 85% sequence identity to SEQ ID NO: 108, the second heavy chain has a sequence set forth in SEQ ID NO: 109 or a sequence having at least 85% sequence identity to SEQ ID NO: 109, and the second light chain has a sequence set forth in SEQ ID NO: 110 or a sequence having at least 85% sequence identity to SEQ ID NO: 110;
H) the first heavy chain of the bispecific antibody has a sequence set forth in SEQ ID NO: 122 or a sequence having at least 85% sequence identity to SEQ ID NO: 122, the first light chain has a sequence set forth in SEQ ID NO: 123 or a sequence having at least 85% sequence identity to SEQ ID NO: 123, the second heavy chain has a sequence set forth in SEQ ID NO: 116 or a sequence having at least 85% sequence identity to SEQ ID NO: 116, and the second light chain has a sequence set forth in SEQ ID NO: 117 or a sequence having at least 85% sequence identity to SEQ ID NO: 117;
I) the first heavy chain of the bispecific antibody has a sequence set forth in SEQ ID NO: 118 or a sequence having at least 85% sequence identity to SEQ ID NO: 118, the first light chain has a sequence set forth in SEQ ID NO: 119 or a sequence having at least 85% sequence identity to SEQ ID NO: 119, the second heavy chain has a sequence set forth in SEQ ID NO: 124 or a sequence having at least 85% sequence identity to SEQ ID NO: 124, and the second light chain has a sequence set forth in SEQ ID NO: 125 or a sequence having at least 85% sequence identity to SEQ ID NO: 125.

37. A method for preparing a multispecific antibody, comprising a step of substituting a CH1/CL of an antibody with a Titin-T chain and an Obscurin-O chain, or a Titin-T chain and an Obscurin-like-O chain; wherein,
more preferably, the Titin-T chain comprises a sequence set forth in any one of SEQ ID NOs: 32, 35-41, 65-76 or a sequence having at least 80% sequence identity to any one of SEQ ID NOs: 32, 35-41, 65-76, and/or
the Obscurin-O chain comprises a sequence set forth in any one of SEQ ID NOs: 33, 42-58, 77-86 or a sequence having at least 80% sequence identity to any one of SEQ ID NOs: 33, 42-58, 77-86; or
the Titin-T chain comprises a sequence set forth in any one of SEQ ID NOs: 32, 35-41, 65-76 or a sequence having at least 80% sequence identity to any one of SEQ ID NOs: 32, 35-41, 65-76, and/or
the Obscurin-like-O chain comprises a sequence set forth in any one of SEQ ID NOs: 34, 59-64 or a sequence having at least 80% sequence identity to any one of SEQ ID NOs: 34, 59-64;
most preferably,
the Titin-T chain comprises a sequence set forth in SEQ ID NO: 68, and the Obscurin-O chain comprises a sequence set forth in SEQ ID NO: 80;
the Titin-T chain comprises a sequence set forth in SEQ ID NO: 73, and the Obscurin-O chain comprises a sequence set forth in SEQ ID NO: 83;
the Titin-T chain comprises a sequence set forth in SEQ ID NO: 76, and the Obscurin-O chain comprises a sequence set forth in SEQ ID NO: 84;
the Titin-T chain comprises a sequence set forth in SEQ ID NO: 76, and the Obscurin-O chain comprises a sequence set forth in SEQ ID NO: 86; or
the Titin-T chain comprises a sequence set forth in SEQ ID NO: 75, and the Obscurin-O chain comprises a sequence set forth in SEQ ID NO: 86.

38. A multispecific antibody, prepared by the method according to claim 37; wherein, the multispecific antibody comprises a first heavy chain variable region (VH1) and a first light chain variable region (VL1), the VH1 and the VL1 forming an antigen-binding site specifically binding to a first antigen, and
a C-terminus of the VH1 is operably linked to an N-terminus of the Titin-T chain, and a C-terminus of the VL1 is operably linked to an N-terminus of the Obscurin-O chain or the Obscurin-like-O chain, or
the C-terminus of the VL1 is operably linked to the N-terminus of the Titin-T chain, and the C-terminus of the VH1 is operably linked to the N-terminus of the Obscurin-O chain or the Obscurin-like-O chain.

39. The multispecific antibody according to claim 38, wherein,
A) the Titin-T chain has a sequence set forth in SEQ ID NO: 32 or further has a sequence having one or more amino acid mutations on positions selected from the group consisting of 8, 20, 22, 25, 26, 39, 3, 11, 13, 40, 42, 45, 47, 49, 56, 58, 66, 70, 75, 77, 79, 81, 82, 83 and 84 and/or 5 amino acid residues of KAGIR added at the N-terminus on the basis of SEQ ID NO: 32; preferably, the Titin-T chain has a sequence having one or more amino acid mutations on positions selected from the group consisting of A8C, V20C, T22C, C25S, A26C, C39T, P3W, S11I, I13L, T22M, G40S, R42K, H45S, Q47E, Q49G, N56S, D58E, M66S and M66K, K70R, L75V, T77S, S79T, G81R, N82M, E83D and F84L and/or a KAGIR amino acid residue added at the N-terminus; and/or
B) the Obscurin-O chain has a sequence set forth in SEQ ID NO: 33 or further has a sequence having one or more amino acid mutations on positions selected from the group consisting of 3, 9, 25, 76, 88, 7, 11, 62, 2, 12, 13, 14, 17, 20, 22, 30, 32, 34, 36, 41, 42, 44, 45, 53, 58, 67, 69, 89, 92, 94 and 97 and/or a DQPQF amino acid residue added at the N-terminus on the basis of SEQ ID NO: 33; preferably, the Obscurin-O chain has a sequence having one or more amino acid mutations on positions selected from the group consisting of A3C, R9C, C25S, C76S, A88C, L7K and L7R, T62K and T62H, G2E, L11K, A12S, F13Y, V14T, V17E, D20L, T22M and T22S, N30D, T32P, Q34E, S36T, Q41K, Q42L, V44I, A45T, A53L, L58V, T62E, A67Q and A67T, G69S, V89L, Q92E, D94G and A97G and/or a DQPQF amino acid residue added at the N-terminus; or
the Obscurin-like-O chain has a sequence set forth in SEQ ID NO: 34 or further has a sequence having one or more amino acid mutations on positions selected from the group consisting of 6, 26, 74, 77, 84 and 86 on the basis of SEQ ID NO: 34; preferably, the Obscurin-like-O chain has a sequence having one or more amino acid mutations on positions selected from the group consisting of C6E, C26S, C77S, V74C, G84C and A86C;
the mutation position in the Titin-T chain is a natural sequence numbering position of the sequence SEQ ID NO: 32; the mutation position in the Obscurin-O chain is a natural sequence numbering position of the sequence SEQ ID NO: 33; the mutation position in the Obscurin-like-O chain is a natural sequence numbering position of the sequence SEQ ID NO: 34;
preferably, wherein,
A) the Titin-T chain has one or more amino acid residue mutations on positions selected from the group consisting of 8, 20, 22, 25, 26 and 39 on the basis of SEQ ID NO: 32, and preferably has one or more amino acid residue mutations on positions selected from the group consisting of A8C, V20C, T22C, C25S, A26C and C39T, and the mutation position in the Titin-T chain is a natural sequence numbering position of SEQ ID NO: 32;
more preferably, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and preferably has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L; the mutation position in the Titin-T chain is a natural sequence numbering position of SEQ ID NO: 35, and/or
B) the Obscurin-O chain has one or more amino acid residue mutations on positions selected from the group consisting of 3, 9, 25, 76 and 88 on the basis of SEQ ID NO: 33, and preferably has one or more amino acid residue mutations on positions selected from the group consisting of A3C, R9C, C25S, C76S and A88C, and the mutation position in the Obscurin-O chain is a natural sequence numbering position of SEQ ID NO: 33;
preferably, the Obscurin-O chain has one or more amino acid residue mutations on positions selected from the group consisting of 7, 11 and 62 on the basis of SEQ ID NO: 45, and preferably has one or more amino acid residue mutations on positions selected from the group consisting of L7K and L7R, T62K and T62H, and K11L, and the mutation position in the Obscurin-O chain is a natural sequence numbering position of SEQ ID NO: 45;
more preferably, the Obscurin-O chain has one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97 on the basis of SEQ ID NO: 50, and preferably has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G, and the mutation position in the Obscurin-O chain is a natural sequence numbering position of SEQ ID NO: 50;
or,
A) the Titin-T chain has one or more amino acid residue mutations on positions selected from the group consisting of 8, 20, 22, 25, 26 and 39 on the basis of SEQ ID NO: 32, and preferably has one or more amino acid residue mutations on positions selected from the group consisting of A8C, V20C, T22C, C25S, A26C and C39T, and the mutation position in the Titin-T chain is a natural sequence numbering position of SEQ ID NO: 32;
more preferably, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and preferably has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L; the mutation position in the Titin-T chain is a natural sequence numbering position of SEQ ID NO: 35, and/or
B) the Obscurin-like-O chain has one or more amino acid residue mutations on positions selected from the group consisting of 6, 26, 74, 77, 84 and 86 on the basis of SEQ ID NO: 34, and preferably has one or more amino acid residue mutations on positions selected from the group consisting of C6E, C26S, C77S, V74C, G84C and A86C, and the mutation position in the Obscurin-like-O chain is a natural sequence numbering position of SEQ ID NO: 34.

40. A conjugate, comprising any one of substances selected from the group consisting of the following (A)-(F):
A) the polypeptide complex according to any one of claims 1 to 12;
B) the multispecific polypeptide complex according to any one of claims 13 to 17;
C) the domain engineered antibody according to any one of claims 18, 20, 27 to 35;
D) the Fab fragment of a domain engineered antibody according to any one of claims 19, 27 to 35;
E) the bispecific antibody according to any one of claims 21 to 36; and
F) the multispecific antibody according to claim 38 or 39.

41. A polypeptide, wherein the polypeptide has a sequence set forth in any one of SEQ ID NOs: 32, 35-41, 65-76 or a sequence having at least 80% sequence identity to any one of SEQ ID NOs: 32, 35-41, 65-76; or
the polypeptide has a sequence set forth in any one of SEQ ID NOs: 33, 42-58, 77-86 or a sequence having at least 80% sequence identity to SEQ ID NOs: 33, 42-58, 77-86; or
the polypeptide has a sequence set forth in any one of SEQ ID NOs: 34, 59-64 or a sequence having at least 80% sequence identity to any one of SEQ ID NOs: 34, 59-64; preferably, the polypeptide can be used to substitute a CH1 and/or a CL of an antibody.

42. Use of a combination of a Titin-T chain and an Obscurin-O chain, or a combination of a Titin-T chain and an Obscurin-like-O chain, in the reduction of mispairing of a light/heavy chain of a multispecific antibody; wherein,
preferably, the multispecific antibody comprises a first heavy chain variable region (VH1) and a first light chain variable region (VL1), the VH1 and the VL1 forming an antigen-binding site specifically binding to a first antigen, and
a C-terminus of the VH1 is operably linked to an N-terminus of the Titin-T chain, and a C-terminus of the VL1 is operably linked to an N-terminus of the Obscurin-O chain or the Obscurin-like-O chain, or
the C-terminus of the VL1 is operably linked to the N-terminus of the Titin-T chain, and the C-terminus of the VH1 is operably linked to the N-terminus of the Obscurin-O chain or the Obscurin-like-O chain;
more preferably, the Titin-T chain comprises a sequence set forth in any one of SEQ ID NOs: 32, 35-41, 65-76 or a sequence having at least 80% sequence identity to any one of SEQ ID NOs: 32, 35-41, 65-76, and/or the Obscurin-O chain comprises a sequence set forth in any one of SEQ ID NOs: 33, 42-58, 77-86 or a sequence having at least 80% sequence identity to any one of SEQ ID NOs: 33, 42-58, 77-86; or
the Titin-T chain comprises a sequence set forth in any one of SEQ ID NOs: 32, 35-41, 65-76 or a sequence having at least 80% sequence identity to any one of SEQ ID NOs: 32, 35-41, 65-76, and/or the Obscurin-like-O chain comprises a sequence set forth in any one of SEQ ID NOs: 34, 59-64 or a sequence having at least 80% sequence identity to any one of SEQ ID NOs: 34, 59-64;
most preferably,
the Titin-T chain comprises a sequence set forth in SEQ ID NO: 68, and the Obscurin-O chain comprises a sequence set forth in SEQ ID NO: 80;
the Titin-T chain comprises a sequence set forth in SEQ ID NO: 73, and the Obscurin-O chain comprises a sequence set forth in SEQ ID NO: 83;
the Titin-T chain comprises a sequence set forth in SEQ ID NO: 76, and the Obscurin-O chain comprises a sequence set forth in SEQ ID NO: 84; the Titin-T chain comprises a sequence set forth in SEQ ID NO: 76, and the Obscurin-O chain comprises a sequence set forth in SEQ ID NO: 86; or
the Titin-T chain comprises a sequence set forth in SEQ ID NO: 75, and the Obscurin-O chain comprises a sequence set forth in SEQ ID NO: 86.

43. The use according to claim 42, wherein,
A) the Titin-T chain has a sequence set forth in SEQ ID NO: 32 or further has a sequence having one or more amino acid mutations on positions selected from the group consisting of 8, 20, 22, 25, 26, 39, 3, 11, 13, 40, 42, 45, 47, 49, 56, 58, 66, 70, 75, 77, 79, 81, 82, 83 and 84 and/or 5 amino acid residues of KAGIR added at the N-terminus on the basis of SEQ ID NO: 32; preferably, the Titin-T chain has a sequence having one or more amino acid mutations on positions selected from the group consisting of A8C, V20C, T22C, C25S, A26C, C39T, P3W, S11I, I13L, T22M, G40S, R42K, H45S, Q47E, Q49G, N56S, D58E, M66S and M66K, K70R, L75V, T77S, S79T, G81R, N82M, E83D and F84L and/or a KAGIR amino acid residue added at the N-terminus; and/or
B) the Obscurin-O chain has a sequence set forth in SEQ ID NO: 33 or further has a sequence having one or more amino acid mutations on positions selected from the group consisting of 3, 9, 25, 76, 88, 7, 11, 62, 2, 12, 13, 14, 17, 20, 22, 30, 32, 34, 36, 41, 42, 44, 45, 53, 58, 67, 69, 89, 92, 94 and 97 and/or a DQPQF amino acid residue added at the N-terminus on the basis of SEQ ID NO: 33; preferably, the Obscurin-O chain has a sequence having one or more amino acid mutations on positions selected from the group consisting of A3C, R9C, C25S, C76S, A88C, L7K and L7R, T62K and T62H, G2E, L11K, A12S, F13Y, V14T, V17E, D20L, T22M and T22S, N30D, T32P, Q34E, S36T, Q41K, Q42L, V44I, A45T, A53L, L58V, T62E, A67Q and A67T, G69S, V89L, Q92E, D94G and A97G and/or a DQPQF amino acid residue added at the N-terminus; or
the Obscurin-like-O chain has a sequence set forth in SEQ ID NO: 34 or further has a sequence having one or more amino acid mutations on positions selected from the group consisting of 6, 26, 74, 77, 84 and 86 on the basis of SEQ ID NO: 34; preferably, the Obscurin-like-O chain has a sequence having one or more amino acid mutations on positions selected from the group consisting of C6E, C26S, C77S, V74C, G84C and A86C;
the mutation position in the Titin-T chain is a natural sequence numbering position of the sequence SEQ ID NO: 32; the mutation position in the Obscurin-O chain is a natural sequence numbering position of the sequence SEQ ID NO: 33; the mutation position in the Obscurin-like-O chain is a natural sequence numbering position of the sequence SEQ ID NO: 34;
preferably, wherein,
A) the Titin-T chain has one or more amino acid residue mutations on positions selected from the group consisting of 8, 20, 22, 25, 26 and 39 on the basis of SEQ ID NO: 32, and preferably has one or more amino acid residue mutations on positions selected from the group consisting of A8C, V20C, T22C, C25S, A26C and C39T, and the mutation position of the Titin-T chain is a natural sequence numbering position of SEQ ID NO: 32;
more preferably, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and preferably has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L; the mutation position in the Titin-T chain is a natural sequence numbering position of SEQ ID NO: 35, and/or
B) the Obscurin-O chain has one or more amino acid residue mutations on positions selected from the group consisting of 3, 9, 25, 76 and 88 on the basis of SEQ ID NO: 33, and preferably has one or more amino acid residue mutations on positions selected from the group consisting of A3C, R9C, C25S, C76S and A88C, and the mutation position in the Obscurin-O chain is a natural sequence numbering position of SEQ ID NO: 33;
preferably, the Obscurin-O chain has one or more amino acid residue mutations on positions selected from the group consisting of 7, 11 and 62 on the basis of SEQ ID NO: 45, and preferably has one or more amino acid residue mutations on positions selected from the group consisting of L7K and L7R, T62K and T62H, and K11L, and the mutation position in the Obscurin-O chain is a natural sequence numbering position of SEQ ID NO: 45;
more preferably, the Obscurin-O chain has one or more amino acid mutations on positions selected from the group consisting of 2, 11, 12, 13, 14, 17, 20, 22, 30, 32, 34, 36, 41, 42, 44, 45, 53, 58, 62, 67, 69, 89, 92, 94 and 97 on the basis of SEQ ID NO: 50, and preferably has one or more amino acid mutations on positions selected from the group consisting of G2E, L11K, A12S, F13Y, V14T, V17E, D20L, T22M and T22S, N30D, T32P, Q34E, S36T, Q41K, Q42L, V44I, A45T, A53L, L58V, K62E, A67Q and A67T, G69S, V89L, Q92E, D94G and A97G, and the mutation position in the Obscurin-O chain is a natural sequence numbering position of SEQ ID NO: 50;
or,
A) the Titin-T chain has one or more amino acid residue mutations on positions selected from the group consisting of 8, 20, 22, 25, 26 and 39 on the basis of SEQ ID NO: 32, and preferably has one or more amino acid residue mutations on positions selected from the group consisting of A8C, V20C, T22C, C25S, A26C and C39T, and the mutation position of the Titin-T chain is a natural sequence numbering position of SEQ ID NO: 32;
more preferably, the Titin-T chain has one or more amino acid mutations on positions selected from the group consisting of 3, 11, 13, 22, 40, 42, 45, 47, 49, 56, 58, 66, 70, 75, 77, 79, 81, 82, 83 and 84 on the basis of SEQ ID NO: 35, and preferably has one or more amino acid mutations on positions selected from the group consisting of P3W, S11I, I13L, T22M, G40S, R42K, H45S, Q47E, Q49G, N56S, D58E, M66S, M66K, K70R, L75V, T77S, S79T, G81R, N82M, E83D and F84L; the mutation position in the Titin-T chain is a natural sequence numbering position of SEQ ID NO: 35, and/or
B) the Obscurin-like-O chain has one or more amino acid residue mutations on positions selected from the group consisting of 6, 26, 74, 77, 84 and 86 on the basis of SEQ ID NO: 34, and preferably has one or more amino acid residue mutations on positions selected from the group consisting of C6E, C26S, C77S, V74C, G84C and A86C, and the mutation position in the Obscurin-like-O chain is a natural sequence numbering position of SEQ ID NO: 34.

44. A nucleic acid molecule, encoding any one of substances selected from the group consisting of the following (A)-(G):
A) the polypeptide complex according to any one of claims 1 to 12,
B) the multispecific polypeptide complex according to any one of claims 13 to 17,
C) the domain engineered antibody according to any one of claims 18, 20, 27-35,
D) the Fab fragment of a domain engineered antibody according to any one of claims 19, 27-35,
E) the bispecific antibody according to any one of claims 21 to 36,
F) the polypeptide according to claim 41, and
G) the multispecific antibody according to claim 38 or 39.

45. A vector, comprising the nucleic acid molecule according to claim 44.

46. A host cell, transformed with the vector according to claim 45; wherein the host cell is selected from the group consisting of prokaryotic cells and eukaryotic cells, preferably eukaryotic cells, and more preferably mammalian cells.

47. A preparation method for any one of substances selected from the group consisting of the following:
the polypeptide complex according to any one of claims 1 to 12, the multispecific polypeptide complex according to any one of claims 13 to 17, the domain engineered antibody according to any one of claims 18, 20, 27 to 35, the Fab fragment of a domain engineered antibody according to any one of claims 19, 27 to 35, the bispecific antibody according to any one of claims 21 to 36, the polypeptide according to claim 41, and the multispecific antibody according to claim 38 or 39; wherein,
the method comprises the following steps:
culturing the host cell according to claim 46 followed by purification and recovery of the polypeptide complex, the multispecific polypeptide complex, the domain engineered antibody, the Fab fragment of a domain engineered antibody, the bispecific antibody,
the polypeptide, or the multispecific antibody.

48. A pharmaceutical composition, comprising one or more pharmaceutically acceptable carriers, excipients or diluents, and any one of substances selected from the group consisting of the following:
the polypeptide complex according to any one of claims 1 to 12, the multispecific polypeptide complex according to any one of claims 13 to 17, the domain engineered antibody according to any one of claims 18, 20, 27 to 35, the Fab fragment of a domain engineered antibody according to any one of claims 19, 27 to 35, the bispecific antibody according to any one of claims 21 to 36, the multispecific antibody according to claim 38 or 39, and the conjugate according to claim 40.

49. Use of a substance in the preparation of a medicament for the treatment or prevention of a disease or condition, wherein the substance is selected from the group consisting of the following substances: the polypeptide complex according to any one of claims 1 to 12, the multispecific polypeptide complex according to any one of claims 13 to 17, the domain engineered antibody according to any one of claims 18, 20, 27 to 35, the Fab fragment of a domain engineered antibody according to any one of claims 19, 27 to 35, the bispecific antibody according to any one of claims 21 to 36, the pharmaceutical composition according to claim 48, the multispecific antibody according to claim 38 or 39, and the conjugate according to claim 40.

50. A detection method, comprising contacting a sample to be tested with any one of substances selected from the group consisting of the following:
the polypeptide complex according to any one of claims 1 to 12, the multispecific polypeptide complex according to any one of claims 13 to 17, the domain engineered antibody according to any one of claims 18, 20, 27 to 35, the Fab fragment of a domain engineered antibody according to any one of claims 19, 27 to 35, the bispecific antibody according to any one of claims 21 to 36, and the multispecific antibody according to claim 38 or 39.
